# EUROPEAN PATENT APPLICATION

(11) **EP 4 538 680 A1**
(43) Date of publication of application: **16.04.2025**
(21) Application number: 24202213.5
(22) Date of filing: 24.09.2024
(51) Int. Cl.: G01N 21/31, A61B 5/1455, G01N 21/47, G01N 21/49

(54) **OPTICAL MEASUREMENT SYSTEMS AND METHODS**

(30) Priority: 29.09.2023 US 202363541754 P; 16.09.2024 US 202418886953
(71) Applicant: Apple Inc., Cupertino, CA 95014 (US)
(72) Inventor: ARBORE, Mark A., Cupertino, 95014 (US); MILES, Alexander A., Cupertino, 95014 (US); TERREL, Matthew A., Cupertino, 95014 (US); HWANG, Victoria, Cupertino, 95014 (US); STEVEN, Samuel, Cupertino, 95014 (US); RIDDER, Trent D., Cupertino, 95014 (US); HILL, Jeffrey T., Cupertino, 95014 (US); MINSHULL, Sinclair A., Cupertino, 95014 (US)
(74) Representative: COPA Copenhagen Patents

(57) **Abstract**

The disclosure relates to embodiments of optical measurement systems that are configured to perform spectroscopic measurements. The optical measurement systems are configured to provide compact arrangements for introducing light into a sample and collecting light returned from the sample. Reducing the size of the launch and/or collection architecture of an optical measurement system may make the overall optical measurement system smaller, thereby providing flexibility in integrating an optical measurement system into various form factors.

## Description

### CROSS-REFERENCE TO RELATED APPLICATIONS

This application claims the benefit under 35 U.S.C. § 119(e) of U.S. Provisional Patent Application No. 63/541,754, filed September 29, 2023, the contents of which are incorporated herein by reference as if fully disclosed herein.

### FIELD

This disclosure relates generally to optical measurement systems that are configured to introduce light into a sample and collect light returned from the sample, and more specifically optical measurement systems that perform spectroscopic measurements using this light.

### BACKGROUND

Optical measurement systems can be used to identify the presence, type, and/or one or more characteristics of objects or substances in the environment surrounding the system. In some instances, an optical measurement system can perform spectroscopic measurements by emitting light at multiple wavelengths and measuring light returned to the system. The relative amounts of light returned at each wavelength may provide information about the nature of the material or materials being measured. The amount of light returned to the optical measurement system may additionally depend on the optical path length of light within the sample. To help improve the accuracy of measurements performed by an optical measurement system, it may be desirable to control the range and distribution of optical path lengths in a sample for light emitted by the optical measurement system. As the number of wavelengths measured by an optical measurement system and the number of unique measurement locations each increase, these optical measurement systems may require increasingly complex architectures to perform accurate measurements. Thus, a compact optical measurement system with improved optical path length control may be desired.

### SUMMARY

Embodiments of the systems, devices, methods, and apparatuses described in the present disclosure are directed to optical measurement systems, as well as components and measurement subsystems thereof, that are configured to determine one or more properties of a sample via a spectroscopic measurement.

Some embodiments described herein are directed to an optical measurement system that includes a sampling interface defining a launch site and a set of collection sites each laterally spaced from the launch site, a launch architecture configured to emit an emission light beam that exits the sampling interface through the launch site, and a collection architecture that includes a set of detector elements positioned to measure light that has entered the sampling interface through the set of collection sites. The set of detector elements may form a set of measurement channels when the launch architecture emits the emission light beam. The emission light beam converges in a first dimension as it exits the sampling interface, and the emission light beam, if emitted into a target sample, projects to a transition region in the target sample that is angle independent for at least one of the set of measurement channels.

Other embodiments are direct to an optical measurement system that includes a sampling interface defining a launch site and a first collection site laterally spaced from the launch site, a launch architecture configured to emit an emission light beam that exits the sampling interface through the launch site, and a collection architecture that includes a first detector element. The collection architecture is configured to collect a first return light beam that enters the sampling interface through the first collection site, and direct the return light beam to the first detector element. Tthe first detector element forms a first measurement channel when the launch architecture emits the emission light beam, the first return light beam diverges in a first dimension as it enters the sampling interface, and the first return light beam, if collected from a target sample, projects from a first transition region in the target sample that is angle independent for the first measurement channel.

Still other embodiments are directed to an optical measurement system that includes a sampling interface defining a launch site and a first collection site laterally spaced from the launch site, a launch architecture configured to emit an emission light beam that exits the sampling interface through the launch site, and a collection architecture that includes a first detector element. The collection is configured to collect a first return light beam that enters the first sampling interface through the collection site, and direct the return light beam to the first detector element. The first detector element forms a measurement channel when the launch architecture emits the emission light beam, the emission light beam converges in a first dimension as it exits the sampling interface, and the return light beam diverges in the first dimension as it enters the sampling interface. The optical measurement system is configured such that, if used to measure a target sample characteristics: the emission light beam projects to a first transition region in the target sample that is angle independent for the measurement channel, and the return light beam projects from a second transition region in the target sample that is angle independent for the measurement channel.

In some embodiments, an optical measurement system includes a sampling interface defining a launch site and a set of collection sites each laterally spaced from the launch site, a launch architecture configured to emit an emission light beam that exits the sampling interface through the launch site into a measured sample, and acollection architecture comprising a set of detector elements positioned to measure light that has entered the sampling interface through the set of collection sites. The emission light beam converges in a first dimension as it exits the sampling interface and is projected to a transition region in the measured sample. The launch architecture includes a beam generator comprising a light source unit and configured to generate a diverging output light beam, and a launch optical subassembly configured to collimate the diverging input light beam in the first dimension to generate a plurality of collimated ray bundles that collectively form the emission light beam and are projected to intersect at the transition region.

Other embodiments are directed to an optical measurement system that includes a sampling interface defining a launch site and a set of collection sites each laterally spaced from the launch site and a launch architecture and a collection architecture. The launch architecture includes a diffuser positioned to receive an input light beam and generate an output light beam that diverges in a first dimension and a set of lens configured to collimate the diverging output light beam in the first dimension to generate an emission light beam that converges in the first dimension. The collection architecture includes a set of detector elements positioned to measure light that has entered the sampling interface through the set of collection sites. The emission light beam is formed from a plurality of collimated ray bundles that are projected, as the emission light beam exits the launch site, to intersect at a transition region in a measured sample.

In some instances, an optical measurement system may include a sampling interface defining a launch site and a set of collection sites each laterally spaced from the launch site, a launch architecture and a collection architecture. The launch architecture includes a photonic integrated circuit configured to generate an input light beam, a diffuser positioned to receive an input light beam and generate an output light beam that diverges in a first dimension, and a set of lens configured to generate, from the output light beam, a plurality of collimated ray bundles that are projected to intersect at a transition region in a measured sample. The collection architecture includes a set of detector elements positioned to measure light that has entered the sampling interface through the set of collection sites.

Still other embodiments are directed to an optical measurement system that includes a sampling interface defining a launch site and a first collection site laterally spaced from the launch site, launch architecture configured to emit an emission light beam that exits the sampling interface through the launch site, and collection architecture that includes a first optical collection subassembly having a first condenser lens, and a first plurality of detector elements. The collection architecture is configured to collect a first return light beam from the first collection site and direct the first return light beam to the first condenser lens and each detector of the first plurality of detector elements measures a corresponding portion of the first return light beam to form a corresponding measurement channel of a first plurality of measurement channels. Additionally, the first return light beam diverges in a first dimension as it enters the first collection site, and the first return light beam, if collected from a target sample, projects from a first transition region in the target sample that is angle independent for each of the first plurality of measurement channels.

In some embodiments, an optical measurement system includes a sampling interface defining a launch site and a collection site laterally spaced from the launch site, a launch architecture configured to emit an emission light beam that exits the sampling interface through the launch site, and a collection architecture that includes: an optical collection subassembly comprising a plurality of condenser lenses, and multiple groups of detector elements. Each of the multiple groups of detector elements is positioned to receive light from a corresponding condenser lens of the plurality of condenser lens. The collection architecture is configured to collect a plurality of return light beams that enter the sampling interface through the collection site. The collection architecture is configured to direct a portion of each of the plurality of return light beams to each of the plurality of condenser lenses, such that each of the multiple groups of detector elements measures light from each of the plurality of light beams.

In other embodiments, an optical measurement system includes a sampling interface defining a launch site and a first collection site laterally spaced from the launch site, a launch architecture configured to emit an emission light beam that exits the sampling interface through the launch site, and a collection architecture. The collection architecture includes a first optical collection subassembly comprising a first condenser lens and configured to receive light returned to the sampling interface through the first collection site, and a first plurality of detector elements positioned to receive light from the first condenser lens. The collection architecture is configured such that, if the collection architecture receives light from a target sample, each of the first plurality of detector elements measures light having a first common path length distribution and a different sampling depth distribution.

Yet other embodiments are directed to an an electromagnetic actuator arrangement that includes a stationary base, a set of suspension elements, a carrier moveably connected to the stationary base via the set of suspension elements, and a first diffuser carried by the carrier. The electromagnetic actuator arrangement also includes a set of actuators mounted to the carrier and controllable to move the carrier relative to the stationary base. The carrier defines a first carrier aperture that extends through the carrier.

In other embodiments, an optical measurement system includes a sampling interface defining a launch site and a first collection site laterally spaced from the launch site, and launch architecture configured to emit an emission light beam that exits the sampling interface through the launch site, where the launch architecture includes a beam generator comprising a diffuser. The diffuser is positioned to receive an input light beam and generate an output light beam that diverges in a first dimension. The optical measurement system includes a collection architecture that has a first set of detector elements positioned to measure light that has entered the sampling interface through the first collection site. The optical measurement system also includes an electromagnetic actuator arrangement that has a stationary base, a carrier moveably connected to the stationary base, and a set of actuators mounted to the carrier and controllable to move the carrier relative to the stationary base. The diffuser is carried by the carrier, the carrier defines a first carrier aperture that extends through the carrier, and the first carrier aperture is positioned such that light measured by the first set of detector elements passes through the first carrier aperture.

In some embodiments, an optical measurement system includes a first measurement subsystem configured to emit a first emission light beam into a sample through a sampling interface, and measure a portion of the first emission light beam that is returned from the sample through the sampling interface. The optical measurement system also includes a second measurement subsystem configured to emit a second emission light beam into the sample through the sampling interface, and measure a portion of the second emission light beam that is returned from the sample through the sampling interface. The optical measurement system further includes an electromagnetic actuator arrangement having a stationary base, a carrier moveably connected to the stationary base, and a set of actuators mounted to the carrier and controllable to move the carrier relative to the stationary base. The first measurement subsystem includes a first diffuser, the second measurement subsystem includes a second diffuser, and the carrier carries the first diffuser and the second diffuser.

Other embodiments are directed to a polarizing beamsplitter that includes a substrate comprising a top surface and a bottom surface, and a polarizer positioned on a first portion of the bottom surface. The top surface defines a first angled facet. The polarizing beamsplitter is configured such that when an incoming light beam is incident on a predetermined location of the bottom surface with a predetermined angle of incidence: the polarizer generates a polarized light beam from the incoming light beam, the first angled facet splits the polarized light beam into a first split light beam and a second split light beam, the first split light beam exits the polarizing beamsplitter through the first angled fact, and at least a portion of the second split light beam exits the polarizing beamsplitter through the bottom surface.

In some embodiments, a polarizing beamsplitter includes a substrate comprising a top surface and a bottom surface, and a polarizer positioned on a first portion of the bottom surface. The top surface defines a first angled facet and a second angled facet. The polarizing beamsplitter is configured such that when an incoming light beam is incident on a predetermined location of the bottom surface with a predetermined angle of incidence: the polarizer generates a polarized light beam from the incoming light beam, the first angled facet splits the polarized light beam into a first split light beam and a second split light beam, the first split light beam exits the polarizing beamsplitter through the first angled fact, and at least a portion of the second split light beam exits the polarizing beamsplitter through the top surface.

In still other embodiments, an optical measurement system includes a sampling interface defining a launch site and a set of collection sites each laterally spaced from the launch site, a launch architecture that includes a beam generator and is configured to emit an emission light beam that exits the sampling interface through the launch site into a measured sample, and a collection architecture that includes a set of detector elements positioned to measure light that has entered the sampling interface through the set of collection sites. The beam generator is configured to generate an input light beam and generate an output light beam from the input light beam, the launch architecture is configured to generate the emission light beam from the output light beam, and the beam generator includes a reference detector and a polarizing beamsplitter. The polarizing beamsplitter includes a substrate with a top surface and a bottom surface, the top surface defining a first angled facet, as well as a polarizer positioned on a first portion of the bottom surface. The polarizing beamsplitter is positioned to receive the input light beam through the polarizer to generate a polarized light beam and is configured to split the polarized light beam into a first split light beam and a second split light beam. The beam generator generates the output light beam from the first split light beam, and the reference detector is positioned to measure at least a portion of the second split light beam.

In some embodiments, a beam shifting optic includes a substrate comprising a top surface and a bottom surface wherein the bottom surface defines at least one angled facet that is angled in a first dimension and the top surface defines a lensed facet that is curved in a second dimension perpendicular to the first dimension. The beam shifting optic is configured such that, when a light beam enters the beam shifting optic through a predetermined input region and at a predetermined angle of incidence: the light beam reflects off of the at least one angled facet to redirect the light beam in the first dimension, and the light beam exits the beam shifting optic through an exit region of the lensed facet to at least partially collimate the light beam.

Other embodiments are directed to beam generator configured to generate an output light beam. The beam generator includes a photonic integrated circuit configured to emit an input light beam, a beam shifting optic, and a diffuser positioned to receive the input light beam and generate the output light beam from the input light beam. The beam shifting optic is positioned to receive the input light beam at a predetermined input region and at a predetermined angle of incidence and configured to laterally shift the input light beam in a first dimension. The beam shifting optic has a substrate with a top surface and a bottom surface, such that the bottom surface defines a first angled facet that is angled in the first dimension. The beam shifting optic is configured such that when the input light beam enters the beam shifting optic through the predetermined input region and at the predetermined angle of incidence, the light beam reflects off of the first angled facet to redirect the light beam in the first dimension, and the light beam exits the beam shifting optic through an exit region of the beam shifting optic.

Still other embodiments are directed to an optical measurement system that includes a sampling interface defining a launch site and a set of collection sites each laterally spaced from the launch site, a launch architecture having a beam generator and configured to emit an emission light beam that exits the sampling interface through the launch site into a measured sample, and a collection architecture having a set of detector elements positioned to measure light that has entered the sampling interface through the set of collection sites. The beam generator is configured to generate an input light beam and generate an output light beam from the input light beam. The launch architecture is configured to generate the emission light beam from the output light beam. The beam generator includes a photonic integrated circuit configured to emit the input light beam and a beam shifting optic configured to laterally shift the input light beam in a first dimension.

In some embodiments, an optical measurement system includes a first measurement subsystem configured to emit a first emission light beam into a sample through a sampling interface, and measure a portion of the first emission light beam that is returned from the sample through the sampling interface. The optical measurement system also includes a second measurement subsystem configured to emit a second emission light beam into the sample through the sampling interface, and measure a portion of the second emission light beam that is returned from the sample through the sampling interface. The optical measurement system includes a set of photonic integrated circuits configured to emit a first input light beam and a second input light beam, and an electromagnetic actuator arrangement comprising a stationary base, a carrier moveably coupled to the stationary base, and at least one diffuser carried by the carrier. The first input light beam is routed through the at least one diffuser to generate a first output light beam and the second input light beam is routed through the at least one diffuser to generate a second output light beam. The first measurement subsystem generates the first emission light beam from the first output light beam, and the second measurement subsystem generates the second emission light beam form the second output light beam.

Other embodiments are directed to an optical system that includes a first measurement subsystem configured to emit a first emission light beam into a sample through a sampling interface, and measure a portion of the first emission light beam that is returned from the sample through the sampling interface. The optical measurement system also includes a second measurement subsystem configured to emit a second emission light beam into the sample through the sampling interface, and measure a portion of the second emission light beam that is returned from the sample through the sampling interface. The optical measurement system includes a photonic integrated circuit configured to emit a first input light beam from a first emission location on the photonic integrated circuit, and further includes a beam redirecting structure configured to route light from the first input light beam between the first measurement subsystem and the second measurement subsystem.

Still other embodiments are directed to an optical measurement system that includes a first measurement subsystem configured to emit a first emission light beam into a sample through a sampling interface, and measure a portion of the first emission light beam that is returned from the sample through the sampling interface. The optical measurement system also includes a second measurement subsystem configured to emit a second emission light beam into the sample through the sampling interface, and measure a portion of the second emission light beam that is returned from the sample through the sampling interface. The optical measurement system includes a photonic integrated circuit that is configured to emit a first input light beam and a second input light beam from a common surface of the photonic integrated circuit. The first measurement subsystem is configured to generate a first output light beam from the first input light beam, and the second measurement subsystem is configured to generate a second output light beam from the first input light beam. The first output light beam and the second output light beam are generated in a staggered relationship relative to the photonic integrated circuit.

In addition to the example aspects and embodiments described above, further aspects and embodiments will become apparent by reference to the drawings and by study of the following description.

### BRIEF DESCRIPTION OF THE DRAWINGS

The disclosure will be readily understood by the following detailed description in conjunction with the accompanying drawings, wherein like reference numerals designate like structural elements, and in which:
FIG. 1A shows a cross-sectional side view of a device incorporating an optical measurement system as described herein. FIG. 1B shows a top view of the device of FIG. 1A.
FIGS. 2A and 2B show partial cross-sectional side views of two variations of optical measurement systems, each having a measurement channel configured to have a different path length distribution. FIG. 2C show graphs of example path length distributions for the measurement channels of the optical measurement systems of FIGS. 2A and 2B
FIG. 3A shows a partial cross-sectional side view of a portion of an optical measurement system that is configured to emit am emission light beam. FIG. 3B shows a perspective view of a portion of the optical measurement system of FIG. 3A.
FIGS. 4A, 4B, 4D, and 4E show partial cross-sectional views of an optical measurement system that is used to measure a target sample as described herein. FIG. 4C and 4F show graphs of example path length distributions for the measurement channels of the optical measurement system of FIGS. 4A, 4B, 4D, and 4E.
FIG. 5A shows a partial cross-sectional side view of a portion of an optical measurement system that is configured to collect a return light beam. FIG. 5B shows a perspective view of a portion of the optical measurement system of FIG. 5A.
FIGS. 6A and 6B show partial cross-sectional views of variations of optical measurement system that are used to measure a target sample as described herein.
FIG. 7 shows a partial cross-sectional side view of an optical measurement system configured to project light toward an angle-independent sample region for a first measurement channel.
FIG. 8 shows a partial cross-sectional side view of an optical measurement system configured to collect light that is projected to emanate from an angle-independent sample region for a first measurement channel.
FIGS. 9A-9C show partial cross-sectional side views of variations of optical measurement systems configured to both project light toward a first angle-independent sample region for at least one measurement channel, and collect light that is projected to emanate from a second angle-independent sample region some or all of the at least one measurement channel. FIG. 9D shows a perspective view of the optical measurement system of FIG. 9C.
FIGS. 10A and 10B show side views of two variations of launch architectures as described herein that are configured to generate an emission light beam that converges in at least one dimension.
FIGS. 11A and 11B show side views of two variations of launch architectures as described herein that are configured to set certain properties of an emission light beam.
FIG. 12 shows a block diagram of example beam generator that may be used with the optical measurement systems described herein to generate an output light beam.
FIG. 13A shows a top view of a variation of a launch architecture that may be used with the optical measurement systems described herein. FIGS. 13B-13F show side views of variations of the launch architecture of FIG. 13A.
FIGS. 14A, 14C, and 14E show side views of variations of collection architectures that may be used with the optical measurement systems described herein. FIGS. 14B, 14D, and 14F show plots of path length and sampling depth distributions for the measurement channels, respectively, of the collection architectures of FIGS. 14A, 14C, and 14E.
FIG. 15A shows a side view of a variation of a collection architecture that incorporates a condenser lens. FIG. 15B shows a plot of path length and sampling depth distributions for the measurement channels of the collection architecture of FIG. 15A.
FIG. 16A and 16B shows side views of a variation of a collection architecture that incorporates a plurality of condenser lenses. FIG. 16C shows a plot of path length and sampling depth distributions for the measurement channels of the collection architecture of FIGS. 16A and 16B.
FIG. 17A shows a side view of another variation of a collection architecture that incorporates a plurality of condenser lenses. FIG. 17B shows a plot of path length and sampling depth distributions for the measurement channels of the collection architecture of FIG. 17A.
FIGS. 18A-18C depict partial top views of collection architectures that may be used with the optical measurement systems described herein.
FIG. 19A shows a top view of an example of electromagnetic actuator arrangement that may be used with the optical measurement systems described herein. FIGS. 19B and 19C show cross-sectional side views of variations of the electromagnetic actuator arrangement of FIG. 19A.
FIGS. 20A-20E show top views of variations of electromagnetic actuator arrangements, such as described herein, that include one or more carrier apertures.
FIG. 21A shows a side view of a portion of an optical measurement system that includes a variation of a polarizing beamsplitter as described herein. FIG. 21B shows a perspective views of the polarizing beamsplitter of FIG. 21A. FIGS. 21C and 2D show perspective views of a beam generator and an optical measurement system, respectively, that incorporate the polarizing beamsplitter of FIG. 21B.
FIG. 22A shows a side view of a portion of an optical measurement system that includes another variation of a polarizing beamsplitter as described herein. FIG. 22B and 22C show perspective views of the polarizing beamsplitter of FIG. 22A.
FIGS. 23-26 show side views of portions of optical measurement systems that include different variations of polarizing beamsplitters as described herein.
FIG. 27A shows a side view, and FIGS. 27B and 27C show perspective views, of a variation of a beam shifting optic as described herein having a curved angled facet. FIG. 27D shows a partial cross-sectional side view of a variation of a beam generator that includes the beam shifting optic of FIGS. 27A-27C.
FIGS. 28A and 28B show side and perspective views, respectively, of a variation of a beam shifting optic as described herein having flat angled facets. FIGS. 28C and 28D show side views of variations of beam generators that include the beam shifting optic of FIGS. 28A and 28B.
FIGS. 29A and 29B show side and perspective views, respectively, of a variation of a beam shifting optic as described herein having a beam redirecting portion. FIGS. 29C shows a side views of a variations of a beam generator that includes the beam shifting optic of FIGS. 29A and 29B.
FIGS. 30A and 30B show side and bottom perspective views, respectively, of a variation of a beam shifting optic as described herein having multiple angled facets on a common surface. FIG. 30C shows a side views of a variations of a beam generator that includes the beam shifting optic of FIGS. 30A and 30B.
FIGS. 31A and 31B show side and bottom perspective views, respectively, of a variation of a beam shifting optic as described herein having multiple angled facets on each of a bottom surface and a top surface.
FIG. 32A shows a side view, and FIGS. 32B and 32C show perspective views, of a variation of a beam shifting optic as described herein having a lensed output region. FIG. 32D shows a partial cross-sectional side view of a variation of a beam generator that includes the beam shifting optic of FIGS. 32A-32C.
FIGS. 33A and 33B show side and perspective views, respectively, of a variation of a beam shifting optic as described herein having a lensed output region and multiple angled facets on each of a bottom surface and a top surface.
FIGS. 34A-34C, 35A-35E, 36, 37A, 37C, 38A-38D, 39A, and 39B show top views of variations of optical measurement systems that have a photonic integrated circuit configured to emit multiple input light beams. FIG. 37B shows a side view of the optical measurement system of FIG. 37A.
FIG. 40A shows a top view of a variation of an optical measurement system that is configured to generate different output light beams from a common input light beam. FIGS. 40B-40E show partial side views of variations of the optical measurement system of FIG. 40A.
FIG. 41A shows a top view of a variation of an optical measurement system that includes an array of mirrors configured to route different input light beams. FIGS. 41B and 41C show partial side views of variations of the optical measurement system of FIG. 41A.
FIG. 42A shows a top view of a variation of an optical measurement system that includes a set of optical displacers configured to adjust respective track lengths of a corresponding set of input light beams. FIGS. 42B and 42C show partial cross-sectional side views of the optical measurement system of FIG. 42A.
FIGS. 43A and 43B show cross-sectional side views of portions of polarizing beamsplitters having immersed polarizers.
It should be understood that the proportions and dimensions (either relative or absolute) of the various features and elements (and collections and groupings thereof) and the boundaries, separations, and positional relationships presented between them, are provided in the accompanying figures merely to facilitate an understanding of the various embodiments described herein and, accordingly, may not necessarily be presented or illustrated to scale, and are not intended to indicate any preference or requirement for an illustrated embodiment to the exclusion of embodiments described with reference thereto.

### DETAILED DESCRIPTION

Reference will now be made in detail to representative embodiments illustrated in the accompanying drawings. It should be understood that the following description is not intended to limit the embodiments to one preferred embodiment. To the contrary, it is intended to cover alternatives, modifications, and equivalents as can be included within the spirit and scope of the described embodiments as defined by the appended claims.

The following disclosure relates to embodiments of optical measurement systems that are configured to perform spectroscopic measurements. The optical measurement systems are configured to provide compact arrangements for introducing light into a sample and collecting light returned from the sample. Reducing the size of the launch and/or collection architecture of an optical measurement system may make the overall optical measurement system smaller, thereby providing flexibility in integrating an optical measurement system into various form factors.

To perform a spectroscopic measurement on a sample, the optical measurement system may perform a measurement sequence of individual measurements. During each individual measurement, the optical measurement system may emit input light (in the form of an emission light beam as described in more detail herein) into a region of the sample, and measures light that is returned to the optical measurement system from the sample (in the form of one or more return light beams as described in more detail herein). The light measured during an individual measurement may represent the relative amount of the input light that is returned to the optical measurement system. The optical measurement system may be configured such that light measured during a given individual measurement may have certain additional properties, such as a particular distribution of optical path lengths and/or a particular distribution of sampling depths. This information may be used by the optical measurement system in determining one or more properties of a sample.

Because light of different wavelengths may interact differently with a given sample, it may be desirable the measurement sequence to include multiple individual measurements performed at different wavelengths. In these instances, the input light may include light of different wavelengths during different individual measurements. In some instances, the optical measurement system may be configured to emit input light having a single wavelength for certain individual measurements. In this way, the measurement sequence may include one or more individual measurements performed using a single wavelength (e.g., a first individual measurement that uses input light of a first wavelength, a second individual measurement that uses input light of a second wavelength, and so on). Additionally or alternatively, the optical measurement system may be configured to emit input light having multiple wavelengths for certain individual measurements. In these instances, the measurement sequence may include one or more individual measurements performed using multiple wavelengths (e.g., a first individual measurement that uses input light of a first plurality of wavelengths, a second individual measurement that uses input light of a second plurality of wavelengths, and so on). The wavelength (or wavelengths) associated with each individual measurement may be used by the optical measurement system in determining one or more properties of the sample.

In some instances it may be desirable for an optical measurement system to be able to measure different regions of a sample without needing to physically move the optical measurement system relative to the sample. Accordingly, in some instances the optical measurement systems described herein may include multiple measurement subsystems, such as will be described in more detail herein. In these instances, each measurement subsystem is capable of performing individual measurements of a different corresponding region of the sample. Specifically, each measurement subsystem is configured to, as part of an individual measurement, i) emit an emission light beam into a sample and ii) collect and measure a portion of the emission light beam that is returned from the sample (e.g., using one or more detector elements). Individual measurements from different measurement subsystems may be performed simultaneously or sequentially, depending on the configuration of the measurement subsystems and the choice of spectroscopic measurement.

Overall, the measurement sequence will include a set of individual measurements performed at one or more regions of a sample using a one or more wavelengths (collectively referred to as the "measurement wavelengths" of the spectroscopic measurement). Collectively, these individual measurements may be analyzed to derive one or more properties of the sample (e.g., using spectroscopic analysis techniques). The optical measurement systems may facilitate a wide range of analytical techniques as will be readily understood by one of ordinary skill in the art.

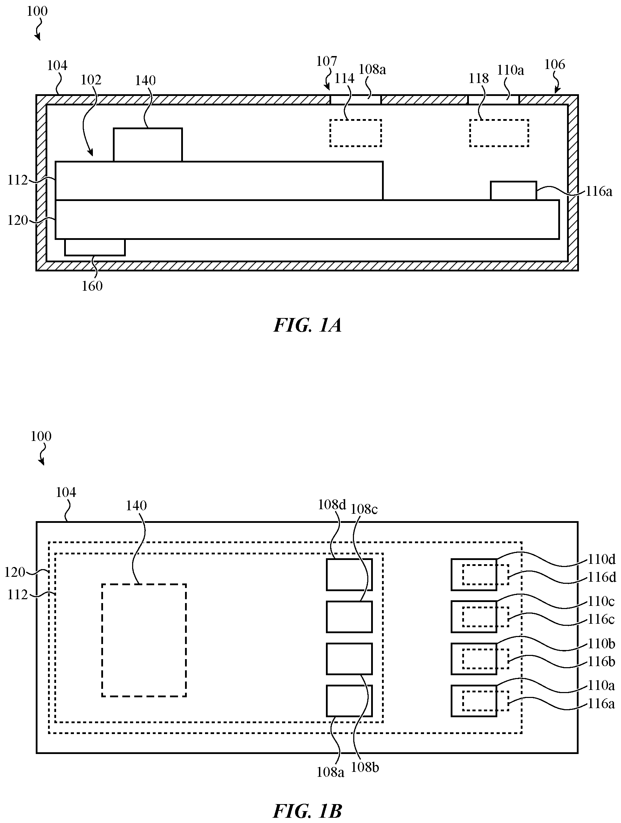

These and other embodiments are discussed below with reference to FIGS. 1A-18C. However, those skilled in the art will readily appreciate that the detailed description given herein with respect to these figures is for explanatory purposes only and should not be construed as limiting.

Generally, the optical measurement systems described herein include a light source unit that is configured to generate light that will be used in a spectroscopic measurement, launch architecture that is configured to introduce the generated light into a sample, collection architecture that is configured to collect light returned from the sample, and one or more detector elements configured to measure light collected by the collection architecture. The optical measurement system includes one or more launch sites from which light is emitted from the optical measurement system. Each launch site may emit a different corresponding light beam from the optical measurement system, and thus the optical measurement system may be configured to emit a single light beam or multiple light beams depending on the number of launch sites. The launch architecture routes light generated by light source unit to the one or more launch sites, and includes the various components (as will be described in more detail herein) that are used to create the light beams emitted from the one or more launch sites. The optical measurement system may be configured to control which launch sites are emitting light at a given time, thereby providing additional flexibility to the optical measurement system in performing spectroscopic measurements. For example, each launch site may be associated with a different measurement subsystem of the optical measurement system (each of which may include a corresponding launch architecture and a collection architecture), and the optical measurement system may be able to control which measurement subsystems are performing individual measurements at a given time.

The optical measurement system further includes one or more collection sites at which light may enter the optical measurement system. Specifically, each collection site represents a different location at which light may be collected from the sample. The collection architecture includes the various components (as will be described in more detail herein) that collect light at the one or more collection sites and directs the collected light to one or more of the detector elements. In instances where the optical measurement system includes a plurality of measurement subsystems, each measurement subsystem may include a corresponding collection architecture. When light is introduced into the sample via one or more launch sites, a portion of that light may be returned to the optical measurement system after interacting with the sample (e.g., via reflections and/or scattering that may occur as light traverses the sample). The proportion of light that is returned to the optical measurement system may depend on the characteristics of the sample, and thus the amount of light collected by the collection sites of an optical measurement system may be measured and used to determine one or more sample properties.

Accordingly, each collection site may be associated with one or more detector elements that may be used to measure a corresponding portion of the light received by that collection site. In some variations, a collection site may be associated with multiple detector elements, in which case different portions of light collected by the collections site are measured by different detector elements. Depending on the number of launch sites, the number of collection sites, and the number of detector elements associated with each collection site, the optical measurement system may be able to separately measure different volumes with a given sample.

Each detector element is able to output a signal that is based on the amount of light received by that detector element, which may depend at least in part on which launch site (or sites) are actively emitting light. Accordingly, the optical measurement systems described herein may be configured to perform individual measurements using one or more measurement channels. A "measurement channel" as used herein refers to a pathway along which light is emitted from the optical measurement system, collected by the optical measurement system, and measured by a detector element of the optical measurement system. A measurement channel includes a detector element and one or more launch sites (including any additional components of the optical measurement system necessary to generate, emit, and collect this light, such as the light source unit, the launch architecture, and the collection architecture), such that when the optical measurement system is used to measure a target sample (as described in more detail herein), each launch site of the measurement channel will simultaneously emit light, and at least a portion of this simultaneously-emitted light will reach and be measured by the detector element. In these instances, the signal generated by the detector element represents the proportion of light emitted by the launch site (or sites) that is collected from the sample and directed to the detector element.

An optical measurement system may be configured to have a single measurement channel, or may include multiple measurement channels. In instances where an optical measurement system includes multiple measurement channels, the optical measurement system may optionally utilize different measurement channels at different times. For example, the optical measurement system may include multiple measurement subsystems, each of which includes a corresponding set of measurement channels. In these instances, a first measurement subsystem may perform a first set of individual measurement using a first set of measurement channels during a first period of time. During a second period of time, a second measurement subsystem may perform a second set of individual measurements using a second set of measurement channels. The first and second sets of individual measurements may be performed as part of a spectroscopic measurement as described herein. As used herein, a "set of" a given element or component, unless otherwise specified, means a set including at least one of that element or component. For example, a set of measurements may include a single measurement or a plurality of measurements.

The various embodiments described here assume that each detector element is always part of the same corresponding measurement channel. In these instances, the optical measurement system is operated in a manner in which each detector element will only measure light that is returned from a given emission light beam. It should be appreciated, however, that an optical measurement system may, if so desired, such that a given detector element is positioned to selectively receive light from two or more launch sites. The properties of light measured by the detector element may vary depending on which launch site is actively emitting light. For the purpose of performing a spectroscopic measurement, this detector element may be considered to form of a first measurement channel when a first launch site is emitting light during a first individual measurement, and may be considered to form a second measurement channel when a second launch site is emitting light during a second individual measurement. Accordingly, the first and second individual measurements may be associated with different properties (e.g., different path length distribution and/or different depth sampling depth distributions).

While each detector element may only be part of a single measurement channel at a given time, it may be possible for a given launch site to be part of multiple measurement channels simultaneously. For example, multiple detector elements may be able to measure different corresponding portions of light emitted from a single launch site. In these instances, each detector element may form a different measurement channel with at least the single launch site. Overall, while the principles described herein are discussed with respect to one or a few measurement channels, it should be appreciated that the optical measurement systems described herein may have several different measurement channels if so desired.

During a spectroscopic measurement, different measurement channels may be used to perform individual measurements that collectively form the spectroscopic measurement. Because each measurement channel represents a different combination of detector elements and launch sites, each measurement channel will output a signal that represents a measurement of a different region within a sample (though it should be appreciated that different measurement channels may measure partially overlapping sampling volumes). In some instances, the output signals of multiple measurement channel may be combined together, such that these output signals are combined into a single combined signal (e.g., a "combined output signal"). It should be appreciated that these output signals may be combined electrically (e.g., the electrical signals from multiple detector elements are combined such that a controller or processor analyzing the output signals receives a single electrical signal) or may be combined digitally (e.g., the controller or processor analyzing the output receives the individual output signals, and digitally combines them such that they are treated as a single output signal for the purpose of sample analysis). Overall, individual measurements from multiple channels may be combined or otherwise collectively analyzed to determine one or more sample properties of the sample being measured.

In general, the design of an optical measurement system as described herein is tailored to perform spectroscopic measurements of samples having certain general sample characteristics. In these instances, the optical measurement system may only perform as intended if used to measure samples having these general sample characteristics. For example, an optical measurement system designed to measure a solid sample may not operate as intended when used to measure a gaseous sample (or vice versa). Accordingly, for ease of illustration, the design and performance of the optical measurement systems described herein are discussed with respect to a target sample. A "target sample" as used herein represents a theoretical sample upon which an optical measurement system will perform a spectroscopic measurement. A target sample has a predetermined set of target sample characteristics, including at least a target scattering coefficient (e.g., a target reduced scattering coefficient), a target absorption coefficient, and a target refractive index, which are representative of the samples that will actually be measured by the optical measurement system.

It should be appreciated that there may be additional sample properties (e.g., the concentration of one or more analytes) that may vary within a target sample while still meeting the predetermined set of target sample characteristics. One or more of these sample properties may be measured by the optical measurement system as part of a spectroscopic measurement as described herein. Additionally, the target sample may have a complex structure with sample characteristics that vary within the sample. For example, a target sample may be assumed to include multiple layers, where each layer has its own corresponding subset of target sample characteristics (e.g., a first layer may have a first target absorption coefficient and a second layer may have a second target absorption coefficient). Accordingly, a given target sample characteristic, such as a target absorption coefficient, may be defined by a single value that represents the entire sample, or may be defined by multiple values that each represent a different corresponding portion of the target sample.

The optical measurement systems described herein may be designed and configured to operate in a particular manner when used to measure a sample having the target sample characteristics (the target scattering coefficient, the target absorption coefficient, etc.). For example, a measurement channel may be optimized to measure a particular sampling volume of a target sample, and the optical measurement system may assume certain operating characteristics (e.g., a distribution of optical path lengths and/or a distribution of sampling depths of light measured by a given measurement channel) when a spectroscopic measurement is performed on the target sample. If the actual sample being measured by an optical measurement system has sample characteristics that significantly deviate from those of the target sample, this measurement channel may no longer operate as intended (e.g., the detector element of measurement channel may no longer receive light during measurement, the accuracy of measurements performed by the measurement channel may be significantly impacted, or the like).

It should be appreciated, however, that in practice the actual samples measured by the optical measurement system may have one or more sample characteristics that vary from those of the target sample. If the sample characteristics of the actual sample being measured is sufficiently close to the predetermined set of target sample characteristics (as may be determined by the accuracy constraints of the optical measurement system), the optical measurement system may still successfully perform a spectroscopic measurement on the actual sample. In other words, the optical measurement system may be optimized for the target sample, but may be used to perform spectroscopic measurements on a variety of actual samples with different sample characteristics. Indeed, it may be possible that in practice the optical measurement system is not actually used to measure a sample that actually meets all of the predetermined set of target sample characteristics.

For the purpose of this application, when particular principles of operation of the optical measurement systems are discussed herein, such as the optical path length distribution and/or the sampling depth distribution of light measured by a given measurement channel, it is assumed that these principles are discussed with relation to a target sample as defined herein. When the optical measurement systems described herein are used to perform a spectroscopic measurement of an actual sample (referred to herein as a "measured sample"), the optical measurement system may analyze the individual measurements assuming that the sample characteristics of the measured sample are the same as the target sample characteristics. Alternatively, if one or more sample characteristics of the measured sample are known (e.g., are previously determined or measured as part of the spectroscopic measurement), the optical measurement system may use known sample characteristics when analyzing the individual measurements.

Additionally, because one or more sampling characteristics of a sample may vary based on the wavelength of light emitted by the optical measurement system, it should be appreciated that the sampling characteristics of a target sample described herein are described with respect to a particular wavelength of light that is referred to herein as a "target wavelength." Accordingly, when the optical measurement system is described herein as performing in a particular manner when measuring a target sample, it is assumed that the measurement is performed using input light having the target wavelength. The target wavelength may be one of the measurement wavelengths of a spectroscopic measurement, such that at least one of the individual measurements performed by a measurement channel (e.g., using the target wavelength and/or other wavelengths sufficiently close to the target wavelength) of the optical measurement system may have a particular path length distribution and/or sampling depth distribution as described herein. Other individual measurements performed by the measurement channel using other wavelengths may have different path length distributions and/or sampling depth distributions, but may still provide useful information as part of a spectroscopic measurement.

In some variations, it may be desirable to configure the optical measurement systems described herein to measure skin. For example, the optical measurement system may be positioned in contact with a user's skin, such that light emitted from the optical measurement system enters the user's skin. Similarly, light may return to the optical measurement system after interacting with the user's skin, and may be measured by one or more measurement channels of the optical measurement system. Accordingly, a spectroscopic measurement may be used to measure one or more properties of a user's skin.

When the optical measurement system is designed to use skin as a measured sample, a target sample may be selected that represents skins. The sampling characteristics of skin may vary from user to user, so the optical measurement system may be designed based on a target sample that represents a selected target skin sample. For example, a target skin sample may have, for a target wavelength as described herein, a target refractive index having a value between 1.3 and 1.5, a target reduced scattering coefficient having a value between 0.5 and 2 mm⁻¹, and a target absorption coefficient having a value between 0.5 and 4 mm⁻¹. In other instances, the optical measurement system may be designed to measure different types of sample, such as food, fluid or gel solutions, or the like. In these instances, the optical measurement system may be designed based on a target sample that is representative of the corresponding type of sample.

### System Overview

The embodiments of the optical measurement systems described herein may be incorporated into a device having a housing. The device, which in some instances is wearable, may operate solely to take measurements using the optical measurement system or may be a multi-functional device capable of performing additional functions, such as will be readily understood by someone of ordinary skill in the art. For example, in some instances the optical measurement system may be incorporated into a smart phone, tablet computing device, laptop or desktop computer, a smartwatch, earphone, headset, head-mounted device, or other wearable, or other electronic device (collectively referred to herein as "electronic devices" for ease of discussion).

The device may include a display (which may be a touchscreen display) that provides a graphical output that is viewable through or at an exterior surface of the device. When the display is configured as a touchscreen, the display may be capable of receiving touch inputs at the exterior surface. The device may include a cover sheet (e.g., a cover glass) positioned over the display that forms at least a portion of the exterior surface. The display is capable of providing graphical outputs and, when configured as a touch screen, receiving touch inputs through the cover sheet. In some embodiments, the display includes one or more sensors (e.g., capacitive touch sensors, ultrasonic sensors, or other touch sensors) positioned above, below, or integrated with the display portion. In various embodiments, a graphical output of the display is responsive to inputs provided to the electronic device. The portable electronic device may include additional components typical of computing devices, including a processing unit, memory, input devices, output devices, additional sensors, and the like.

FIGS. 1A and 1B show an example of a device 100 that houses an optical measurement system 102 as described herein. FIG. 1A shows a side view of a device 100 comprising, for example, a housing 104 having a top exterior surface 106 (the housing 104 is depicted as a cross-section in FIG. 1A to reveal a side view of components of the optical measurement system 102 that are positioned within the housing 104). This top exterior surface 106 defines a sampling interface 107 for the optical measurement system 102, through which light generated by the optical measurement system 102 can be emitted from the optical measurement system 102 and the device 100. Light may also pass through the sampling interface 107 to re-enter the optical measurement system 102 and the device 100. While the same surface of the device 100 is used as a sampling interface 107 for emission and collection of light from the optical measurement system, it should be appreciated that the sampling interface 107 may span multiple surfaces of the device 100 such that light may be emitted and/or collected from different surfaces of the device 100 (e.g., light is emitted from the optical measurement system 102 at a portion of the sampling interface 107 on a first surface of the device 100 and light is collected by the optical measurement system 102 at a second sampling interface at a second portion of the sampling interface 107 on a second surface of the device 100). Additionally or alternatively, light may be emitted from multiple different surfaces of the device 100 and or collected from multiple different surfaces of the device.

The sampling interface 107 includes at least one window that defines a set of launch sites 108a-108d of the optical measurement system 102 and a set of collection sites 110a-110d of the optical measurement system 102. In the variation shown in FIGS. 1A and 1B, the device includes a plurality of launch sites 108a-108d and a plurality of collection sites 1 10a-1 10d. While FIGS. 1A and 1B depict an equal number (e.g., four) of launch sites 108a-108d and collection sites 1 10a-1 10d, in other instances the sampling interface 107 has an unequal number of launch sites 108a-108d and collection sites 110a-110d. Each window that defines a launch site and/or a collection site is transparent at any wavelength or wavelengths used by the measurement channels of the optical measurement system 102 that are associated with the launch site and/or collection site. For example, during a spectroscopic measurement, a given measurement channel will perform one or more individual measurements using a set of wavelengths (collectively the "measurement wavelengths" for that measurement channel, which may some or all of the measurement wavelengths of the spectroscopic measurement performed by the optical measurement system), as described in more detail herein. Accordingly, any window defining a launch site may be transparent at the measurement wavelengths for all of the measurement channels that include the launch site. Similarly, any window defining a collection site may be transparent at the measurement wavelengths for all of the measurement channels that include the collection site.

In some instances, each of the set of launch sites 108a-108d and each of the collection sites 110a-110d is defined by a different corresponding window. For example, a first launch site 108a may be defined by a first window, a second launch site may be defined by a second window, a first collection site 110a may be defined by a third window, a second collection site 110b may be defined by a fourth window, and so on. In these instances, the individual windows defining the various launch sites and collections sites may be separated from each other by one or more opaque portions of the housing (i.e., that absorb or otherwise block light transmission at the measurement wavelengths used by the optical measurement system 102). In other variations, some or all of the launch sites 108a-108d and/or collection sites 110a-110d are defined in a common window (e.g., using a mask that is opaque at the measurement wavelengths that is deposited on the window to define apertures that form the various launch sites and/or collection sites). Additionally or alternatively, the device 100 may include barriers, baffles, or other light-blocking structures (not shown) that may at least partially define some or all of the launch sites 108a-108d and collection sites 110a-1 10d. These light-blocking structures may block stray light and act as a guide to limit the paths that light can take within the optical measurement system 102 before reaching a given launch site or collection site at the sampling interface 107.

The optical measurement system 102 is capable of generating light and emitting light through the set of launch sites 108a-108d during a spectroscopic measurement. Specifically, the optical measurement system 102 may include a light source unit 140 that is configured to generate light in a range of wavelengths (including the measurement wavelengths used to perform the various individual measurements of the spectroscopic measurement). The light source unit 140 includes a set of light sources (not shown), each of which is selectively operable to emit light at a corresponding set of wavelengths. Each light source may be any component capable of generating light at one or more particular wavelengths, such as a light-emitting diode or a laser. A laser may include a semiconductor laser, such as a laser diode (e.g., a distributed Bragg reflector laser, a distributed feedback laser, an external cavity laser), a quantum cascade laser, or the like. A given light source may be single-frequency (fixed wavelength) or may be tunable to selectively generate one of multiple wavelengths (i.e., the light source may be controlled to output different wavelengths at different times). The set of light sources may include any suitable combination of light sources, and collectively may be operated to generate light at any of a plurality of different wavelengths.

To the extent the light source unit 140 is capable of generating multiple different wavelengths, the light source unit 140 may be configured to generate different wavelengths of light simultaneously and/or sequentially. In some instances, such as the variation shown in FIGS. 1A and 1B, the optical measurement system 102 comprises a photonic integrated circuit 112. In these instances, the light source unit 140 may be integrated into a photonic integrated circuit 112 or may be separate from the photonic integrated circuit 112 and couple light into the photonic integrated circuit 112.

The photonic integrated circuit 112 routes light generated by the light source unit 140, and launches light from the photonic integrated circuit 112 to form one or more light beams. For example, the photonic integrated circuit 112 may include one or more outcouplers (e.g., an edge coupler, a vertical output coupler, or the like) for launching light from the photonic integrated circuit 112. The photonic integrated circuit 112 may be configured to emit a single light beam, or may be configured to emit multiple light beams. For example, in instances where the sampling interface 107 includes multiple launch sites 108a-108d, it may be desirable for the photonic integrated circuit 112 to emit a different light beam for each individual launch site of the set of launch sites 108a-108d (e.g., a first light beam for the first launch site 108a, a second light beam for the second launch site 108b, and so on). For example, in instances where the optical measurement system 102 includes multiple measurement subsystems, different launch sites 108a-108d may be associated with different measurement subsystems, which may allow the optical measurement system 102 to perform individual measurements at different locations of a measured sample.

In some of these variations, the optical measurement system may individually control the timing and/or properties of some or all of the light beams emitted the photonic integrated circuit 112. Specifically, in some instances the emission of different light beams from the photonic integrated circuit 112 is individually controllable. For example, the photonic integrated circuit 112 may be controllable to selectively launch a first set of light beams independently of a second set of light beams. Accordingly, at any given time the optical measurement system 102 may control whether i) only the first set of light beams (and not the second set of light beams) is launched from the photonic integrated circuit 112, ii) only the second set of light beams (and not the first set of light beams) is launched from the photonic integrated circuit 112, or iii), in instances when the photonic integrated circuit 112 is capable of emitting both sets of light beams simultaneously, both the first and the second sets of light beams are simultaneously launched by the photonic integrated circuit 112. Additionally or alternatively, the photonic integrated circuit 112 may be able to generate light beams with different light properties, such as intensity, phase, and/or wavelength. Overall, individual control of different beams or different groups of beams may reduce the amount of stray light that is lost within the optical system, may allow for selective control of light emission through the individual launch sites 108a-108d (e.g., allowing some launch sites to emit light while other launch sites are not actively emitting light), and/or may allow for light properties such as intensity, phase, or wavelength to be selectively varied between different launch sites 108a-108d.

It should be appreciated that in some variations, the optical measurement system 102 may include multiple photonic integrated circuits, each of which may include a different corresponding light source unit. In these instances, different photonic integrated circuits may be used to generate different light beams. The different photonic integrated circuits may be used to direct light beams to different subsets of the launch sites 108a-108d (e.g., as part of different measurement subsystems or different groups of measurement subsystems), which may provide flexibility in routing light beams to different launch sites 108a-108d.

The optical measurement system 102 may include additional light modification components between the photonic integrated circuit 112 and the sampling interface 107. These light modification components collectively act to route light from the photonic integrated circuit 112 to the various launch sites 108a-108d. For example, these light modification components may act to redirect, combine (e.g., such that multiple light beams launched from the photonic integrated circuit 112 are combined into a single light beam), split (e.g., such that a single light beam is split into multiple individual light beams), change the divergence of, reshape, or otherwise modify the light beams launched from the photonic integrated circuit 112. Examples of light modification components include lenses (which change the divergence and/or direction of a light beam), diffusers, mirrors, beamsplitters, polarizers, or the like. For the purpose of illustration, a first set of optical components is depicted schematically as box 114 positioned between the photonic integrated circuit 112 and the sampling interface 107. It should be appreciated that in some instances the sampling interface 107 itself may act as a light modification component (e.g., it may have an integrated lens or the like that can change the divergence and/or direction of the light passing therethrough). Collectively, the photonic integrated circuit 112, the sampling interface 107, and any intervening light modification components 114 may form a launch architecture of the optical measurement system 102 and cooperate to determine the characteristics of light emitted from each of the launch sites 108a-108d. Examples of different launch architectures are described in more detail herein.

The optical measurement system further comprises one or more detector groups 116a-116d, each of which includes a corresponding set of detector elements. Each of the one or more detector groups 116a-116d is positioned within the device 100 to receive light that has entered the device 100 (and thereby the optical measurement system 102) through the sampling interface 107 (e.g., via one or more of the set of collection sites 1 10a-1 10d). Each of the one or more detector groups 116a-116d includes one or more detector elements configured to measure light received by the optical measurement system 102 (e.g., light that has been emitted from the optical measurement system toward a measured sample and returned to the optical measurement system) during a measurement. Each detector element is capable of generating an output signal that represents an amount of light measured by that detector element (and thus forms an output signal of a measurement channel as described herein). Accordingly, a detector group that includes multiple detector elements may generate multiple corresponding output signals.

It should be appreciated that in some instances the output of two or more detector elements may be combined, such that the signals generated by the two or more detector element are combined into a single output signal, such as described in more detail herein. Overall, the light measured by the set of detector groups during a measurement sequence of individual measurement may be analyzed to determine one or more properties of the sample being measured. Light may be measured by a given detector element (or a combined group of detector elements) while a corresponding emission site (including one or more launch sites 108) is emitting light, which may represent how the emitted light interacts with the sample. Light may optionally also be measured while the corresponding emission site is not actively emitting light, which may measure background light incident on the detector element and/or dark current for use in a background correction operation.

In some variations the optical measurement system 102 comprises one or more light modification components positioned between the sampling interface 107 and one or more of the detector groups 116a-116d. These light modification components collectively act to route light from the various collection sites 110a-110d to the detector groups 116a-116d. For example, these light modification components may act to redirect, combine (e.g., such that multiple light beams collected by one or more collection sites 110a-110d are combined into a single light beam), split (e.g., such that a single light beam is split into multiple individual light beams), change the divergence of, reshape, or otherwise modify the light beams collected by the set of collection sites 110a-110d. Examples of light modification components include lenses (which change the divergence and/or direction of a light beam), diffusers, mirrors, beamsplitters, polarizers, or the like. For the purpose of illustration, a second set of optical components is depicted schematically in FIG. 1A as box 118 positioned between the sampling interface 107 and the one or more detector groups 116a-116d.

For each of the set of collection sites 1 10a-1 10d, the light modification components 118 may control how light entering the optical measurement system 102 via that collection site is routed to one or more detector elements of the detector groups 116a-116d. As an example, if light entering a first collection site 110a has a first set of characteristics (e.g., enters the optical measurement system 102 a particular location of the first collection site 110a with a particular angle of incidence) it may be routed by the light modification components 118 to a first detector element (e.g., a first detector element of a first detector group 116a). If light entering the first collection site 110a has a different second set of characteristics (e.g., a different combination of entry location and angle of incidence), it may be routed to a second detector element (e.g., a second detector element of the first detector group 116a). Collectively, the one or more detector groups 116a-116d, the sampling interface 107, and any intervening light modification components 118 may form one or more collection architectures of the optical measurement system 102 and cooperate to determine the characteristics of light collected by each of the collection sites 1 10a-1 10d and measured by the one or more detector groups 116a-116d. Examples of different collection architectures are described in more detail herein.

In some instances where the optical measurement system 102 includes a photonic integrated circuit 112, the photonic integrated circuit 112 and one or more of the detector groups 116a-116d are mounted to a common component. For example, in the variation shown in FIGS. 1A and 1B, the optical measurement system 102 comprises an interposer 120. In these instances, the photonic integrated circuit 112 and the one or more detector groups 116a-116d are all mounted on the interposer 120, which in turn may act as an electrical interface for these components (e.g., to route power, control, and/or other signals to and/or from the components). In some instances, the interposer also acts as a heat sink. In variations where the optical measurement system 102 includes multiple photonic integrated circuits, some or all of the photonic integrated circuits may be mounted on the interposer 120. In other variations, the photonic integrated circuit 112 is mounted to a separate component than some or all of the detector groups 116a-116d. In still other variations, some or all of the detector groups 116a-116d are directly mounted on (or otherwise integrated into) a portion the photonic integrated circuit 112.

Also shown in FIG. 1A is a controller 160. The controller 160 is configured to control the operation of the optical measurement system 102 to perform a spectroscopic measurement. Specifically, the controller 160 is operatively coupled to various components of the optical measurement to control operation thereof. For example, the controller 160 may be operatively connected to the light source unit 140, and may control the light source unit 140 to generate light at one or more wavelengths as needed during a spectroscopic measurement. Similarly, the controller 160 may be operatively connected to the one or more detector groups 116a-116d to receive the output signals generated by the various detector elements. Indeed, the controller 160 may control the operation of any active component (e.g., controllable phase shifters, optical switches, moving components) during a spectroscopic measurement. Additionally, the controller 160 may be configured to process the output signals received during a spectroscopic measurement to determine one or more sample properties of a sample being measured.

The controller 160 may include any suitable combination of hardware, software, and/or firmware as may be necessary to control the various operations of the optical measurement system 102. For example, the controller 160 may include one or more processors and memory. Memory can include one or more non-transitory computer-readable storage mediums, for storing computer-executable instructions, which, when executed by one or more computer processors, for example, can cause the computer processors to perform the techniques that are described here (such as controlling the individual components of the optical measurement system 102 to perform a spectroscopic measurement). A computer-readable storage medium can be any medium that can tangibly contain or store computer-executable instructions for use by or in connection with the instruction execution system, apparatus, or device. In some examples, the storage medium is a transitory computer-readable storage medium. In some examples, the storage medium is a non-transitory computer-readable storage medium. The non-transitory computer-readable storage medium can include, but is not limited to, magnetic, optical, and/or semiconductor storages. Examples of such storage include magnetic disks, optical discs, as well as persistent solid-state memory such as flash, solid-state drives, and the like.

Similarly, the one or more processors can include, for example, dedicated hardware as defined herein, a computing device as defined herein, a processor, a microprocessor, a programmable logic array (PLA), a programmable array logic (PAL), a generic array logic (GAL), a complex programmable logic device (CPLD), an application-specific integrated circuit (ASIC), a field-programmable gate array (FPGA), or any other programmable logic device (PLD) configurable to execute an operating system and operation of the optical measurement system 102. The specific details and arrangements of a controller 160 that may control the various operations of the optical measurement system 102 will readily be understood by one of ordinary skill in the art, and thus will not be described in further detail herein.

The optical measurement systems described herein, such as the optical measurement system 102 of FIGS. 1A and 1B, may perform a spectroscopic measurement on a sample to determine one or more sample properties of the measured sample. The optical measurement system may utilize one or more measurement channels to perform a measurement sequence that includes set of individual measurements. Specifically, each individual measurement represents a measurement performed by a particular measurement channel over a sampling duration and using light of a particular wavelength or plurality of wavelengths. The spectroscopic measurement may include multiple individual measurements that are sequentially performed by the same measurement channel (e.g., at different corresponding wavelengths). Additionally or alternatively, the spectroscopic measurement may include individual measurements that are performed by multiple measurement channels (i.e., each of the multiple measurement channels performs one or more corresponding individual measurements). Individual measurements from different measurement channels may be performed concurrently (e.g., during the same sampling period or during partially overlapping sampling periods) or sequentially as may be desired. Collectively, the spectroscopic measurement may include multiple individual measurements performed across a range of wavelengths (i.e., the measurement wavelengths of the spectroscopic measurement), which may be combined or collectively analyzed to determine one or more sample properties of the measured sample.

During each individual measurement, the optical measurement system emits light and measures light returned to the optical measurement system for a given measurement channel. The relative amount of light returned for a given individual measurement may depend on the sample being measured and thus may provide information about one or more properties of the sample. Specifically, a portion of light introduced into a sample may be absorbed as it travels through the sample. The amount of light absorbed depends at least in part on the contents of the sample (e.g., the presence and concentration of different substances within the sample) as well as the optical path length of the light (i.e., the length that light travels within the sample). Accordingly, it may be desirable to understand and control the path length of light measured by the individual measurement channels of the optical measurement systems described herein.

For example, the optical measurement systems may be configured to measure a volume-scattering sample, such as skin. In these instances, the launch site(s) and collection site(s) associated with a given measurement channel may be positioned such that light introduced into the sample will scatter within the sample before returning to the optical measurement system. Individual photons may scatter differently within a volume-scattering sample, and thus each photon measured by a measurement channel may travel a different corresponding path (with a corresponding path length) through the sample. Accordingly, each measurement channel may be associated with a corresponding "path length distribution", which represents a probability distribution that represents the likelihood that photons of light measured by the measurement channel (i.e., introduced into a sample from the optical measurement system and collected by a detector element of the measurement channel) have a particular optical path length for a sample having certain properties (e.g., a target sample as described herein). It should be appreciated that the path length distribution of light measured by a measurement channel may vary based on the sample characteristics of the sample being measured. An individual measurement performed on a target sample may have a certain path length distribution for a given measurement channel, and this path length distribution may change as the measurement channel is used to measured different measured samples (e.g., having sampling characteristics that vary from the target sampling characteristics).

The characteristics of the path length distribution of a given measurement channel may impact the accuracy of individual measurements performed by the measurement channel. Wide variations in the range of measured optical path lengths may make it more difficult to accurately measure certain sample properties (e.g., the presence and/or concentration of a particular substance within the sample). For example, a narrower distribution (i.e., with less dispersion) may provide higher confidence that light measured by the measurement channel has a particular optical path length. Accordingly, it may be desirable to design one or more the measurement channels of an optical measurement system to provide a narrow path length distribution for measurements performed on a target sample (e.g., using a target wavelength as described herein).

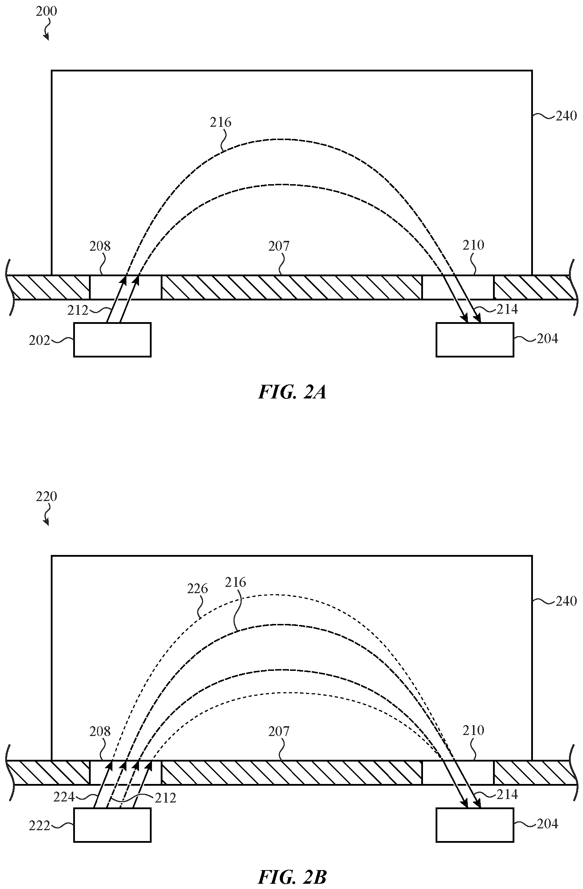

FIGS. 2A-2C illustrate how the design of a measurement channel may impact the path length distribution of measurements performed by that measurement channel. Specifically, FIG. 2A shows one example of a measurement channel of a first optical measurement system 200. The first optical measurement system 200 may be configured in any manner as described above with respect to optical measurement system 102 of FIGS 1A and 1B. Specifically, the first optical measurement system 200 includes launch architecture (depicted schematically as box 202), collection architecture (depicted schematically as box 204), and a sampling interface 207 defining a launch site 208 and a collection site 210.

The launch architecture 202 is configured to generate an emission light beam 212 that exits the sampling interface 207 at the launch site 208. Similarly, the collection architecture 204 is configured to collect a return light beam 214 through the collection site 210, and direct this return light beam 214 to a detector element (not shown) of the measurement channel. It should be appreciated that the collection site 210 may receive additional light beyond the return light beam 214, but that this light is not measured by the detector element of the measurement channel shown in FIG. 2A. In some instances, some of this light may be measured by other measurement channels of the first optical measurement system 200.

The emission light beam 212 may have particular beam properties as it exits the launch site 208, including a beam size (i.e., the dimensions of the light beam as it exits the launch site 208), beam shape (i.e., the shape of the light beam as it exits the launch site 208), and beam vergence (e.g., the amount of divergence of the light beam as it exits the launch site 208). When a measured sample is placed against the sampling interface 207, such as the target sample 240 as shown in FIG. 2A, the emission light beam 212 will have these beam properties as it enters the sample. Similarly, the return light beam 214 will also have a set of beam properties (i.e., beam size, beam shape, and beam vergence) as it exits the sample and enters the collection site 210. Because the beam vergence of a light beam depends on the refractive index of the medium carrying the beam of light, the actual beam vergence of the emission light beam 212 as it exits the launch site 208 depend on what is positioned against the launch site 208 when as the emission light beam exits the launch site 208. Accordingly, when values of beam vergence are used to characterize a light beam as described herein, these values reflect the beam vergence of that light beam as measured in air (e.g., with a refractive index of 1) unless otherwise specified.

Collectively, the beam properties of the emission light beam 212 and the return light beam 214 may at least partially determine the path length distribution, for a given sample, of light measured by the measurement channel. For example, when the first optical measurement system 200 is used to measure a target sample 240 as shown in FIG. 2A, the emission light beam 212 will penetrate into the target sample 240 as it exits the sampling interface 207. As the light interacts with the target sample 240, at least a portion of the light will be returned to the sampling interface 207 (e.g., via scattering) at the set of ray positions/angles that define the return light beam 214. The amount of light that returns to the optical measurement system 200 as the return light beam 214 depends at least in part on the target sample characteristics (e.g., the target scattering coefficient, the target absorption coefficient, and the target refractive index).

While individual photons may take different paths through the target sample 240, collectively the light measured by the detector element (i.e., the collected return light beam 214) will predominantly travel through a particular volume of target sample 240. For example, FIG. 2A includes a sampling volume 216 of possible optical paths for the measurement channel shown in FIG. 2A. Specifically, the sampling volume 216 represents the possible regions of the sample that may be measured by the measurement channel (i.e., by a photon in the emission light beam 212 returning to the first optical measurement system 200 as part of the return light beam 214) with a minimum threshold probability. In other words, it may be possible for an individual photon measured by the measure channel to travel outside of the sampling volume 216, but the likelihood of that happening is below the minimum threshold probability.

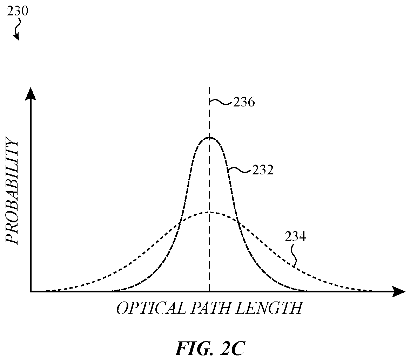

Individual photons may take a range of different possible optical paths in the target sample 240, and each possible optical path has a corresponding likelihood of occurrence for a given photon. Collectively, it may be more likely that photons will follow optical paths having a given optical path length (or range of optical path lengths). For example, FIG. 2C shows a plot 230 of probability as a function of optical path length, which represents the relative probability that a photon of light collected by a measurement channel traversed a particular optical path length. Specifically, plot 230 includes a path length distribution 232 of the measurement channel of the first optical measurement system 200 shown in FIG. 2A. The measurement channel shown in FIG. 2A is configured to generate a collimated emission light beam 212 and collect a collimated return light beam 214, each of which has a relatively narrow beam width. Accordingly, the path length distribution 232 of this measurement channel has a relatively narrow distribution around a median path length 236. As used herein, a "median path length" of a path length distribution refers to the path length that represents the 50% value of a cumulative distribution function that is based on the path length distribution. In other words, a given photon measured by a measurement channel has a 50% probability of traveling an optical path length in the sample that is shorter than the median path length.

Conversely, FIG. 2B shows a measurement channel of a variation of a second optical measurement system 220. The second optical measurement system 220 may be configured the same as the first optical measurement system 200 (e.g., with the same sampling interface 207 and collection architecture 204), except that the second optical measurement system 220 includes a different launch architecture 222 configured to generate a collimated emission light beam 224 that has a wider beam width than the collimated emission light beam 212 (shown in phantom in FIG. 2B for comparison) generated by the launch architecture 202 of FIG. 2A. By increasing the beam width of the collimated emission light beam 224, the measurement channel of the second optical system 220 will measure a larger sampling volume 226 (with the same minimum threshold probability) than the sampling volume 216 (shown in FIG. 2B for comparison) measured by the measurement channel of the first optical measurement system 200. Returning to FIG. 2C, the measurement channel of the second optical measurement system 220 has a second path length distribution 234 with a wider distribution, even though the path length distributions 232, 234 may have the same median path length 236. Similarly, changing the beam vergence (e.g., increasing the divergence) of the emission and/or collection beam of a measurement channel may increase the measured volume, but may negatively impact the path length distribution for that measurement channel.

### Optical Measurement Systems with Increased Volume Sampling

For many optical measurement systems there is a tradeoff between the sampling volume measured by a measurement channel and the shape of the path length distribution for that measurement channel. A narrower path length distribution may provide for a better understanding of the optical path length of measured light, which may improve the accuracy of spectroscopic measurements. A larger sampling volume, on the other hand, may make the optical measurement system less susceptible to local heterogeneities in the measured sample and/or coherent noise associated with the sampling volume. Accordingly, it may be desirable to provide an optical measurement system with one or more measurement channels designed to measure a larger sampling volume while still maintaining a narrow path length distribution.

Specifically, in some variations of the optical measurement systems described herein, the optical measurement systems are configured to emit a converging emission light beam into a measured sample. Depending on the configuration of the optical measurement system, such as will be described in more detail herein, a converging emission light beam may increase the sampling volume of a target sample for one or more measurement channels without significantly changing the path length distributions of these measurement channels.

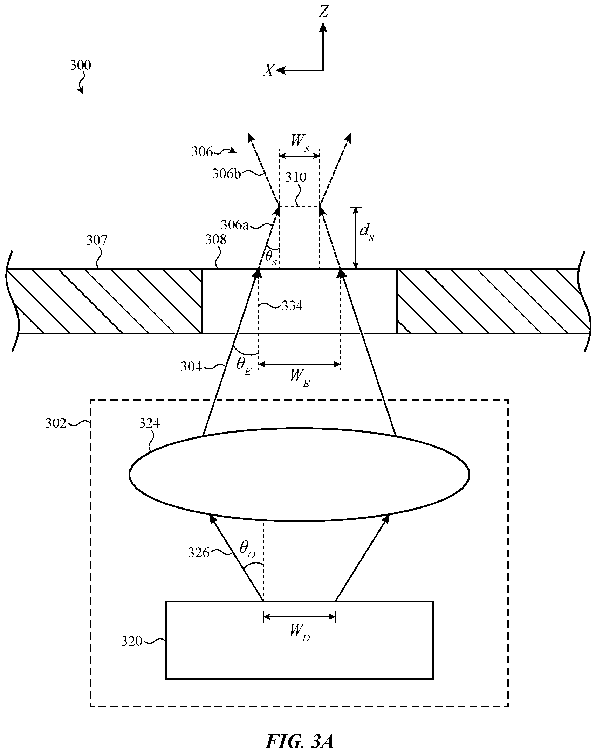

FIG. 3A shows a partial cross-sectional side view of a portion of a variation of an optical measurement system 300 as described herein. The optical measurement system 300 includes a sampling interface 307 having a launch site 308, such as described in more detail herein, and launch architecture 302. The launch architecture 302 is configured to generate and emit an emission light beam 304 that exits the sampling interface 307 through the launch site. While the sampling interface 307 is not depicted in FIG. 3A as being part of the launch architecture 302, it should be appreciated that in some variations the sampling interface 307 may be configured to shape or otherwise steer the emission light beam 304 (and may thereby be part of the launch architecture).

The emission light beam 304 has certain beam properties as it exits the sampling interface 307, including a beam width W_{E} and a beam vergence half-angle θ_{E} along a first dimension (e.g., along an XZ plane using the cartesian coordinate system depicted in FIG. 3A). The beam width W_{E} represents the size of the emission light beam 304 along the first dimension as it exits the optical measurement system 300 and enters a sample (not shown) positioned in contact with the sampling interface 307. Similarly, the beam vergence half-angle θ_{E} represents the half-angle at which the emission light beam 304 converges along the first dimension as it exits the optical measurement system 300 and enters the sample. While not shown in FIG. 3A, it should be appreciated that the beam vergence of the emission light beam 304 may have different values it passes through different materials. For example, the launch site 308 may be formed from a material (e.g., a sapphire crystal, glass, or the like), and the emission light beam 304 may have a different beam vergence when passing through this material as compared to is beam vergence when passing through different media on either side of the launch site 308 (e.g., air present against an interior side of the launch site 308 and/or a sample positioned against an exterior side of the launch site 308).

While the emission light beam 304 is shown as passing through the launch site 308 at a normal incidence (e.g., the beam direction 334 of the emission light beam 304 is perpendicular to an outer surface of the launch site 308), it should be appreciated that in some instances the emission light beam 304 may pass through the launch site 308 at a non-normal incidence (e.g., the beam direction 334 of the emission light beam 304 is positioned at a non-perpendicular angle relative to an outer surface of the launch site 308).

When a sample is placed against the launch site 308, the emission light beam 304 projects a projected emission light beam 306 into the sample. The projected emission light beam 306 represents the path the emission light beam 304 would take through the sample if no scattering were to occur within the sample. In other words, the projected emission light beam 306 represents the initial trajectories of photons of the emission light beam 304 as they enter the sample. Accordingly, while the actual photons of the emission light beam 304 may undergo one or more scattering events after entering the sample, the projected emission light beam 306 may be beneficial in understanding the emission light beam 304 as it leaves the optical measurement system 300.

Specifically, the emission light beam 304 may be configured to project the projected emission light beam 306 to a transition region 310 in a target sample. The transition region 310 represents the location at which the projected emission light beam 306 transitions from a converging beam (as illustrated by converging portion 306a of the projected emission light beam 306) to a diverging beam (as illustrated by diverging portion 306b of the projected emission light beam 306) along a first dimension.

The projected emission light beam 306 will have, at the transition region 310, a beam width Ws and a beam vergence half-angle θ_{S} along the first dimension. Additionally, the transition region 310 will be located at a depth ds in the target sample. It should be appreciated that the beam width Ws and beam vergence half-angle θ_{S}, as well as the depth ds, may depend on the beam characteristics of the emission light beam 304 (e.g., the beam width W_{E}, beam vergence half-angle θ_{E}, and beam direction 334) as well as certain sample characteristics (e.g., the refractive index) of the target sample. For example, a refractive index change at the interface between the launch site 308 and a sample may cause the emission light beam 304 to refract as it enters the sample. While the transition region 310 is shown in FIG. 3A as being parallel to an exterior surface of the sampling interface 307, it should be appreciated that that in other instances the transition region 310 may, depending on the properties of the emission light beam 304, be angled at a non-zero angle relative to the sampling interface 307.

Increasing the beam vergence half-angle θ_{E} and/or the beam width W_{E} of the emission light beam 304 may increase the etendue of the emission light beam 304 (and thereby the projected emission light beam 306), which may increase the sampling volume measured by one or more measurement channels of the optical measurement system 300. For example, in some variations the optical measurement system 300 is configured to emit a converging emission light beam 304 having a beam vergence half-angle θ_{E} of at least five degrees, as measured in air, along a first dimension as the emission light beam 304 exits the launch site 308. In some of these variations, the converging emission light beam has a beam vergence half-angle θ_{E} of at least fifteen degrees, as measured in air, along the first dimension as the emission light beam 304 exits the launch site 308.

Similarly, in some variations the optical measurement system 300 is configured to emit an emission light beam 304 that projects a projected emission light beam 306 into a target sample (such as discussed in more detail herein), wherein the projected emission light beam 306 has, at the transition region 310, a beam vergence half-angle θ_{S} of at least three degrees, as measured in the target sample, along a first dimension. In other words, at any given point along the transition region 310, the transition region 310 is projected to receive projected rays of the projected emission light beam 306 that span at least six degrees (e.g., ± three degrees around a center ray projected to pass through that point), as measured in the target sample. In some of these variations, the beam vergence θ_{S} is at least ten degrees along the first dimension, as measured in the target sample.

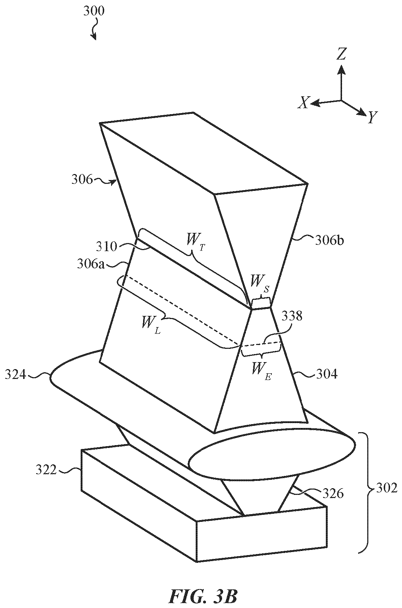

It should be appreciated that the emission light beam 304, and thereby the projected emission light beam 306, may have different beam properties in other dimensions. For example, FIG. 3B shows a perspective view of the optical measurement system 300 of FIG. 3A. The sampling interface 307 is not shown in FIG. 3B for sake of illustration, and line 338 represents the point at which the emission light beam 304 exits the sampling interface. As shown there, the emission light beam 304 may have a first beam width W_{E} in a first dimension (e.g. in the XZ plane shown in FIG. 3B) as the emission light beam 304 exits the sampling interface, and a second beam width W_{L} in a second dimension perpendicular to the first dimension (e.g., in the YZ plane shown in FIG. 3B) as the emission light beam 304 exits the sampling interface. In some variation, the second beam width W_{L} is larger than the first beam width W_{E}. In some of these variations, the second beam width W_{L} is at least four times larger than the first beam width W_{E}. In some of these variations, the second beam width W_{L} is at least eight times larger than the first beam width W_{E}. As a result, the projected emission light beam 306 may also be projected to have a second beam width W_{T} in the second dimension that is larger than the beam width Ws in the first dimension.

Similarly, the emission light beam 304 may have a different beam vergence (e.g., beam vergence half-angle θ_{E}) along the first dimension than it does along the second dimension. For example, while the emission light beam 304 (and thereby the projected emission light beam 306) may converge in the first dimension, the emission light beam 304 may be collimated in the second dimension. Alternatively, the emission light beam 304 may diverge in the second dimension. In still other variations, the emission light beam 304 may be configured to converge in the second dimension. In these instances, the beam vergence of the emission light beam 304 in the second dimension may be the same as or different than the beam vergence in the first dimension.

The properties of the emission light beam 304 and the projected emission light beam 306 may impact the accuracy of measurements perfumed by the optical measurement system 300. For example, the optical measurement systems described herein may emit an emission light beam that is configured, such that when the emission light beam is introduced into a target sample, the transition region is positioned at an angle-independent sample region for at least one measurement channel. As used herein, a sample region is considered to be "angle independent" for a given measurement channel if the path length distribution of light projected to pass through that sample region is independent of the angle at which the light is projected to pass through the sample region.

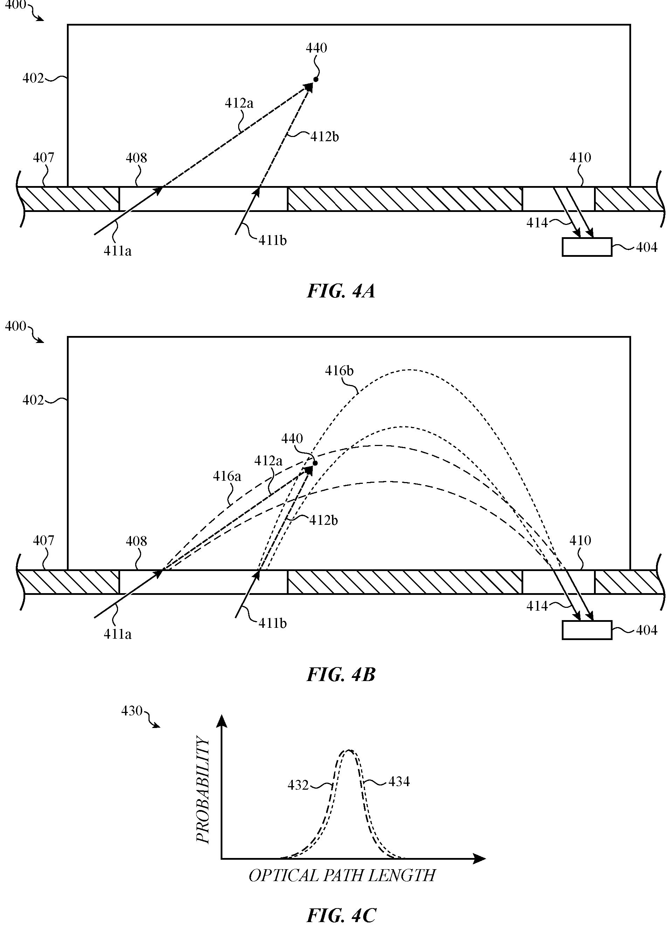

FIGS. 4A-4F illustrate the concept of an angle-independent sample region. FIGS. 4A and 4B show partial cross-sectional views of an optical measurement system 400 that is used to measure a target sample 402 using light a target wavelength. Specifically, the optical measurement system 400 includes collection architecture (depicted schematically as box 404), and a sampling interface 407 defining a launch site 408 and a collection site 410 such as described herein. The optical measurement system 400 includes a first measurement channel, and the collection architecture 404 is configured to collect a return light beam 414 from the target sample 402 through the collection site 410, and direct this return light beam 414 to a detector element (not shown) of the first measurement channel.

Also shown in FIG. 4A is a first sample region 440 that is angle independent for the first measurement channel. To illustrate this, two example rays, a first ray 411a and a second ray 411b, are depicted in FIG. 4A. The first ray 411a and the second ray 411b are both projected to pass through the first sample region 440 as part of an emission light beam. Specifically, the first ray 411a has a first projected ray 412a that represents the projected trajectory of the first ray 411a in the target sample 402 if no scattering were to occur. Similarly, the second ray 411b has a second projected ray 412b. The first projected ray 412a and the second projected ray 412b each intersect with the first sample region 440.

If light were to be introduced into the target sample 402 along the trajectories of the first ray 411a and the second ray 411b, individual photons would scatter in the target sample 402 as described herein before being collected as part of the return light beam 414. FIG. 4B shows a first sampling volume 416a of possible optical paths for the first measurement channel that originate from the trajectory of the first ray 411a, and a second sampling volume 416b of possible optical paths for the first measurement channel that originate from the trajectory of the second ray 411b. Accordingly photons that enter the target sample 402 along the trajectory of the first ray 411a may sample different (but partially overlapping) volumes of the target sample 402 as compared to photons that enter the target sample 402 along the trajectory of the second ray 411b.

Even though light traveling along the trajectories of the first ray 411a and the second ray 411b sample different sampling volumes 416a, 416b, the light will have approximately the same path length distributions. For example, FIG. 4C shows a plot 430 of probability as a function of optical path length. Specifically, plot 430 includes a first path length distribution 432 and a second path length distribution 434 of the first measurement channel of the optical measurement system 400. The first path length distribution 432 represents the path length distribution for light that enters the target sample 402 along the trajectory of the first ray 411a and is collected as part of the return light beam 414 (e.g., is measured by the first measurement channel). The second path length distribution 434 represents the path length distribution for light that enters the target sample 402 along the trajectory of the second ray 411b and is measured by the first measurement channel.

The first path length distribution 432 is approximately the same as the second path length distribution 434. For the purpose of purpose of this application, a plurality of path length distributions (each associated with a corresponding median path length) is considered to be "approximately the same" if the median path lengths of the plurality of path length distributions are all within 10% (e.g., ± 10%) of the average of the median path lengths. Similarly, additional rays (not shown) that are projected to cross through the first sample region 440 from other angles may also have corresponding path length distributions that are approximately the same as the first path length distribution 432 and the second path length distribution 434. Accordingly, if these various rays were combined into a light beam, the overall path length distribution of the light beam would be approximately the same as the individual path length distributions associated with each ray. In this way, light projected to travel through the first sample region 440 is independent of the angle at which the light projected to travel through the sample, the first sample region 440 is considered to be an angle-independent sample region for the measurement channel.

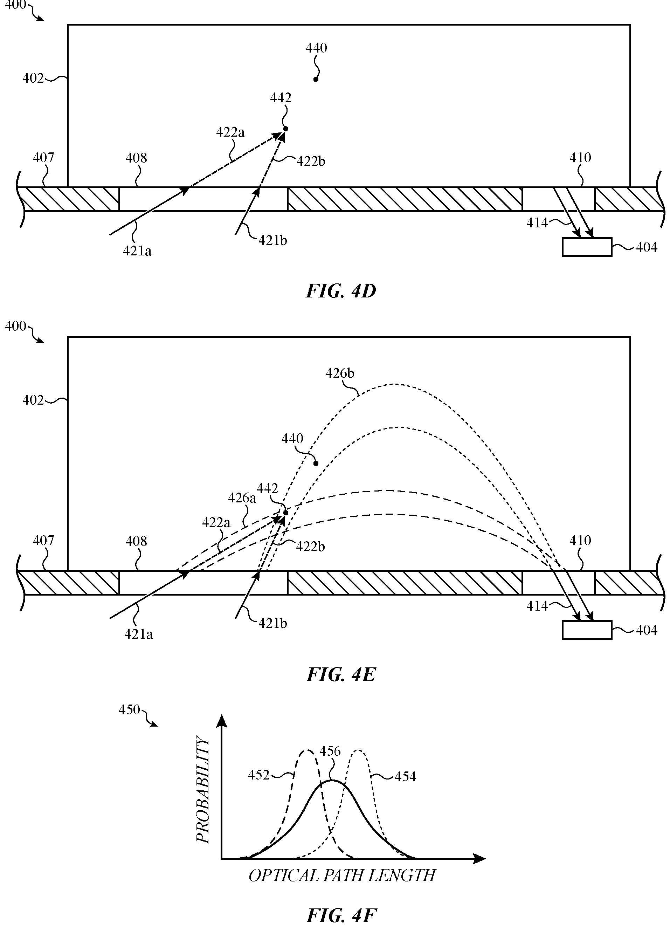

It should be appreciated that other regions of the target sample 402 will not be angle independent for the first measurement channel. FIGS. 4D and 4E also depict partial cross-sectional side views of the optical measurement system 400 of FIGS. 4A and 4B, and highlights a second sample region 442 that is not angle independent for the first measurement channel. Two example rays, a third ray 421a and a fourth ray 421b are depicted in FIGS. 4D and 4E that are projected to pass through the second sample region 442 (e.g., corresponding third and fourth projected rays 422a, 422b intersect with the second sample region 442). If light were to be introduced into the target sample 402 along the trajectories of the third ray 421a and the fourth ray 421b, individual photons would scatter in the target sample 402 as described herein before being collected as part of the return light beam 414. FIG. 4E shows a third sampling volume 426a of possible optical paths for the first measurement channel that originate from the trajectory of the third ray 421a, and a fourth sampling volume 426b of possible optical paths for the first measurement channel that originate from the trajectory of the fourth ray 421b.

Light sampling the third sampling volume 426a and the fourth sampling volume 426b will have different path length distributions. FIG. 4F shows a plot 450 of probability as a function of optical path length. Specifically, plot 450 includes a third path length distribution 452 and a fourth path length distribution 454 of the first measurement channel of the optical measurement system 400. The third path length distribution 452 represents the path length distribution for light that enters the target sample 402 along the trajectory of the third ray 421a and is measured by the first measurement channel. The fourth path length distribution 454 represents the path length distribution for light that enters the target sample 402 along the trajectory of the fourth ray 421b and is measured by the first measurement channel.

The third path length distribution 452 is different than the fourth path length distribution 454 in one or more ways. For example, as shown in FIG. 4F, the third path length distribution 452 and the fourth path length distribution 454 have sufficiently different median path lengths such that the third and fourth path length distributions 452, 454 are not approximately the same. If light is simultaneously introduced along the trajectories of the third and fourth rays 421a, 421b, the overall path length distribution 456 of light measured by the first measurement channel will be a combination of the third path length distribution 452 and the fourth path length distribution 454, which may significantly spread out the overall path length distribution as compared to either of the third or fourth path length distributions 452, 454. Introducing light along additional trajectories that are projected to pass through the second sample region 442 will further spread the shape of the overall path length distribution 456. Because different rays of the emission light beam are associated with different path length distributions, the second sample region 442 is an angle-dependent sample region 442.

Returning to FIGS. 3A and 3B, if the transition region 310 is positioned at an angle-independent region of a target sample for a given measurement channel, the entire emission light beam 304 is projected to intersect the angle-independent region of the target sample. Specifically, the path length distributions of the individual rays forming the emission light beam 304 (e.g., across the beam vergence half-angle θ_{E} of the emission light beam 304 and the beam vergence half-angle θ_{S} of the projected emission light beam 306) will have approximately the same path length distribution for a target wavelength. As a result, the beam vergence half-angle θ_{E} of the emission light beam 304 may be increased to increase the overall volume of a target sample measured by a measurement channel without significantly changing the path length distribution of light measured by that measurement channel.

In these instances, it may be desirable to configure the emission light beam 304 to have a relatively large beam vergence half-angle θ_{E}. To generate such an emission light beam 304 while maintaining a compact form factor, the launch architecture 302 may include a beam generator 320 and a launch optical subassembly 324 that includes one or more lens elements. The beam generator 320 is configured to generate an output light beam 326 that diverges in at least one dimension (e.g., at least the first dimension). For example, as shown in FIG. 3A, the output light beam 326 has a beam width W_{D} in the first dimension and diverges with a beam vergence half-angle θ_{O} in the first dimension. The output light beam 326 may diverge, converge, or may be collimated as may be desired (and depending on the configuration of the beam generator 320.

The launch optical subassembly 324 is configured to receive and shape the output light beam 326 to form the emission light beam 304. While the launch optical subassembly 324 is shown in FIGS. 3A and 3B as being formed from a single cylinder lens, it should be appreciated that the launch optical subassembly 324 may include any suitable number or combination of lens elements as needed to form the emission light beam 304 from the output light beam 326. Accordingly, the relationship between the beam vergence half-angle θ_{E} of the output light beam 326 and the beam vergence half-angle θ_{O} of the emission light beam 304 along the first dimension may depend on the optical magnification of the launch optical subassembly 324 in the first dimension. For example, if the optical assembly 324 provides a 1x optical magnification power in the first dimension, the beam vergence half-angle θ_{E} of the output light beam 326 may be same as the beam vergence half-angle θ_{O} of the emission light beam 304. Examples of the beam generator 320 and launch optical subassembly 324 will be described in more detail herein.

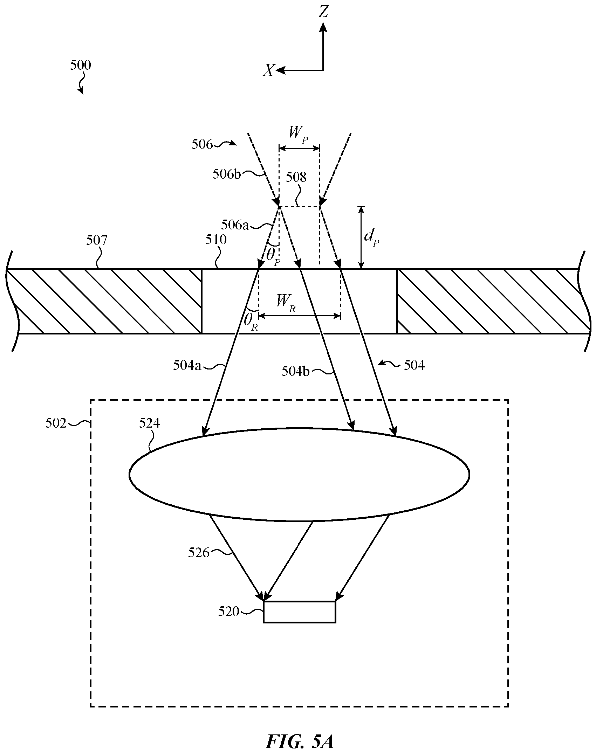

In some variation, the optical measurement systems described herein may be configured to, for at least one measurement channel, collect and measure a return light beam that is projected to intersect an angle-independent sample region. FIG. 5A shows a partial cross-sectional side view of a portion of one such variation of an optical measurement system 500 as described herein. The optical measurement system 500 includes a sampling interface 507 having a collection site 510, such as described in more detail herein, and collection architecture 502. The collection architecture 502 is configured to collect one or more return light beams that enter the sampling interface 507 through the collection site 510, and is further configured to direct each return light beam to a corresponding detector element. While the sampling interface 507 is not depicted in FIG. 5A as being part of the collection architecture 502, it should be appreciated that in some variations the sampling interface 507 may be configured to shape or otherwise steer the return beam (or beams) collected from the collection site 510 (and may thereby be part of the collection architecture).

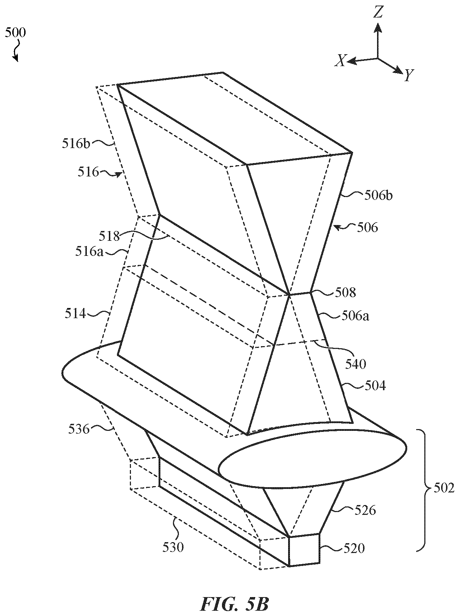

FIG. 5A depicts a first return light beam 504 that is collected by the collection architecture 502 when launch architecture (not shown) of the optical measurement system 500 emits one or more emission light beams into a measured sample (not shown). In the variation shown in FIG. 5A, the collection architecture 502 further includes a collection optical subassembly 524 and a first detector element 520. The collection optical subassembly 524 includes one or more lens elements, and is configured to receive and shape the first return light beam 504 to form a first measurement light beam 526. The collection optical subassembly 524 is further configured to direct the first measurement light beam 526 to the first detector element 520. In other words, the first return light beam 504 defines a first set of position and angle pairs on the collection site 510. Any light entering the optical measurement system 500 through the collection site 510 at a position/angle pair associated with the first return light beam 504 will be directed (e.g., via the collection optical subassembly 524 and the first measurement light beam 526) to and measured by the first detector element 520 as part of a first measurement channel of the optical measurement system 500. While the collection optical subassembly 524 is shown in FIGS. 5A and 5B as being formed from a single cylinder lens, it should be appreciated that the collection optical subassembly 524 may include any suitable number or combination of lens elements as needed to form the first measurement light beam 526 from the first return light beam 504. Furthermore, while the collection optical subassembly 524 is shown as having positive magnification, it should be appreciated that the collection optical subassembly 524 may be configured to have negative magnification, which in some instances may allow for the collection optical subassembly 524 to be configured with fewer lenses.

In some variations the first return light beam 504 diverges along a first dimension (e.g., along an XZ plane using the cartesian coordinate system depicted in FIG. 5A) as it enters the sampling interface 507. Specifically, the collection architecture 502 may be configured such that the first return light beam 504 has certain beam properties as it enters the sampling interface 507, including a beam width W_{R} and a beam vergence half-angle θ_{R} along the first dimension. The beam width W_{R} represents the size of the first return light beam 504 along the first dimension as it exits a sample (not shown) positioned in contact with the sampling interface 507 and enters the optical measurement system 500. Similarly, the beam vergence half-angle θ_{R} represents the divergence of the first return light beam 504 along the first dimension as it exits the sample and enters the optical measurement system 500. While the first return light beam 504 is shown as passing through the collection site 510 at a normal incidence, it should be appreciated that in some instances the first return light beam 504 may pass through the collection site 510 at a non-normal incidence.

When a sample is placed against the collection site 510, the first return light beam 504 may be projected to emanate from a first projected return light beam 506 in the sample. The first projected return light beam 506 represents the path that light would travel through the sample, if no scattering were to occur within the sample, in order to be part of the first return light beam 504. In other words, the first projected return light beam 506 represents an extension of the trajectories of the first return light beam 506 as it exits the sample. In practice, however, actual photons collected as part of the first return light beam 504 may take paths that deviate from the projected return light beam 506.

The first turn light beam 504 may be configured such that the first projected return light beam 506 has a transition region 508 (also referred to herein as a "first transition region 508") in a target sample. The first transition region 508 represents the location at which the first projected return light beam 506 transitions from a diverging beam (as illustrated by diverging portion 506a of the first projected return light beam 506) to a converging beam (as illustrated by converging portion 506b of the first projected return light beam 506) along a first dimension. For example, in some variations the collection optical subassembly 524 is configured to image a region within the sample onto the first detector element 520. Accordingly, the first detector element 520 may be positioned at an image plane of the collection optical subassembly 524, and the transition region 508 is positioned at the object plane of the collection optical subassembly 524. In this way, rays that are projected to emanate from a common point in the transition region 508 (e.g., first and second rays 504a, 504b of the first return light beam 504) will be directed to a common position on the first detector element 520.

The first projected return light beam 506 will have, at the transition region 508, a beam width W_{P} and a beam vergence half-angle θ_{P} along the first dimension, will be located at a depth d_{P} in the target sample. It should be appreciated that the beam width W_{P} and beam vergence half-angle θ_{P}, as well as the depth d_{P}, may depend on the beam characteristics of the first return light beam 504 (e.g., the beam width W_{R}, beam vergence half-angle θ_{R}, and beam direction of the first return light beam 504) as well as certain sample characteristics (e.g., the refractive index) of the target sample. For example, a refractive index change at the interface between the collection site 510 and a sample may cause light to refract as it exits the sample and enters the sampling interface 507 to form the first return light beam 504. While the transition region 508 is shown in FIG. 5A as being parallel to an exterior surface of the sampling interface 507, it should be appreciated that that other instances the transition region 508 may, depending on the properties of the first return light beam 504, be angled at a non-zero angle relative to the sampling interface 507.

Increasing the beam vergence half-angle θ_{R} and/or the beam width W_{R} of the first return light beam 504 may increase the etendue of the first return light beam 504 (and thereby the first projected return light beam 506), which may increase the sampling volume measured by the measurement channel associated with the first detector element 520. For example, in some variations the optical measurement system 500 is configured to collect and measure, using the collection architecture 502 and the first detector element 520, a first return light beam 504 having a beam vergence half-angle θ_{R} of at least at least five degrees, as measured in air, along a first dimension as the first return light beam 504 enters the collection site 510. In some of these variations, the diverging return light beam has a beam vergence half-angle θ_{R} of at least fifteen degrees, as measured in air, along the first dimension as the first return light beam 504 exits the launch site 308.

Similarly, in some variations the optical measurement system 500 is configured to collect and measure a first return light beam 504 that collects a first projected return light beam 506 from a target sample (such as discussed in more detail herein), wherein the first projected return light beam 506 has, at the transition region 508, a beam vergence half-angle θ_{P} of at least three degrees along a first dimension, as measured in the target sample. In other words, at any given point along the transition region 508, the first return light beam 504 is projected to receive, from that point, projected rays that span at least six degrees (e.g., ± three degrees around a center ray projected to pass through that point). In some of these variations, the beam vergence half-angle θ_{P} is at least ten degrees along the first dimension, as measured in the target sample.

It should be appreciated that the first return light beam 504, and thereby the first projected return light beam 506, may have different beam properties in other dimensions. For example, FIG. 5B shows a perspective view of the optical measurement system 500 of FIG. 5A. The sampling interface 507 is not shown in FIG. 5B for sake of illustration, and line 540 represents the point at which the first return light beam 504 enters the sampling interface. As shown there, the beam width of first return light beam 504 may be different in a first dimension (e.g. in the XZ plane shown in FIG. 5B) than in a second dimension perpendicular to the first dimension (e.g., in the YZ plane shown in FIG. 5B) as the first return light beam 504 enters the sampling interface. In some variation, first return light beam 504 has a beam width in the second dimension that is at least four times larger than the beam width in the first dimension. In some of these variations, the beam width in the second dimension is at least eight times larger than the beam width in the first dimension.

Similarly, the first return light beam 504 may have a different beam vergence along the first dimension (e.g., beam vergence half-angle θ_{R}) than it does along the second dimension. For example, while the first return light beam 504 (and thereby the first projected return light beam 506) may diverge in the first dimension as it enters the sampling interface, the first return light beam 504 may be collimated in the second dimension. Alternatively, the first return light beam 504 may converge in the second dimension. In still other variations, the first return light beam 504 may be configured to diverge in the second dimension. In these instances, the beam vergence of the first return light beam 504 in the second dimension may be the same as or different than the beam vergence in the first dimension.

In some instances, the collection architecture 502 may be configured to simultaneously collect multiple return light beams, each of which is measured by a different detector element. In this way, the optical measurement system 500 may be able to measure different portions of light emitted by a given emission light beam (e.g., the emission light beam 304 of the variation of the optical measurement system 300 described with respect to FIGS. 3A and 3B) to form different measurement channels of the optical measurement system 500. In some of these variations, the same collection optical subassembly (e.g., the collection optical subassembly 524) may be configured to simultaneously capture multiple return light beams.

For example, FIG. 5B shows a second detector element 530 (shown in phantom for ease of illustration). The collection architecture 502 optical measurement system 500 is configured to collect a second return light beam 514 that is directed to, and measured by, the second detector element 530 as part of a second measurement channel of the optical measurement system 500. Specifically, the collection optical subassembly 524 is configured to receive and shape the second return light beam 514 to form a second measurement light beam 536. The collection optical subassembly 524 is further configured to direct the second measurement light beam 536 to the second detector element 530. In other words, the second return light beam 514 defines a second set of position and angle pairs on the collection site 510. Any light entering the optical measurement system 500 through the collection site 510 at a position/angle pair associated with the second return light beam 514 will be directed (e.g., via the collection optical subassembly 524 and the second measurement light beam 536) to and measured by the second detector element 530.

The collected second return light beam 514 may be similar to the first return light beam 504 (e.g., it may be a diverging light beam that is projected from a second projected light beam 516 having a second transition region 518 between a diverging portion 516a and a converging portion 516b of the second projected light beam 516). The first transition region 508 may be positioned at a different region with a measured sample than the second transition region 518, such that the first detector element 520 and the second detector element 530 (and thereby the first and second measurement channels of the optical measurement system 500) measure light that is projected to emanate from different regions within a sample. The collection optical subassembly 524 may further be associated with additional detector elements (e.g., a third detector element), such that the collection optical subassembly 524 collects additional return light beams, each associated with a corresponding projected return light beam having a transition region.

The optical measurement system may be configured such that, when the collection architecture 502 is used to collect a set of return light beams (e.g., the first return light beam 504, the second return light beam 514, and so on) from a target sample, the corresponding transition regions of some or all of these beams are positioned at an angle-independent sample region for their corresponding measurement channels. In other words, the first transition region 508 is positioned at a region of a target sample that is angle independent for a first measurement channel defined by the first detector element 520. In this way, the first entire return light beam 504 is projected to intersect a first angle-independent region of the target sample (e.g., that is angle independent with respect to light measured by the first measurement channel). Similarly, the second transition region 518 is positioned at a region of the target sample that is angle independent for a second measurement channel defined by the second detector element 530. Accordingly, the entire second return light beam 514 is projected to intersect a second angle-independent region of the target sample (e.g., that is angle independent with respect to light measured by the second measurement channel).

In this way, the path length distribution for each measurement channel is independent of the range of angles at which the measurement channel collects light. Specifically, the path length distributions of the individual rays measured by the first detector element 520 (e.g., across the beam vergence half-angle θ_{R} of the first return light beam 504 and the beam vergence half-angle θ_{P} of the projected return light beam 506) will have approximately the same path length distribution for a target wavelength. Similarly, the path length distributions of the individual rays measured by the second detector element 530 will have approximately the same path length distribution for a target wavelength. Accordingly, increasing these beam vergences increase the etendue of the return light beams 504, 514, and may thereby increase the sampling volume measured by these measurement channels, without significantly changing the respective path length distributions of light measured by these measurement channels.

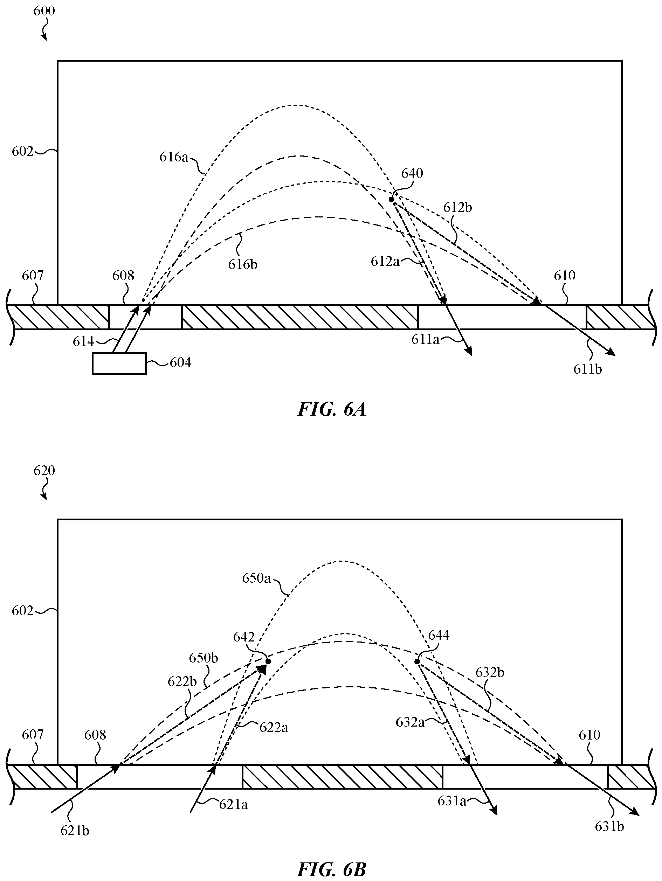

For a given measurement channel, light may be projected to be emitted toward and/or be collected from angle-independent sample regions. FIGS. 6A-6B illustrates collection of light that is projected to emanate an angle-independent sample region. Specifically, FIG. 6A shows a partial cross-sectional view of an optical measurement system 600 that is used to measure a target sample 602. Specifically, the optical measurement system 600 includes launch architecture (depicted schematically as box 604), and a sampling interface 607 defining a launch site 608 and a collection site 610 such as described herein. The optical measurement system 600 includes a first measurement channel, and the launch architecture is configured to emit an emission light beam 614 into the target sample 602 through the launch site 608. The first measurement channel also includes a detector element (not shown) that is configured to receive a portion of the light from the emission light beam 614 that is returned to the optical measurement system through the collection site 610.

FIG. 6A shows first sample region 640 that is angle independent for the first measurement channel on the collection side of the measurement channel. To illustrate this, two example rays (a first ray 611a and a second ray 611b) are depicted in FIG. 6A that represent two possible rays that may be collected by the first measurement channel. The first ray 611a and the second ray 611b are both projected to pass through the first sample region 640 (e.g., via first and second projected rays 612a, 612b, respectively, that intersect with the first sample region 640). Also shown in FIG. 6A is a first sampling volume 616a that represents the possible optical paths for the first measurement channel that enter the target sample 602 via the emission light beam 614 and exit the target sample 602 along the trajectory of the first ray 611a (e.g., at the position and ray angle of the first ray 611a). Similarly, a second sampling volume 616b represents the possible optical paths for the first measurement channel that enter the target sample 602 via the emission light beam 614 and exit the target sample 602 along the trajectory of the second ray 611b (e.g., at the position and ray angle of the second ray 611b).

Because the first sample region 640 is angle independent, light collected along the trajectory of the first ray 611a will have approximately the same path length distribution as that of light collected along the trajectory of the second ray 611b (e.g., similar to the first and second path length distributions 432, 434 depicted in the plot 430 of FIG. 4C). Additional rays may be collected that are projected to intersect with the first sample region 640, but will not significantly change the overall path length distribution of the first measurement channel. Even though light traveling along the trajectories of the first ray 611a and the second ray 611b sample different sampling volumes 616a, 616b, the light will have approximately the same path length distribution, thereby allowing the optical measurement system to increase the beam vergence of a collected return light beam for a given measurement channel while still maintaining a desired path length distribution.

In some instances, an optical measurement system may be configured to, for a given measurement channel, both i) emit light that is projected to intersect with an angle-independent sample region and ii) collect light is projected to intersect with a second angle-independent sample region. FIG. 6B shows a partial cross-sectional view of another variation of an optical measurement system 620 that is used to measure the target sample 602 of FIG. 6A. Specifically, the optical measurement system 620 includes a sampling interface 607 defining a launch site 608 and a collection site 610 such as described herein.

The optical measurement system 620 may be configured to project a first emission light beam (the boundaries of which are represented by first and second rays 621a, 621b and corresponding first and second projected rays 622a, 622b) toward a first sample region 642 that is angle independent for a first measurement channel. Similarly, the optical measurement system 620 is configured to collect a first return light beam (the boundaries of which are represented by third and fourth rays 631a, 631b and corresponding third and fourth projected rays 632a, 632b) that is measured by a detector element of the first measurement channel. The first return light beam is projected to emanate from a second sample region 644 that is angle independent for the first measurement channel. In these instances, any light that is measured by the first measurement channel will enter the target sample 602 along a trajectory that intersects the first angle-independent sample region 642 (e.g., along the trajectory of the first ray 621a, the second ray 621b, or another ray that is projected to intersect the first sample region 642) and exit the target sample 602 along a trajectory that intersects the second angle-independent sample region 644 (e.g., along the trajectory of the third ray 621a, the fourth ray 621b, or another ray that is projected to intersect the second sample region 644).

Regardless of the combination of entry and exit trajectories, light measured by the first measurement channel of the optical measurement system 620 will have approximately the same path length distribution. For example, FIG. 6B shows a first sampling volume 650a that represents the possible optical paths for the first measurement channel that enter the target sample 602 along the trajectory of the first ray 621a and exits the target sample 602 along the trajectory of the third ray 631a. Similarly, a second sampling volume 650b represents the possible optical paths for the first measurement channel that enter the target sample 602 along the trajectory of the second ray 621b and exits the target sample 602 along the trajectory of the fourth ray 631b. Light collected from the first sampling volume 650a and the second sampling volume 650b will have approximately the same path length distribution (e.g., similar to the first and second path length distributions 432, 434 depicted in the plot 430 of FIG. 4C). Similarly, any other combination of trajectories (e.g., entering the target sample 602 along the trajectory of the first ray 621a and exiting along the trajectory of the fourth ray 631b, entering along the trajectory of the second ray 621a and exiting along the trajectory of the third ray 631a) will also have approximately the same path length distribution. In this way, the optical measurement system may increase the etendue associated both the launch and collection of light into a sample.

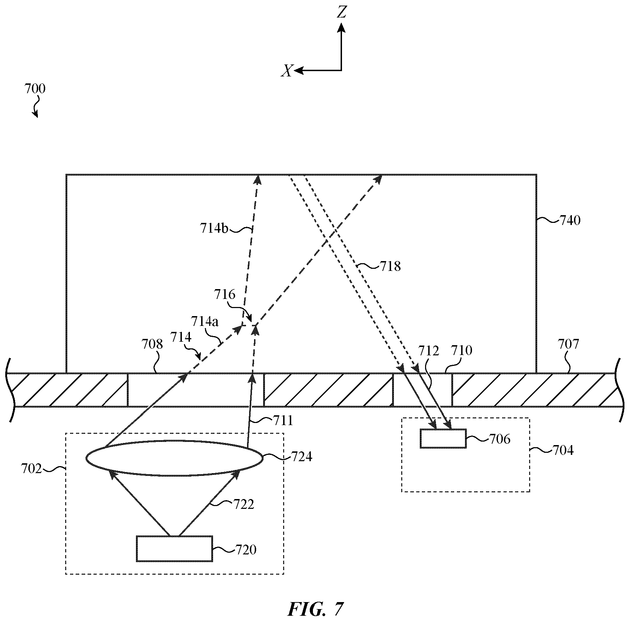

FIGS. 7-9D show examples of optical measurement systems that are configured to project light toward and/or collect light projected from angle-independent sample regions for one or more measurement channel. FIG. 7 shows a partial cross-sectional side view of an optical measurement system 700 configured to project light toward an angle-independent sample region for a first measurement channel. Specifically, the optical measurement system 700 includes a sampling interface 707 having a launch site 708 and a collection site 710, such as described in more detail herein, launch architecture 702 and collection architecture 704. The launch architecture 702 is configured to generate and emit an emission light beam 711 that exits the sampling interface 707 through the launch site 708. Similarly, the collection architecture 704 includes a detector element 706, and is configured to collect a return light beam 712 that enters the sampling interface 707 through the collection site 710 and to direct the return light beam 712 to the detector element 706.

When the launch architecture 702 emits the emission light beam 711 into a target sample 740 (or another measured sample have a predetermined set of sample characteristics), at least a portion of the emission light beam 711 will be returned to the optical measurement system 700 as part of the return light beam 712. In other words, the detector element 706 measures a portion of the emission light beam 711 that is returned to the optical measurement system 700 from the sample being measured. In this way, the detector element 706 forms a first measurement channel when the launch architecture emits the emission light beam 711.

The launch architecture 702 is configured such that the emission light beam 711 projects a projected emission light beam 714 into the sample being measured. The emission light beam 711 and the projected emission light beam 714 may have any characteristics as described above with respect to the emission light beam 304 and the projected emission light beam 306 of FIGS. 3A and 3B. Specifically, the emission light beam 711 converges along a first dimension (e.g., along an XZ plane using the cartesian coordinate system depicted in FIG. 7) as it exits the sampling interface 707. In this way, the emission light beam 711 projects a projected emission light beam 714 that has a converging portion 714a along the first dimension as it exits the sampling interface 707 and transitions to a diverging portion 714b at a transition region 716. In some instances, such as shown in FIG. 7, the launch architecture 702 may be configured such that the emission light beam 711 (and thereby the projected emission light beam 714) is angled toward the collection site 710 along the first dimension. This may preferentially direct light toward the collection site 710, which may increase the collection efficiency of the first measurement channel.

The optical measurement system 700 is further configured such that, when the emission light beam 711 is emitted into a target sample 740, the transition region 716 coincides with an angle-independent sample region for the first measurement channel. In this way, each ray of the light from the emission light beam 711 is projected to intersect the angle-independent sample region for the first measurement channel. Accordingly, light measured by the detector element 706 while the launch architecture is emitting the emission light beam 711 (i.e., light measured by the first measurement channel) will have approximately the same path length distribution regardless of where individual photons exit the sampling interface 707.

The return light beam 712 is projected to emanate from a projected return light beam 718 in the measured sample (e.g., the target sample 740). As shown in FIG. 7, the emission light beam 711 and the return light beam 712 are configured such that the projected return light beam 718 intersects in the first dimension with the diverging portion 714b of the projected emission light beam 714. While individual photons may not stay within the projected light pathways, configuring the emission and return light beams 711, 712 in this manner may promote collection of light that has passed through the transition region 716 of the projected emission light beam 714. In the variation shown in FIG. 7, the collection architecture 704 is configured to collect a return light beam 712 that is collimated in the first dimension as it enters the sampling interface 707, however it should be appreciated that the return light beam 712 may alternatively converge or diverge along the first dimension. It should also be appreciated that the optical measurement system 700 may include any components, such as those described herein, that may be needed to generate the emission light beam 711 and collect the return light beam 712. For example, the launch architecture 702 is shown in FIG. 7 as having a beam generator 720 configured to generate an output light beam 722 and a launch optical subassembly 724 that includes one or more lens elements configured to generate the emission light beam 711 from the output light beam 722).

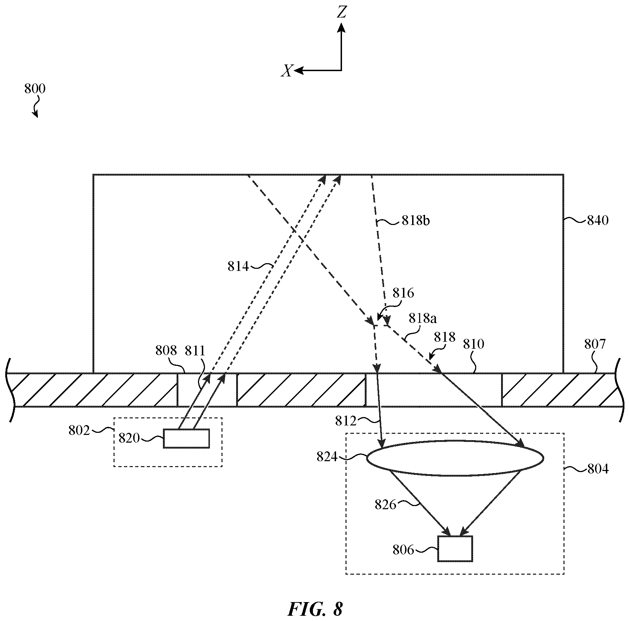

FIG. 8 shows a partial cross-sectional side view of an optical measurement system 800 configured to collect light that is projected to emanate from an angle-independent sample region for a first measurement channel. Specifically, the optical measurement system 800 includes a sampling interface 807 having a launch site 808 and a collection site 810, such as described in more detail herein, launch architecture 802 and collection architecture 804. The launch architecture 802 is configured to generate and emit an emission light beam 811 that exits the sampling interface 807 through the launch site 808. Similarly, the collection architecture 804 includes a detector element 806, and is configured to collect a return light beam 812 that enters the sampling interface 807 through the collection site 810 and to direct the return light beam 812 to the detector element 806.

When the launch architecture 802 emits the emission light beam 811 into a target sample 840 (or another measured sample have a predetermined set of sample characteristics), at least a portion of the emission light beam 811 will be returned to the optical measurement system 800 as part of the return light beam 812. In other words, the detector element 806 measures a portion of the emission light beam 811 that is returned to the optical measurement system 800 from the sample being measured. In this way, the detector element 806 forms a first measurement channel of the optical measurement system 800 when the launch architecture emits the emission light beam 811.

The return light beam 812 is projected to emanate from a projected return light beam 818 in the measured sample (e.g., the target sample 840). The return light beam 812 and the projected return light beam 818 may have any characteristics as described above with respect to the return light beam 504 and the projected return light beam 506 of FIGS. 5A and 5B. Specifically, the return light beam 812 diverges along a first dimension (e.g., along an XZ plane using the cartesian coordinate system depicted in FIG. 8) as it enters the sampling interface 807. In this way, the emission light beam 812 is projected to emanate from a projected return light beam 818 that has a diverging portion 818a along the first dimension as it enters the sampling interface 807 and transitions from a converging portion 818b at a transition region 816. In some instances, such as shown in FIG. 8, the collection architecture 804 may be configured such that the return light beam 812 (and thereby the projected return light beam 818) is angled away from the launch site 808 in the first dimension. This may promote collection of light coming from the direction of the launch site 808, which may increase the collection efficiency of the first measurement channel.

The optical measurement system 800 is further configured such that, when the emission light beam 811 is emitted into a target sample 840, the transition region 816 coincides with an angle-independent sample region for the first measurement channel. In this way, each ray of the light collected as part of the return light beam 812 is projected to have emanated from the angle-independent sample region for the first measurement channel. Accordingly, light measured by the detector element 806 while the launch architecture is emitting the emission light beam 811 (i.e., light measured by the first measurement channel) will have approximately the same path length distribution regardless of where individual photons enter the sampling interface 807 as part of the return light beam 812.

The launch architecture 802 is configured to generate an emission light beam 811, such that a projected emission light beam 814 intersects with the converging portion 818b of the projected return light beam 818. While individual photons may not stay within the projected light pathways, configuring the emission and return light beams 811, 812 in this manner may promote collection of light that has passed through the transition region 816 of the projected return light beam 818. In the variation shown in FIG. 8, the launch architecture 802 is configured to emit an emission light beam 811 that is collimated in the first dimension. It should be appreciated, however, that the emission light beam 812 may alternatively converge or diverge along the first dimension.

It should also be appreciated that the optical measurement system 800 may include any components, such as those described herein, that may be needed to generate the emission light beam 811 and collect the return light beam 812. For example, the launch architecture 802 is shown in FIG. 8 as having a beam generator 820 configured to generate the emission light beam 811. The collection architecture 804 is shown in FIG. 8 as having a collection optical subassembly 824 includes one or more lens elements, and is configured to receive and shape the return light beam 812 to form a measurement light beam 826 that is incident on the detector element 806.

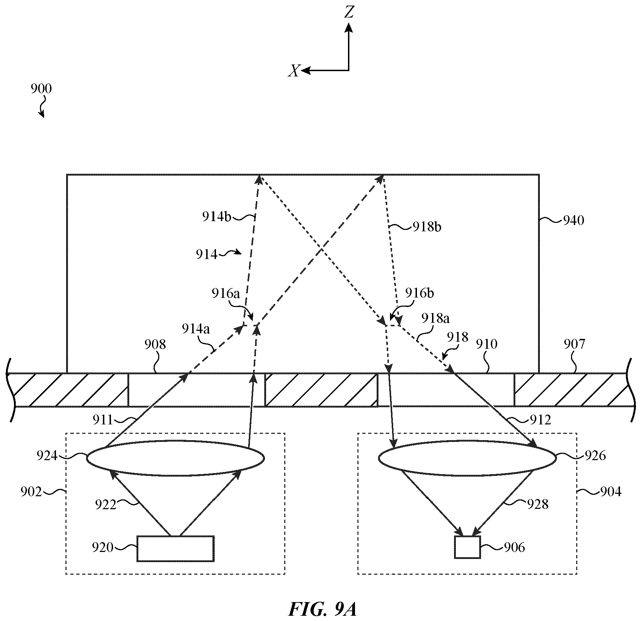

FIG. 9A shows a partial cross-sectional side view of an optical measurement system 900 configured to both i) project light toward a first angle-independent sample region for a first measurement channel, and ii) collect light that is projected to emanate from a second angle-independent sample region for the first measurement channel. Specifically, the optical measurement system 900 includes a sampling interface 907 having a launch site 908 and a collection site 910, such as described in more detail herein, launch architecture 902 and collection architecture 904. The launch architecture 902 is configured to generate and emit an emission light beam 911 that exits the sampling interface 907 through the launch site 908. Similarly, the collection architecture 904 includes a detector element 906, and is configured to collect a return light beam 912 that enters the sampling interface 907 through the collection site 910 and to direct the return light beam 912 to the detector element 906.

When the launch architecture 902 emits the emission light beam 911 into a target sample 940 (or another measured sample have a predetermined set of sample characteristics), at least a portion of the emission light beam 911 will be returned to the optical measurement system 900 as part of the return light beam 912. In other words, the detector element 906 measures a portion of the emission light beam 911 that is returned to the optical measurement system 900 from the sample being measured. In this way, the detector element 906 forms a first measurement channel when the launch architecture 902 emits the emission light beam 911.

The launch architecture 902 is configured such that the emission light beam 911 projects a projected emission light beam 914 into the sample being measured. The emission light beam 911 and the projected emission light beam 914 may have any characteristics as described above with respect to the emission light beam 304 and the projected emission light beam 306 of FIGS. 3A and 3B. Specifically, the emission light beam 911 converges along a first dimension (e.g., along an XZ plane using the cartesian coordinate system depicted in FIG. 9A) as it exits the sampling interface 907. In this way, the emission light beam 911 projects a projected emission light beam 914 that has a converging portion 914a along the first dimension as it exits the sampling interface 907 and transitions to a diverging portion 914b at a first transition region 916a. In some instances, such as shown in FIG. 9A, the launch architecture 902 may be configured such that the emission light beam 911 (and thereby the projected emission light beam 914) is angled toward the collection site 910 along the first dimension. This may preferentially direct light toward the collection site 910, which may increase the collection efficiency of the first measurement channel.

The optical measurement system 900 is further configured such that, when the emission light beam 911 is emitted into a target sample 940, the first transition region 916a coincides with a first angle-independent sample region for the first measurement channel. In this way, each ray of the light from the emission light beam 911 is projected to intersect the angle-independent sample region for the first measurement channel. Accordingly, light measured by the detector element 906 while the launch architecture is emitting the emission light beam 911 (i.e., light measured by the first measurement channel) will have the approximately same path length distribution regardless of where individual photons exit the sampling interface 907.

Similarly, the return light beam 912 is projected to emanate from a projected return light beam 918 in the measured sample (e.g., the target sample 940). The return light beam 912 and the projected return light beam 918 may have any characteristics as described above with respect to the return light beam 504 and the projected return light beam 506 of FIGS. 5A and 5B. Specifically, the return light beam 912 diverges along a first dimension (e.g., along the XZ plane of FIG. 9A) as it enters the sampling interface 907. In this way, the return light beam 912 is projected to emanate from a projected return light beam 918 that has a diverging portion 918a along the first dimension as it enters the sampling interface 907 and transitions from a converging portion 918b at a second transition region 916b. In some instances, such as shown in FIG. 9A, the collection architecture 904 may be configured such that the return light beam 912 (and thereby the projected return light beam 918) is angled away from the launch site 908 in the first dimension. This may promote collection of light coming from the direction of the launch site 908, which may further increase the collection efficiency of the first measurement channel.

The optical measurement system 900 is further configured such that, when the emission light beam 911 is emitted into a target sample 940, the second transition region 916b coincides with a second angle-independent sample region for the first measurement channel. In this way, each ray of the light collected as part of the return light beam 912 is projected to have emanated from the angle-independent sample region for the first measurement channel. Accordingly, light measured by the detector element 906 while the launch architecture is emitting the emission light beam 911 (i.e., light measured by the first measurement channel) will have approximately the same path length distribution regardless of where individual photons enter the sampling interface 907 as part of the return light beam 912.

The launch architecture 902 and collection architecture 904 may be configured such that the diverging portion 914b of the projected emission light beam 914 intersects with the converging portion 914c of the projected return light beam 918. This may help promote collection of light that has passed through both the first transition region 916a and the second transition region 916b within the target sample 940. To generate the emission light beam 911, the launch architecture 902 may include a beam generator 920 and a launch optical subassembly 924 that includes one or more lens elements, which may be configured in any suitable manner such as described herein. The beam generator 920 is configured to generate an output light beam 922 that diverges in at least one dimension (e.g., at least the first dimension), and is received and shaped by the launch optical subassembly 924 to form the emission light beam 911. Similarly, the collection architecture 904 may include a collection optical subassembly 926 that has one or more lens elements. The collection optical subassembly 926 may be configured in any suitable manner such as described herein, and is configured to receive and shape the return light beam 912 to form a first measurement light beam 928 that is incident on the detector element 906.

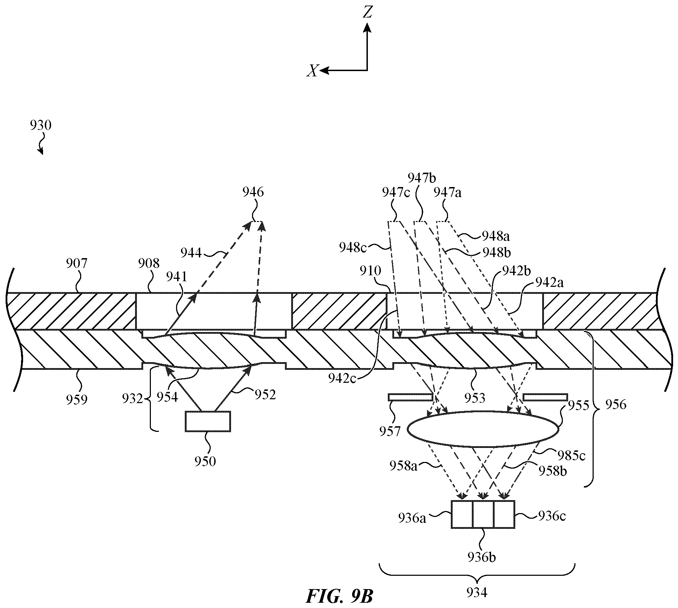

While the optical measurement system 900 shown in FIG. 9A is depicted as including a single detector element 906, in other variations the optical measurement systems described herein may include multiple detector elements that simultaneously form multiple measurement channels. For example, FIG. 9B shows a partial cross-sectional side view of another variation of an optical measurement system 930 as described herein. The optical measurement system 930 is similar to the optical measurement system 900 of FIG. 9A, except that the optical measurement system 930 is configured to both i) project light toward a sample region that is angle independent for a first plurality of measurement channels, and ii) collect light that is projected to emanate from a multiple samples region, each of which is angle independent for a different corresponding measurement channel.

Specifically, the optical measurement system 930 includes a sampling interface 907 having a launch site 908 and a collection site 910, such as described in more detail herein, launch architecture 932 and collection architecture 934. The launch architecture 932 is configured to generate and emit an emission light beam 941 that exits the sampling interface 907 through the launch site 908. Similarly, the collection architecture 934 includes a plurality of detector element 936a-936c, and is configured to collect a plurality of return light beam 942a-942c that enter the sampling interface 907 through the collection site 910 and to direct the return light beams 942a-942c to the detector element 936a-936c. For example, the plurality of detector elements 936a-936c is shown in FIG. 9B as including three detector elements: a first detector element 936a, a second detector element 936b, and a third detector element 936c.

The collection architecture 934 is configured to collect a first return light beam 942a that is directed to and measured by the first detector element 936a, a second return light beam 942b that is directed to and measured by the second detector element 936b, and a third return light beam 942c that is directed to and measured by the third detector element 936c. Each of the plurality of return light beams 942a-942c defines a different set of position and angle pairs on the collection site 910, such that light entering the sampling interface 907 at a given position/angle pair (e.g., at a particular angle of incidence for a particular location) will be routed to a given detector element of the plurality of detector elements 936a-936c. It should be appreciated that different return light beams may overlap on the collection site 910. For example, for light entering the sampling interface at a particular location on the collection site 910 where the first and second return light beams 942a, 942b overlap, photons entering the location with a first angle of incidence may be routed to the first detector element 936a, and photons entering the location with a different second angle of incidence may be routed to the second detector element 936b. It should be appreciated that the plurality of detector elements 936a-936c may alternatively include two or four or more detector elements as may be desired.

When the launch architecture 932 emits the emission light beam 941 into a target sample or another measured sample have a predetermined set of sample characteristics (not shown), at least a portion of the emission light beam 941 will be returned to the optical measurement system 930 as part of each of the plurality of return light beams 942a-942c. In other words, the each of the plurality of detector elements 936a-936c measures a different corresponding portion of the emission light beam 941 that is returned to the optical measurement system 930 from the sample being measured. In this way, each of the plurality of detector element 936a-936c forms a different measurement channel when the launch architecture 932 emits the emission light beam 941 (e.g., the first detector element 936a forms a first measurement channel, the second detector element 936b forms a second measurement channel, and the third detector element 936c forms a third measurement channel).

The launch architecture 932 is configured such that the emission light beam 941 projects a projected emission light beam 944 into the sample being measured. The emission light beam 941 and the projected emission light beam 944 may have any characteristics as described above with respect to the emission light beam 304 and the projected emission light beam 306 of FIGS. 3A and 3B. Specifically, the emission light beam 941 converges along a first dimension (e.g., along an XZ plane using the cartesian coordinate system depicted in FIG. 9B) as it exits the sampling interface 907. In this way, the emission light beam 941 projects a projected emission light beam 944 that has a converging portion along the first dimension as it exits the sampling interface 907 and transitions to a diverging portion (only the converging portion is depicted in FIG. 9B) at an emission transition region 946. In some instances, such as shown in FIG. 9B, the launch architecture 932 may be configured such that the emission light beam 941 (and thereby the projected emission light beam 944) is angled toward the collection site 910 along the first dimension. This may preferentially direct light toward the collection site 910, which may increase the collection efficiency of the first measurement channel.

The optical measurement system 930 is further configured such that, when the emission light beam 941 is emitted into a target sample (not shown) as described herein, the emission transition region 946 coincides with a first sample region that is angle independent for at least one of the plurality of measurement channels. In some instances, the sample region is angle independent for each of the plurality of measurement channels. Specifically, the emission transition region 946 coincides with a first sample region that is angle independent for each of the first, second, and third measurement channels. In this way, light measured by each of the plurality of detector element 936a-936c while the launch architecture is emitting the emission light beam 941 will have approximately the same path length distribution regardless of where individual photons exit the sampling interface 907. It should be appreciated, however, that the path length distribution of light measured by the first detector element 936a may still be different (e.g., have a different shape and/or median path length) than the path length distribution of light measured by the second detector element 936b and/or the third detector element 936c.

In other instances, the emission transition region 916 may coincide with a first sample region that is not angle independent for every measurement channel. For example, the first sample region may be angle independent for the second measurement channel (e.g., light measured by the second detector element 936b), but may not be angle independent for the first and third measurement channels (e.g., light measured by the first and third detector elements 936a, 936c, respectively). In these instances, it may be desirable to lose some path length control in return for increasing the collection efficiency of these angle-dependent measurement channels. This angle dependency may be accounted for when determining a sample property (e.g., information from the angle-dependent measurement channels may be given less weight than angle-independent measurement channels).

The plurality of return light beams 942a-942c is projected to emanate from a plurality of projected return light beams 948a-948c in the measured sample such that each return light beam is projected to emanate from a corresponding projected return light beam. Specifically, the first return light beam 942a is projected to emanate from a first projected return light beam 948a, the second return light beam 942b is projected to emanate from a second projected return light beam 948b, and the third return light beam 942c is projected to emanate from a third projected return light beam 948c. Each of return light beams 942a-942c and its corresponding projected return light beam may have any characteristics as described above with respect to the return light beam 504 and the projected return light beam 506 of FIGS. 5A and 5B.

In the variation shown in FIG. 9B, each of the return light beams 942a-942c diverges along a common first dimension (e.g., along the XZ plane of FIG. 9B) as it enters the sampling interface 907. In this way, each of the plurality of return light beams 942a-942c is projected to emanate from a projected return light beam that has a diverging portion along the first dimension as it enters the sampling interface 907 and transitions from a converging portion (only the diverging portions of the projected return light beams 948a-948c are shown in FIG. 9B) at a corresponding collection transition region. Accordingly, the plurality of return light beams 942a-942c is associated with a plurality of collection transition regions 947a-947c (e.g., the first projected return light beam 948a has a first collection transition region 947a, the second projected return light beam 948b has a second collection transition region 947b, and the third projected return light beam 948c has a third collection transition region 947c).

The optical measurement system 930 is further configured such that, when the emission light beam 941 is emitted into a target sample, the plurality of collection transition region 947a-947c coincides with a plurality of sample regions that are angle independent for the respective measurement channels of the plurality of measurement channels. Specifically, the first collection transition region 947a coincides with a second sample region that is angle independent for the first measurement channel (e.g., light measured by the first detector element 936a), the second collection transition region 947b coincides with a third sample region that is angle independent for the second measurement channel (e.g., light measured by the second detector element 936b), and the third collection transition region 947c coincides with a fourth sample region that is angle independent for the third measurement channel (e.g., light measured by the third detector element 936c). In this way, each ray of the light collected as part of a return light beam for a given measurement channel is projected to have emanated from a sample region that is angle independent for that measurement channel. Accordingly, each detector element of the plurality of detector elements 936a-936c may increase the amount of light collected it collects while maintaining the path length control as discussed herein.

Although not shown in FIG. 9B, it should be appreciated that the launch architecture 932 and collection architecture 934 may be configured such that the diverging portion of the projected emission light beam 914 intersects with the converging portions of each of the projected return light beams 948a-948c. This may, for each of measurement channel, help promote collection of light that has passed through both the launch transition region 936 and a corresponding one of the collection transition regions 947a-947c within a measured sample.

In the variation shown in FIG. 9B, the collection architecture 934 includes a collection optical subassembly 956. The collection optical subassembly 956 includes one or more lens elements and is configured to receive and shape the plurality of return light beams 942a-942c to form a plurality of measurement light beams 958a-958c that are incident on the plurality of detector elements 936a-936c. Specifically, the collection optical subassembly 956 shapes the first return light beam 942a into a first measurement light beam 958a that is measured by the first detector element 936a, shapes the second return light beam 942b into a second measurement light beam 958b that is measured by the second detector element 936b, and shapes the third return light beam 942c into a third measurement light beam 958c that is measured by the third detector element 936c.

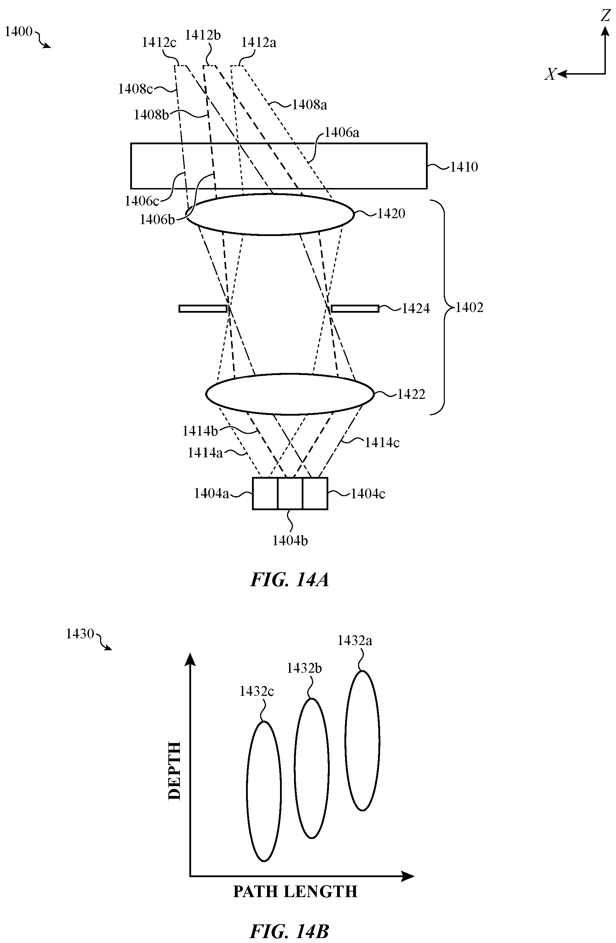

The collection optical subassembly 956 may be configured in any suitable manner that defines the return light beams 942a-942c and routes each of these return light beams 942a-942c to a corresponding detector element of the plurality of detector elements 936a-936c. In the variation shown in FIG. 9B, the collection optical subassembly 956 includes an imaging lens 953 and a relay lens 955 separated by an aperture 957. In some variations, the imaging lens 953 and the relay lens 955 are each configured as cylinder lenses, which may allow for the profile of the return light beams 942a-942c to be extended in a second dimension perpendicular to the first dimension. Examples of collection optical subassemblies suitable for use with the optical measurement system 930 of FIG. 9B are described in more detail herein with respect to FIGS. 14A-17B.

To generate the emission light beam 941, the launch architecture 932 may include a beam generator 950 and a launch optical subassembly 954 that includes one or more lens elements (shown in FIG. 9B as a single cylinder lens), which may be configured in any suitable manner such as described herein. The beam generator 950 is configured to generate an output light beam 952 that diverges in at least one dimension (e.g., at least the first dimension), and is received and shaped by the launch optical subassembly 954 to form the emission light beam 941. In instances, one or more lenses of the launch optical subassembly may be formed as part of one or more common substrates with one or more lens of the collection optical subassembly 956. For example, in the variation shown in FIG. 9B, the lens of the launch optical subassembly 954 and the imaging lens 953 of the collection optical subassembly 956 are formed as part of a common substrate 959. For example, a first portion of the substrate 959 may be etched to define the lens of the launch optical subassembly 954 and a second portion of the substrate 959 may be etched to define the imaging lens 953. Forming imaging lens 953 and the lens of the launch optical subassembly 954 from different portions of a common substrate 959 may allow for precise relative positioning between these lenses within the optical measurement system 930.

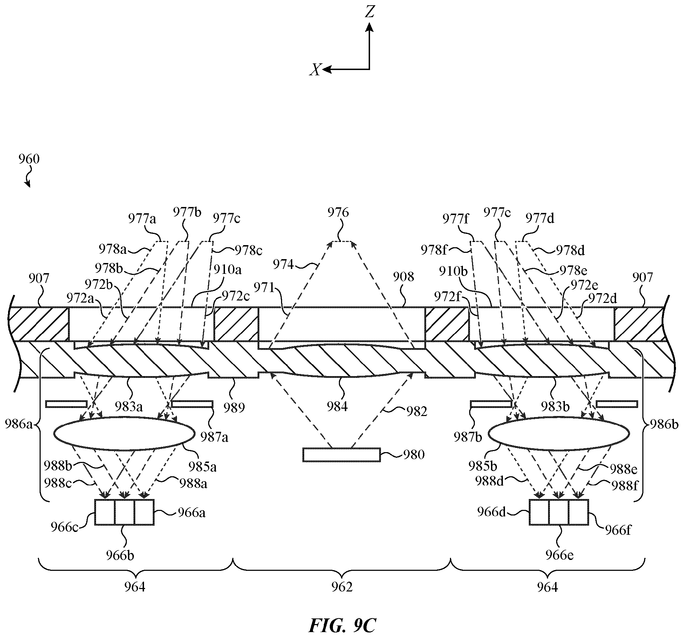

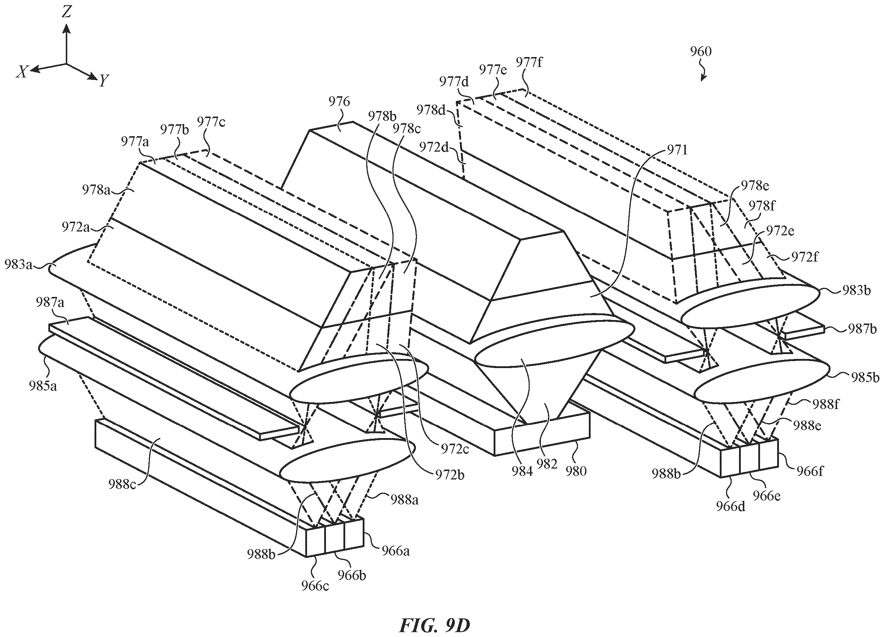

The optical measurement system 930 is configured such that light returned from an emission light beam 941 is collected by a single collection site 910, but in other variations an optical measurement system as described herein may be configured to use multiple collection sites to measure light returned from a single emission light beam. For example, FIGS. 9C and 9D show partial cross-sectional side and perspective views, respectively, of another variation of an optical measurement system 960. The optical measurement system 960 is similar to the optical measurement system 930 of FIG. 9B, except that the optical measurement system 960 is configured to project an emission light beam and collect different portions of return light using multiple collection sites. Each collection site may be associated with a different set of measurement channels. In some variations, the optical measurement system 960 may be configured to project light toward a sample region that is angle independent for both i) one or more measurement channels associated with a first collection site and ii) one or more measurement channels associated with a second collection site. Additionally or alternatively, the optical measurement system 960 is configured to collect light that is projected to emanate from multiple samples regions, each of which is angle independent for a different corresponding measurement channel.

Specifically, the optical measurement system 960 includes a launch architecture 962, collection architecture 964, and a sampling interface 907 having a launch site 908, a first collection site 910a, and a second collection site 910b, such as described in more detail herein. In the variation shown in FIGS. 9C and 9D, the launch site 908 is positioned between the first collection site 910a and the second collection site 910b. The launch architecture 962 is configured to generate and emit an emission light beam 971 that exits the sampling interface 907 through the launch site 908. Similarly, the collection architecture 964 includes a first set of detector elements 966a-966c associated with the first collection site 910a and a second set of detector elements 966d-966f associated with the second collection site 910b. The collection architecture 964 is configured to collect a first set of return light beams 972a-972c that enter the sampling interface through the first collection site 910a, and is configured to route these return light beams 972a-972c to the first plurality of detector elements 966a-966c. Similarly, the collection architecture 964 is configured to collect a second set of return light beam 972d-972f that enter the sampling interface 907 through the second collection site 910b, and is configured to direct these return light beams 972d-972f to the second set of detector element 966d-966f.

The first set of detector elements 966a-966c is shown in FIGS. 9C and 9D as having the same number of detector elements (e.g., three) as the second set of detector elements 966d-966f. This may allow the launch architecture 962 and the collection architecture 964 to have a symmetric configuration, such as described in more detail herein, which allow for improved light collection at different path length distributions. In other instances, the first set of detector elements 966a-966c may have a different number of detector elements than the second set of detector elements 966d-966f. In the example shown in FIGS. 9C and 9D, the first set of detector elements 966a-966c includes a first plurality of detector elements (e.g., a first detector element 966a, a second detector element 966b, and a third detector element 966c, which may be arranged such that the second detector element 966b is positioned between the first and third detector elements 966a, 966c along the first dimension). The second set of detector elements 966d-966f includes a second plurality of detector elements (e.g., a fourth detector element 966d, a fifth detector element 966e, and a sixth detector element 966f, which may be arranged such that the fifth detector element 966e is positioned between the fourth and sixth detector elements 966d, 966f along the first dimension).

In these instances, the collection architecture is configured to collect a first plurality of return light beams 972a-972c (e.g., a first return light beam 972a, a second return light beam 972b, and a third return light beam 972c) through the first collection site 910a. Each of the first plurality of return light beams 972a-972c defines a different set of position and angle pairs on the first collection site 910a, such that light entering the sampling interface 907 at a given position/angle pair (e.g., at a particular angle of incidence for a particular location) will be routed to a given detector element of the first plurality of detector elements 966a-966b, such as described in more detail herein. Similarly, the collection architecture is configured to collect a second plurality of return light beams 972d-972f (e.g., a fourth return light beam 972d, a fifth return light beam 972e, and a sixth return light beam 972f) through the second collection site 910b. Each of the second plurality of return light beams 972d-972f defines a different set of position and angle pairs on the second collection site 910b, such that light entering the sampling interface 907 at a given position/angle pair (e.g., at a particular angle of incidence for a particular location) on the second collection site 910b will be routed to a given detector element of the second plurality of detector elements 966d-966f. Overall, light collected by the first return light beam 972a is measured by the first detector element 966a, light collected by the second return light beam 972b is measured by the second detector element 966b, and so on.

When the launch architecture 962 emits the emission light beam 971 into a target sample or another measured sample have a predetermined set of sample characteristics (not shown), a first portion of the emission light beam 941 will be returned to the optical measurement system 960 via the first collection site 910a as part of the first set of return light beams 972a-972c. A second portion of the emission light beam 941 will be concurrently returned to the optical measurement system 960 via the second collection site 910b as part of the second set of return light beams 972d-972f. In other words, the each of the first and second sets of detector elements 976a-976c, 976d-976f measures a different corresponding portion of the emission light beam 971 that is returned to the optical measurement system 960 from the sample being measured. In this way, each of the first and second sets of detector elements 976a-976c, 976d-976f forms a different measurement channel when the launch architecture 962 emits the emission light beam 971. For example, the optical measurement system 970 shown in FIGS. 9C and 9D includes a plurality of measurement channels (e.g., six measurement channels) when the launch architecture 962 emits the emission light beam 971, where three measurement channels concurrently measure light received through the first collection site 910a and three measurement channels concurrently measure light received through the second collection site 910b. Specifically, the first detector element 966a forms a first measurement channel, the second detector element 966b forms a second measurement channel, and so on.

The launch architecture 962 is configured such that the emission light beam 971 projects a projected emission light beam 974 into the sample being measured. The emission light beam 971 and the projected emission light beam 974 may have any characteristics as described above with respect to the emission light beam 304 and the projected emission light beam 306 of FIGS. 3A and 3B. Specifically, the emission light beam 971 converges along a first dimension (e.g., along an XZ plane using the cartesian coordinate system depicted in FIGS. 9C and 9D) as it exits the sampling interface 907. In this way, the emission light beam 971 projects a projected emission light beam 974 that has a converging portion along the first dimension as it exits the sampling interface 907 and transitions to a diverging portion (only the converging portion is depicted in FIGS. 9C and 9D) at an emission transition region 976. In the variation shown in FIGS. 9C and 9D, the projected emission light beam 974 exits the sampling interface 907 at normal incidence, which may result in the same amount of light being directed toward each of the first and second collection sites 910a, 910b. In other instances the launch architecture 962 may be configured such that the emission light beam 971 (and thereby the projected emission light beam 974) is angled toward one of the collection sites along the first dimension. For example, if the emission light beam 971 is angled toward the first collection site 910a, light may be preferentially direct toward the first collection site 910a, which may prioritize collection of light by the first collection site 910a over collection of light by the second collection site 910b.

The optical measurement system 960 is further configured such that, when the emission light beam 971 is emitted into a target sample (not shown) as described herein, the emission transition region 976 coincides with a first sample region that is angle independent for at least one of the plurality of measurement channels. In some instances, the sample region is angle independent for at least one measurement channel associated with the first collection site 910a and is also angle independent for at least one measurement channel associated with the second collection site 910b. In some of these instances, the sample region is angle independent for each of the plurality of measurement channels (e.g., the six measurement channels associated with the first and second plurality of detector elements 966a-966c, 966d-966f). In this way, light measured by each of the plurality of the first and second plurality of detector element 966a-966c, 966d-966f while the launch architecture is emitting the emission light beam 941 will have approximately the same path length distribution regardless of where individual photons exit the sampling interface 907.

In other instances, the emission transition region 976 may coincide with a first sample region that is not angle independent for every measurement channel associated with the first and second sets of detector elements 966a-966c, 966d-966f. For example, the first sample region may be angle independent for the second measurement channel (e.g., light measured by the second detector element 966b), but may not be angle independent for the first and third measurement channels (e.g., light measured by the first and third detector elements 966a, 966c, respectively). Similarly, the first sample region may be angle independent for the fifth measurement channel (e.g., light measured by the fifth detector element 966c), but may not be angle independent for the fourth and sixth measurement channels (e.g., light measured by the fourth and sixth detector elements 966d, 966f, respectively). This angle dependency may be accounted for when determining a sample property, such as described herein.

In some instances, the optical measurement system 960 is configured such some or all of the first set of detector elements 966a-966c are configured to measure a path length distribution of light that is approximately the same as the path length distribution of light measured by a corresponding detector element of the second set of detector elements 966d-966f. For example, in one example the first detector element 966a and the fourth detector element 966d (and thereby the first and fourth measurement channels) are configured to measure light with approximately the same path length distribution (e.g., a first path length distribution). Additionally or alternatively, the second detector element 966b and the fifth detector element 966e (and thereby the second and fifth measurement channels) may be configured to measure light with approximately the same path length distribution (e.g., a second path length distribution, which may be different than the first path length distribution). Similarly, the third detector element 966c and the sixth detector element 966f (and thereby the third and sixth measurement channels) may be configured to measure light with approximately the same path length distribution (e.g., a third path length distribution, which may be different than the first and second path length distributions).

In these instances, measurement channels that are configured to measure light of approximately the same path length distribution may be combined, which may allow the outputs of multiple detector elements to be treated as a single signal for the purpose of determining one or more sample properties as described herein. For example, the output signals of the first and fourth measurement channels may be combined to form a first combined output signal, the output signals of the second and fifth measurement channels may be combined to form a second combined output signal, and the output signals of the third and sixth measurement channels may be combined to form a third combined output signal. Overall, this may increase the amount of light that is collected (and across a larger sampling volume) for a given path length distribution (or set of path length distributions), which may improve the accuracy of spectroscopic measurements performed by the optical measurement system 970.

With regard to the collection architecture 964, the first set of return light beams 972a-972c is projected to emanate from a first set of projected return light beams 978a-978c and the second set of return light beams 972d-972f is projected to emanate from a second set of projected return light beams 978d-978f. Specifically, the first return light beam 972a is projected to emanate from a first projected return light beam 978a, the second return light beam 972b is projected to emanate from a second projected return light beam 978b, the third return light beam 972c is projected to emanate from a third projected return light beam 978c, the fourth return light beam 972d is projected to emanate from a fourth projected return light beam 978d, the fifth return light beam 972e is projected to emanate from a fifth projected return light beam 978e, and the sixth return light beam 972f is projected to emanate from a sixth projected return light beam 978f. Each of return light beams 972a-972c and its corresponding projected return light beam may have any characteristics as described above with respect to the return light beam 504 and the projected return light beam 506 of FIGS. 5A and 5B.

In the variation shown in FIGS. 9C and 9D, each of the first and second sets of return light beams 972a-972c, 972d-972f diverges along a common first dimension (e.g., along the XZ plane of FIG. 9C) as it enters the sampling interface 907. In this way, each of the first and second sets of return light beams 972a-972c, 972d-972f is projected to emanate from a projected return light beam that has a diverging portion along the first dimension as it enters the sampling interface 907 and transitions from a converging portion (only the diverging portions of the return light beams 942a-942c are shown in FIGS. 9C and 9D) at a corresponding collection transition region. Accordingly, the first and second sets of return light beams 972a-972c, 972d-972f are associated with corresponding first and second sets of collection transition regions 977a-997c, 997d-977f.

In the example shown in FIGS. 9C and 9D, in which the first set of return light beams 972a-972c includes a first plurality of return light beams 972a-972c, the first plurality of return light beams 972a-972c is associated with a first plurality of collection transition regions 977a-977c (e.g., the first projected return light beam 978a has a first collection transition region 977a, the second projected return light beam 978b has a second collection transition region 977b, and the third projected return light beam 978c has a third collection transition region 977c). Similarly, when the second set of return light beams 972d-972f includes a second plurality of return light beams 972d-972f, the second plurality of return light beams 972d-972f is associated with a second plurality of collection transition regions 977d-977f (e.g., the fourth projected return light beam 978d has a fourth collection transition region 977d, the fifth projected return light beam 978e has a fifth collection transition region 977e, and the sixth projected return light beam 978f has a sixth collection transition region 977f.

The optical measurement system 930 may be further configured such that, when the emission light beam 971 is emitted into a target sample, the first and second sets of collection transition region 977a-977c, 977d-977f coincide with a plurality of sample regions that are angle independent for the respective measurement channels of the plurality of measurement channels. In the example in which the optical measurement system 970 includes six concurrent measurement channels, the six collection transition regions 977a-977f may coincide with six angle-independent sample regions. Specifically, the first collection transition region 977a coincides with a second sample region that is angle independent for the first measurement channel (e.g., light measured by the first detector element 967a), the second collection transition region 977b coincides with a third sample region that is angle independent for the second measurement channel (e.g., light measured by the second detector element 966b), the third collection transition region 977c coincides with a fourth sample region that is angle independent for the third measurement channel (e.g., light measured by the third detector element 966c), and so on. In this way, each ray of the light collected as part of a return light beam for a given measurement channel is projected to have emanated from a sample region that is angle independent for that measurement channel. Accordingly, each detector element of the first and second sets of detector elements 976a-976c, 976d-976f may increase the amount of light collected it collects while maintaining the path length control as discussed herein.

Although not shown in FIGS. 9C and 9D, it should be appreciated that the launch architecture 962 and collection architecture 964 may be configured such that the diverging portion of the projected emission light beam 974 intersects with the converging portions of each of the first and second sets of projected return light beams 978a-978c, 978d-978f. This may, for each measurement channel, help promote collection of light that has passed through both the launch transition region 976 and a corresponding one of the collection transition regions 977a-977f within a measured sample.

To collect light from the first and second collection sites 910a, 910b, the collection architecture 964 includes a first collection optical subassembly 986a and a second collection optical subassembly 986b, each of which includes one or more lens elements. The first collection optical subassembly 986a is configured to the receive and shape the first set of return light beams 972a-972c to form a first set of measurement light beams 988a-988c that is incident on the first set of detector elements 966a-966c. Similarly, the second collection optical subassembly 986b is configured to the receive and shape the second set of return light beams 972d-972f to form a second set of measurement light beams 988d-988f that is incident on the first set of detector elements 966d-966f.

The first and second collection optical subassemblies 986a, 986b may be configured in any suitable manner such as described herein. As shown in FIGS. 9C and 9D, the first collection optical subassembly 986a may include a first imaging lens 983a and a first relay lens 985a separated by a first aperture 957a, and the second collection optical subassembly 986b may include a second imaging lens 983b and a second relay lens 985b separated by a second aperture 957b. In some variations, the first and second imaging lenses 953a, 953b and the first and second relay lenses 955a, 955b are each configured as cylinder lenses, which may allow for the profile of the first and second sets of return light beams 972a-972c, 972d-972f to be extended in a second dimension perpendicular to the first dimension (e.g., along the Y axis in the cartesian coordinate system shown in FIG. 9D). Examples of collection optical subassemblies suitable for use with the optical measurement system 970 of FIGS. 9C and 9D are described in more detail herein with respect to FIGS. 14A-17B.

To generate the emission light beam 971, the launch architecture 962 may include a beam generator 980 and a launch optical subassembly 984 that includes one or more lens elements (shown in FIGS. 9C and 9D as a single cylinder lens), which may be configured in any suitable manner such as described herein. The beam generator 980 is configured to generate an output light beam 982 that diverges in at least one dimension (e.g., at least the first dimension), and is received and shaped by the launch optical subassembly 984 to form the emission light beam 971. In instances, one or more lenses of the launch optical subassembly may be formed as part of one or more common substrates with one or more lens of the first and/or second collection optical subassembly 986a, 986b. For example, in the variation shown in FIG. 9C, the lens of the launch optical subassembly 984, the first imaging lens 983a of the first collection optical subassembly 986a, and the second imaging lens 983b of the second collection optical subassembly 986b are formed as part of a common substrate 989 (the portions of the substrate 989 connecting these lenses are not shown in FIG. 9D for ease of illustration). For example, a first portion of the substrate 989 may be etched to define the lens of the launch optical subassembly 984, a second portion of the substrate 989 may be etched to define the first imaging lens 983a, and a third portion of the substrate 989 may be etched to define the second imaging lens 983b. Forming these lens from different portions of a common substrate 989 may allow for precise relative positioning between these lenses within the optical measurement system 970.

### Launch Architectures for use in Optical Measurement Systems

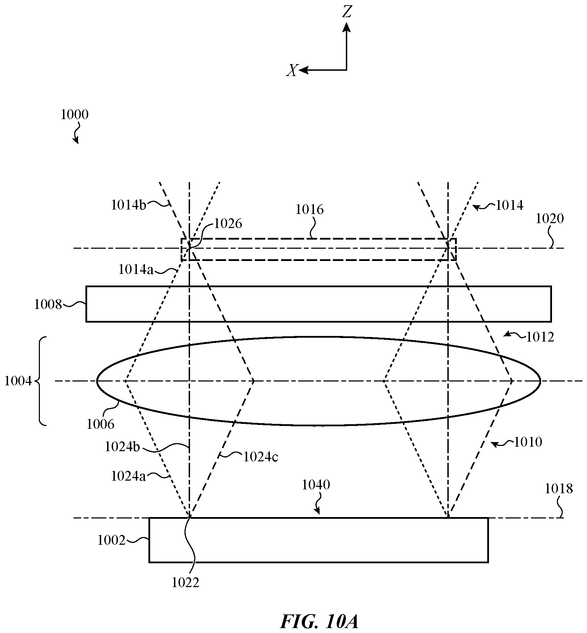

The optical measurement systems described herein may use a range of possible launch architectures to generate an emission light beam. Generally, such as described with respect to FIGS. 3A, 3B and 7-9D, a launch architecture may include a beam generator that is configured to generate an output beam, and a launch optical subassembly that is configured to receive and shape the output beam to form the emission light beam. Examples of launch architectures are described herein with respect to FIGS. 10A-13F. It should be appreciated that these launch architectures may be utilized as part of the optical measurement systems described herein with respect to FIGS. 1A-9D (e.g., may be used to generate any of the output light beams and the emission light beams described with respect to these figures), or may be utilized as part of any other optical measurement system in which it is desirable to emit an emission light beam as described herein.

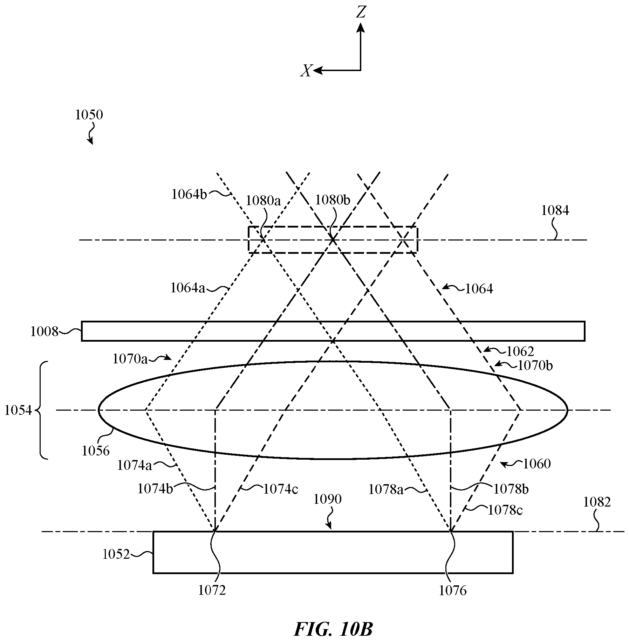

FIGS. 10A and 10B show side views of two variations of launch architectures that are configured to generate an emission light beam that converges in at least one dimension. FIG. 10A shows a first variation of a launch architecture 1000 having a beam generator 1002 and an imaging launch optical subassembly 1004. The imaging launch optical subassembly 1004 is shown schematically in FIG. 10A as including a single lens 1006, though it should be appreciated that other variations of the imaging launch optical subassembly 1004 may include multiple lens elements. The lens 1006, as well as any of the additional lenses of the imaging launch optical subassembly 1004 may be configured as a one-dimensional lens such as a cylinder lens.

The beam generator 1002 is configured to generate an output light beam 1010 that diverges in a first dimension (e.g., along an XZ plane using the cartesian coordinate system depicted in FIG. 10A), such as described in more detail herein. The imaging launch optical subassembly 1004 is configured to receive the output light beam 1010 and shape the output light beam 1010 to generate an emission light beam 1012 that converges in the first dimension. When the launch architecture 1000 is incorporated into an optical measurement system having a sampling interface (e.g., a launch site 1008 is shown in FIG. 10A), the emission light beam 1012 may project a projected emission light beam 1014 to a transition region 1016 in a measured sample that is positioned in contact with the launch site 1008. As discussed in more detail herein, the transition region 1016 represents the point at which a converging portion 1014a (e.g., converging in the first dimension) of the projected emission light beam 1014 exiting the launch site 1008 changes to a diverging portion 1014b (e.g., diverging in the first dimension) of the projected emission light beam 1014.

To project the projected emission light beam 1014 to the transition region 1016, the imaging launch optical subassembly 1004 is configured to image, in the first dimension, an emission surface 1040 of the beam generator 1002 to the transition region 1016. Specifically, if an emission surface 1040 of the beam generator (e.g., a surface of a diffuser forming the output light beam 1010, a surface of the photonic integrated circuit forming the output light beam 1010, or the like) is positioned at a focal plane 1018 of the launch optical subassembly 1004, light emitted by this emission surface will be focused at an image plane 1020 that defines the transition region 1016. For example, rays of the output light beam 1010 emanating from a first point 1022 on the beam generator 1002 along the first dimension (e.g., three rays 1024a-1024c are shown in FIG. 10A for sake of illustration) will be projected to a corresponding second point 1026 in the image plane 1020 (and will thereby be projected to the same second point 1026 in the transition region 1016).

In these instances, the beam width of the emission light beam 1012 in the first dimension, and thereby the corresponding beam width of the projected emission light beam 1014 (e.g., at the transition region 1016) in the first dimension, will depend on the beam width of the output light beam 1010 in the first dimension. In other words, increasing the beam width of the output light beam 1010 in the first dimension will increase the corresponding beam widths of the first emission light beam 1012 and the projected emission light beam 1014. The beam vergence of the emission light beam 1012 in the first dimension, and thereby the corresponding beam vergence of the projected emission light beam 1014 (e.g., at the transition region 1016), will depend on the beam vergence of output light beam 1010. In this way, increasing the divergence of the output light beam 1010 will increase the beam vergence at which the emission light beam converges.

FIG. 10B shows a second variation of a launch architecture 1050 having a beam generator 1052 and a collimating launch optical subassembly 1054. The collimating launch optical subassembly 1054 is shown schematically in FIG. 10B as including a single lens 1056, though it should be appreciated that other variations of the collimating launch optical subassembly 1054 may include multiple lens elements. The lens 1056, as well as any of the additional lenses of the collimating launch optical subassembly 1054 may be configured as a one-dimensional lens such as a cylinder lens. In general, the collimating launch optical subassembly 1054 has a focal length, and will collimate light that is received from a distance corresponding to the focal length.

The beam generator 1052 is configured to generate an output light beam 1060 that diverges in a first dimension (e.g., along an XZ plane using the cartesian coordinate system depicted in FIG. 10B), such as described in more detail herein. The collimating launch optical subassembly 1054 is configured to receive the output light beam 1060 and shape the output light beam 1060 to generate an emission light beam 1062 that converges in the first dimension. When the launch architecture 1050 is incorporated into an optical measurement system having a sampling interface (e.g., a launch site 1008 is shown in FIG. 10B), the emission light beam 1062 may project a projected emission light beam 1064 to a transition region 1066 in a measured sample that is positioned in contact with the launch site 1008. As discussed in more detail herein, the transition region 1066 represents the point at which a converging portion 1064a (e.g., converging in the first dimension) of the projected emission light beam 1064 exiting the launch site 1008 changes to a diverging portion 1064b (e.g., diverging in the first dimension) of the projected emission light beam 1064.

To project the projected emission light beam 1064 to the transition region 1066, the collimating launch optical subassembly 1054 is configured to collimate, in the first dimension, light exiting an emission surface 1090 of the beam generator 1002 to the transition region 1016. Specifically, the collimating launch optical subassembly 1054 will generate a plurality of collimated ray bundles 1070a-1070b that collectively form the projected emission light beam 1064. Specifically, the plurality of collimated ray bundles 1070a-1070b are projected to intersect at the transition region 1066 of the projected emitted light beam 1064. In the variation shown in FIG. 10B, the plurality of ray bundles 1070a-1070b are each parallel ray bundles such that the individual rays within each bundle are parallel.

The individual rays of each collimated ray bundle emanate from a different a point on the emission surface 1090 of the beam generator 1052 (e.g., a surface of a diffuser forming the output light beam 1010, a surface of the photonic integrated circuit forming the output light beam 1010, or the like). Rays emitted from a given point on the emission surface 1090 are at least partially collimated in the first dimension by the collimating launch optical subassembly 1054 to form a corresponding ray bundle. For example, two ray bundles - a first ray bundle 1070a and a second ray bundle 1070b-are shown in FIG. 10B. The first ray bundle 1070a includes a light emanating from a first point 1072 on an emission surface 1090 of the beam generator 1052 (represented by light rays 1074a-1074c). The light rays 1074a-1074c exit the first point 1072 at multiple different angles, but are collimated by the collimating launch optical subassembly 1054 to form the first ray bundle 1070a. Similarly, the second ray bundle 1070b includes a light emanating from a second point 1076 on the emission surface 1090 of the beam generator 1052 (represented by light rays 1078a-1078c). The light rays 1078a-1078c exit the second point 1076 at multiple different angles, but are collimated by the collimating launch optical subassembly 1054 to form the second ray bundle 1070b.

While individual photons may not follow the trajectory of the projected emitted light beam 1064, each of ray bundles 1070a-1070b is projected to reach the transition region 1066 at a different angle of incidence such that the ray bundles 1070a-1070b are projected to cross at the transition region 1066. Specifically, each point in the transition region 1066 receives a ray from each of the plurality of ray bundles 1070a-1070b that corresponds to a different emission angle from the surface of the beam generator 1052. For example, a first point 1080a in the transition region 1066 receives all rays from the output light beam 1060 that exit the emission surface 1090 of the beam generator 1052 at a first angle (e.g., ray 1074a emanating from the first point 1072 of the emission surface 1090 at the first angle and ray 1078a emanating from the second point 1076 of the emission surface 1090 at the first angle). Similarly, a second point 1080b in the transition region 1066 receives all rays from the output light beam 1060 that exit the emission surface 1090 of the beam generator 1052 at a second angle (e.g., ray 1074b emanating from the first point 1072 of the emission surface 1090 at the second angle and ray 1078b emanating from the second point 1076 of the emission surface 1090 at the second angle).

In this way, the beam width of the emission light beam 1062 in the first dimension, and thereby the corresponding beam width of the projected emission light beam 1064 (e.g., at the transition region 1066) in the first dimension, will depend on the beam vergence of the output light beam 1060 in the first dimension. In other words, increasing the beam vergence of the output light beam 1060 in the first dimension will increase the beam width of the projected emission light beam 1064 at the transition region 1066. Conversely, the beam vergence of emission light beam 1062 in the first dimension, and thereby the corresponding beam vergence of the projected emission light beam 1064 (e.g., at the transition region 1066) in the first dimension, will depend on the beam width of output light beam 1010 in the first dimension. In this way, increasing the size of the output light beam 1010 in the first dimension will increase the beam vergence at which the emission light beam 1062 converges in that dimension. Overall, this may improve the wavelength-dependency associated with the beam size of the emission light beam 1062, such as described herein in more detail.

The emission surface 1090 of the beam generator 1052 may be placed at a first distance (represented by line 1082) from the collimating launch optical subassembly 1054, which will result in the transition region 1066 being positioned at a second distance (represented by line 1084) from the collimating launch optical subassembly 1054 when the emission light beam 1062 is emitted into a target sample as described herein. The relative placement of the emission surface 1090 relative to the collimating launch optical subassembly 1054, as well as the focal length of the collimating launch optical subassembly 1054, may be adjusted to change one or more parameters of the emission light beam 1062.

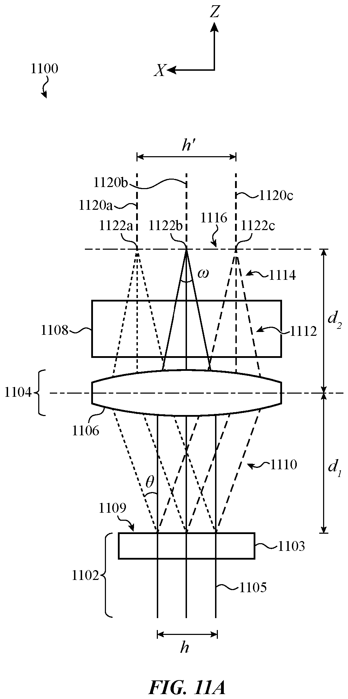

For example, FIG. 11A shows a variation of a launch architecture 1100 having a beam generator 1102 and a collimating launch optical subassembly 1104. The collimating launch optical subassembly 1104 is shown schematically in FIG. 11A as including a single lens 1106, but it should be appreciated that the collimating launch optical subassembly 1104 may be configured in any manner such as described with respect to the launch architecture 1050 of FIG. 10B. In the variation shown in FIG. 11A, the beam generator 1102 includes a diffuser 1103. The diffuser 1103 is positioned to receive an input light beam 1105 (which may be generated and shaped by other portions of the beam generator 1102 not shown in FIG. 11A). When the input light beam 1105 is incident on a rear surface of the diffuser 1103, the diffuser 1103 will generate an output light beam 1110 that diverges in at least one dimension (e.g., a first dimension along the XZ plane using the cartesian coordinate system depicted in FIG. 11A). Accordingly, a front surface of the diffuser 1103 acts an emission surface 1109 for the beam generator 1102, from which the output light beam 1110 is emitted.

The collimating launch optical subassembly 1104 is positioned to receive the output light beam 1110 and shape the output light beam 1110 to form an emission light beam 1112. Specifically, the emission light beam 1112 includes a plurality of ray bundles that are projected (e.g., via a projected emission light beam 1114 that is projected from a launch site 1108 of the optical measurement systems described herein) to cross at a transition region 1116, such as described in more detail with respect to the launch architecture 1050 of FIG. 10B. In the variation shown in FIG. 11A, the emission surface 1109 of the beam generator 1102 is positioned at a telecentric position relative to the collimating launch optical subassembly 1104 (e.g., the emission surface 1109 is placed at a distance d₁ corresponding to the focal length of the collimating launch optical subassembly 1104). In this instance, the emission light beam 1112 is formed from a plurality of parallel ray bundles that are projected to cross at the transition region 1116.

In some variations, the beam generator 1002 may be configured such the angle content of the output light beam 1110 is constant across the output light beam 1110 in the first dimension. In other words, at each point of the emission surface 1109, the output light beam 1110 includes a range of angles in the first dimension that are centered around a center ray (e.g., an angle range of ± 15 degrees relative to the center ray). When the center rays are all parallel, such as shown in FIG. 11A, the output light beam 1110 may have the same angle content across the emission surface 1109. For example, if the input light beam 1105 is collimated along the first dimension, each ray incident on the diffuser 1103 will generate, at a corresponding location of the emission surface 1109, a set of rays having a common range of emission angles, in the first dimension, around a corresponding center ray. Collectively, the center rays are parallel across the emission surface 1109, thereby providing an output light beam 1110 that has angle content that is spatially independent.

In these instances, the transition region 1116 will be positioned at a second distance d₂ (when accounting for any changes in refractive index between the launch site 1108 and the measured sample) from the collimating launch optical subassembly 1104, and this second distance d₂ corresponds to the focal length of the collimating launch optical subassembly 1104.

Conversely, if the input light beam 1105 is converging or diverging in a first dimension when it reaches the diffuser 1103, there will be a position-dependent range of emission angles on the emission surface 1109 of the beam generator 1102 in the first dimension. This may alter the distance at which the transition region 1116 is positioned within a measured sample (e.g., a target sample as described herein). For example, if the input light beam 1105 is converging in the first dimension when it reaches the diffuser 1103, the angle emitted by the peripheral portions of the output light beam 1110 may be biased, in the first dimension, toward the center of the output light beam 1110. In other words, the center rays at the emission surface 1109 may be angled, in the first dimension, toward a center of the output light beam 1110. The center rays may have progressively steeper angles (e.g., higher deviation from normal incidence) in a direction from the center of the output light beam 1110 to the periphery of the output light beam 1110 along the first dimension.

In these instances, the transition region 1116 will be positioned at a second distance d₂ (when accounting for any changes in refractive index between the launch site 1108 and the measured sample) from the collimating launch optical subassembly 1104 that is less than the focal length of the collimating launch optical subassembly 1104. Accordingly, reducing the focal length of the collimating launch optical subassembly 1104 and/or increasing the converge of the input light beam 1105 may bring the transition region 1116 closer to the launch site 1108, which may be useful in positioning the transition region 1116 at a particular location in a target sample (e.g., at a sample region that is angle independent for one or more measurement channels, such as described herein). In other instances, the input light beam 1105 may be diverging, in which case the transition region 1116 will be positioned at a second distance d₂ that is greater than the focal length of the collimating launch optical subassembly 1104

In the variation shown in FIG. 11A, the beam width of the output light beam 1110 in the first dimension depends on a corresponding beam width h of the input light beam 1105 in the first dimension, and a beam vergence half-angle θ of the output light beam 1110 depends on the diffusing properties of the diffuser 1103. As discussed in more detail with respect to the launch architecture 1050 of FIG. 10B, the beam width *h'* in the first dimension of the projected emission light beam 1114 at the transition region 1116 depends on the beam vergence half-angle θ of the output light beam 1110 in the first dimension. The angular divergence ω in the first dimension of the projected emission light beam 1114 at the transition region 1116 depends on the beam width h of the input light beam 1105 in the first dimension.

When the emission surface 1109 of a beam generator 1102 is positioned at a telecentric position relative to the collimating launch optical subassembly 1104 (and the ray bundles forming the emission light beam 1112 are each parallel ray bundles), each point of the transition region 1116 is projected to receive light with the same angle content. Specifically, each point of the transition region 1116 is projected to receive light having a range of angles (e.g., ω degrees) centered in the first dimension around a corresponding center ray. Collectively, the center rays (three center rays 1120a-1120c are shown in FIG. 11A for the sake of illustration) across the transition region 1116 are parallel.

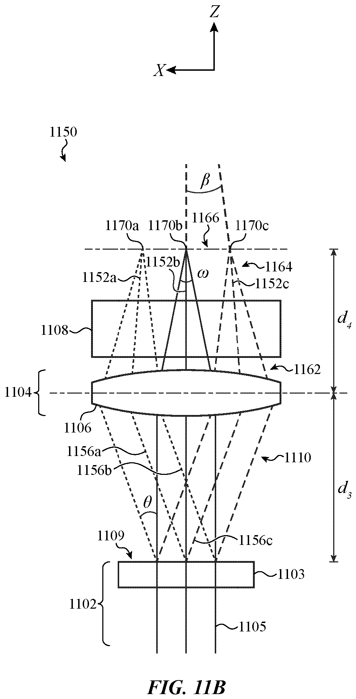

Conversely, FIG. 11B shows another variation of a launch architecture 1150, which is configured the same as the launch architecture 1100 (with like components labeled the same) except that the emission surface 1109 of the beam generator 1102 is positioned at a hyper-telecentric position relative to the collimating launch optical subassembly 1104. Specifically, the emission surface 1109 is positioned at a distance d₃ from the collimating launch optical subassembly 1104, where d₃ is larger than the focal length of the collimating launch optical subassembly 1104. In these instances, the collimating launch optical subassembly 1104 will generate, from light emitted from each position of the emission surface 1109, a corresponding ray bundle that converges in a first dimension (e.g., along an XZ plane using the cartesian coordinate system depicted in FIG. 11B) with a vergence angle β. For example, a first converging ray bundle (represented by rays 1152a-1 152c) is formed from light (represented by rays 1156a-1 156c) emitted at first point 1154 of the emission surface 1109. Accordingly, the collimating launch optical subassembly 1104 will form, from the output light beam 1110, an emission light beam 1162 that is made up of a plurality of converging ray bundles. Each of the converging ray bundles exit the collimating launch optical subassembly 1104 at different angles, such that the converging ray bundles are projected (e.g., via a projected emission light beam 1164 that is projected from a launch site 1108 of the optical measurement systems described herein) to cross at a transition region 1116.

Accordingly, each point of the transition region 1116 is projected to receive light with the different angle content. Specifically, each point of the transition region 1116 is projected to receive light having a range of angles (e.g., ω degrees) centered in the first dimension around a corresponding center ray. Collectively, the center rays (three center rays 1152a-1152c corresponding to the rays of the first converging ray bundle are shown in FIG. 11B for the sake of illustration) converge with a beam vergence half-angle β. In this way, the rays at the periphery of the emission light beam 1162 are preferentially angled toward a center of the emission light beam 1162 in the first dimension.

Positioning the emission surface 1109 of the beam generator 1102 at a hyper-telecentric position relative to a collimating launch optical subassembly 1104, such as shown in the launch architecture 1150 of FIG. 11B, may help to balance collection efficiency across the emission light beam 1162 for one or more measurement channels. Any of the launch architectures 902, 932, and 962 of the optical measurement systems 900, 930, and 960 of FIGS. 9A, 9B, and 9C-9D, respectively, may be configured to be the same as the launch architecture 1150 of FIG. 11B. Using the optical measurement system 960 of FIGS. 9C-9D as an example, the launch optical subassembly 984 of the optical measurement system 960 may be configured as a collimating launch optical subassembly.

If an emission surface of the beam generator 980 is positioned at a telecentric position relative to the launch optical subassembly 984, there may be a slight difference in median path length (and thereby collection efficiency) across the transition region 976 for light measured by a given measurement channel of the optical measurement system 960. For the first measurement channel (e.g., light measured by the first detector element 966a), light projected to pass through the leftmost portion of the transition region 976 along the first dimension (e.g., corresponding to point 1122a in the transition region 1116 shown in FIG. 11A) will have a shorter median path length than light projected to pass through the center of the transition region 976 along the first dimension (e.g., corresponding to point 1122b in the transition region 1116 shown in FIG. 11A). Similarly, light projected to pass through the rightmost portion of the transition region 976 along the first dimension (e.g., corresponding to point 1122c in the transition region 1116 shown in FIG. 11A) will have a longer median path length than light projected to pass through the center of the transition region 976 along the first dimension. The opposite occurs for the fourth measurement channel (e.g., light measured by the fourth detector element 966d). Specifically, light projected to pass through the leftmost portion of the transition region 976 along the first dimension will have a longer median path length than light projected to pass through the center of the transition region 976 along the first dimension, which in turn has a longer median path length than light projected to pass through the rightmost portion of the transition region 976 along the first dimension. In these instances, the first measurement channel will preferentially receive light projected to pass through the leftmost region of the transition region 976, while the fourth measurement channel will preferentially receive light projected to pass through the rightmost region of the transition region 976.

Positioning the emission surface of the beam generator 980 at a hyper-telecentric position relative to the launch optical subassembly 984 may balance the median path lengths across the transition region 975. In these instances, light projected to pass through the leftmost portion of the transition region 975 (e.g., corresponding to point 1170a in the transition region 1166 shown in FIG. 11B) will be somewhat angled away from the first detector element 966a in the first dimension. This may increase the median path length of light measured by the first measurement channel for light projected to pass through the leftmost portion of the transition region 975. Similarly, light projected to pass through the rightmost portion of the transition region 975 (e.g., corresponding to point 1170c in the transition region 1166 shown in FIG. 11B) will be somewhat angled toward from the first detector element 966a in the first dimension. This may decrease the median path length of light measured by the first measurement channel for light projected to pass through the leftmost portion of the transition region 975. Depending on the selection of the vergence half-angle β of the converging ray bundles, the emission light beam 971 may be configured such that light measured by the first measurement channel has the same median pathlength regardless of whether the measured light is projected to have passed through the leftmost portion, the rightmost portion, or the center (e.g., corresponding to point 1170b in the transition region 1166 shown in FIG. 11B) of the transition region 975.

Returning to FIG. 11B, the configuration of the output light beam 1110 may at least partially control the distance between collimating launch optical subassembly 1104 and the transition region 1166 of the projected emission light beam 1164. When the beam generator 1002 is configured such the angle content of the output light beam 1110 is constant across the output light beam 1110 in the first dimension, the transition region 1166 will be positioned at a distance d₄ from the collimating launch optical subassembly 1104 that corresponds to the focal length of the collimating launch optical subassembly 1104. Conversely, if the output light beam 1110 has a position-dependent range of emission angles on the emission surface 1109 of the beam generator 1102 in the first dimension, the distance d₄ will be different than the focal length of the collimating launch optical subassembly 1104. For example, if the input light beam 1105 is converging, the distance d₄ will be less than the focal length of the collimating launch optical subassembly 1104. Conversely, if the input light beam 1105 is diverging, the distance d₄ will be greater than the focal length of the collimating launch optical subassembly 1104.

When the launch architectures of the optical measurement systems described herein utilize a beam generator to generate an output light beam, it should be appreciated that beam generator may include any number of and combination of components as necessary to generate an output light beam as will be readily understood by someone of ordinary skill in the art. Generally, the beam generator includes one or more light sources that generate light. In some instances, the beam generator includes one or more light modification components (e.g., lens, mirrors, beamsplitters, polarizers, or the like) that shape and/or redirect the light generated by the one or more light sources to form the output light beam.

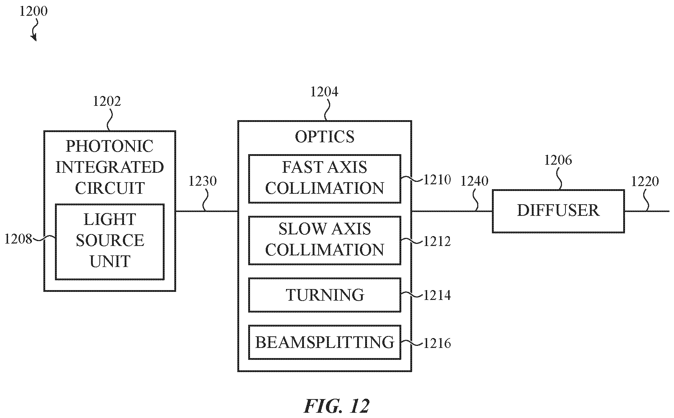

For example, FIG. 12 shows a block diagram of example beam generator 1200 that may be used with the optical measurement systems described herein to generate an output light beam 1220. As shown there, the beam generator 1200 may include a photonic integrated circuit 1202, an optical unit 1204, and a diffuser 1206. The photonic integrated circuit 1202 is configured to emit a first light beam 1230. For example, the photonic integrated circuit 1202 may include a light source unit 1208, such as described herein, which generates the light used to form the first light beam 1230. Specifically, at least a portion of the light generated by the light source unit 1208 may be routed within the photonic integrated circuit 1202 (e.g., using one or more waveguides) to a first set of outcouplers (e.g., a single outcoupler or a plurality of outcouplers). Light may exit the photonic integrated circuit 1202 via the first set of outcouplers to form the first light beam 1230.

The diffuser 1206 is configured to configured to receive an input light beam 1240, and to generate the output light beam 1220 therefrom. Specifically, the diffuser 1206 may be any optical diffuser that diffuses incoming light. In some variations, the diffuser 1206 may be a transmissive diffuser, such that the output light beam 1220 is formed as the input light beam 1240 passes through the diffuser 1206. Specifically, light will be scattered and/or refracted, depending on the configuration of the diffuser 1206, as it passes through the diffuser 1206. Light passing through a given location of the diffuser will generate diffused light having an angular spread (referred to herein as the "diffuser angle") in multiple dimensions. As a result, the beam vergence of the output light beam 1220 may depend on the diffuser angle. For example, in some variations the diffuser angle may have a half-angle of at least at least five degrees, as measured in air (e.g., light passing through the diffuser will be diffused at least five degrees, as measured in air, relative to a center beam along one or more dimension), which may result in the diffuser 1206 generating an output light beam 1220 having a beam vergence half-angle of at least at least five degrees, as measured in air, in at least one dimension. In other variations, the diffuser angle may have a half-angle of at least at least fifteen degrees, as measured in air, which may result in the diffuser 1206 generating an output light beam 1220 having a beam vergence half-angle of at least at least fifteen degrees, as measured in air, in at least one dimension.

In some variations, the diffuser 1206 may be positioned to receive light directly from the photonic integrated circuit 1202, in which case the first light beam 1230 may act as the input light beam 1240 to the diffuser 1206. Alternatively, the optical unit 1204 may collectively act to shape and/or redirect at least a portion of the first light beam 1230 to form the input light beam 1240. In the variation shown in FIG. 12, the optical unit 1204 is configured to perform collimation in one or more dimensions (e.g., fast axis collimation 1210 and slow axis collimation 1212), beam turning 1214, and beamsplitting 1216, though it should be appreciated that the optical unit 1204 may alternatively be configured to perform any suitable combination of these functions. Generally, the optical unit 1204 includes one or more light modification components such as described herein, such as one or more lenses, which collectively perform the functions of the optical unit 1204.

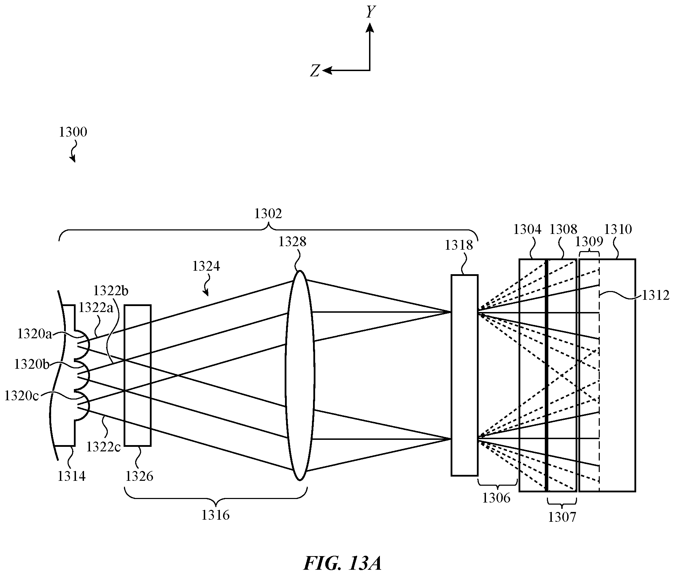

FIGS. 13A-13F depict variations of beam generators that may be used with the launch architectures described herein. Specifically, FIG. 13A depicts a top view of a launch architecture 1300 that may be used with the optical measurement systems described herein. The launch architecture 1300 includes a beam generator 1302 and a launch optical subassembly 1304. The beam generator 1302 is configured to generate an output light beam 1306. The launch optical subassembly 1304 is configured to receive and shape the output light beam 1306 to form an emission light beam 1307. The emission light beam 1307 may be emitted from an optical measurement system via a launch site 1308 of a sampling interface, such that the emission light beam 1307 projects a projected emission light beam 1309 into a target sample 1310 that is positioned in contact with the launch site 1308.

The emission light beam 1307 (and thereby the projected emission light beam 1309 when the emission light beam 1307 is emitted into a target sample 1310) may be configured in any suitable manner as described herein with respect to FIGS. 3A-3B and 9A-9D. For example, the emission light beam 1307 may converge in a first dimension, such it projects a projected emission light beam 1309 that transitions, in the first dimension, between a converging portion and a diverging portion. In these instances, FIG. 13A depicts a second dimension (e.g., along the YZ plane of the cartesian coordinate system shown there) of the emission light beam 1307 perpendicular to the first dimension, and line 1312 represents the transition region. In the variation shown in FIG. 13A, the emission light beam 1307 diverges in the second dimension (e.g., the beam generator 1302 generates an output light beam 1306 that diverges in the second dimension and the launch optical subassembly 1304 is configured as a one-dimensional set of lenses that does not change the divergence in the second dimension when generating the emission light beam 1307). Accordingly, the emission light beam 1307 projects a projected emission light beam 1309 that diverges in the second dimension.

To generate the output light beam 1306, the beam generator 1302 includes a photonic integrated circuit 1314, an optical unit 1316, and a diffuser 1318. The photonic integrated circuit 1314 includes a set of outcouplers, each of which is configured to emit a corresponding light beam from the photonic integrated circuit 1314. Specifically, the photonic integrated circuit 1314 includes one or more light sources (not shown) configured to generate light that is emitted via the set of outcouplers. In the variation shown in FIG. 13A, the photonic integrated circuit 1314 includes a plurality of output couplers 1320a-1320c that is configured to emit a plurality of individual light beams 1322a-1322c. The plurality of individual light beams 1322a-1322c at least partially overlap and collectively form an input light beam 1324. The input light beam 1324 is received by the diffuser 1318, which diffuses the input light beam 1324 to generate the output light beam 1306.

The plurality of outcouplers 1320a-1320c may be configured in any suitable manner, and may be include edge couplers, a vertical output couplers, or the like. For example, in the variation shown in FIG. 13A, each of the plurality of outcouplers 1320a-1320c is configured as an edge coupler, such that a corresponding portion of a side surface of the photonic integrated circuit 1314 acts as an output facet from which a corresponding light beam of the plurality of individual light beams 1322a-1322c may be emitted. For example, each of the plurality of output couplers 1320a-1320c include a portion of a side of the photonic integrated circuit 1314 that is shaped to form an on-chip lens, such as described in U.S. Patent Publication No. US2023/0089758A1, titled "Light Output Devices and Light Outputting Methods for Optical Systems", the contents of which are hereby incorporated by reference in their entirety.

Each of the plurality of individual light beams 1322a-1322c may, upon exiting the photonic integrated circuit 1314, diverge differently in different dimensions. For example, the plurality of individual light beams 1322a-1322c may, upon exiting the photonic integrated circuit 1314, have a higher divergence in a first dimension (hereinafter referred to as the "fast axis) than its divergence in a second dimension perpendicular to the first dimension (hereinafter referred to as the "slow axis"). As a result, the resulting input light beam 1324 may have a fast axis and a slow axis upon exiting the photonic integrated circuit 1314. In variations where the launch architecture 1300 is used to generate an emission light beam 1307 that is wider in one dimension, such as described with respect to the launch architecture 302 of FIGS. 3A and 3B, the input light beam 1324 may also be wider in one dimension. In some of these variations, the input light beam 1324 may be wider along the slow axis than it is along the fast axis.

In some variations, the optical unit 1316 may be configured to at least partially collimate the input light beam 1324 along the fast and slow axes. For example, in the variation shown in FIG. 13A, the optical unit 1316 includes a fast axis collimating lens 1326 that is configured to at least partially collimate the input light beam 1324 along its fast axis and a slow axis collimating lens 1328 that is configured to at least partially collimate the input light beam 1324 along its slow axis. As shown in FIG. 13A, the slow axis collimating lens 1328 collimates each of the plurality of individual light beams 1322a-1322c along the second dimension. Additionally, the slow axis collimating lens 1328 may be positioned relative to the photonic integrated circuit 1314 such that the collimated light beams 1322a-1322c intersect at the diffuser 1318. In these instances, the size of the input light beam 1324 along the second dimension (as shown in FIG. 13A) as it enters the diffuser 1318 will be independent of the number of outcouplers 1320a-1320c and emitted individual light beams 1322a-1322c. Additionally, to the extent that different outcouplers of the plurality of outcouplers 1320a-1320c emit light beams 1322a-1322c having different phases (e.g., one or more phase shifters are used to alter the relative phases of these light beams 1322a-1322c), the size of the input light beam 1324 along the second dimension as it enters the diffuser 1318 will be independent of the relative phases of the individual light beams 1322a-1322c. As a result, the size of the output light beam 1306 generated by the diffuser 1318 will also be independent of the number and relative phases of the plurality of individual light beams 1322a-1322c.

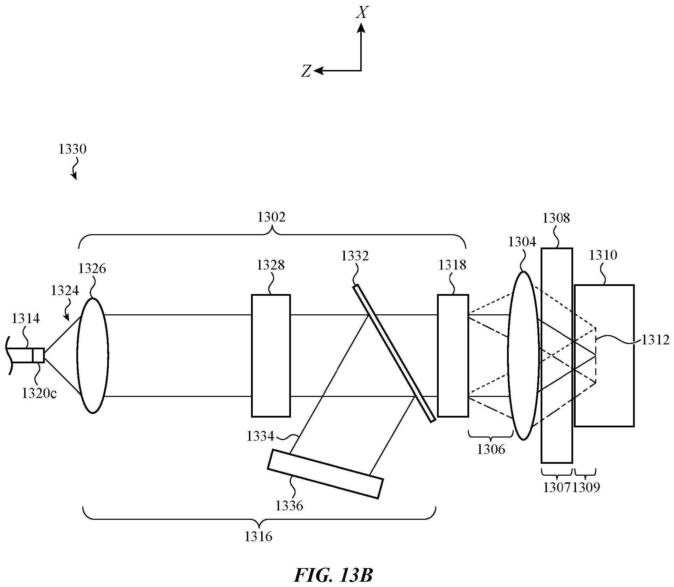

FIGS. 13B-13F show side views of different variations of the launch architecture 1300 of FIG. 13A. As discussed herein with respect to FIGS. 11A and 11B, the vergence angle of the input light beam 1324 as it reaches the diffuser 1318 may impact the placement of the transition region 1312 of the projected emission light beam 1309. For example, FIG. 13B shows a side view of a first variation of a launch architecture 1330. The launch architecture 1330 may be configured the same as the launch architecture 1300 (with like components labeled the same), and may be configured such that the input light beam 1324 is collimated in the first dimension (e.g., along the XZ plane shown in FIG. 13B) when it reaches the diffuser 1318.

The input light beam 1324 may diverge along the first dimension (e.g., along the fast axis) as it exits the photonic integrated circuit 1314. In the variation shown in FIG. 13B, the fast axis collimating lens 1326 fully collimates the input light beam 1324 in the first dimension (e.g., along the fast axis). The slow axis collimating lens 1328 does not alter the input light beam 1324 in the first dimension, such that the input light beam 1324 is collimated as it reaches the diffuser 1318. In instances where the launch optical subassembly 1304 is configured as a collimating launch optical subassembly (such as shown in FIG. 13B), the launch optical subassembly 1304 will generate, using the input light beam 1324, an emission light beam 1307 that converges in the first dimension. Additionally, the emission light beam 1307 will project a projected emission light beam 1309 with a transition region that is located at a distance corresponding to the focal length of the launch optical subassembly 1304, such as described in more detail herein.

When the optical measurement systems described herein utilize individual measurements of different wavelengths as part of a spectroscopic measurement, the launch architectures described herein may be used to generate an emission light beam is formed from different wavelengths at different times (and/or multiple wavelengths at the same time). In some instances, changing the wavelength of the emission light beam may alter one or more properties of the emission light beam. For example, when the photonic integrated circuit 1314 of launch architecture 1330 emits an input light beam 1324 as shown in FIG. 13B, the divergence of the input light beam 1324 in the first dimension (e.g., the fast axis) may depend on the wavelength of light emitted by the photonic integrated circuit 1314. Longer wavelengths of light may have a larger diffraction angle than shorter wavelengths of light. After collimation by the fast axis collimating lens 1326, the beam width of the input light beam 1324 in the first dimension may depend on the wavelength of light (e.g., longer wavelengths will have a wider beam width in the first dimension).

When the launch optical subassembly 1304 is configured as a collimating launch optical subassembly as shown in FIG. 13B, these wavelength-dependent changes in beam width of the input light beam 1324 will not change the size of the transition region 1312 in the first dimension. As discussed with respect to FIGS. 11A and 11B, the beam width in the first dimension of the projected emission light beam 1309 at the transition region 1312 depends on the beam vergence half-angle of the output light beam 1306 in the first dimension. The beam vergence half-angle of the output light beam 1306 is determined by the properties of the diffuser 1318, not the size of the input light beam 1324. Instead, wavelength-dependent changes in beam width of the input light beam 1324 may impact the angular divergence, in the first dimension, of the projected emission light beam 1309 at the transition region 1312.

In the variation shown in FIG. 13B, the optical unit 1316 may also include a beamsplitter 1332 positioned between the slow axis collimating lens 1328 and the diffuser 1318. The beamsplitter 1332 is configured to split off a portion of the input light beam 1324 as a reference light beam 1334. At least a portion of the reference light beam 1334 may be directed to a reference detector 1336, which may measure an intensity of the reference light beam 1334. Measurements from the reference detector 1336 may be used by the optical measurement systems described herein (e.g., using a controller) to account for fluctuations in the intensity of the reference light beam 1334 (which may represent the power of the emission light beam 1307).

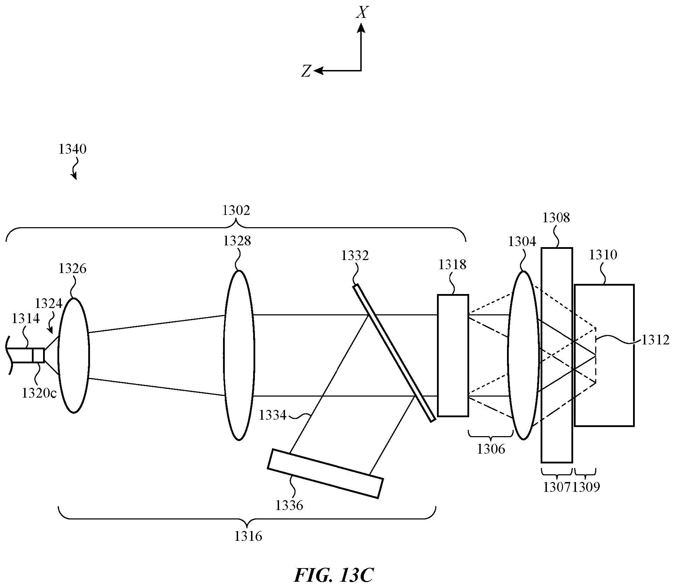

FIG. 13C shows a side view of another variation of a launch architecture 1340. The launch architecture 1340 may be configured the same as the launch architecture 1330 of FIG. 13B (with like components labeled the same), and may also be configured such that the input light beam 1324 is collimated in the first dimension (e.g., along the XZ plane shown in FIG. 13C) when it reaches the diffuser 1318. In the variation of FIG. 13C, the fast axis collimating lens 1326 partially collimates the input light beam 1324 in the first dimension, such that the input light beam 1324 is still diverging in the first dimension after exiting the fast axis collimating lens 1326. The slow axis collimating lens 1328 may also act to collimate the input light beam 1324 in the first dimension (e.g., the slow axis collimating lens 1328 may be configured as a crossed cylinder lens), such that the input light beam 1324 is collimated in the first dimension after exiting the slow axis collimating lens 1328. This arrangement may make the launch architecture 1340 less sensitive to misalignment of the fast axis collimating lens 1326.

In some of these variations, the slow axis collimating lens 1328 and beamsplitter 1332 may be positioned and configured to image the fast axis collimating lens 1326 onto the reference detector 1336. In these instances, vertical misalignments of the fast axis collimating lens 1326 relative to the photonic integrated circuit 1314 (e.g., along the X-axis show as shown in FIG. 13C) may still result in the reference light beam 1334 being incident on the same portion of the reference detector 1336. In this way, measurements performed by the reference detector 1336 may be less sensitive to misalignment of the fast axis collimating lens 1326.

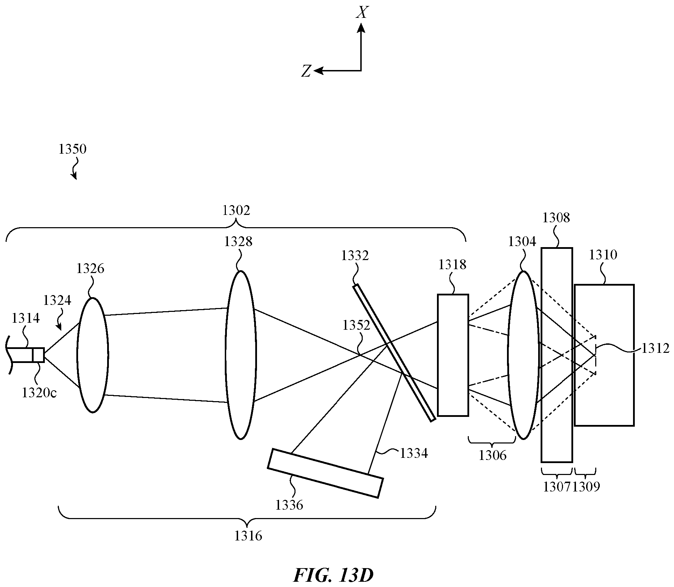

FIG. 13D shows a side view of another variation of a launch architecture 1350. The launch architecture 1350 may be configured the same as the launch architecture 1340 of FIG. 13C (with like components labeled the same), except that the beam generator 1302 is configured to generate an input light beam 1324 that is diverging in the first dimension (e.g., along the XZ plane shown in FIG. 13D) when it reaches the diffuser 1318. In the variation of FIG. 13D, the fast axis collimating lens 1326 partially collimates the input light beam 1324 in the first dimension, such that the input light beam 1324 is still diverging after exiting the fast axis collimating lens 1326. The slow axis collimating lens 1328 focuses the input light beam 1324 in the first dimension to a focus point 1352 between the slow axis collimating lens 1328 and the diffuser 1318. As the input light beam 1324 pass through the focus point 1352, the input light beam 1324 transitions from a converging beam in the first dimension to a diverging beam the first dimension. As a result, the input light beam 1324 will diverge in the first dimension as it enters the diffuser 1318.

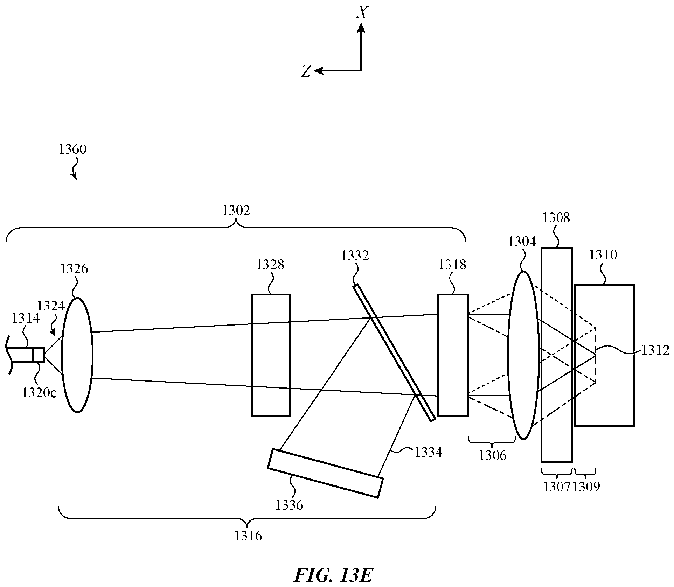

FIG. 13E shows a side view of another variation of a launch architecture 1360 that is configured to generate an input light beam 1324 that diverges in the first dimension as it enters the diffuser 1318. Specifically, the launch architecture 1360 may be configured the same as the launch architecture 1350 of FIG. 13D (with like components labeled the same), except that the slow axis collimating lens 1328 does not alter the input light beam 1324 in the first dimension. In this way, the input light beam 1324 (which is partially collimated, but still diverging after passing through the fast axis collimating lens 1326) will continue to diverge as it reaches the diffuser 1318.

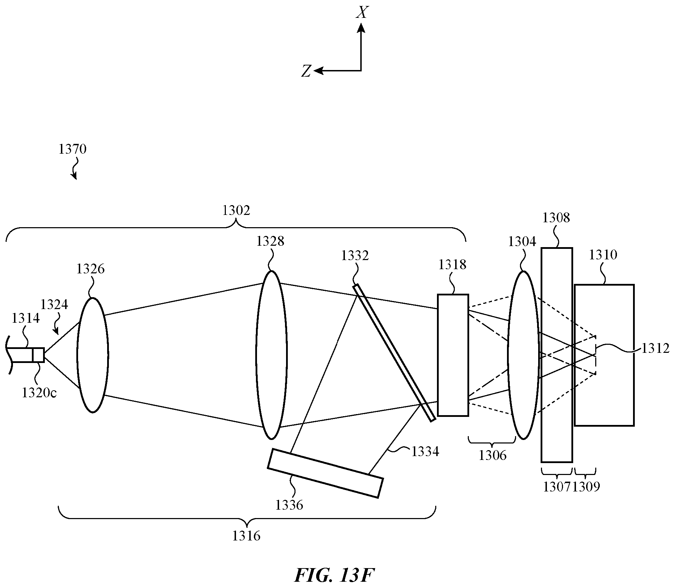

FIG. 13F shows a side view of another variation of a launch architecture 1370. The launch architecture 1370 may be configured the same as the launch architecture 1340 of FIG. 13C (with like components labeled the same), except that the beam generator 1302 is configured to generate an input light beam 1324 that is converging in the first dimension (e.g., along the XZ plane shown in FIG. 13F) when it reaches the diffuser 1318. In the variation of FIG. 13F, the fast axis collimating lens 1326 partially collimates the input light beam 1324 in the first dimension, such that the input light beam 1324 is still diverging after exiting the fast axis collimating lens 1326. The slow axis collimating lens 1328 focuses the input light beam 1324 in the first dimension to a focus point (not shown) positioned beyond the diffuser 1318. The input light beam 1324 will reach the diffuser 1318 before the focus point, and thus the input light beam 1324 will converge in the first dimension as it enters the diffuser 1318.

### Collection Architectures for use in Optical Measurement Systems

The optical measurement systems described herein may use a range of possible collection architectures to collect and direct light to one or more detector elements. Generally, such as described with respect to FIGS. 5A, 5B and 7-9D, a collection architecture includes one or more sets of detector elements and one or more collection optical subassemblies, each of which is configured to collect a set of return light beams and to direct the set of return light beams to a corresponding set of detector elements. Examples of collection architectures are described herein with respect to FIGS. 14A-18C. It should be appreciated that the launch architectures may be utilized as part of the optical measurement systems described herein with respect to FIGS. 1A-9D (e.g., may be used to collect any of the return light beams described with respect to these figures), or may be utilized as part of any other optical measurement system in which it is desirable to collect a return light beam as described herein.

When a collection architecture includes a collection optical subassembly that is configured to direct light to a plurality of detector elements (e.g., such as described above with respect to FIGS. 5B and 9B-9D), such that each detector element may collect light having different properties. In addition to measuring light having different path length distributions, it may be possible for different detector elements to collect light with a different sampling depth distribution. As used herein, a "sampling depth distribution" of a measurement channel refers to a probability distribution that represents the likelihood that a photon of light measured by the measurement channel (i.e., introduced into a sample from the optical measurement system and collected by a detector element of the measurement channel) penetrate to a particular sample depth in a sample having certain properties (e.g., a target sample as described herein). As used herein, a "median sampling depth" of a sampling depth distribution refers to the sampling depth that represents the 50% value of a cumulative distribution function that is based on the sampling depth distribution. In other words, a given photon measured by a measurement channel has a 50% probability of reaching a sampling depth in the sample that is shorter than the median sampling depth.

Accounting for the sampling depth distribution of light collected by a measurement channel may be advantageous in instances where a measured sample has different sample characteristics and/or sample properties as a function of sample depth. For example, a measured sample may include multiple layers, where each layer has different sample characteristics and/or sample properties. In these instances, it may be desirable to understand and/or control the depth to which measure light has penetrated a measured sample.

Accordingly, the collection architectures described herein may be configured to adjust the relative sampling depth distributions and path length distributions of light measured by different measurement channels. For example, FIG. 14A shows an example of a collection architecture 1400 that may be used with the optical measurement systems described herein. Specifically, the collection architecture 1400 includes a collection optical subassembly 1402 and a plurality of detector elements 1404a-1404c that define a plurality of measurement channels of an optical measurement system. The collection optical subassembly 1402 is configured to collect a plurality of return light beams 1406a-1406c (e.g., when a launch architecture of an optical measurement system, such as those described herein, emits light into a measured sample) from a measured sample through a collection site 1410 of a sampling interface, and is configured to direct each of the return light beams 1406a-1406c to a corresponding detector element of the plurality of detector elements 1404a-1404c.

The plurality of return light beams 1406a-1406c is projected to emanate from a plurality of projected return light beams 1408a-1408c in the measured sample such that each return light beam is projected to emanate from a corresponding projected return light beam. Specifically, a first return light beam 1406a is projected to emanate from a first projected return light beam 1408a and is measured by a first detector element 1404a, a second return light beam 1406b is projected to emanate from a second projected return light beam 1408b and is measured by a second detector element 1404b, and a third return light beam 1406c is projected to emanate from a third projected return light beam 1408c and is measured by a third detector element 1404c. Each of return light beams 1406a-1406c and its corresponding projected return light beam may have any characteristics as described above with respect to the return light beam 504 and the projected return light beam 506 of FIGS. 5A and 5B.

In the variation shown in FIG. 14A, each of the return light beams 1406a-1406c diverges along a common first dimension (e.g., along the XZ plane of FIG. 14A) as it enters a sampling interface (e.g., the collection site 1410) of an optical measurement system. In this way, each of the plurality of return light beams 1406a-1406c is projected to emanate from a projected return light beam that has a diverging portion along the first dimension as it enters the sampling interface and transitions from a converging portion (only the diverging portions of the return light beams 1406a-1406c are shown in FIG. 14A) at a corresponding collection transition region. Accordingly, the plurality of return light beams 1406a-1406c is associated with a plurality of collection transition regions 1412a-1412c (e.g., the first projected return light beam 1408a has a first collection transition region 1412a, the second projected return light beam 1408b has a second collection transition region 1412b, and the third projected return light beam 1408c has a third collection transition region 1412c). In some variations, such as described in more detail with respect to the optical measurement systems of FIGS. 5A-5B and 8-9D, some or all of these transition regions 1412a-1412c may be positioned at angle-independent sample regions for the respective measurement channels when the collection architecture 1400 is used to collect light from a target sample.

Depending on the configuration of the emission light beam, the measurement channels formed by the plurality of detector elements 1404a-1404c may measure light having different path length distributions and different sampling depth distributions. For example, FIG. 14B shows an example plot 1430 representing the range of path lengths and sampling depths of light collected by each of the measurement channels of collection architecture 1400 when the collection architecture 1400 is incorporated as the collection architecture 934 of the optical measurement system 930 of FIG. 9B. Specifically, plot 1430 includes a first distribution 1432a that represents the range of possible path lengths and sampling depth combinations (with a minimum threshold probability) for light measured by the first detector element 1404a, a second distribution 1432b that represents the range of possible path lengths and sampling depth combinations (with the minimum threshold probability) for light measured by the second detector element 1404b, and a third distribution 1432c that represents the range of possible path lengths and sampling depth combinations (with the minimum threshold probability) for light measured by the third detector element 1404c. As shown, light measured by the first detector element 1404a has, on average, a longer optical path length and a deeper sampling depth than light measured by the second detector element 1404b. Similarly, light measured by the second detector element 1404b has, on average, a longer optical path length and a deeper sampling depth than light measured by the third detector element 1404c.

The design of the collection optical subassembly 1402 may at least partially control the relative sampling depth distributions of the measurement channels associated with the plurality of detector elements 1404a-1404c. For example, in FIG. 14A, the collection optical subassembly 1042 includes an imaging lens 1420, a relay lens 1422, and an aperture 1424 positioned between the imaging lens 1420 and the relay lens 1422. The collection optical subassembly 1402 is configured to receive and shape the plurality of return light beams 1406a-1406c to form a corresponding plurality of measurement light beams 1414a-1414c, each of which is measured by a corresponding detector element of the plurality of detector elements 1404a-1404c.

In the variation shown in FIG. 14A, the aperture 1424 is placed at an object telecentric position in the first dimension, such that the collection optical subassembly 1402 acts an object telecentric lens in the first dimension. In these instances, each of the return light beams 1406a-1406c has the same angle content (e.g., the same beam direction and beam vergence) in the first dimension as it enters the collection site 1410, but collects the angle content from a different spatial position on the collection site 1410. In this way, the plurality of detector elements 1404a-1404c each measure light having the same angle content in the first dimension, but that is projected to emanate from different collection transition regions.

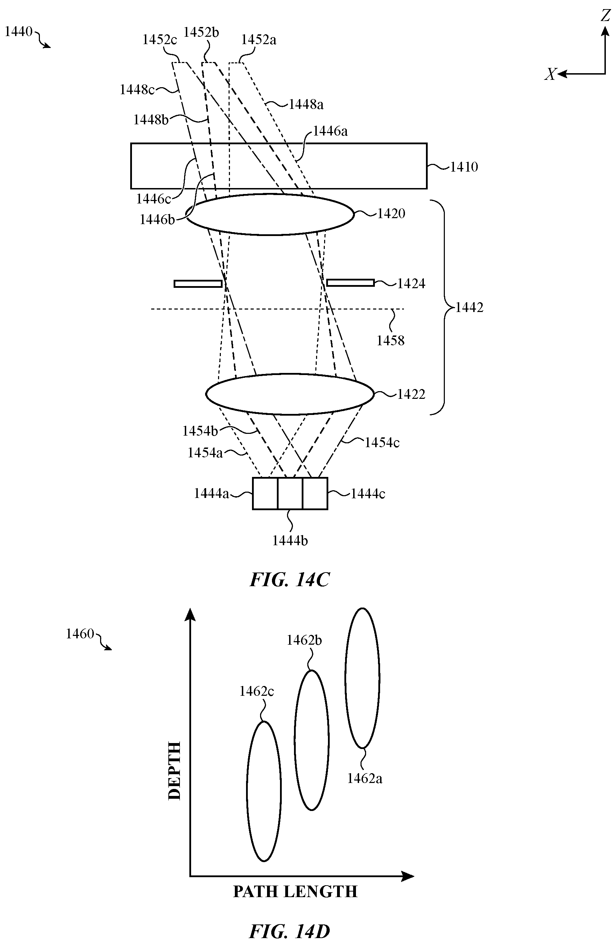

If, however, the aperture 1412 is placed at a non-telecentric position, the plurality of return light beams 1406a-1406c may have different angle content. For example, FIG. 14C shows another variation of a collection architecture 1440 as described herein. The collection architecture 1440 includes a collection optical subassembly 1442 and a plurality of detector elements 1444a-1444c. The collection architecture 1440 is otherwise configured the same as the collection architecture 1400 of FIG. 14A, except that the collection optical subassembly 1442 includes an imaging lens 1420, a relay lens 1422, and an aperture 1424, where the aperture 1424 is positioned closer to the sampling interface than an object telecentric position (which is depicted in FIG. 14C as line 1458 for comparison).

The collection architecture 1440 is configured to collect a plurality of return light beams 1446a-1446c from a collection site 1410. The plurality of return light beams 1446a-1446c is projected to emanate from a plurality of projected return light beams 1448a-1448c in a measured sample, where the projected return light beams 1448a-1448c have a corresponding plurality of collection transition regions 1452a-1452c. The collection optical subassembly 1442 is configured to receive and shape the plurality of return light beams 1446a-1446c to form a corresponding plurality of measurement light beams 1454a-1454c, each of which is measured by a corresponding detector element of the plurality of detector elements 1444a-1444c.

Due to the positioning of the aperture 1424, each of the plurality of return light beams 1446a-1446c has different angle content. Whereas the center rays of return light beams 1406a-1406c in FIG. 14A may be parallel in a first dimension, the center rays of the return light beams 1446a-1446c in FIG. 14C may converge in the first dimension. Specifically, when the plurality of return light beams 1446a-1446c includes three return light beams (e.g., with a second return light beam 1446b positioned between a first return light beam 1446a and a third return light beam 1446c), the center rays of the first and third return light beams 1446a, 1446c may be angled toward the center ray of the second return light beam 1446b.

Changing the angle content of the return light beams 1446a-1446c may change the relative sampling depths of collected light that is projected to travel through the plurality of transition regions 1452a-1452c. Specifically, placing the aperture 1424 closer to the sampling interface than an object telecentric position may increase the differences in median sampling depths between the measurement channels corresponding to the plurality of detector elements 1444a-1444c. For example, FIG. 14D shows an example plot 1460 representing the range of path lengths and sampling depths of light collected by each of the measurement channels of collection architecture 1440 when the collection architecture 1440 is incorporated as the collection architecture 934 of the optical measurement system 930 of FIG. 9B. Specifically, plot 1460 includes a first distribution 1462a that represents the range of possible path lengths and sample depth combinations (with a minimum threshold probability) for light measured by the first detector element 1444a, a second distribution 1462b that represents the range of possible path lengths and sample depth combinations (with the minimum threshold probability) for light measured by the second detector element 1444b, and a third distribution 1462c that represents the range of possible path lengths and sample depth combinations (with the minimum threshold probability) for light measured by the third detector element 1444c.

Light measured by the first detector element 1444a has, on average, a deeper sampling depth than light measured by the second detector element 1444b, but the difference in median sampling depth between the first distribution 1462a and the second distribution 1462b of plot 1460 is greater than the difference in median sampling depth between the first distribution 1432a and the second distribution 1432b of plot 1430. Similarly, light measured by the second detector element 1444b has, on average, a longer optical path length and a deeper sampling depth than light measured by the third detector element 1444c, but the difference in median sampling depth between the second distribution 1462b and the third distribution 1462c of plot 1460 is greater than the difference in median sampling depth between the second distribution 1432b and the third distribution 1432c of plot 1430. Additionally, in instances where the plurality of transition regions 1452a-1452c are positioned at sample regions in a target sample that are angle independent for their corresponding measurement channels, the collection architecture 1440 may adjust these relative depth differences without significantly impacting the path length distributions measured by the plurality of detector elements 1444a-1444c. For example, the first, second and third transition regions 1452a-1452c may be angle independent for respective first, second, and third measurement channels that are respectively measured by the plurality of detector elements 1444a-1444c.

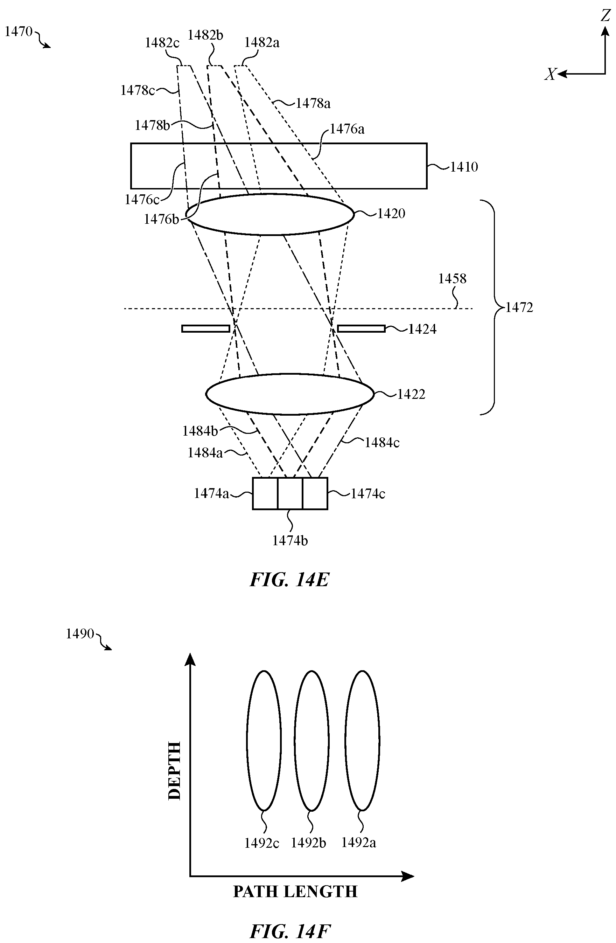

FIG. 14E shows another variation of a collection architecture 1470 as described herein. The collection architecture 1470 includes a collection optical subassembly 1472 and a plurality of detector elements 1474a-1474c. The collection architecture 1470 is otherwise configured the same as the collection architecture 1400 of FIG. 14A, except that the collection optical subassembly 1472 includes an imaging lens 1420, a relay lens 1422, and an aperture 1424, where the aperture 1424 is positioned farther from the sampling interface than an object telecentric position (depicted in FIG. 14E as line 1458 for comparison).

The collection architecture 1470 is configured to collect a plurality of return light beams 1476a-1476c that enter the collection architecture 1470 at a collection site 1410. The plurality of return light beams 1476a-1476c is projected to emanate from a plurality of projected return light beams 1478a-1478c in a measured sample, where the projected return light beams 1478a-1478c have a corresponding plurality of collection transition regions 1482a-1482c. The collection optical subassembly 1472 is configured to receive and shape the plurality of return light beams 1476a-1476c to form a corresponding plurality of measurement light beams 1484a-1484c, each of which is measured by a corresponding detector element of the plurality of detector elements 1474a-1474c.

In these instances, each of the plurality of return light beams 1446a-1446c has different angle content. Whereas the center rays of return light beams 1446a-1446c in FIG. 14A may converge in a first dimension, the center rays of the return light beams 1476a-1746c in FIG. 14C may diverge in the first dimension. Specifically, when the plurality of return light beams 1476a-1476c includes three return light beams (e.g., with a second return light beam 1476b positioned between a first return light beam 1476a and a third return light beam 1476c), the center rays of the first and third return light beams 1476a, 1476c may be angled away from the center ray of the second return light beam 1476b.

Changing the angle content of the return light beams 1476a-1476c in this way may decrease the difference in median sampling depth between the measurement channels corresponding to the plurality of detector elements 1474a-1474c. For example, FIG. 14F shows an example plot 1490 representing the range of path lengths and sampling depths of light collected by each of the measurement channels of collection architecture 1470 when the collection architecture 1470 is incorporated as the collection architecture 934 of the optical measurement system 930 of FIG. 9B. Specifically, plot 1490 includes a first distribution 1492a that represents the range of possible path lengths and sampling depth combinations (with a minimum threshold probability) for light measured by the first detector element 1474a, a second distribution 1492b that represents the range of possible path lengths and sampling depth combinations (with the minimum threshold probability) for light measured by the second detector element 1474b, and a third distribution 1492c that represents the range of possible path lengths and sampling depth combinations (with the minimum threshold probability) for light measured by the third detector element 1474c.

In the variation shown in FIG. 14F, the light measured by each of the plurality of detector elements 1474a-1474c has approximately the same median sampling depth. For the purpose of purpose of this application, a plurality of sampling depth distributions (each associated with a corresponding median sampling depth) is considered to be "approximately the same" if the median sampling depths of the plurality of sampling depth distributions are all within 10% (e.g., ± 10%) of the average of the median sampling depths. It should be appreciated that, in other instances, that there may be differences in median sampling depths between the first, second, and third distributions 1492a-1492c of plot 1490, but that these differences may be less than the corresponding differences between the distributions 1432a-1432c of plot 1430.

Accordingly, the positioning of the aperture 1424 between the imaging lens 1420 and the relay lens 1422 of a collection optical subassembly may be selected to achieve a desired distribution of median sampling depths for a given set of measurement channels. Additionally, because there may be a tradeoff between collection efficiency and sampling depth (e.g., the amount of light returned may decrease as a function of sampling depth), this balance may be taken into account when selecting a relative aperture 1424 position. In some variations, the collection optical subassembly may comprise an actuator that is configured to selectively move the aperture 1424. In these instances, the actuator may selectively move the aperture 1424 between an object telecentric position (as depicted into FIG. 14A) and one or more non-object telecentric positions (such as depicted in FIGS. 14C and 14E).

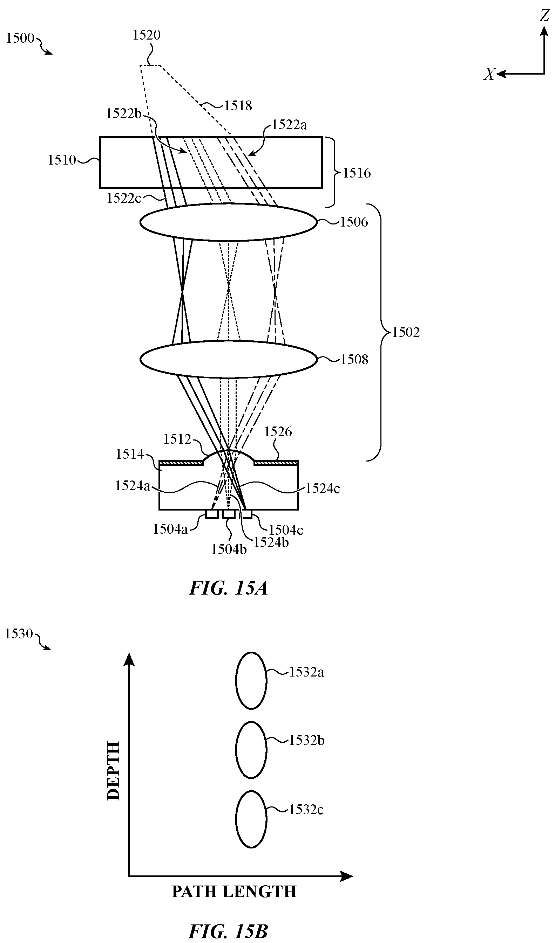

In the variation shown in FIGS. 14A-14E, different detector elements associated with a collection optical subassembly may measure light having different path length distributions and different sampling depth distributions. In some instances, it may be desirable to configure a collection architecture to measure, using different detector elements, light having approximately the same path length distribution and different sampling depth distributions. FIG. 15A depicts one such variation of a collection architecture 1500 as described herein. The collection architecture 1500 includes a collection optical subassembly 1502 and a plurality of detector elements 1504a-1504c, where the collection optical subassembly 1502 includes a condenser lens 1512. Light passing through the condenser lens 1512 is measured by the plurality of detector elements 1504a-1504c.

The collection optical subassembly 1502 is configured to collect a return light beam 1516 (e.g., when a launch architecture of an optical measurement system, such as those described herein, emits light into a measured sample) from a measured sample through a collection site 1510 of a sampling interface, and is configured to direct the return light beam 1516 to the condenser lens 1512. The return light beam 1516 is projected to emanate from a projected return light beam 1518 that has a diverging portion along a first dimension (e.g., along the XZ plane shown in FIG. 15A) as it enters the collection site 1510, and that transitions from a converging portion (only the diverging portion is shown in FIG. 15A) at a transition region 1520. The return light beam 1516 and its corresponding projected return light beam 1518 may have any characteristics as described above with respect to the return light beam 504 and the projected return light beam 506 of FIGS. 5A and 5B.

The collection optical subassembly 1502 further includes a set of lenses (e.g., including an imaging lens 1506 and relay lens 1508) that is configured to the image return light beam 1518 onto the condenser lens 1512. For example, the collection optical subassembly 1502 may be configured such that the condenser lens 1512 is positioned at a focal plane of the set of lenses. Additionally, the collection optical subassembly 1502 may be configured to position, when the collection optical subassembly 1502 collects the return light beam 1516 from a target sample, the transition region 1520 of the projected light return light beam 1518 at an image plane of the set of lenses. In these instances, the angle at which a ray of light is incident upon the condenser lens 1512 corresponds to the angle at which the ray of light enters the sampling interface 1510.

The condenser lens 1512 is configured to generate, from the return light beam 1518, a plurality of measurement light beams 1524a-1524c, each of which is measured by a corresponding detector of the plurality of detector elements 1504a-1504c. Each of the measurement light beams 1524a-1524c includes light that enters the collection site 1510 with a different corresponding range of angles. For example, FIG. 15A depicts a first ray bundle 1522a that represents the rays of light in the return light beam 1516 that enter the collection site 1510 at a first angle, a second ray bundle 1522b that represents the rays of light in the return light beam 1516 that enter the collection site 1510 at a second angle different from the first angle, and a third ray bundle 1522c that represents the rays of light in the return light beam 1516 that enter the collection site 1510 at a third angle different from the first and second angles.

The location at which an individual ray is incident upon the plurality of detector elements 1504a-1504c is dependent on the angle at which that ray is incident on the condenser lens 1512. For example, rays of the first ray bundle 1522a, after passing through the imaging lens 1506 and the relay lens 1508, are incident on the condenser lens 1512 with a first common angle, and form part of a first measurement light beam 1524a that is measured by a first detector element 1504a. Similarly, rays of the second ray bundle 1522b, after passing through the imaging lens 1506 and the relay lens 1508, are incident on the condenser lens 1512 with a second common angle, and form part of a second measurement light beam 1524b that is measured by a second detector element 1504b. Rays of the third ray bundle 1522c, after passing through the imaging lens 1506 and the relay lens 1508, are incident on the condenser lens 1512 with a third common angle, and form part of a third measurement light beam 1524c that is measured by a third detector element 1504c.

Because the return light beam 1516 is imaged onto the condenser lens 1512, each ray measured by the plurality of detector elements is projected to have crossed the transition region 1520. The collection architecture 1500 may be configured such that the transition region 1520 is positioned, for a target sample, at a sample region that is angle-independent for each of the plurality of detector elements 1504a-1504c. In these instances, because the plurality of detector elements 1504a-1504c each measure light that is projected to cross the same transition region 1520, the detector elements 1504a-1504c will measure light having approximately the same path length distribution. Additionally, because the plurality of detector elements 1504a-1504c each measured light that is projected to cross the transition region 1520 at a different range of angles, the plurality of detector elements 1504a-1504c will measure light having different sampling depth distributions.

For example, FIG. 15B shows an example plot 1530 representing the range of path lengths and sampling depths of light collected by each of the measurement channels of collection architecture 1500 when the collection architecture 1500 is incorporated as the collection architecture 904 of the optical measurement system 900 of FIG. 9A. Specifically, plot 1530 includes a first distribution 1532a that represents the range of possible path lengths and sampling depth combinations (with a minimum threshold probability) for light measured by the first detector element 1504a, a second distribution 1532b that represents the range of possible path lengths and sampling depth combinations (with the minimum threshold probability) for light measured by the second detector element 1504b, and a third distribution 1532c that represents the range of possible path lengths and sampling depth combinations (with the minimum threshold probability) for light measured by the third detector element 1504c. As shown there, the first, second, and third distributions 1532a-1532c have approximately the same path length distribution, but the first detector element 1504a measures light that has, on average, a deeper sampling depth than light measured by the second detector element 1504b. Similarly, the second detector element 1504b measures light that has, on average, a deeper sampling depth than light measured by the third detector element 1504c. These distributions may improve the accuracy of measurements performed by the optical measurement systems described herein.

In some variations, the condenser lens 1512 may be configured as an immersion condenser lens. In these variations, the condenser lens 1512 may be formed from a surface of a substrate 1514. The plurality of detector elements 1504a-1504c may also be formed on the substrate 1514 (e.g., using one or more epitaxial layers grown on the substrate 1514). Examples of detectors with immersion lenses are described in more detail in U.S. Patent Publication No. US2022/0037543A1, titled "Wideband Back-Illuminated Electromagnetic Radiation Detectors", the contents of which are hereby incorporated by reference in their entirety. Additionally, in some variations the substrate 1514 may further include an aperture layer 1526 formed from a light-blocking material and at least partially surrounding the condenser lens 1512. The aperture layer 1526 may define an aperture of the condenser lens 1512.

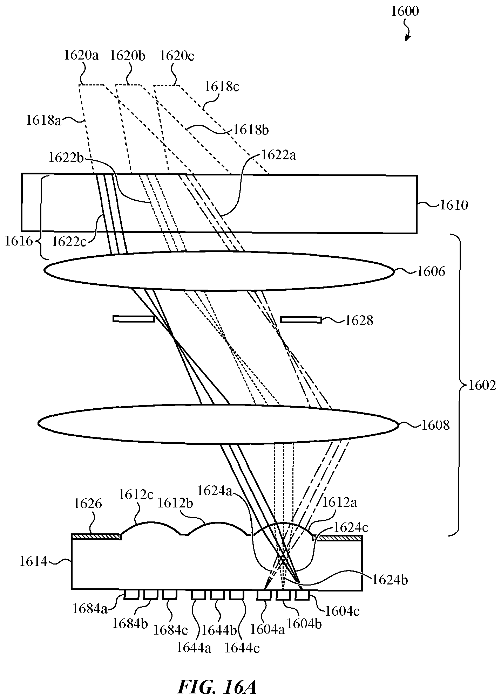

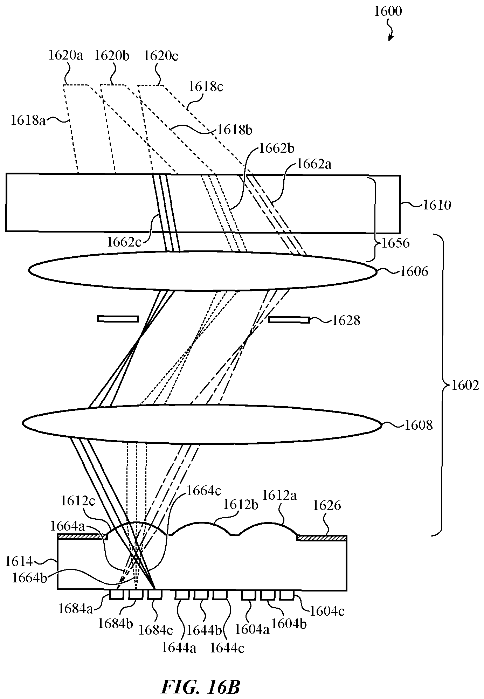

In some variations, a collection optical subassembly may comprise a plurality of immersion lens, each of which is associated with a different plurality of detector elements. In these instances, each plurality of detector elements may measure light having a common path length distribution and different sampling depth distributions, whereas different pluralities of detector elements may measure light having different path length distributions. FIGS. 16A and 16B depicts one such variation of a collection architecture 1600 as described herein. The collection architecture 1600 includes a collection optical subassembly 1602 that has a plurality of condenser lenses 1612a-1612c. The collection architecture 1600 further includes multiple groups of detector elements, each of which is positioned to receive light from a different condenser lens of the plurality of condenser lenses 1612a-1612c.

In the example shown in FIGS. 16A and 16B, the collection architecture 1600 comprises three condenser lenses 1612a-1612c and three pluralities of detector elements. Specifically, a first plurality of detector elements 1604a-1604c is positioned to measure light passing through the first condenser lens 1612a, a second plurality of detector elements 1644a-1644c is positioned to measure light passing through the second condenser lens 1612b, and a third plurality of detector elements 1684a-1684c is positioned to measure light passing through the third condenser lens 1612c. Each of the pluralities of detector elements 1604a-1604c, 1644a-1644c, and 1684a-1684c are shown in FIGS. 16A and 16B as having three detector elements, though it should be appreciated that in some instances one or more of the pluralities of detector elements may have two or four or more detector elements.

The collection optical subassembly 1602 is configured to collect a plurality of return light beams (e.g., when a launch architecture of an optical measurement system, such as those described herein, emits light into a measured sample) from a measured sample through a collection site 1610 of a sampling interface. Specifically, the plurality of return light beams includes a first return light beam 1616 (shown in FIG. 16A), a second return light beam (not shown), and a third return light beam 1656. The plurality of return light beams may at least partially overlap as light enters the collection site 1610 of the sampling interface. The collection optical subassembly 1602 is further configured to direct the first return light beam 1616 to the first condenser lens 1612a, direct the second return light beam to the second condenser lens 1612b, and the third return light beam to the third condenser lens 1612c.

Each of the plurality of return light beams is proj ected to emanate from a corresponding projected return light beam. For example, the first return light beam 1616 is projected to emanate from a first projected return light beam 1618a, the second return light beam is projected to emanate from a second projected return light beam 1618b, and the third return light beam 1656 is projected to emanate from a third projected return light beam 1618c. Each of the plurality of projected return light beams 1618a-1618c may include a diverging portion that diverges along a first dimension (e.g., along the XZ plane shown in FIGS. 16A and 16B) as it enters the collection site 1610. Additionally, these diverging portions transitions from corresponding converging portions (only the diverging portions are shown in FIGS. 16A and 16B) at a plurality of transition region 1620a-1620c. Each of the return light beams and its corresponding projected return light beam may have any characteristics as described above with respect to the return light beam 504 and the projected return light beam 506 of FIGS. 5A and 5B.

The collection optical subassembly 1602 further includes a set of lenses (e.g., including an imaging lens 1606 and relay lens 1608 separated by an aperture 1628) that is configured to the image the plurality of return light beams onto the plurality of condenser lenses 1612a-1612c. For example, the collection optical subassembly 1602 may be configured such that the each of the plurality of condenser lenses 1612a-1612c is positioned at a focal plane of the set of lenses. Additionally, the collection optical subassembly 1602 may be configured to position, when the collection optical subassembly 1602 collects the plurality of return light beams from a target sample, each of the plurality of transition regions 1620a-1620c of the projected light return light beams 1618a-1618c at an image plane of the set of lenses. In these instances, the selection of which condenser lens receives a given ray of light depends on which of the transition regions 1620a-1620c the ray is projected to intersect. Additionally, the angle at which a ray of light is incident upon a given condenser lens corresponds to the angle at which the ray of light enters the sampling interface via the collection site 1610.

As described in more detail below, each of the condenser lenses 1612a-1612c is configured to generate, from its corresponding return light beam, a plurality of measurement light beams that is measured by the plurality of detector elements associated with the condenser lens. Each of plurality of measurement light beams corresponds to a different range of angles selected from the return light beam. For example, as shown in FIG. 16A, a first detector element 1604a of the first plurality of detector elements 1604a-1604c measures a first ray bundle 1622a of the first return light beam 1616 that includes rays projected to intersect the first transition region 1620a and entering the collection site 1610 at a first range of angles. A second detector element 1604b of the first plurality of detector elements 1604a-1604c measures a second ray bundle 1622b of the first return light beam 1616 that includes rays projected to intersect the first transition region 1620a and entering the collection site 1610 at a second range of angles (different from the first range of angles). A third detector element 1604c of the first plurality of detector elements 1604a-1604c measures a third ray bundle 1622c of the first return light beam 1616 that includes rays projected to intersect the first transition region 1620a and entering the collection site 1610 at a third range of angles (different from the first and second range of angles).

Similarly, as shown in FIG. 16B a first detector element 1684a of the third plurality of detector elements 1684a-1684c measures a first ray bundle 1662a of the third return light beam 1656 that includes rays projected to intersect the third transition region 1620c and entering the collection site 1610 at the first range of angles. A second detector element 1684b of the third plurality of detector elements 1684a-1684c measures a second ray bundle 1662b of the third return light beam 1656 that includes rays projected to intersect the third transition region 1620c and entering the collection site 1610 at the second range of angles. A third detector element 1684c of the third plurality of detector elements 1684a-1684c measures a third ray bundle 1662c of the third return light beam 1656 that includes rays projected to intersect the third transition region 1620c and entering the collection site 1610 at the third range of angles.

In this way, each plurality of detector elements may measure light having a common path length distribution and a different sampling depth distribution, such as described herein with respect to the collection architecture 1500 of FIG. 15A. The collection architecture 1600 may be configured such that the each of the plurality of transition regions 1620a-1620c is positioned, for a target sample, at a corresponding sample region that is angle-independent for a corresponding plurality of detector elements. Specifically, the first transition region 1620a may be angle independent for each of the first plurality of detector elements 1604a-1604c, the second transition region 1620b may be angle independent for each of the second plurality of detector elements 1644a-1644c, and the third transition region 1620c may be angle independent for each of the third plurality of detector elements 1684a-1684c. In these instances, the path length distribution may be approximately the same within a given plurality of detector elements, but may vary between different pluralities of detector elements.

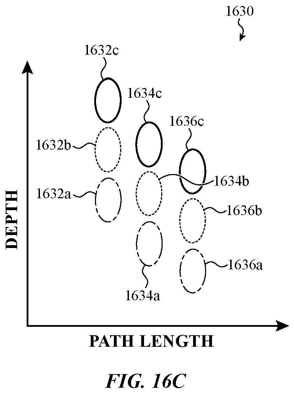

FIG. 16C shows an example plot 1630 representing the range of path lengths and sampling depths of light collected by each of the measurement channels of collection architecture 1600 when the collection architecture 1600 is incorporated as the collection architecture 934 of the optical measurement system 930 of FIG. 9B. Specifically, plot 1630 includes a first group of distributions 1632a-1632c that respectively represents the ranges of possible path lengths and sampling depth combinations (with a minimum threshold probability) for light measured by the first plurality of detector elements 1604a-1604c. Plot 1630 further includes a second group of distributions 1634a-1634c corresponding to the second plurality of detector elements 1644a-1644c, and a third group of distributions 1636a-1636c corresponding to the third plurality of detector elements 1684a-1684c.

As shown there, the first plurality of detector elements 1604a-1604c may measure light having a common path length distribution, but different sampling depth distributions. Similarly, the second plurality of detector elements 1644a-1644c may measure light having a common path length distribution, but different sampling depth distributions. That said, the second plurality of detector elements 1644a-1644c may measure light having, on average, a longer optical path length than light measured by the first plurality of detector elements 1604a-1604c. Overall, the various detector elements may selectively measure different combinations of optical path length and sampling depth. Additionally, the relative position of the aperture 1628 may be adjusted to alter the relative sampling depth differences between the different pluralities of detector elements, such as described herein with respect to FIGS. 14A-14F.

As with the condenser lens 1512 of FIG. 15A, the plurality of condenser lenses 1612a-1612c may be formed as part of a common substrate 1614 to form a plurality of immersion condenser lenses. In these variations, the first, second, and third pluralities of detector elements 1604a-1604c, 1644a-1644c, 1684a-16840c may be formed on the substrate 1614. Additionally, the substrate 1614 may further include an aperture layer 1626 such as described in more detail herein.

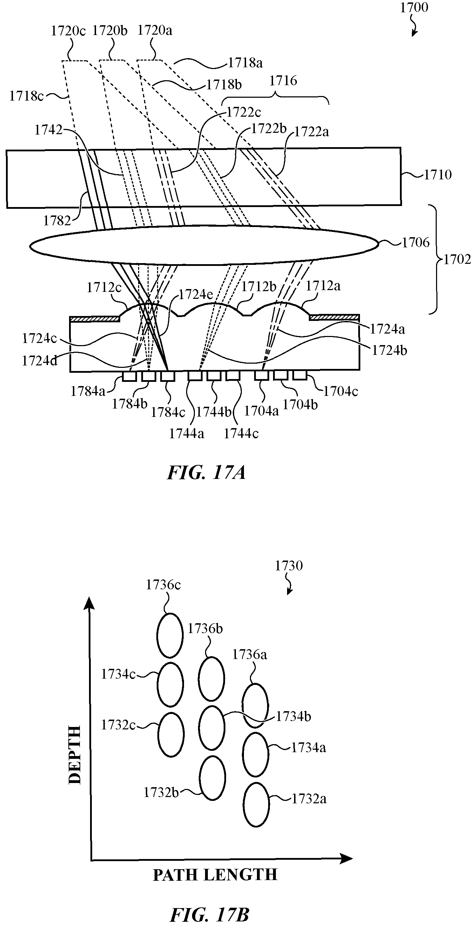

In other variations of the collection architectures described herein, a collection optical subassembly may be configured such that a plurality of detector elements associated with a condenser lens measure light having a common sampling depth distribution, but different path length distributions. FIG. 17A depicts one such variation of a collection architecture 1700 as described herein. The collection architecture 1700 includes a collection optical subassembly 1702 that has a plurality of condenser lenses 1712a-1712c (which may be a plurality of immersion condenser lenses formed on a common substrate as discussed in more detail herein). The collection architecture 1700 further includes multiple groups of detector elements, each of which is positioned to receive light from a different condenser lens of the plurality of condenser lenses 1712a-1712c.

In the example shown in FIG. 17A, the collection architecture 1700 comprises three condenser lenses 1712a-1712c and three pluralities of detector elements. Specifically, a first plurality of detector elements 1704a-1704c is positioned to measure light passing through the first condenser lens 1712a, a second plurality of detector elements 1744a-1744c is positioned to measure light passing through the second condenser lens 1712b, and a third plurality of detector elements 1784a-1784c is positioned to measure light passing through the third condenser lens 1712c. Each of the pluralities of detector elements 1704a-1704c, 1744a-1744c, and 1784a-1784c are shown in FIG. 17A as having three detector elements, though it should be appreciated that in some instances one or more of the pluralities of detector elements may have two or four or more detector elements.

The collection optical subassembly 1702 is configured to collect a plurality of return light beams (e.g., when a launch architecture of an optical measurement system, such as those described herein, emits light into a measured sample) from a measured sample through a collection site 1710 of a sampling interface. Specifically, the plurality of return light beams includes a first return light beam 1716, a second return light beam (a portion of which, specifically ray bundle 1742, is depicted in FIG. 17A), and a third return light beam (a portion of which, specifically ray bundle 1782, is depicted in FIG. 17A). The plurality of return light beams may at least partially overlap as light enters the collection site 1710 of the sampling interface. The collection optical subassembly 1702 is further configured to direct a portion of each of the plurality of return light beams to each of the plurality of condenser lenses 1712a-1712c.

Each of the plurality of return light beams is projected to emanate from a corresponding projected return light beam. For example, the first return light beam 1716 is projected to emanate from a first projected return light beam 1718a, the second return light beam is projected to emanate from a second projected return light beam 1718b, and the third return light beam is projected to emanate from a third projected return light beam 1718c. Each of the plurality of projected return light beams 1718a-1718c may include a diverging portion that diverges along a first dimension (e.g., along the XZ plane shown in FIG. 17A) as it enters the collection site 1710. Additionally, these diverging portions transitions from corresponding converging portions (only the diverging portions are shown in FIG. 17A) at a plurality of transition region 1720a-1720c. Each of the return light beams and its corresponding projected return light beam may have any characteristics as described above with respect to the return light beam 504 and the projected return light beam 506 of FIGS. 5A and 5B.

The collection optical subassembly 1702 further includes a set of lenses (e.g., including a lens 1706) that is configured to direct a portion of each of plurality of return light beams onto each of the plurality of condenser lenses 1712a-1712c. For example, the collection optical subassembly 1702 may be configured to collimate each of the return light beams and direct the collimated return light beams toward the plurality of condenser lenses 1712a-1712c. Specifically, each of the collimated return light beams may be incident upon the plurality of condenser lenses at a different angle. Accordingly, each of the plurality of condenser lenses 1712a-1712c receives a collimated portion of each of the plurality of return light beams, where these collimated portions enter the condenser lens at different angles. Each condenser lens will direct each collimated portion to a different detector element.

For example, the first return light beam 1716 includes a first ray bundle 1722a that enters the collection site 1710 at a first range of angles, a second ray bundle 1722b that enters the collection site 1710 site at a second range of angles different from the first range of angles, and a third ray bundle 1722c that enters the collection site 1710 at a third range of angles different from the first and second ranges of angles. The first ray bundle 1722a may be directed to the first condenser lens 1712a, which directs the first ray bundle 1722a (e.g., as a first measurement light beam 1724a) to a first detector element 1704a of the first plurality of detector elements 1704a-1704c. The second ray bundle 1722b may be directed to the second condenser lens 1712b, which directs the second ray bundle 1722b (e.g., as a second measurement light beam 1724b) to a first detector element 1744a of the second plurality of detector elements 1744a-1744c. The third ray bundle 1722c may be directed to the third condenser lens 1712c, which directs the third ray bundle 1722c (e.g., as a third measurement light beam 1724c) to a first detector element 1784a of the third plurality of detector elements 1784a-1784c. Accordingly, a first detector element from each of the first, second, and third pluralities of detector elements 1704a-1704c, 1744a-1744c, 1784a-1784c measures light from the first return light beam 1716. Similarly, a second detector element from each of the first, second, and third pluralities of detector elements 1704a-1704c, 1744a-1744c, 1784a-1784c measures light from the second return light beam. A third detector element from each of the first, second, and third pluralities of detector elements 1704a-1704c, 1744a-1744c, 1784a-1784c measures light from the third return light beam.

As a result, each plurality of detector elements measures light from a different return light beams, but with the same angle content. For example, the third condenser lens 1712c receives the third ray bundle 1722c (e.g., that enters the collection site 1710 at the third range of angles), a ray bundle 1742 from the second return light beam that enters the collection site 1710 with the third range of angles, and a ray bundle 1782 from the third return light beam that enters the collection site 1710 with the third range of angles. Accordingly, the third plurality of detector elements 1784a-1784c may include the first detector element 1784a that receives the third ray bundle 1722c (e.g., as the third measurement light beam 1724c), a second detector element 1784b that receives the ray bundle 1742 (e.g., as a fourth measurement light beam 1724d), and a third detector element 1784c that receives the ray bundle 1782 (e.g., as a fifth measurement light beam 1724e).

The collection optical subassembly 1702 may be configured to position, when the collection optical subassembly 1702 collects the plurality of return light beams from a target sample, each of the plurality of transition regions 1720a-1720c may be positioned at a corresponding sample region that is angle independent for a detector element from each of the first, second, and third pluralities of detector elements 1704a-1704c, 1744a-1744c, 1784a-1784c. For example, the first transition region 1720a may be angle independent for a first detector element of each of the pluralities of detector elements (e.g., a first detector element 1704a of the first plurality of detector elements 1704a-1704c, a first detector element 1744a of the second plurality of detector elements 1744a-1744c, and a first detector element 1784a of the third plurality of detector elements 1784a-1784c. Additionally, because these detector elements all measure light projected to emanate from the same transition region 1720a, they may measure light having approximately the same path length distributions but different sampling depth distributions.

Similarly, the second transition region 1720b may be angle independent for a second detector element of each of the pluralities of detector elements (e.g., a second detector element 1704b of the first plurality of detector elements 1704a-1704c, a second detector element 1744b of the second plurality of detector elements 1744a-1744c, and a third detector element 1784c of the third plurality of detector elements 1784a-1784c). The third transition region 1720c may be angle independent for a third detector element of each of the pluralities of detector elements (e.g., a third detector element 1704c of the first plurality of detector elements 1704a-1704c, a third detector element 1744c of the second plurality of detector elements 1744a-1744c, and a third detector element 1784c of the third plurality of detector elements 1784a-1784c).

Overall, the first, second, and third pluralities of detector elements 1704a-1704c, 1744a-1744c, 1784a-1784c may collectively measure light having the same set of path length distributions and sampling depth distributions as that collected by the first, second, and third pluralities of detector elements 1604a-1604c, 1644a-1644c, and 1684a-1684c described herein with respect to collection architecture 1600. FIG. 17B shows an example plot 1730 representing the range of path lengths and sampling depths of light collected by each of the measurement channels of collection architecture 1700 when the collection architecture 1700 is incorporated as the collection architecture 934 of the optical measurement system 930 of FIG. 9B. Specifically, plot 1730 includes a first group of distributions 1732a-1732c that respectively represents the ranges of possible path lengths and sampling depth combinations (with a minimum threshold probability) for light measured by the first plurality of detector elements 1704a-1704c. Plot 1730 further includes a second group of distributions 1734a-1734c corresponding to the second plurality of detector elements 1744a-1744c, and a third group of distributions 1736a-1736c corresponding to the third plurality of detector elements 1784a-1784c.

As shown there, the first, second, and third detector elements 1704a, 1704b, 1704c of the first plurality of detector elements 1704a-1704c may measure light having a first path length distribution, a second path length distribution, and a third path length distribution, respectively. The first, second, and third detector elements 1744a, 1744b, 1744c of the second plurality of detector elements 1744a-1744c may also measure light having the first path length distribution, the second path length distribution, and third path length distribution, respectively. The second plurality of detector elements 1744a-1744c, measure light that, on average, has a deeper sampling depth than light measured by the first plurality of detector elements 1704a-1704c. Similarly, the first, second, and third detector elements 1784a, 1784b, 1784c of the third plurality of detector elements 1784a-1784c may also measure light having the first path length distribution, the second path length distribution, and third path length distribution, respectively. The third plurality of detector elements 1784a-1784c measure light that, on average, has a deeper sampling depth than light measured by the second plurality of detector elements 1744a-1744c.

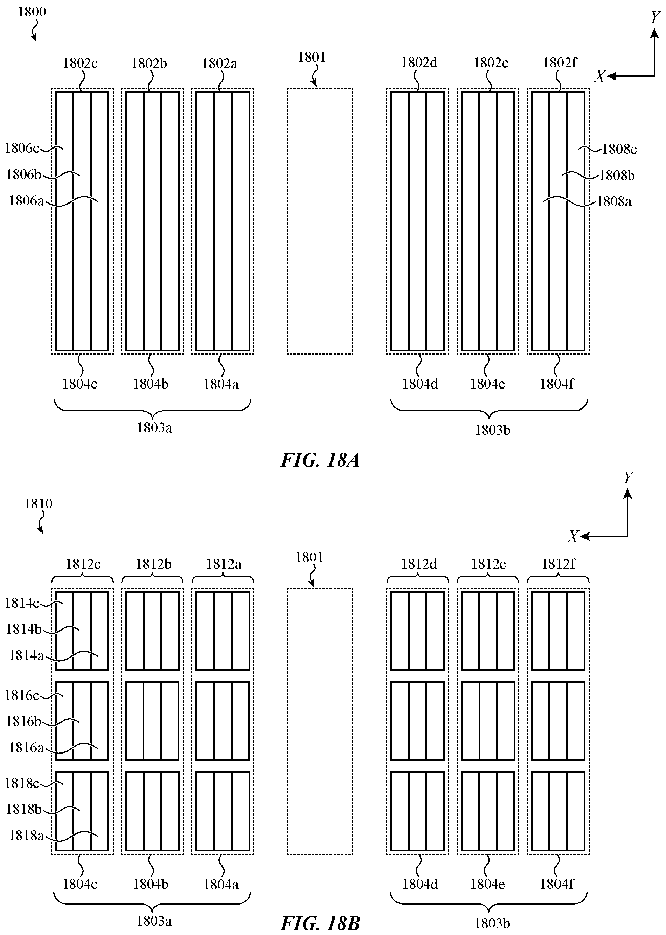

In some instances, it may be desirable for a collection architecture to collect and measure, from the same emission light beam, multiple copies of a given return light beam. Each of these copies may be aligned along a second dimension of the collection architecture, and collectively may approximate a single return light beam having a higher aspect ratio. This principle, which may be applied to any of the variations of the optical measurement systems described herein, is illustrated in FIGS. 18A-18C. FIGS. 18A-18C depict top views of different variations of collection architectures that utilize condenser lenses, such as described above with respect to the collection architectures of FIGS. 15A-17B.

For example, FIG. 18A shows a first variation of a collection architecture 1800. As shown there, the collection architecture 1800 includes two optical collection subassembly on opposite sides of a launch architecture (not shown). The launch architecture is configured to emit an emission light beam from an optical measurement system (schematically represented by rectangle 1801). As shown there, the emission light beam 1801 is longer in a second dimension (e.g., along the Y axis shown in FIG. 18A) than in a first dimension (e.g., along the X axis shown in FIG. 18B), such as described in more detail with respect to the emission light beam 304 of FIGS. 3A and 3B. The emission light beam 1801 is emitted into a measured sample (e.g., through a launch site of a sampling interface), and the collection architecture 1800 collects a portion of the light from the emission light beam 1801 that is returned to the optical measurement system. Specifically, a first collection optical subassembly 1803a collects light returned from a first side of the launch architecture (e.g., light that enters the optical measurement system via a first collection site of the sampling interface) and a second collection optical subassembly 1803b collects light returned from a second side of the launch architecture opposite the first side (e.g., light that enters the optical measurement system via a second collection site of the sampling interface).

As shown there, the first collection optical subassembly 1803a includes at least one condenser lens (e.g., a first plurality of condenser lenses 1804a-1804c), each of which is positioned to direct light to a different corresponding plurality of detector elements. The first collection optical subassembly 1803a may further include additional lens or other components (e.g., such as those described in more detail herein with respect to the collection architectures of FIGS. 15A-17B) configured to direct light from the sampling interface to the at least one condenser lens. In the variation shown FIG. 18A, a first condenser lens 1804a is positioned to direct light to each of a first plurality of detector elements 1802a, a second condenser lens 1804b is positioned to direct light to each of a second plurality of detector elements 1802b, and a third condenser lens 1804c is positioned to direct light to each of a third plurality of detector elements 1802c.

Similarly, the second collection optical subassembly 1803b includes at least one condenser lens (e.g., a second plurality of condenser lenses 1804d-1804f), each of which is positioned to direct light to a different corresponding plurality of detector elements. The second collection optical subassembly 1803b may also include additional lens or other components (e.g., such as those described in more detail herein with respect to the collection architectures of FIGS. 15A-17B) configured to direct light from the sampling interface to the at least one condenser lens. In the variation shown in FIG. 18A, a fourth condenser lens 1804d is positioned to direct light to each of a fourth plurality of detector elements 1802d, a fifth condenser lens 1804e is positioned to direct light to each of a fifth plurality of detector elements 1802e, and a sixth condenser lens 1804f is positioned to direct light to each of a sixth plurality of detector elements 1802f.

In some variations, the collection architecture 1800 is symmetrically configured around the launch architecture, such that each detector element associated with the first collection optical subassembly 1803a measures light having approximately the same path length and sampling depth distributions as a corresponding detector element associated with the first collection optical subassembly 1803a. This may allow output signals to be combined without altering the path length and sampling depth distributions associated with the output signals. For example, a first detector element 1806a of the third plurality of detector elements 1802c may measure light having approximately the same path length distribution and sampling depth distribution as light measured by a first detector element 1808a of the sixth plurality of detector elements 1802f. The output signals of these measurement channels may be combined to form a first combined output signal. Similarly, a second detector element 1806b of the third plurality of detector elements 1802c may measure light having approximately the same path length distribution and sampling depth distribution as light measured by a second detector element 1808b of the sixth plurality of detector elements 1802f. The output signals of these measurement channels may be combined to form a second combined output signal. Additionally, third detector elements 1806c, 1808c of the third and sixth pluralities of detector elements 1802c, 1802f may measure light having the path length distribution and sampling depth distribution. The output signals of these measurement channels may be combined to form a third combined output signal.

In the variation shown in FIG. 18A, each of the pluralities of detector elements 1802a-1802f is configured as a one-dimensional array of detector elements. Accordingly, to improve collection efficiency of the optical measurement system, the length of each detector element along the second dimension depends on the length of the emission light beam 1801 along the second dimension. A longer emission light beam 1801, as well as longer corresponding detector elements, may increase the amount of light that is measured by a given detector element while still maintain the path length and sampling depth distribution associated with that detector element. In practice, however, it may be difficult (e.g., from a manufacturing or yield perspective) to create detector elements having such a length or aspect ratio.

Accordingly, in some variations it may be desirable to replace each detector element of the collection architecture of FIG. 18A with multiple shorter detector elements. FIG. 18B shows one such variation of a collection architecture 1810. The collection architecture 1810 is the same as the collection architecture 1800 of FIG. 18A, except that the pluralities of detector elements 1802a-1802f is replaced with different pluralities of detector elements 1812a-1812f, each of which is configured as two-dimensional array of detector elements. In these instances, the light measured by individual detector elements may have approximately the same sampling depth and path length distributions within a column (e.g., along the second dimension) and may have different sampling depths and/or path length distributions between rows (e.g., along the second dimension).

For example, a third plurality of detector elements 1812c is positioned to receive light from the third condenser lens 1804c. The third plurality of detector elements 1812c includes a first group of detector elements 1814a-1814c, a second group of detector elements 1816a-1816c, and a third group of detector elements 1818a-1818c. A first detector element from each of these groups (e.g., detector elements 1814a, 1816a, and 1818a) may measure light having approximately the same path length distribution and sampling depth distribution, and the output signals from these measurement channels may be combined as described herein. In this way, these detector elements approximate the light received by the detector element 1806a of FIG. 18A, except that individual detector element may have a smaller length along the second dimension (and thereby, a smaller aspect ratio). Similarly, the output signals of detector elements 1814b, 1816b, and 1818b may be combined to approximate the light measured by the detector element 1806b of FIG. 18A.

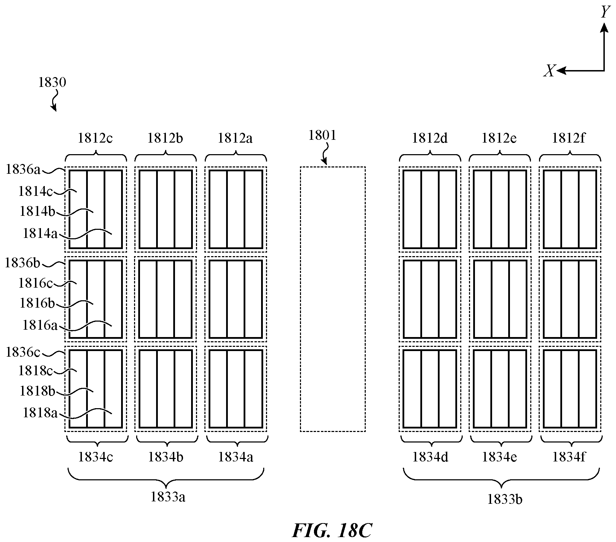

In other variations, one or more components of the collection optical subassemblies may be replicated along the second dimension. FIG. 18C shows one such variation of a collection architecture 1820. The collection architecture 1810 is the same as the collection architecture 1800 of FIG. 18B, except that the first and second collection optical subassemblies 1813a, 1813b are replaced with different first and second collection optical subassemblies 1823a, 1823b. Specifically, each of the plurality of condenser lenses 1804a-1804f is replaced with a one-dimensional array of condenser lens 1834a-1834f. For example, instead of third condenser lens 1804c of FIG. 18B, the first collection optical subassembly 1833a includes a third one-dimensional array of condenser lenses 1834c. Specifically, the one-dimensional array of condenser lenses 1834c includes a first condenser lens 1836a positioned over the first group of detector elements 1814a-1814c of the first plurality of detector elements 1812c, a second condenser lens 1836b positioned over the second group of detector elements 1816a-1816c of the first plurality of detector elements 1812c, and a third condenser lens 1836c positioned over the third group of detector elements 1818a-1818c of the first plurality of detector elements 1812c.

In this way, each of the first and second collection optical subassemblies 1833a and 1833b may include a two-dimensional array of condenser lenses. In variations where one of these collection optical subassemblies includes additional lenses or components positioned over the condenser lenses these components may or may not be replicated along the second dimension. Using the imaging lens 1606 of the collection architecture 1600 of FIGS. 16A and 16B as an example, in some variations the first collection optical subassembly 1833a may include a single imaging lens that is positioned over all of the condenser lens, and performs the function of imaging lens 1606 for every condenser lens. In other variations, multiple imaging lenses may be positioned along the second dimension, where each of these lenses performs the function of imaging lens 1606 for a different subset of the condenser lenses. For example, a first imaging lens may direct light to a first condenser lens of each of the one-dimensional arrays of condenser lenses 1834a-1834c (e.g., condenser lens 1836a), a second imaging may direct light to a second condenser lens of each of the one-dimensional arrays of condenser lenses 1834a-1834c (e.g., condenser lens 1836b), and so on. Accordingly, there is flexibility in the design of the optical measurement systems described herein to increase the effective aspect ratio of light emitted into and measured from a measured sample.

### Optical Measurement Systems with Moving Diffusers

In various embodiments of the optical measurement systems described herein, an optical measurement system may include a diffuser as part of its launch architecture. In some of these variations, the optical measurement system may be configured to move the diffuser within the optical measurement system while performing a spectroscopic measurement. Specifically, moving a diffuser, either within an individual measurement or between successive measurements, may act to reduce noise associated with measurements performed by the optical measurement system. When the light source of the light source units described herein include coherent light sources, such as lasers, measurements performed using coherent illumination may be subject to coherent noise (also referred to herein as "speckle" noise).

Specifically, the interference of coherent light as it scatters through a sample may result in spatial intensity variations of light received by a detector element. These spatial variations may reduce the signal-to-noise ratio of light measured by the various measurement channels of the optical measurement systems described herein. When the diffuser is configured to receive an input light beam (e.g., input light beam 1105 of FIGS. 11A and 11B or input light beam 1240 of FIG. 12) and generated a diverging output light beam therefrom (e.g., output light beam 1110 of FIGS. 11A and 11B or output light beam 1220 of FIG. 12), movement of the diffuser relative to the input light beam may cause the input light beam to be incident on a different portion of the diffuser. This may change the distribution of phase changes applied to the input light beam as it passes through the diffuser and generates the output light beam. The optical measurement system may be used to perform otherwise identical measurements, but measurements taken with the diffuser at different diffuser positions may have different speckle noise states (e.g., different spatial intensity variations). Accordingly, multiple measurements taken while the diffuser is at different positions may be analyzed or otherwise combined to average out some of the speckle noise. This may thereby increase the SNR of spectroscopic measurements performed by the optical measurement system.

Accordingly, any of the optical measurement systems described herein that include a diffuser may also incorporate an electromagnetic actuator arrangement that is configured to the move the diffuser relative to a stationary base. It should be appreciated that the term "stationary," when used herein in the context of an electromagnetic actuator arrangement, is intended as a relative term to indicate that positioning of a particular component that is fixed within the electromagnetic actuator arrangement. Specifically, the electromagnetic actuator arrangement may include one or more "moveable" components that may be moved relative to other "stationary" components of the electromagnetic actuator arrangement. When such an electromagnetic actuator arrangement is incorporated into an optical measurement system (or a device incorporating the optical measurement system), it should be appreciated that this arrangement (including any stationary components thereof) need not remain stationary within the overall optical measurement system or the device incorporating the optical measurement system. Indeed, the entire electromagnetic actuator arrangement may be moveable within the optical measurement system, and the electromagnetic actuator arrangement may be controllable to create relative movement between its stationary and moveable components. As a result, a given component may be stationary within the electromagnetic actuator arrangement, but may not be stationary in the context of the overall optical measurement system or device.

It should be appreciated that the electromagnetic actuator arrangements described herein may be used to move a diffuser within any of the optical measurement systems described herein, such as those described herein with respect to FIGS. 1A-18C. The electromagnetic actuator arrangements may similarly be utilized as part of any other optical measurement system in which it is desirable to actively move a diffuser. Similarly, it should also be appreciated that the optical measurement systems described herein with respect to FIGS. 1A-18C may utilize other arrangements, if so desired, to move a diffuser relative to other components of the optical measurement system.

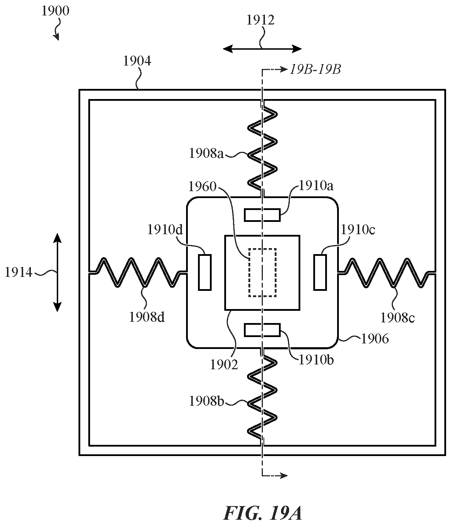

FIG. 19A shows an example of components of an electromagnetic actuator arrangement 1900 that is configured to move a diffuser 1902 relative to a stationary base 1904. To move the diffuser 1902 relative to the stationary base 1904, the electromagnetic actuator arrangement 1900 includes a carrier 1906 that is moveably connected to the stationary base 1904 via a set of suspension elements 1908a-1908d. The suspension elements 1908a-1908d, while represented in FIG. 19A by a generic spring symbol, may include one or more flexures, sheet springs, or the like that are each connected to both the stationary base 1904 and the carrier 1906. In some instances, the stationary base 1904, the carrier 1906, and the set of suspension elements 1908a-1908d may be monolithic. For example, these components may be formed from a common sheet of material (e.g., a metal), in which some of the material is removed from the sheet to define the carrier 1906 and the set of suspension elements 1908a-1908d.

The diffuser 1902 may be mounted to the carrier 1906, such that movement of the carrier 1906 relative to the stationary base 1904 also moves the diffuser 1902 relative to the stationary base 1904. To move the carrier 1906 and diffuser 1902 relative to the stationary base 1904, the electromagnetic actuator arrangement 1900 includes a set of actuators 1910a-1910d. Each of these actuators 1910a -1910d is controllable to apply a force to the carrier 1906 relative to the stationary base 1904. Depending on the collective forces applied to the carrier 1906 by the set of actuators 1910a-1910d, the carrier 1906 may move in one or more directions relative the stationary base 1904.

For example, the carrier 1906 may have a planar shape (such as when the carrier 1906 is formed from a planar sheet of material) and the set of actuators 1910a-1910d may be configured to move the carrier 1906 in multiple planar directions (e.g., within the plane defined by the carrier 1906), such as a first planar direction 1912 and a second planar direction 1914 perpendicular to the first planar direction 1912. The set of actuators 1910a-1910d may be controlled to collectively apply forces along one or both of the planar directions 1912, 1914 to move the carrier 1906 accordingly. For example, each of a first actuator 1910a and a second actuator 1910b may be controllable to apply a corresponding force to the carrier 1906 along the second planar direction 1914, and thereby control movement of the carrier 1906 along the second planar direction 1914. Similarly, each of a third actuator 1910c and a fourth actuator 1910d may be controllable to apply a force to the carrier 1906 along the first planar direction 1912, and thereby control movement of the carrier 1906 along the first planar direction 1912. Collectively, the set of actuators 1910a-1910d may selectively move carrier 1906 in both planar directions 1912, 1914 simultaneously.

Each of the set of actuators 1910a-1910d may be any actuator capable of applying a force to the carrier 1906 relative to the stationary base 1904. For example, the set of actuators 1910a-1910d may each be configured as a voice coil motor (VCM) actuator that includes a magnet and a coil. Either the magnet or coil is connected to (and moveable with) the carrier 1906, and the other of which is stationary with respect to the electromagnetic actuator arrangement 1900. The magnet and coil are positioned sufficiently close to each other such that the magnet and coil generate a Lorentz force when current is driven through the coil. This Lorentz force is applied to the carrier 1906 relative to the stationary base 1904 to assist in moving the carrier 1906.

It should be appreciated that different actuators may have different coils that interact with a common magnet, or vice versa. Additionally, various actuators within the set of actuators 1910a-1910d may be individually or jointly controlled. For example, in some variations the first and second actuators 1910a, 1910b may be jointly controlled (e.g., the coils from these actuators receive a common voltage or current), and the third and fourth actuators 1910c, 1910d may also be jointly controlled. In these variations, the first and second actuators 1910a, 1910b may be independently controlled relative to the third and fourth actuators 1910c, 1910d, which allows for independent control over movement along the second planar direction 1914 and the first planar direction 1912, respectively.

The suspension elements 1908a-1908d may be configured to facilitate movement of the carrier 1906 in certain directions while limiting motion of the carrier 1906 in other directions. For example, certain suspension elements 1908a-1908d may be configured with higher stiffness in certain directions, which may limit the directions in which carrier 1906 may be moved relative to the stationary base 1904. For example, in the electromagnetic actuator arrangement 1900 shown in FIG. 19A, the set of suspension elements 1908a-1908d may have relatively lower stiffness in the first and second planar directions 1912, 1914, but have relatively higher stiffness in an out-of-plane direction perpendicular to the first and second planar directions 1912, 1914. In these instances, the set of suspension elements 1908a-1908d may be more resistant to out-of-plane movement, thereby restricting unintentional movement in the out-of-plane direction.

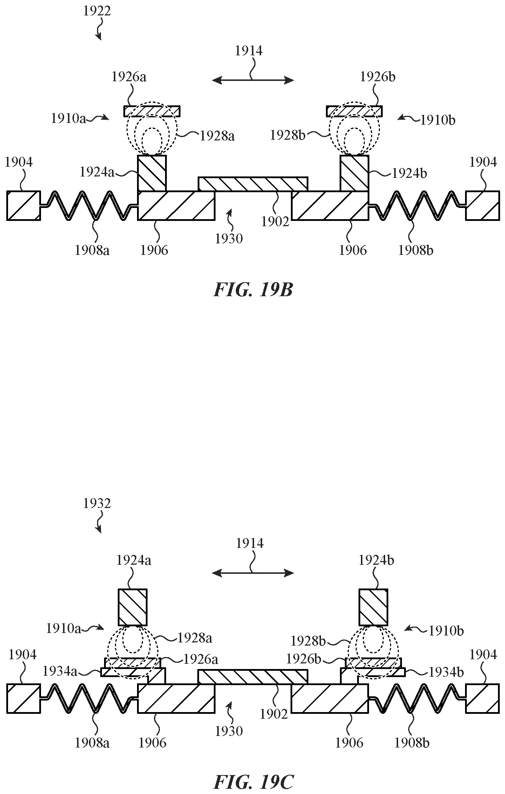

FIGS. 19B and 19C show cross-sectional side views (taken along line 19B-19B) of variations of the electromagnetic actuator arrangement 1900 of FIG. 19A. Specifically FIG. 19B shows a first variation of an electromagnetic actuator arrangement 1922 in which the first actuator 1910a and a second actuator 1910b each include a magnet and a coil. Specifically, the first actuator 1910a has a first magnet 1924a and a first coil 1926a and the second actuator 1910b has a second magnet 1924b and a second coil 1926b. In the variation shown in FIG. 19B, both the first magnet 1924a and the second magnet 1924b are mounted to the carrier 1906 (thereby mounting the first and second actuators 1910a, 1910b to the carrier 1906). The first coil 1926a and the second coil 1926b are mounted to a stationary component within the electromagnetic actuator arrangement 1922 (e.g., to a mounting structure that is fixed relative to the stationary base 1904) such that first coil 1926a is positioned within a magnetic field 1928a of the first magnet 1924a and the second coil 1926b is positioned within a magnetic field 1928b of the second magnet 1924b. When current is run through either or both of the coils 1926a, 1926b, a corresponding Lorentz force will be generated along the second direction 1914.

While the first and second magnets 1924a, 1924b are shown in FIG. 19B as being mounted to the carrier 1906, in other variations one or both of the first and second coils 1926a, 1926b may instead be mounted to the carrier 1906. For example, FIG. 19C shows a second variation of an electromagnetic actuator assembly 1932 that is configured as described with respect to the electromagnetic actuator arrangement 1900 of FIG. 19A. In this variation, the first and second actuators 1910a, 1910b may be the same as the variation shown in FIG. 19B, except that the first and second coils 1926a, 1926b are instead mounted to the carrier 1906, and the first and second magnets 1924a, 1924b are mounted to a stationary component within the electromagnetic actuator assembly 1932.

In instances where a coil of an actuator is mounted to a carrier, the electromagnetic actuators described herein may be configured to route current to the coil to facilitate operation of the actuator. In some variations, one or more electrical traces may be carried along the carrier 1906. For example, to power a coil mounted to the carrier 1906, one or more electrical traces may be carried by the stationary base 1904, along one or more of the first set of suspension elements 1908a-1908d, and to the carrier 1906. In other variations, flexible cables or sheets (such as a ribbon cable or the like) may provide electrical connections to one or both of the carriers.

In some variations, a coil or magnet may be mounted directly to a carrier (such as the first and second magnets 1924a, 1924b in the variation of the launch architecture 222 shown in FIG. 19B). In other variations, the coil or magnet may be mounted indirectly to a carrier via an intervening structure. For example, in FIG. 19C, the electromagnetic actuator assembly 1932 may include one or more mounting structures (e.g., a first mounting structure 1934a connecting the first coil 1926a to the carrier 1906 and a second mounting structure 1934b connecting the second coil 1926b to the carrier 1906). These mounting structures may allow for a physical connection between the carrier 1906 and an actuator (and thus allowing the actuator to apply moving forces to the carrier), while providing flexibility in determining the relative sizes of the actuator and the carrier 1906.

For example, a mounting structure may extend laterally (e.g., along the first and/or second directions 1912, 1914) past a peripheral edge of the carrier 1906, which may allow the portion of the actuator mounted to the carrier to also extend laterally past the peripheral edge of the carrier 1906. In the variation shown in FIG. 19C, the first coil 1926a is positioned such that extends laterally past a peripheral edge of the carrier 1906, such that at least a portion of the first coil 1926a is suspended above a portion of suspension element 1908a. In these instances, it may not be necessary for a surface (e.g., a top surface or a bottom surface) of the carrier 1906 to be sized to accommodate the entire first coil 1926a, so long as the carrier 1906 has sufficient space to connect to the first mounting structure 234a. Accordingly, the size of the carrier 1906 may be reduced as compared to instances in which one or more actuators are mounted directly to the carrier 1906.

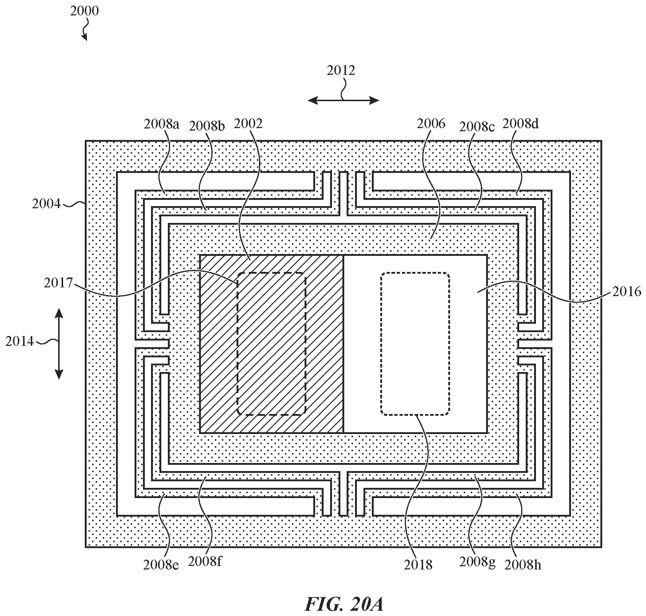

In some variations, such as shown in FIGS. 19B and 19C, the diffuser 1902 is positioned over (or at least partially within) an aperture 1930 defined to extend through the carrier 1906. Specifically the diffuser 1902 is positioned to at least partially block the aperture 1930, such that light passing through the diffuser 1902 will also pass through the aperture 1930. In some variations where the carrier 1906 also includes one or more carrier apertures, such as described herein with respect to FIGS. 20A-20E, some or all of these carrier apertures may be formed from a portion of the aperture 1930 that is not otherwise blocked by the diffuser 1902.

Depending on the particular configuration of the optical measurement systems described herein, there may be a relatively small lateral separation between light exiting and re-entering the sampling interface. For example, the lateral separation between an emission light beam as it enters a sample and a return light beam as it is collected from a sample may be on the order of millimeters, or in some cases even less than a millimeter. In these instances it may be difficult to incorporate an electromagnetic actuator arrangement, such as those described with respect to FIGS. 19A-19C and 20A-20E, without components of the electromagnetic actuator arrangement (e.g., one or more portions of a carrier, a suspension element, an actuator, or the like) potentially blocking or otherwise interfering with light collected by the optical measurement system.

Accordingly, in some variations, an electromagnetic actuator arrangement may define one or more carrier apertures extending therethrough. Each carrier aperture may be positioned such that light measured by one or more measurement channels first passes through the carrier aperture. In this way, light may be collected by a collection architecture, such as described herein, such that one or more return light beam entering the optical measurement site through a collection site passes through a carrier aperture of the carrier before being measured by one or more corresponding detector elements. Accordingly, the carrier may still generate movement of the diffuser without the electromagnetic actuator arrangement interfering with collection of light by the optical measurement system.

FIGS. 20A-20E show top views of example variations of electromagnetic actuator arrangements as described herein. FIG. 20A shows a top view of a first variation of an electromagnetic actuator arrangement 2000 that includes a carrier 2006 that is configured to move a diffuser 2002 relative to a stationary base 2004. The carrier 2006 is movably connected to the stationary base 2004 by a set of suspension elements 2008a-2008h. The diffuser 2002 is carried by (e.g., mounted to, formed on, or otherwise connected to) to the carrier 2006, such that the diffuser 2002 moves with the carrier 2006. The electromagnetic actuator arrangement 2000 further includes a set of actuators (not shown), such as the set of actuators 1910a-1910d described with respect to FIGS. 19A-19C, that is mounted to the carrier 2006 and controls relative movement between carrier 2006 and the stationary base 2004. The set of actuators may be configured to move the carrier 2006 along a single direction (e.g., either a first planar direction 2012 or a second planar direction 2014 as shown in FIG. 20A), or may be configured to move the carrier 2006 in multiple different planar directions (e.g., both the first planar direction 2012 and the second planar direction 2014). A controller (e.g., controller 160 of the optical measurement system 102 of FIGS. 1A and 1B) may be operatively coupled to the set of actuators to direct movement of the carrier 2006.

In the variation shown in FIG. 20A, the carrier 2006 at least partially defines a carrier aperture 2016 that extends through the carrier 2006. In some variations, the diffuser 2002 may also at least partially define the carrier aperture 2016 (e.g., a side of the diffuser 2002 may define a corresponding side of the carrier aperture 2016). When the electromagnetic actuator arrangement 2000 is incorporated into an optical measurement system as described herein, the electromagnetic actuator arrangement 2000 may be positioned such that the diffuser 2002 receives an input light beam 2017 as part of a launch architecture of the optical measurement system. Similarly, the carrier aperture 2016 may be positioned such that at least one light beam 2018, which is collected by a collection architecture of the optical measurement system, passes through the carrier aperture 2016.

For example, the electromagnetic actuator arrangement 2000 may be incorporated into a variation of the optical measurement system 900 shown in FIG. 9A. In these instances, the beam generator 920 may incorporate the diffuser 2002 (e.g., such as described herein with respect to FIGS. 11A-13F), such that light passing through the diffuser 2002 forms the output light beam 922. In this way, the electromagnetic actuator arrangement 2000 may be positioned such that the launch optical subassembly 924 is positioned between the electromagnetic actuator arrangement 2000 and the sampling interface 907.

Additionally, the electromagnetic actuator arrangement 2000 may be positioned such that light collected through the collection site 910 will pass through the carrier aperture 2016. Depending on the configuration and placement of the collection optical subassembly 926 (e.g., its relative placement to the beam generator 920), the light beam 2018 passing through the carrier aperture 2016 may be any portion of the light traveling between the collection site 910 and the detector element 906. For example, in some variations the carrier 2006 may be positioned between the collection optical subassembly 926 and the collection site 910, such that the return light beam 912 passes through the carrier aperture 2016. In other variations, the carrier 2006 may be positioned between the collection optical subassembly 926 and the detector element 906, such that the first measurement light beam 928 passes through the carrier aperture 2016. In still other variations, the carrier 2006 may be positioned to at least partially surround a portion of the collection optical subassembly 926. For example, the carrier 2006 may be positioned such that one or more lenses of the collection optical subassembly 926 are at least partially positioned within the carrier aperture 2016. Additionally or alternatively, the carrier 2006 may be positioned between two or more lenses of the collection optical subassembly 926. Overall, the carrier aperture 2016 may be sized such that, as the diffuser 2002 is moved between the different diffuser positions during operation of the optical measurement system 900, the carrier 2006 will not contact the components of the collection architecture 904 or otherwise interfere with collection of light by the collection architecture 904.

In some variations, the stationary base 2004, the carrier 2006, and the set of suspension elements 2008a-2008h may be monolithic. For example, these components may be formed from a common sheet of material (e.g., a metal), in which some of the material is removed from the sheet to define the stationary base 2004, carrier 2006 and the set of suspension elements 2008a-2008h. Accordingly, in these instances the set of suspension elements 2008a-2008h is configured as a set of flexures formed from corresponding portions of the sheet. The flexures may have relatively lower stiffness in the movement direction (or directions) of the carrier 2006 (and thereby the movement directions of the diffuser 2002) to facilitate this movement. Similarly, the flexures may have relatively higher stiffness in other directions (e.g., in an out-of-plane direction perpendicular to the first and second planar directions 2012, 2014) to limit unintentional movement of the carrier 2006 in these directions.

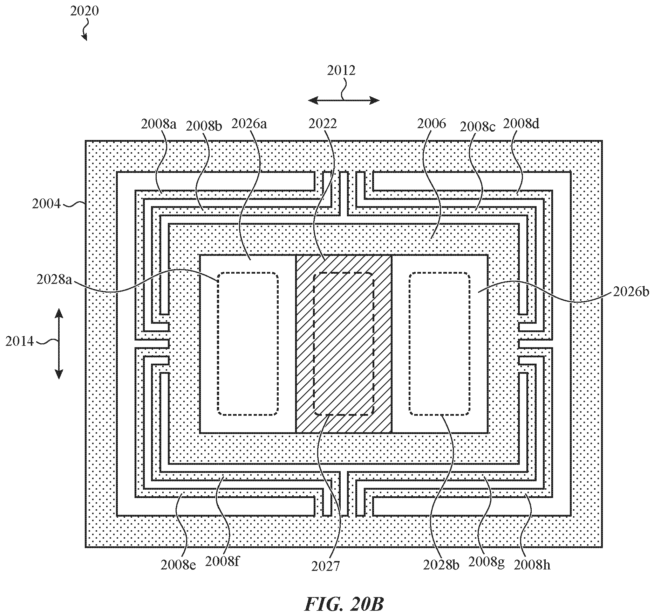

The electromagnetic actuator arrangement 2000 may, in other instances, be similarly incorporated into the optical measurement systems 700 and 930 described with respect to FIGS. 7 and 9B. In variations where an optical measurement system as described herein is configured to collect light through multiple collection sites to measure light returned from a single emission light beam, it may be desirable to configure the carrier 2006 to include multiple carrier apertures extending through the carrier 2006. FIG. 20B shows a top view of one such variation of an electromagnetic actuator arrangement 2020 that includes a stationary base 2004, a carrier 2006, a set of suspension elements 2008a-2008h, and a set of actuators (not shown) configured to move the carrier 2006 relative to the stationary base 2004. These components may be configured the same as described with respect to the electromagnetic actuator arrangement 2000 of FIG. 20A, except that the carrier 2006 is configured to carry a diffuser 2022 that is positioned between a first carrier aperture 2026a and a second carrier aperture 2026b. Each of the first carrier aperture 2026a and the second carrier aperture 2026b are defined to extend through different portions of the carrier 2006. Accordingly, the carrier 2006 may at least partially define the first carrier aperture 2026a and the second carrier aperture 2026b. In some instances, the diffuser 2022 may also at least partially define one or both of the first and second carrier apertures 2026a, 2026b (e.g., a first side of the diffuser 2022 may define a corresponding side of the first carrier aperture 2026a and an opposite second side of the diffuser 2022 may define a corresponding side of the second carrier aperture 2026b .

When the electromagnetic actuator arrangement 2020 is incorporated into an optical measurement system as described herein, the electromagnetic actuator arrangement 2020 may be positioned such that the diffuser 2022 receives an input light beam 2027 as part of a launch architecture of the optical measurement system. The first carrier aperture 2026a and the second carrier apertures 2026b may be accordingly positioned such that light collected by a collection architecture of the optical measurement system will pass through the first and second carrier apertures 2026a, 2026b. Specifically, a first set of light beams (represented by first light beam 2028a) collected from a first collection site will pass through the first carrier aperture 2026a, while a second set of light beams (represented by second light beam 2028b) collected from a second collection site will pass through the second carrier aperture 2026b. In this way, light may be directed through the diffuser 2022 to assist in generating an emission light beam as described herein, and the diffuser 2022 may be moved between different positions as part of a spectroscopic measurement (e.g., to generate different speckle noise states).

For example, in some instances the electromagnetic actuator arrangement 2020 may be incorporated into a variation of the optical measurement system 960 shown in FIGS. 9C and 9D. In these instances, the beam generator 980 may incorporate the diffuser 2022 (e.g., such as described herein with respect to FIGS. 11A-13F), such that light passing through the diffuser 2022 forms the output light beam 982. In this way, the electromagnetic actuator arrangement 2020 may be positioned such that the launch optical subassembly 984 is positioned between the electromagnetic actuator arrangement 2020 and the sampling interface 907 (e.g., the launch site 908).

Additionally, the electromagnetic actuator arrangement 2020 may be positioned such that light collected through the first collection site 910a will pass through the first carrier aperture 2026a. Depending on the configuration and placement of the first collection optical subassembly 986a (e.g., its relative placement to the beam generator 980), the first set of light beams 2028a passing through the first carrier aperture 2026a may be any portion of the light traveling between the first collection site 910a and the first set of detector elements 966a-966c, such as described in more detail with respect to FIG 20A. In some instances one or more lenses or other components of the first collection optical subassembly 986a may be at least partially positioned within the first carrier aperture 2026a. For example, in some variations the first relay lens 985a may be positioned at least partially within the first carrier aperture 2026a.

Similarly, the electromagnetic actuator arrangement 2020 may be positioned such that light collected through the second collection site 910b will pass through the second carrier aperture 2026b. Depending on the configuration and placement of the second collection optical subassembly 986b, the second set of light beams 2028b passing through the second carrier aperture 2026b may be any portion of the light traveling between the second collection site 910b and the second set of detector elements 966d-966f, such as described in more detail with respect to FIG 20A. In some instances where one or more lenses or other components of the first collection optical subassembly 986a are positioned at least partially within the first carrier aperture 2026a, a similar set of components of the second collection assembly 986b may also be positioned at least partially within the second carrier aperture 2026b. For example, in some variations, the second relay lens 985b may be at least partially positioned with the second carrier aperture 2026b, such that the first and second relay lenses 985a, 986b are each at least partially positioned within a respective carrier aperture.

In other instances, the optical measurement systems described herein may be divided into multiple measurement subsystems, each of which is configured to i) emit an emission light beam into a sample and ii) collect and measure a portion of the emission light beam that is returned from the sample (e.g., one or more return light beams each measured by a corresponding set of detector elements). Indeed, each individual measurement subsystem may be configured in any manner as described above with respect to the optical measurement systems of FIGS. 1A-18C. In an illustrative example, an optical measurement system may include a first measurement subsystem and a second subsystem, each of which includes the various components of the optical measurement system 960 of FIGS. 9C and 9D. The first measurement subsystem and second measurement subsystem may each include a corresponding launch architecture configured the same as launch architecture 962 of optical measurement system 960. Accordingly, each measurement subsystem is operable to generate a corresponding emission light beam. Similarly, first measurement subsystem and second measurement subsystem may each include a corresponding a collection architecture that is configured the same as the collection architecture 964 of optical measurement system 960.

The measurement subsystems may be controlled independently or may controlled together. Each measurement subsystem may be positioned at a different location within the optical measurement system, and thus may measure a different region of a given sample. The measurement subsystems may be sufficiently spaced from each other such that light emitting into a measured sample by one measurement subsystem will not be measured by the measurement channels of another measurement subsystem. In this way, multiple measurement subsystems may be operated simultaneously without light from one measurement subsystem impacting the measurements of the other measurement subsystems. Measuring different regions of a sample with different measurement subsystems may facilitate faster and/or more accurate spectroscopic measurements.

For example, a particular region of a measured sample may have an unexpected local heterogeneity that causes one or more sample characteristics in that region to significantly deviate beyond what is expected for the measured sample. If a first measurement subsystem measures light that has traversed through this region, the unexpected change in sampling characteristics may impact the accuracy of measurements taken by the first measurement subsystem. Conversely, the second measurement subsystem may measure light that has traversed through another region of the sample, and the accuracy of these measurements may not be impacted by the unexpected heterogeneity. Accordingly, using multiple measurement subsystems may reduce the impact that localized sample heterogeneities will have on the accuracy of the overall spectroscopic measurement.

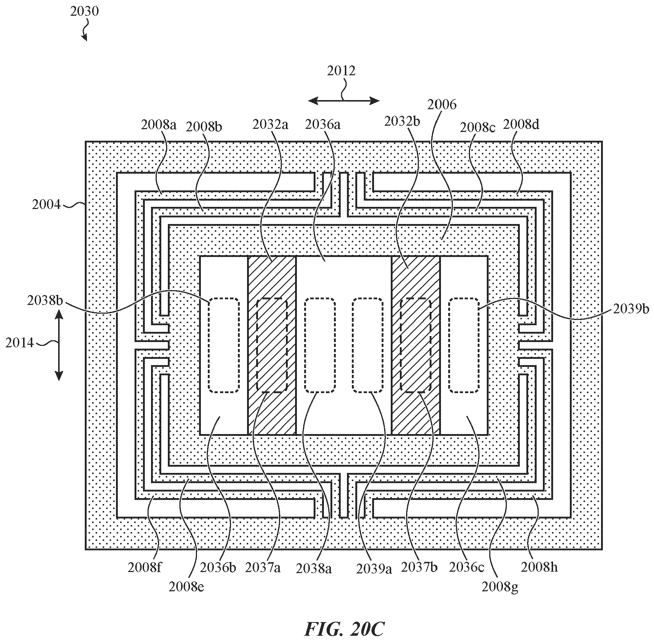

When an optical measurement system as described herein utilizes multiple measurement subsystems, it should be appreciated that different measurement subsystems may share certain components, such as an electromagnetic actuator arrangement as described herein. For example, FIG. 20C shows a top view of one such variation of an electromagnetic actuator arrangement 2030 that is configured to be utilized by multiple measurement subsystems. Specifically, the electromagnetic actuator arrangement 2030 includes a stationary base 2004, a carrier 2006, a set of suspension elements 2008a-2008h, and a set of actuators (not shown) configured to move the carrier 2006 relative to the stationary base 2004. These components may be configured the same as the electromagnetic actuator arrangement 2000 of FIG. 20A, except that the carrier 2006 is configured to carry a first diffuser 2032a and a second diffuser 2032b and defines a set of carrier apertures 2036a-2036c that extend through the carrier 2006.

The first diffuser 2032a may form a portion of a beam generator of a first measurement subsystem. Specifically, the first diffuser 2032a is positioned to receive a first input light beam 2037a as part of a launch architecture of the first measurement subsystem. The first diffuser 2032a may generate a first diverging output beam from the input light beam 2037a. This diverging output beam may be used to generate a first emission light beam that is emitted by the optical measurement system when the first measurement subsystem is used to perform a measurement. Similarly, the second diffuser 2032b may form a portion of a beam generator of a second measurement subsystem. Specifically, the second diffuser 2032b is positioned to receive a second input light beam 2037b as part of a launch architecture of the second measurement subsystem. The second diffuser 2032b may generate a second diverging output beam from the second input light beam 2037b. This diverging output beam may be used to generate a second emission light beam that is emitted by the optical measurement system when the second measurement subsystem is used to perform a measurement.

The set of carrier apertures 2036a-2036c may each be positioned such that light received by one or more of the measurement subsystems passes the carrier 2006 via that carrier aperture. For example, in the variation shown in FIG. 20C, the set of carrier apertures 2036a-2036c includes a plurality of carrier apertures (e.g., a first carrier aperture 2036a, a second carrier apertures 2036b, and a third carrier apertures 2036c). In this example, the first carrier aperture 2036a is positioned between first diffuser 2032a and the second diffuser 2032b. The first diffuser 2032a is positioned between the first carrier aperture 2036a and the second carrier aperture 2036b, whereas the second diffuser 2032b is positioned between the first carrier aperture 2036a and the third carrier apertures 2036c.

The number and relative position of the set of carrier apertures 2036a-2036c may depend at least in part on the configuration of the first and second measurement subsystems. For example, the first and second measurement subsystems may each be configured to collect light through multiple collection sites to measure light returned from a corresponding emission light beam, such as described herein with respect to the optical measurement system 960 of FIGS. 9C and 9D. For example, the first measurement subsystem may be configured to collect a first set of light beams (represented by light beam 2038a) through a first collection site of a sampling interface (not shown) and collect a second set of light beams (represented by light beam 2038b) through a second collection site of the sampling interface. The first measurement subsystem may include a first set of detector elements positioned to measure the first set of light beams 2038a and a second set of detector elements positioned to measure the second set of light beams 2038b.

Similarly, the second measurement subsystem may be configured to collect a third set of light beams (represented by light beam 2039a) through a third collection site of the sampling interface and collect a fourth set of light beams (represented by light beam 2039b) through a fourth collection site. The second measurement subsystem may include a third set of detector elements positioned to measure the third set of light beams 2039a and a fourth set of detector elements positioned to measure the fourth set of light beams 2039b.

When the electromagnetic actuator arrangement 2030 is incorporated into such an optical measurement system, the first carrier aperture 2036a may be positioned such that the first set of light beams 2038a and the third set of light beams 2039a each pass through the first carrier aperture 2036a before they are measured by the first and third sets of detector elements, respectively. In this way, at least some of light measured by the first measurement subsystem and at least some of the light measured by the second measurement subsystem will pass through the same carrier aperture. Additionally, the second carrier aperture 2036b may be positioned such that second set of light beams 2038b will pass through the second carrier aperture 2036b before being measured by the second set of detector elements. Similarly, the third carrier aperture 2036c may be positioned such that the fourth set of light beams 2039b will pass through the third carrier aperture 2036c before reaching the fourth set of detector elements.

While shown in FIG. 20C as having three carrier apertures, the set of carrier apertures 2036a-2036c may include any suitable number of carrier apertures as may be desired. For example, if the first and second measurement subsystems are each configured to collect a single set of light beams through a single collection site, one or more of the set of carrier apertures 2036a-2036c shown in FIG. 20C may be omitted. For example, if the first measurement subsystem is configured to only collect the first set of light beams 2038a (e.g., and not the second set of light beams 203 8b) and the second measurement subsystem is configured to collect the third set of light beams 2039a (e.g., and not the fourth set of light beams 2039b), the set of carrier apertures 2036a-2036c may include a single carrier aperture. Accordingly, the carrier 2006 may include only the first carrier aperture 2036a positioned between the first diffuser 2032a and second diffuser 2032b (e.g., and not the second carrier aperture 2036b or the third carrier aperture 2036c). In this way, the first and third set of light beams 2038a, 2039a may be collected through the first carrier aperture 2036a.

In another example, the first measurement subsystem may be configured to collect the second set of light beams 2038b (in addition to or as an alternative to the first set of light beams 2038a). In such an example, the set of carrier apertures 2036a-2036c may include only the first carrier aperture 2036a and the second carrier aperture 2036b (e.g., and not the third carrier aperture 2036c). Accordingly, the second set of light beams 2038b may be collected through the second carrier aperture 2036b, and the third set of light beams 2039a may be collected through the first carrier aperture 2036a.

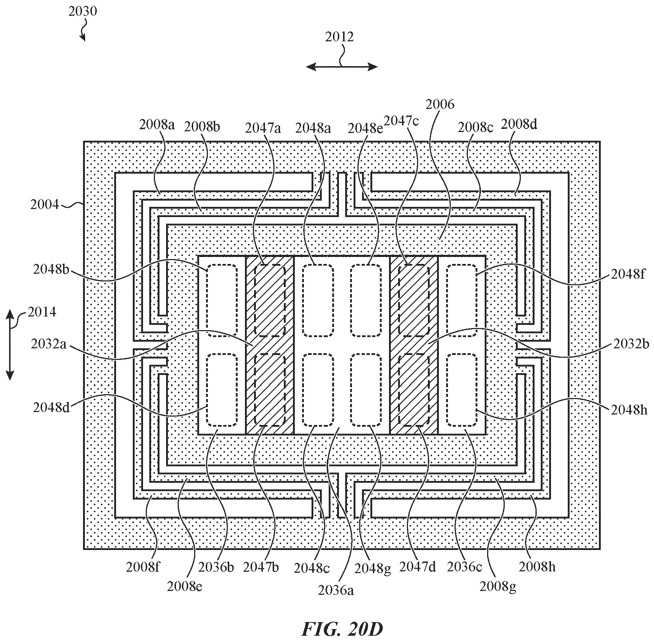

While the first diffuser 2032a and second diffuser 2032b are shown in FIG. 20C as each receiving a single input light beam, it should be appreciated that in other instances a single diffuser may receive input light beams from multiple measurement subsystems. For example, FIG. 20D shows an example in which the electromagnetic actuator arrangement 2030 of FIG. 20C is incorporated into an optical measurement system having four measurement subsystems. As shown there, the first diffuser 2032a is positioned to receive a first input light beam 2047a from a first measurement subsystem and a second input light beam 2047b from a second measurement subsystem. The second diffuser 2032b is positioned to receive a third input light beam 2047c from a third measurement subsystem and a fourth input light beam 2047d from a fourth measurement subsystem.

In the variation shown in FIG. 20D, each of the four measurement subsystems is configured to collect light from multiple collection sites to measure light returned from a corresponding emission light beam (e.g., generated from a respective input light beam of the input light beams 2047a-2047d). Accordingly, each measurement subsystem is configured to collect and measure two sets of light beams, each of which is collected from a different collection site of a sampling interface (not shown) of the optical measurement system. For example, the first measurement subsystem collects a first set of light beams 2048a that passes through the first carrier aperture 2036a and collects a second set of light beams 2048b that passes through the second carrier aperture 2036b. The second measurement subsystem collects a third set of light beams 2048c that passes through the first carrier aperture 2036a and collects a fourth set of light beams 2048d that passes through the second carrier aperture 2036b. The third measurement subsystem collects a fifth set of light beams 2048e that passes through the first carrier aperture 2036a and collects a sixth set of light beams 2048f that passes through the third carrier aperture 2036c. The fourth measurement subsystem collects a seventh set of light beams 2048g that passes through the first carrier aperture 2036a and collects an eight set of light beams 2048h that passes through the third carrier aperture 2036c. In such an example, each of the measurement subsystems collects a corresponding set of light beams that passes through first carrier aperture 2036a.

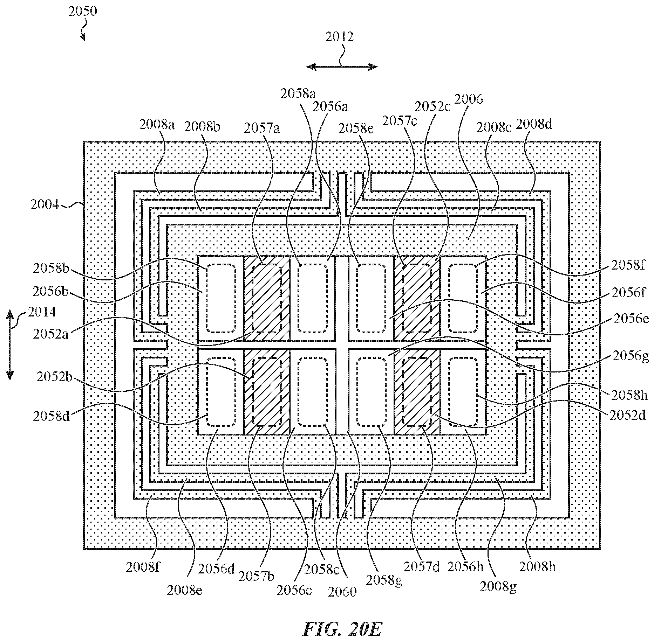

In still other instances, the electromagnetic actuator arrangements described herein may be configured such that any light passing through the electromagnetic actuator arrangement (through a diffuser or a carrier aperture) from one measurement subsystem is optically isolated from light passing the electromagnetic actuator arrangement from other measurement subsystems. For example, FIG. 20E shows a variation of an electromagnetic actuator arrangement 2050. The electromagnetic actuator arrangement 2050 includes a stationary base 2004, a carrier 2006, a set of suspension elements 2008a-2008h, and a set of actuators (not shown) configured to move the carrier 2006 relative to the stationary base 2004. These components may be configured the same as the electromagnetic actuator arrangement 2000 of FIG. 20A, except that the electromagnetic actuator arrangement 2050 includes multiple optically isolated diffuser arrangements.

Each diffuser arrangement includes a diffuser and at least one carrier aperture defined to extend through the carrier 2006, and may be associated with a different corresponding measurement subsystem. Accordingly, the carrier 2006 may carry a plurality of diffusers 2052a-2052d, each of which may be used to generate a diverging output beam for a corresponding measurement subsystem. For example, a first diffuser arrangement may include a first diffuser 2052a that is positioned between a first carrier aperture 2056a and a second carrier aperture 2056b. The first diffuser arrangement may be associated with a first measurement subsystem such that the first diffuser 2052a receives a first input light beam 2057a from the first measurement system. The first measurement subsystem may collect a first set of light beams 2058a through the first carrier aperture 2056a and a second set of light beams 2058b through the second carrier aperture 2056b. Similarly, the second diffuser arrangement may include a second diffuser 2052b that is positioned between a third carrier aperture 2056c and a fourth carrier aperture 2056d. The second diffuser arrangement may be associated with a second measurement subsystem such that the second diffuser 2052b receives a second input light beam 2057b from the second measurement system. The second measurement subsystem may collect a third set of light beams 2058c through the third carrier aperture 2056c and a fourth set of light beams 2058d through the fourth carrier aperture 2056d.

The first and second diffuser arrangements may be separated by an optical barrier 2060 positioned between the first and second diffuser arrangements. The optical barrier 2060 may be formed from a material that is opaque at the wavelength or wavelengths of light generated by the optical measurement system. In some instances, the optical barrier 2060 may be formed as a monolithic piece with the carrier 2006, such as when the carrier 2006, the suspension elements 2008a-2008h, and the stationary base 2004 are formed from a common sheet of material. In other instances the optical barrier 2060 may formed separately from and attached to the carrier 2006. The optical barrier 2060 may provide optical isolation between the first and second diffuser arrangements, which may limit crosstalk between adjacent measurement subsystems.

The carrier 2006 may further include a third diffuser arrangement (e.g., having a third diffuser 2052c positioned between a fifth carrier aperture 2056e and a sixth carrier aperture 2056f) that is associated with a third measurement subsystem. The third measurement subsystem may direct a third input light beam 2057c toward the third diffuser 2052c, and may collect a fifth set of light beams 2058e and a sixth set of light beams 2058f through the fifth carrier aperture 2056e and the sixth carrier aperture 2056f. The optical barrier 2060 may separate the third diffuser arrangement from any or all of the other diffuser arrangements (e.g., the first and/or second diffuser arrangements).

Similarly, the carrier 2006 may further include a fourth diffuser arrangement (e.g., having a fourth diffuser 2052d positioned between a seventh carrier aperture 2056g and an eighth carrier aperture 2056h) that is associated with a fourth measurement subsystem. The fourth measurement subsystem may direct a fourth input light beam 2057d toward the fourth diffuser 2052d, and may collect a seventh set of light beams 2058g and a sixth set of light beams 2058h through the seventh carrier aperture 2056g and the eighth carrier aperture 2056h. The optical barrier 2060 may separate the fourth diffuser arrangement from any or all of the other diffuser arrangements (e.g., the first, second and/or third diffuser arrangements). Depending on the configuration of the optical barrier 2060, one or more portions of the barrier may be oriented along a first planar direction 2012. Additionally or alternatively, one or more portions of the barrier may be oriented along a second planar direction 2014.

### Polarizing Beamsplitters and Optical Measurement Systems Incorporating the Same

Also described herein are polarizing beamsplitters. In some instances, it may be desirable to split a light beam into multiple separate light beams. In this way, the individual light beams may be used for different purposes with an optical measurement system, such as the optical measurement systems described herein. For example, in the variations of the launch architectures described herein with respect to 13B-13F, the optical unit 1316 includes a beamsplitter 1332 that is configured to split off a portion of the input light beam 1324 as a reference light beam 1334. By measuring at least a portion of the reference light beam 1334 with a reference detector 1336, the optical measurement systems described herein may determine the intensity of the reference light beam 1334. This intensity may also indicate a corresponding intensity of the input light beam 1324, and thus may be used to determine the intensity of an emission light beam that is introduced into a sample.

Accordingly, it may be desirable to configure a beamsplitter to have a particular sampling ratio (e.g., the relative percentage of light that is split off of the light beam). For example, if a beamsplitter splits an incoming light beam with a 5% sampling ratio (and assuming no optical losses occur as part of the beam splitting), the beamsplitter will split the incoming light beam into a first split light beam with 95% of the intensity of the incoming light beam and a second split beam with 5% of the intensity of the incoming light beam. In this way, the relative intensity between the first split light beam and the second split light beam may be known. In the instance of a 5% sampling ratio, there will be a 19:1 ratio between the intensities of the first and second split light beams. The design of a beamsplitter may be selected to achieve a desired sampling ratio depending on the needs of the optical measurement system.

Additionally, in some instances it may be desirable to polarize a beam of light. For example, any of the optical measurement systems described herein may be configured to emit polarized light into a measured sample. Depending on the nature and type of the sample being measured, there may be polarization-dependent behavior of light as it interacts with a measured sample. In these instances, illuminating a sample with light having a predetermined polarization state may help to improve the accuracy of spectroscopic measurement performed by an optical measurement system. Accordingly, in some variations of the optical measurement systems described herein, the optical measurement system (or one or more measurement subsystems thereof) may include a launch architecture the generates a polarized emission light beam. In this way, the emission light beam may have a predetermined polarization state as the emission light beam exits a sampling interface of the optical measurement system.

When a launch architecture of an optical measurement system is configured to both i) split off a reference light beam and ii) generate a polarized emission light beam, it may be desirable for the polarization to occur before collecting the reference light beam. If a polarizer were to polarize the emission light beam after the reference light beam has already been collected, imperfections in the polarizer and/or changes to the operation of the polarizer over time (e.g., that may occur with aging and/or changes in temperature) may alter the relative intensity between the reference light beam and the emission light beam. Accordingly, measurements performed by a reference detector (e.g., reference detector 1336 of FIGS. 13B-13F) are less likely to accurately reflect the intensity of light being introduced into the measured sample, which may reduce the accuracy of spectroscopic measurements performed by optical measurement system.

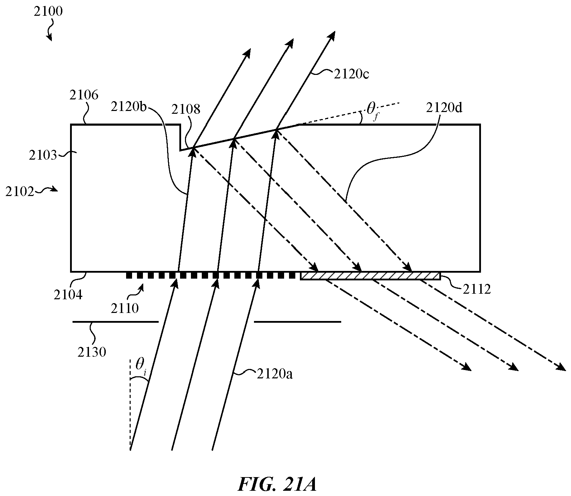

Conversely, it may also be desirable for polarization of the emission light beam to occur as close as possible to the sampling interface, which may reduce the likelihood that other components of the optical measurement system unintentionally alter the polarization of the emission light beam. Accordingly, a beamsplitter that also functions as a polarizer may balance these interests. FIGS. 21A-26 illustrate different variations of polarizing beamsplitters as described herein. It should be appreciated that any of the optical measurement systems described herein, such as those described with respect to FIGS. 1A-20E may be configured to include one or more polarizing beamsplitters as described herein. It should also be appreciated that the polarizing beamsplitters described herein may be utilized as part of any other optical measurement system in which it is desirable to both polarizing and split incoming light.

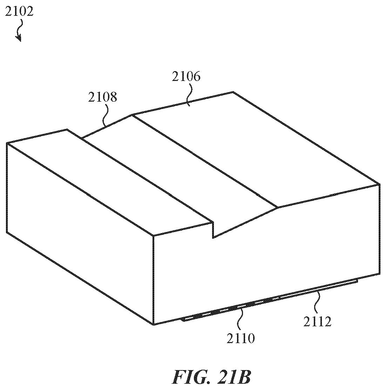

FIG. 21A shows a side view of a portion of an optical measurement system 2100 that includes a polarizing beamsplitter 2102. FIG. 21B shows a perspective view of the polarizing beamsplitter 2102. The polarizing beamsplitter 2102 may be positioned within the optical measurement system 2100 to receive an incoming light beam 2120a and to split the incoming light beam 2120a into a first split light beam 2120c and a second split light beam 2120d. Each of the first and second split light beams 2120c, 2120d are polarized, such that the polarizing beamsplitter 2102 outputs two split light beams having the same polarization state.

Specifically, the polarizing beamsplitter 2102 may be formed form a substrate 2103. The substrate 2103 may be formed from a material that is transparent at any wavelength (or wavelengths) that will be received by the polarizing beamsplitter 2102 (e.g., any of the measurement wavelengths used by an optical measurement system, simultaneously or sequentially, to generate an emission light beam). Additionally it may be desirable for the substrate 2103 to be formed from a material having a relatively low refractive index, which may facilitate Fresnel reflections with a surface of the substrate 2103 as described in more detail herein. For example, the substrate 2103 may be formed from a glass wafer, such as fused silica.

The substrate 2103 may include a bottom surface 2104 and a top surface 2106 opposite the bottom surface 2104. A bottom surface 2104 may be flat, and may be positioned within the optical measurement system 2100 to receive the incoming light beam 2120a. The top surface 2106 may be processed or otherwise shaped to define an angled facet 2108. The angled facet 2108 may be orientated at a non-zero wedge angle θ*_{f}* relative to the bottom surface 2104, such that the angled facet 2108 is not parallel to the bottom surface 2104. While the angled facet 2108 is not parallel to the bottom surface 2104, it should be appreciated that other portions of the top surface 2106 may be parallel to the bottom surface 2104.

The polarizing beamsplitter 2102 further includes a polarizer 2110 positioned on a first portion of the bottom surface 2104. The polarizer 2110 may include any suitable structure capable of polarizing light passing therethrough. As one non-limiting example, the polarizer 2110 may include a wire grid polarizer, which may be lithographically defined on the bottom surface 2104. The polarizer 2110 acts to polarize the incoming light beam 2120a as it enters the polarizing beamsplitter 2102.

Specifically, the polarizing beamsplitter 2102 may be positioned in the optical measurement system 2100 such that the incoming light beam 2120a is incident on the first portion of the bottom surface 2104 that includes the polarizer 2110. Accordingly, the polarizing beamsplitter 2102 (as well as the other polarizing beamsplitters described herein) may be designed to operate assuming that light will enter the polarizing beamsplitter 2102 at a predetermined location and a predetermined angle of incidence. For example, the optical measurement system 2100 may be configured such that the incoming light beam 2120a is incident on the predetermined location of the bottom surface 2104 at the predetermined angle of incidence. In some variations, the predetermined angle of incidence is a non-normal angle of incidence θ*ᵢ*. It may be desirable for the non-normal angle of incidence θ*ᵢ* to be relatively close to normal, which may improve the performance of the polarizer 2110, but sufficiently far from normal to reduce back reflections that may occur as the incoming light beam 2120a enters the polarizing beamsplitter 2102. For example, in some variations, the angle of incidence θ*ᵢ* may be between ten and twenty degrees (e.g., relative to normal incidence). In some variations, the optical measurement system 2100 may include an input aperture 2130 that is positioned, such that incoming light beam 2120a passes through the input aperture 2130 before reaching the polarizing beamsplitter 2102. In these instances, the input aperture 2130 may help confine the angle and position of the incoming light beam 2120a as it reaches the bottom surface 2104.

The incoming light beam 2120a enters the polarizing beamsplitter 2102 through the polarizer 2110, thereby polarizing the incoming light beam 2120a to generate a polarized light beam 2120b. The polarized light beam 2120b will travel from the bottom surface 2104 to the top surface 2106, where the polarized light beam 2120b will be incident on the angled facet 2108. The angled facet 2108 is angled (e.g., at the wedge angle θ*_{f}*) such that, when the polarized light beam 2120b is incident on the angled facet 2108, a Fresnel reflection will occur. In these instances, a first portion of the polarized light beam 2120b will exit polarizing beamsplitter 2102 via the angled facet 2108 to form the first split light beam 2120c. A second portion of the polarized light beam 2120b will reflect off of the angled facet 2108 to form the second split light beam 2120d.

The magnitude of the Fresnel reflection (e.g., how much light is transmitted as compared to how much light is reflected) at the angled facet 2108 may at least partially determine the sampling ratio of the polarizing beamsplitter 2102. The magnitude of the Fresnel reflection depends at least in part on the angle of incidence of the polarized light beam 2120b on the angled facet 2108 (which depends on the input angle θ*ᵢ* and the wedge angle θ*_{f}*), as well change in refractive index at the angled facet (e.g., the difference between the refractive index of the substrate 203 and the refractive index of the medium in contact with the angled facet 2108). Accordingly, these properties may be selected to achieve a desired sampling ratio for the polarizing beamsplitter 2102. In some variations the angled facet 2108 is uncoated, such that the angled facet is in contact with air during operation of the polarizing beamsplitter 2102.

In the variation shown in FIG. 21A, the second split light beam 2120d exits the polarizing beamsplitter 2102 through the bottom surface 2104 of the polarizing beamsplitter 2102. Specifically, the second split light beam 2120d may exit the polarizing beamsplitter through a second portion of the bottom surface 2104 that is not covered by the polarizer. In some of these variations, the polarizing beamsplitter 2102 includes an anti-reflection ("AR") coating 2112 positioned on the second portion of the bottom surface 2104. The AR coating 2112 may be laterally spaced from the polarizer 2110 (e.g., not overlapping) such that the AR coating 2112 and the polarizer 2110 are positioned side-by-side on the bottom surface 2104. The polarizing beamsplitter 2102 may be configured such that the second split light beam 2120d is incident on the AR coating 2112, such that the second split light beam 2120d passes through the AR coating 2112. The AR coating 2112 may be designed to reduce internal reflections (and thereby optical losses) that may otherwise occur as the second split light beam 2120d exits the polarizing beamsplitter 2102 via the bottom surface 2104. For example, the AR coating 2112 may include a multi-layer AR coating that is optimized for the wavelength or range of wavelengths that will be received by the polarizing beamsplitter 2102.

Overall, the light forming the first and second split light beams 2120c, 2120d will have passed through the polarizer 2110 once as the incoming light beam 2120a first enters the polarizing beamsplitter 2102. To prevent the second split light beam 2120d from also passing through the polarizer 2110 (which may alter the intensity of the second split light beam 2120d), the polarizing beamsplitter 2102 may be configured such that the second split light beam 2120d does not overlap the incoming light beam 2120a at the bottom surface 2104. Accordingly, the input angle θ*ᵢ*, the wedge angle θ*_{f},* and the thickness of the substrate 2103 may be selected to achieve a desired beam separation between the incoming light beam 2120a and the second split light beam 2120d at the bottom surface 2104.

In some variations of the optical measurement systems described herein, the polarizing beamsplitter 2102 of FIGS. 21A and 21B may be incorporated into a beam generator to both i) polarize an input light beam and ii) sample a portion of the input light beam. Accordingly, the polarizing beamsplitter 2102 may generate a polarized input light beam (corresponding to the first split light beam 2120c in FIG. 21A) that may be used to generate a polarized output light beam that is emitted by the beam generator. A launch optical subassembly may generate a polarized emission light beam from the polarized output light beam, such that the emission light beam introduced into a measured sample may have a predetermined polarization state. Additionally, the polarizing beamsplitter 2102 may generate a reference light beam (corresponding to the second split light beam 2120d in FIG. 21A) that may be measured by a reference detector, such as described in more detail herein.

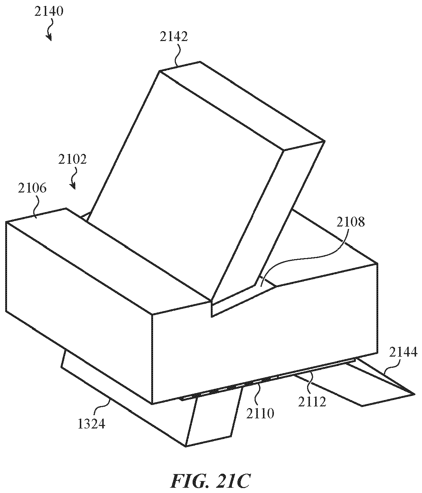

For example, FIG. 21C shows an instance in which the polarizing beamsplitter 2102 of FIGS. 21A and 21B may be incorporated into a beam generator 2140 of a launch architecture. Specifically, the beam generator 2140 may be configured in any manner as described herein with respect to the beam generators 1302 described herein with respect to FIGS. 13A-13F, except that beamsplitter 1332 has been replaced by the polarizing beamsplitter 2102 of FIGS. 21A and 21B. The other components of the beam generator 2140 are not depicted shown in FIG. 21C. The polarizing beamsplitter 2102 may be positioned to receive the input light beam 1324 generated by the photonic integrated circuit 1314 (e.g., after it has passed through slow axis collimating lens 1328). The input light beam 1324 enters the polarizing beamsplitters 2102 through the polarizer 2110, and the polarizing beamsplitter 2102 will generate a polarized input light beam 2142 that will be received by the diffuser 1318, and a polarized reference light beam 2144 that will be directed toward reference detector 1336. In this way, the beam generator 2140 will function the same as described with respect to FIGS. 13A-13F, except that the light measured by the reference detector 1336 and the output light beam generated by the beam generator 2140 may both be polarized.

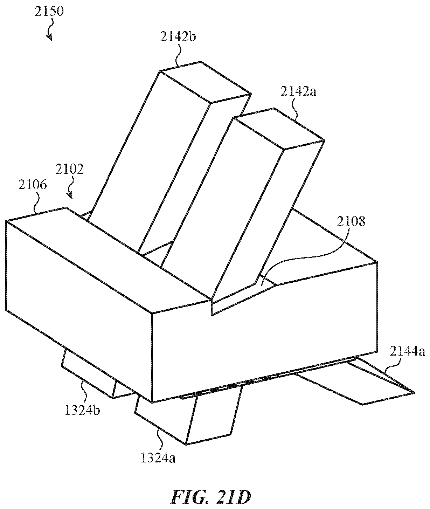

In some variations, the polarizing beamsplitters described herein may be shared by multiple measurement subsystems of an optical measurement system as described herein. For example, FIG. 21D shows an instance in which the polarizing beamsplitter 2102 of FIGS. 21A and 21B is incorporated into an optical measurement system 2150 having a first measurement subsystem and a second measurement subsystem. Specifically, the first measurement subsystem may include a first beam generator that is configured to generate a first output light beam from a first input light beam 1324a. Similarly, the second measurement subsystem may include a second beam generator that is configured to generate a second output light beam from a second input light beam 1324b. For example, each of the first and second beam generators may be configured in any manner such as described herein with respect to FIGS. 13A-13F.

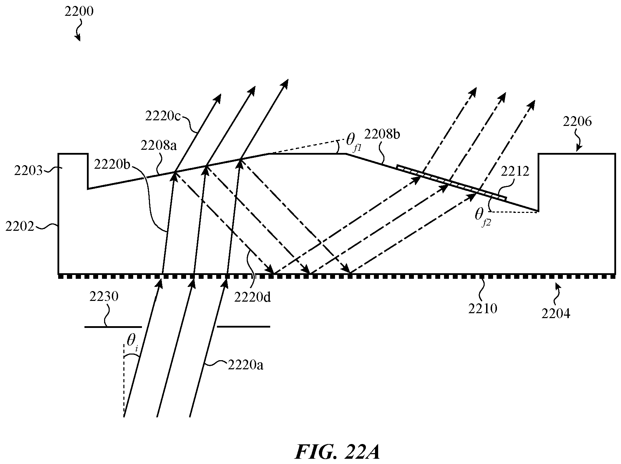

The polarizing beamsplitter 2102 may act as a beamsplitter for both the first and second beam generators. For example, the polarizing beamsplitter 2102 may be positioned to receive the first input light beam 1324a through a first region of the polarizer 2110 and to receive the second input light beam 1324b through a second region of the polarizer 2110. Light of the first input light beam 1324a entering the bottom surface 2104 of the polarizing beamsplitter 2102 will be split between a first polarized input light beam 2142a and a first polarized reference light beam 2144a, such as described herein with respect to FIG. 21C. Similarly, light of the second input light beam 1324b entering the bottom surface 2104 of the polarizing beamsplitter 2102 will be split between a second polarized input light beam 2142b and a second polarized reference light beam (not shown). Accordingly, the first and second polarized input light beam 2142a, 2142b may be used to generate corresponding polarized output light beams in the first and second measurement subsystems. The first and second polarized reference light beams may be measured by corresponding reference detectors of the first and second measurement subsystems. Similar principles may be applied to the polarizing beamsplitters described herein with respect to FIGS. 22A-26.

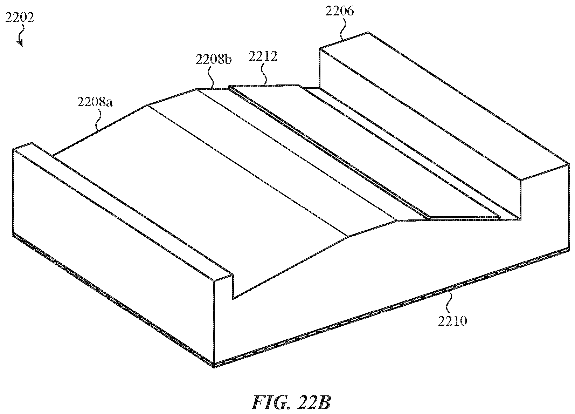

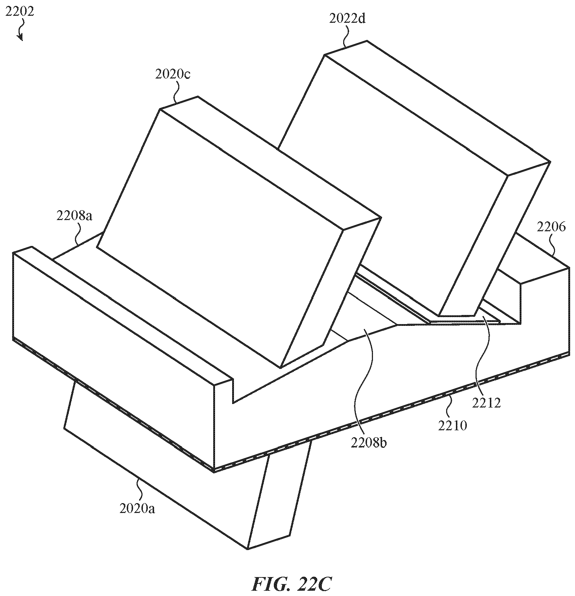

In some variations, it may be desirable for a polarizing beamsplitter to split an incoming light beam such that first and second split light beams exit the polarizing beamsplitter from opposite sides. For example, FIG. 22A shows an optical measurement system 2200 that includes a polarizing beamsplitter 2202. FIGS. 22B and 22C show perspective views of the polarizing beamsplitter 2202. The polarizing beamsplitter 2202 may be positioned within the optical measurement system 2100 to receive an incoming light beam 2220a (such as shown in FIGS. 22A and 22C) and to split the incoming light beam 2220a into a first split light beam 2220c and a second split light beam 2220d. The polarizing beamsplitter 2202 is configured such that the first split light beam 2220c and the second split light beam 2220d both exit the same side of the polarizing beamsplitter.

Specifically, the polarizing beamsplitter 2202 may be formed from a substrate 2203 (which may be configured in any manner as described herein with respect to the substrate 2103 of FIGS. 21A-21D) having a bottom surface 2204 and a top surface 2206. The bottom surface 2204 may be flat, and may be positioned within the optical measurement system 2200 to receive the incoming light beam 2220a. The top surface 2206 may be processed or otherwise shaped to define a first angled facet 2208a and a second angled facet 2208b. The first angled facet 2208a may be orientated at a first non-zero wedge angle θ*_{f1}* relative to the bottom surface 2204, such that the first angled facet 2208a is not parallel to the bottom surface 2204. Similarly, the first angled facet 2208a may be orientated at a second non-zero wedge angle θ*_{f2}* relative to the bottom surface 2104, such that the second angled facet 2208b is not parallel to the bottom surface 2204. In some instances, one or more portions of the top surface 2206 (e.g., a region between the first angled facet 2208a and the second angled facet 2208b) may be parallel to the bottom surface 2204.

The polarizing beamsplitter 2202 further includes a polarizer 2210 (e.g., a wire grid polarizer or the like) positioned on the bottom surface 2204. Because the second split light beam 2220d does not exit the polarizing beam-splitter through the bottom surface 2204, in some variations the polarizer 2210 may cover the entire bottom surface 2204 of the polarizing beamsplitter 2202. This may reduce processing complexity associated with manufacturing the polarizing beamsplitter 2202. The polarizer 2210 may polarize the incoming light beam 2220a as it enters the polarizing beamsplitter 2202.

The polarizing beamsplitter 2202 may be positioned in the optical measurement system 2200 such that the incoming light beam 2220a is incident on the bottom surface 2204 at a non-normal angle of incidence θ*ᵢ*, such as described herein with respect to the polarizing beamsplitter 2102 of FIGS. 21A-21D. Additionally, the optical measurement system 2200 may include an aperture 2230, such as described in more detail herein, positioned to limit the properties of the incoming light beam 2220a that is incident on the polarizing beamsplitter 2202.

The incoming light beam 2220a enters the polarizing beamsplitter 2202 through the polarizer 2210, thereby polarizing the incoming light beam 2220a to generate a polarized light beam 2220b. The polarized light beam 2220b will travel from the bottom surface 2204 to the top surface 2206, where the polarized light beam 2120b will be incident on the first angled facet 2208a. The first angled facet 2208a is angled (e.g., at the first wedge angle θ*_{f1}*) such that, when the polarized light beam 2220b is incident on the first angled facet 2208a, a Fresnel reflection will occur. As part of this Fresnel reflection, a first portion of the polarized light beam 2220b will exit polarizing beamsplitter 2202 via the first angled facet 2208a to form the first split light beam 2220c. A second portion of the polarized light beam 2220b will reflect off of the first angled facet 2208a to form the second split light beam 2220d. The various properties of the polarizing beamsplitter 2202 may be selected to achieve a desired sampling ratio. In some variations the first angled facet 2208a is uncoated, such that the first angled facet 2208a is in contact with air during operation of the polarizing beamsplitter 2202.

After reflecting off the first angled facet 2208a, the second split light beam 2220d will be directed toward the bottom surface 2204. Instead of the second split light beam 2220d exiting the polarizing beamsplitter 2202 via the bottom surface 2204, the polarizing beamsplitter 2202 may be configured such that the second split light beam reflects, via total internal reflection, off of the bottom surface 2204. In this way, the second split light beam 2220d may be redirected back toward the top surface 2206. Specifically, the polarizing beamsplitter 2202 may be configured such that the second split light beam 2220d is incident on the second angled facet 2208b after reflecting off of the bottom surface 2204. Because the second split light beam 2220d does not exit the bottom surface 2204, it is not necessary to provide beam separation between the incoming light beam 2202a and the second split light beam 2220d at the bottom surface.

As shown in FIGS. 22A and 22C, the second split light beam 2220d exits the polarizing beamsplitter 2202 through the top surface 2206 via the second angled facet 2208b. In some of these variations, the second angled facet 2208b may be at least partially covered with an AR coating 2212, such that the second split light beam 2220d passes through the AR coating 2212 as it exits the polarizing beamsplitter 2202. The AR coating 2212 may be designed to reduce internal reflections (and thereby optical losses) that may otherwise occur as the second split light beam 2220d exits the polarizing beamsplitter 2202 via the second angled facet 2208b.

Because the first split light beam 2220c and the second split light beam 2220d exit the polarizing beamsplitter 2202 through the same surface (e.g., the top surface 2206), it may be desirable for the first and second split light beams 2220c, 2220d to not overlap as they exit the polarizing beamsplitter 2202. Accordingly, the input angle θ*ᵢ*, the first wedge angle θ*_{f1}*, the second wedge angle θ*_{f2}*, and the thickness of the substrate 2103 may be selected to achieve a desired beam separation between the first split light beam 2120c and the second split light beam 2120d at the top surface 2206.

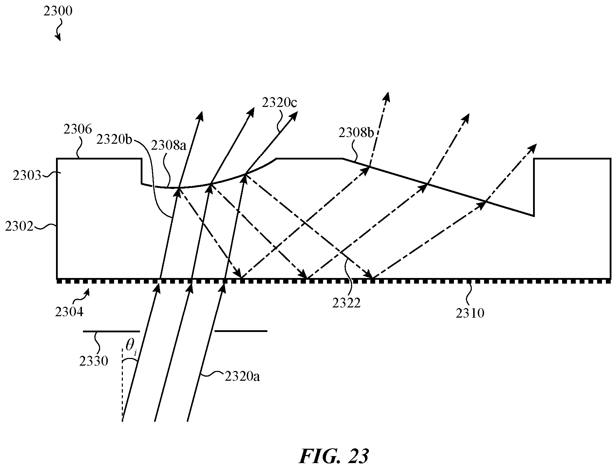

The polarizing beamsplitters described herein with respect to FIGS. 21A-22C, 25, and 26 are shown as having angled facets that are formed as flat surfaces. However, it should be appreciated that in some instances, a polarizing beamsplitter such as described herein may include one or more angled facets that are formed as curved surfaces. For example, FIG. 23 shows a side view of a portion an optical measurement system 2300 that includes a polarizing beamsplitter 2302. The polarizing beamsplitter 2302 may be configured similar to the polarizing beamsplitter 2202 of FIGS. 22A-22C. Specifically, the polarizing beamsplitter 2302 may be formed from a substrate 2303 having a bottom surface 2304 and a top surface 2306. A polarizer 2304 may be positioned on the bottom surface 2304, and the top surface 2306 defines a first angled facet 2308a and a second angled facet 2308b. The polarizing beamsplitter 2302 may further include an AR coating (not shown) deposited on the second angled facet 2308b.

The polarizing beamsplitter 2302 may be positioned to receive an incoming light beam 2320a (e.g., that passes through an aperture 2330 and is incident on the bottom surface 2304 at an input angle θ*ᵢ*), which generates a polarized light beam 2320b as the incoming light beam 2320a passes through the polarizer 2310. The polarized light beam 2320b is split into a first split light beam 2320c that exits the polarizing beamsplitter 2302 via the first angled facet 2308a, and a second split light beam 2320d that exits the polarizing beamsplitter 2302 via the second angled facet 2308b (e.g., after reflecting off of the back surface 2304). The first angled facet 2308a is formed from a surface that is curved in a first dimension, which in the variation shown in FIG. 23 is a curved surface that is concave along the first dimension.

The concave surface of the first angled facet 2308a may act to increase the divergence of the polarized light beam 2320b in the first dimension as it is split into the first split light beam 2320c and the second split light beam 2320d. For example, in instances where the incoming light beam 2320a is collimated a first dimension, the first split light beam 2320c will diverge in the first dimension as it exits the polarizing beamsplitter 2302. Similarly, the second split light beam 2320d will diverge as it is reflected from the first angled facet 2308a. If the second angled facet 2308b is a flat surface (such as shown in FIG. 23), the second split light beam 2320d will diverge in the first dimension as it exits the polarizing beamsplitter 2302. In other variations, the second angled facet 2308b may be curved in the first dimension to change the divergence of the second split light beam 2320d as it exits the second angled facet 2308b. For example, the second angled facet 2308b may include a curved surface that is convex in the first dimension, which may reduce the divergence of the second split light beam 2320d as it exits the second angled facet 2308b (e.g., to at least partially collimate or focus the second split light beam 2320d in the first dimension). Conversely, the second angled facet 2308b may include a curved surface this concave in the first dimension, which may further increase the divergence of the second split light beam 2320d in the first dimension as it exits the second angled facet 2308b. Accordingly, a curved second angled facet 2308b may provide for independent control of the divergence of the first and second split light beams 2320c, 2320d in the first dimension.

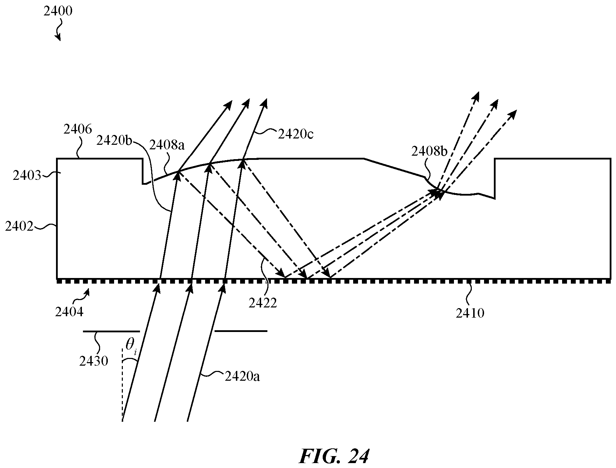

In other variations, the first angled facet may be a curved surface that is convex in a first dimension. FIG. 24 shows a side view of a portion an optical measurement system 2400 that includes a polarizing beamsplitter 2402. The polarizing beamsplitter 2402 may be configured similar to the polarizing beamsplitter 2202 of FIGS. 22A-22C. Specifically, the polarizing beamsplitter 2402 may be formed from a substrate 2403 having a bottom surface 2404 and a top surface 2406. A polarizer 2410 may be positioned on the bottom surface 2404, and the top surface 2406 defines a first angled facet 2408a and a second angled facet 2408b. The polarizing beamsplitter 2402 may further include an AR coating (not shown) deposited on the second angled facet 2408b.

The polarizing beamsplitter 2402 may be positioned to receive an incoming light beam 2420a (e.g., that passes through an aperture 2430 and is incident on the bottom surface 2404 at an input angle θ*ᵢ*), which generates a polarized light beam 2420b as the incoming light beam 2420a passes through the polarizer 2410. The polarized light beam 2420b is split into a first split light beam 2420c that exits the polarizing beamsplitter 2402 via the first angled facet 2408a, and a second split light beam 2420d that exits the polarizing beamsplitter 2402 via the second angled facet 2408b (e.g., after reflecting off of the bottom surface 2404). The first angled facet 2408a is formed from a curved surface that is convex in a first dimension.

The convex surface of the first angled facet 2408a may act to decrease the divergence of the polarized light beam 2420b in the first dimension as it is split into the first split light beam 2420c and the second split light beam 2420d. For example, in instances where the incoming light beam 2420a is collimated a first dimension, the first split light beam 2420c will converge in the first dimension as it exits the polarizing beamsplitter 2402. Similarly, the second split light beam 2420d will converge as it is reflected from the first angled facet 2408a. Depending on the dimensions of the polarizing beamsplitter 2402, the second split light beam 2320d may also converge as it exits the second angled facet 2408b (e.g., when the second angled facet 2408b is flat). In other instances, the second angled facet 2408b may be curved in the first dimension (e.g., with a concave curve as shown in FIG. 24 or with a convex curve).

When the polarizing beamsplitters described with respect to FIGS. 23 and 24 are incorporated into a beam generator as described herein, the curved surface of the first angled facet may help to shape an input light beam in a particular dimension. For example, the beam generators 1302 described with respect to FIGS. 13A-13F are configured to adjust an input light beam 1324 along its fast axis such that is has a particular beam divergence and beam size as it reaches the diffuser 1318. Accordingly, when the polarizing beamsplitter 2302 or the polarizing beamsplitter 2402 is used in place of beamsplitter 1332, the curved first angled facet (e.g., the concave first angled facet 2308a or the convex first angled facet 2308b) may, in addition to polarizing the input light beam 1324, cooperate with the remaining components of the beam generator 1302 to adjust the beam divergence and beam size of the input light beam 1324 along the fast axis as it reaches the diffuser 1318. Additionally, the curved angled facets may reduce the likelihood that etalons will be generated in the polarizing beamsplitters described herein.

In some variations, it may be desirable to configure a polarizing beamsplitter to increase the relative intensity between the first split light beam and the second split light beam output by the polarizing beamsplitter. For example, when a polarizing beamsplitter is used to split off a reference light beam that is measured by a reference detector, there may be limits to the intensity of light that may be measured by the reference detector without saturating the reference detector. Depending on the intensity requirements of the emission light beam, it may be possible that the sampling ratio of a particular polarizing beamsplitter (e.g., 5%) may result in saturation of the reference detector.

For example, to help reduce the sampling ratio of the polarizing beamsplitters described with respect to FIGS. 21A-24, these polarizing beamsplitters may include an AR coating positioned on the angled facet that splits the polarized light beam into a first split light beam and a second split light beam. For example, this angled facet may be the angled facet 2108 of polarizing beamsplitter 2102, the first angled facet 2208a of polarizing beamsplitter 2202, the first angled facet 2308a of polarizing beamsplitter 2302, or the first angled facet 2408a of polarizing beamsplitter 2402. In these instances, the AR coating may be designed to partially reduce the Fresnel reflection. Whereas AR coatings are typically designed to eliminate reflections at an interface, this AR coating is designed to intentionally allow some reflection in order to split the polarized light beam.

For example, the AR coating may be designed as a single-layer quarter-wave coating. The quarter-wave coating may be formed from a material having a lower refractive index than the refractive index of the substrate of the polarizing beamsplitter. For example, in some variations, the substrate of a polarizing beamsplitter as described herein may be formed from fused silica, and the AR coating may be formed from a single layer of magnesium fluoride. The thickness of the AR coating may depend on the range of wavelengths that will be received by the polarizing beamsplitter. For example, the polarizing beamsplitter may be configured to operate over a broad range of wavelengths, such as a wavelength range spanning at least 500 nm. In some instances, the polarizing beamsplitter may be configured to operate over a range of wavelengths spanning at 1000 nm. Accordingly, the thickness of the AR coating may be selected as a quarter of a selected wavelength that falls within the range of wavelengths. The selected wavelength may be selected to balance the perform of the AR coating across the range of wavelengths. While there may be a small wavelength-dependency of the transmissivity of the AR coating, this dependency may be sufficiently small to be within acceptable tolerances for a given optical measurement system.

Accordingly, when an angled facet is split a beam of light, a single-layer AR coating positioned on the angled may reduce the amount of light that is split off as compared to instances in which the angled facet is uncoated. In some instances, the single-layer AR coating may be positioned to cover the entire top surface of the substrate of a polarizing beamsplitter (e.g., the top surface 2106 of the polarizing beamsplitter 2102), which may simplify the manufacturing of the polarizing beamsplitter. In instances where the top surface of the substrate of a polarizing beamsplitter also defines a second angled facet (e.g., the second angled facet 2208b of polarizing beamsplitter 2202, the second angled facet 2308b of polarizing beamsplitter 2302, or the second angled facet 2408b of polarizing beamsplitter 2402), the single-layer AR coating may also cover the second angled facet. In instances where a split light beam exits the polarizing beamsplitter via the second angled facet, the single-layer AR coating may also result in a Fresnel reflection occurring at the second angled facet. Accordingly, the polarizing beamsplitter (or the optical measurement system incorporating the polarizing beamsplitter) may be configured to direct or absorb this stray light so that it doesn't interfere with the rest of the optical measurement system.

In some variations where a polarizing beamsplitter defines two angled facets in a top surface of a substrate, the two angled facets may be covered with different AR coatings. For example, in the variation of the polarizing beamsplitter 2202 described with respect to FIGS. 22A-22C, the first angled facet 2208a may be coated with a first AR coating (not shown) and the second angled facet 2208b may be coated with a different second AR coating (e.g., AR coating 2212). In these instances, the first AR coating may be configured to have a lower transmissivity than the second AR coating. For example, the first AR coating may be a single-layer quarter wave AR coating and the second AR coating may be a multi-layer AR coating. Accordingly, the first AR coating may facilitate splitting of a polarized light beam 2220b into a first split light beam 2220c and a second split light beam 2220d, whereas the second AR coating may reduce stray light that may reflect off of the second angled facet 2208b as the second split light beam 2220d exits the polarizing beamsplitter 2102.

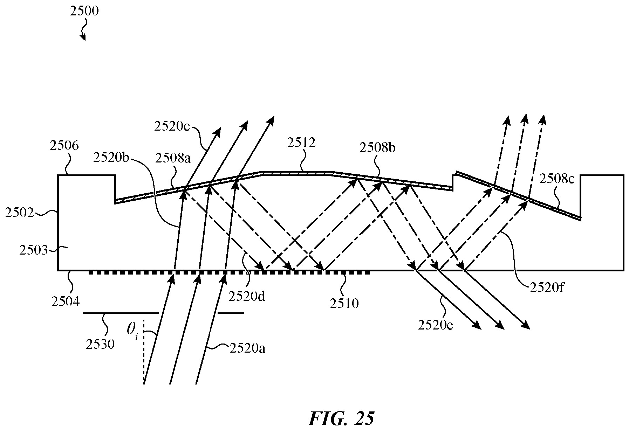

In some variations, a polarizing beamsplitter may be configured to split a beam of light multiple times. For example, FIG. 25 shows a side view of a portion of an optical measurement system 2500 that includes a polarizing beamsplitter 2502. The polarizing beamsplitter 2502 may be positioned within the optical measurement system 2500 to receive an incoming light beam 2520a and to split the incoming light beam 2520a into a first split light beam 2520c and a second split light beam 2520d. The polarizing beamsplitter is further configured to split the second split light beam 2520d into a third split light beam 2520e and a fourth split light beam 2520f. Overall, the polarizing beamsplitter 2502 will emit three split beams (e.g., the first split light beam 2520c, the third split light beam 2520e, and the fourth split light beam 2520f). In the variation shown in FIG. 25, the polarizing beamsplitter 2502 is configured such that the first split light beam 2520c and the fourth split light beam 2620f each exit the same side of the polarizing beamsplitter 2502, whereas the third split light beam 2220e exits an opposite side of the polarizing beamsplitter 2502.

Specifically, the polarizing beamsplitter 2502 may be formed from a substrate 2503 (which may be configured in any manner as described herein with respect to the substrate 2103 of FIGS. 21A-21D) having a bottom surface 2504 and a top surface 2506. The bottom surface 2504 may be flat, and may be positioned within the optical measurement system 2500 to receive the incoming light beam 2520a. The top surface 2506 may be processed or otherwise shaped to define a first angled facet 2508a, a second angled facet 2508b, and a third angled facet 2508c. Each of the first, second, and third angled facets 2508a-2508c are oriented at corresponding non-zero wedge angles (e.g., a first wedge angle, a second wedge angle, and third wedge angle, respectively), such that each of these angled facets is not parallel to the bottom surface. In some instances, one or more portions of the top surface 2506 (e.g., a region between the first angled facet 2508a and the second angled facet 2508b) may be parallel to the bottom surface 2504.

The polarizing beamsplitter 2502 further includes a polarizer 2510 (e.g., a wire grid polarizer or the like) positioned on a first portion of the bottom surface 2504. The polarizer 2510 may polarize the incoming light beam 2520a as it enters the polarizing beamsplitter 2502. Specifically, the polarizing beamsplitter 2502 may be positioned in the optical measurement system 2500 such that the incoming light beam 2520a is incident on the first portion of the bottom surface 2504 at a non-normal angle of incidence θ*ᵢ*, such as described herein with respect to the polarizing beamsplitter 2102 of FIGS. 21A-21D. Additionally, the optical measurement system 2500 may include an aperture 2530, such as described in more detail herein, positioned to limit the properties of the incoming light beam 2520a that is incident on the polarizing beamsplitter 2502.

The incoming light beam 2520a enters the polarizing beamsplitter 2502 through the polarizer 2510, thereby polarizing the incoming light beam 2520a to generate a polarized light beam 2520b. The polarized light beam 2520b will travel from the bottom surface 2504 to the top surface 2506, where the polarized light beam 2520b will be incident on the first angled facet 2508a. The first angled facet 2508a is angled (e.g., at the first wedge angle) such that, when the polarized light beam 2520b is incident on the first angled facet 2508a, a Fresnel reflection will occur. As part of this Fresnel reflection, a first portion of the polarized light beam 2520b will exit polarizing beamsplitter 2502 via the first angled facet 2508a to form the first split light beam 2520c. A second portion of the polarized light beam 2520b will reflect off of the first angled facet 2508a to form the second split light beam 2520d.

After reflecting off the first angled facet 2508a, the second split light beam 2520d will be directed toward the bottom surface 2504. The polarizing beamsplitter 2502 may be configured such that the second split light 2520d beam reflects off of the bottom surface 2504 and is redirected back toward the top surface 2506. In some instances, the second split light beam 2520d is angled such that it reflects via total internal reflection. In these instances, it is not necessary to provide beam separation between the incoming light beam 2502a and the second split light beam 2520d at the bottom surface 2504.

The polarizing beamsplitter 2202 may be configured such that the second split light beam 2520d is incident on the second angled facet 2508b after reflecting off of the bottom surface 2504. The second angled facet 2508b may be angled (e.g., via the second wedge angle), such that the second split light beam 2520d is redirected toward a second portion of the bottom surface 2504 that may not be covered by the polarizer 2510. A second Fresnel reflection will occur as the second split light beam 2520d is incident upon the second portion of the bottom surface 2504. Accordingly, a first portion of the second split light beam 2520d will exit the polarizing beamsplitter 2502 through the bottom surface 2504 to form the third split light beam 2520e. A second portion of the second split light beam 2520d will be reflected off the bottom surface 2504 to form the fourth split light beam 2520f.

The polarizing beamsplitter 2502 may be configured such that the fourth split light beam 2520f, after reflecting off the bottom surface 2504 is incident on the third angled facet 2508f. The third angled facet 2508c may be angled (e.g., via the third wedge angle) such that the fourth split light beam 2520f exits the polarizing beamsplitter 2502 via through the third angled facet 2508c. Because the fourth split light beam 2520f represents a fraction of the intensity of the second split light beam 2520d, the fourth split light beam 2520f may result in an even smaller sampling ratio as compared to the examples of polarizing beamsplitters described with respect to FIGS. 21A-24.

In instances where the polarizing beamsplitter 2502 is configured to split off a reference light beam as part of beam generator, the first split light beam 2520c may be used to form an output light beam generated by the beam generator The fourth split light beam 2520f may be used as the reference light beam. For example, if the polarizing beamsplitter 2502 is used in place of the beamsplitter 1332 of FIGS. 13B-13F, the polarizing beamsplitter 2502 may receive the input light beam 1324 as the incoming light beam 2520a. The polarizing beamsplitter 2502 will generate the first split light beam 2520c, which may be directed to the diffuser 1318 to generate output light beam 1306. The polarizing beamsplitter 2502 will generate the fourth split light beam 2520f, which may act as the reference light beam 1334 (and is thereby directed to a reference detector 1336). In some instances, the optical measurement system may not have use for the third split light beam 2520e, in which case the optical measurement system may treat the third split light beam 2520e as a waste beam. In these instances, the third split light beam 2520e may be directed to an absorber or another portion of the optical measurement system such that this light does not interfere with the spectroscopic measurement performed by the optical measurement system.

While the first, second, and third angled facets 2508a-2508c are shown in FIG. 25 as being formed at flat surfaces, it should be appreciated that some or all of these angled facets may be curved in a first dimension, such as describe with respect to FIGS. 23 and 24. Additionally, one or more surfaces of the substrate 2503 may be include an AR coating configured to adjust the magnitude of reflections occurring at the various interfaces of the polarizing beamsplitter 2502. For example, the second split light beam 2520d is split at a second portion of the bottom surface 2504 of the substrate 2503, which is shown in FIG. 25 as being uncoated. In other variations, the second portion of the bottom surface 2504 may include an AR coating (e.g., a single layer quarter-wave coating as described herein) that is configured to partially reduce the Fresnel reflection at the bottom surface 2504. This may change the relative intensity between the third split light beam 2520e and the fourth split light beam 2520f to further reduce the intensity of the fourth split light beam 2520f.

Similarly, one or more portions of the top surface 2506 may be coated with an AR coating. For example, in the variation shown in FIG. 25, an AR coating 2512 may cover each of the first, second, and third angled facets 2508a-2508c. This AR coating 2512 may be a single layer quarter-wave coating as described herein, which may change the relative intensity between the first split light beam 2520c and the second split light beam 2520d, which will also reduce the intensity of the fourth split light beam 2520f. In some variations, the third angled facet 2508c (and in some instances, the second angled facet 2508b) may instead be covered with a second AR coating (not shown), such as a multi-layer AR coating, that has a higher transmissivity than the AR coating 2512. This may act to reduce stray light that may be generated as the fourth split light beam 2520f exits the third angled facet 2508c. In still other variations, the first angled facet 2508a may be uncoated, and the third angled facet 2508c (and optionally the second angled facet 2508b) may be coated with an AR coating.

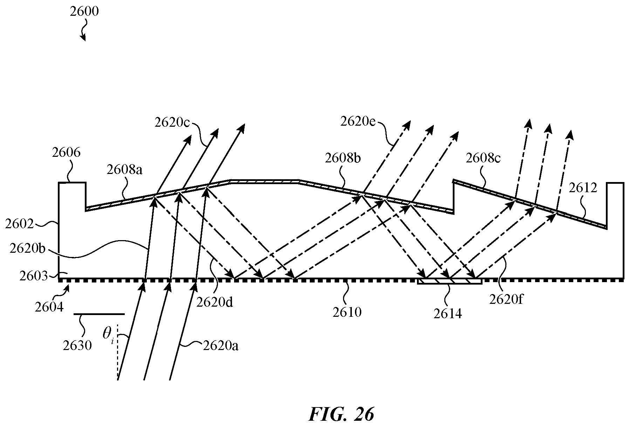

FIG. 26 shows a side view of a portion of an optical measurement system 2600 that includes another variation of a polarizing beamsplitter 2602 as described herein. The polarizing beamsplitter 2602 may be positioned within the optical measurement system 2600 to receive an incoming light beam 2620a and to split the incoming light beam 2620a into a first split light beam 2620c and a second split light beam 2620d. The polarizing beamsplitter is further configured to split the second split light beam 2620d into a third split light beam 2620e and a fourth split light beam 2620f. Overall, the polarizing beamsplitter 2602 will emit three split beams (e.g., the first split light beam 2620c, the third split light beam 2620e, and the fourth split light beam 2620f). In the variation shown in FIG. 26, the polarizing beamsplitter 2602 is configured such that the first split light beam 2620c the third split light beam 2620e, and the fourth split light beam 2620f each exit the same side of the polarizing beamsplitter 2602.

Specifically, the polarizing beamsplitter 2602 may be formed from a substrate 2603 (which may be configured in any manner as described herein with respect to the substrate 2103 of FIGS. 21A-21D) having a bottom surface 2604 and a top surface 2606. The bottom surface 2604 may be flat, and may be positioned within the optical measurement system 2600 to receive the incoming light beam 2620a. The top surface 2606 may be processed or otherwise shaped to define a first angled facet 2608a, a second angled facet 2608b, and a third angled facet 2608c. Each of the first, second, and third angled facets 2608a-2608c are oriented at corresponding non-zero wedge angles (e.g., a first wedge angle, a second wedge angle, and third wedge angle, respectively), such that each of these angled facets is not parallel to the bottom surface 2604. In some instances, one or more portions of the top surface 2606 (e.g., a region between the first angled facet 2608a and the second angled facet 2608b) may be parallel to the bottom surface 2604.

The polarizing beamsplitter 2602 further includes a polarizer 2610 (e.g., a wire grid polarizer or the like) positioned on a first portion of the bottom surface 2604. The polarizer 2610 may polarize the incoming light beam 2620a as it enters the polarizing beamsplitter 2602. Specifically, the polarizing beamsplitter 2602 may be positioned in the optical measurement system 2600 such that the incoming light beam 2620a is incident on the first portion of the bottom surface 2604 at a non-normal angle of incidence θ*ᵢ*, such as described herein with respect to the polarizing beamsplitter 2102 of FIGS. 21A-21D. Additionally, the optical measurement system 2600 may include an aperture 2630, such as described in more detail herein, positioned to limit the properties of the incoming light beam 2620a that is incident on the polarizing beamsplitter 2602.

The incoming light beam 2620a enters the polarizing beamsplitter 2602 through the polarizer 2610, thereby polarizing the incoming light beam 2620a to generate a polarized light beam 2620b. The polarized light beam 2620b will travel from the bottom surface 2604 to the top surface 2606, where the polarized light beam 2620b will be incident on the first angled facet 2608a. The first angled facet 2608a is angled (e.g., at the first wedge angle) such that, when the polarized light beam 2620b is incident on the first angled facet 2608a, a first Fresnel reflection will occur. As part of this Fresnel reflection, a first portion of the polarized light beam 2620b will exit polarizing beamsplitter 2602 via the first angled facet 2608a to form the first split light beam 2620c. A second portion of the polarized light beam 2620b will reflect off of the first angled facet 2608a to form the second split light beam 2620d.

After reflecting off the first angled facet 2608a, the second split light beam 2620d will be directed toward the bottom surface 2604. The polarizing beamsplitter 2602 may be configured such that the second split light 2620d beam reflects off of the bottom surface 2604 and is redirected back toward the top surface 2606. In some instances, the second split light beam 2620d is angled such that it reflects via total internal reflection. In these instances, it is not necessary to provide beam separation between the incoming light beam 2602a and the second split light beam 2620d at the bottom surface 2604.

The polarizing beamsplitter 2602 may be configured such that the second split light beam 2620d is incident on the second angled facet 2608b after reflecting off of the bottom surface 2504. The second angled facet 2608b may be angled (e.g., via the second wedge angle), such that a second Fresnel reflection occurs at the second angled facet 2608b to split the second split light beam 2620d. Specifically, a first portion of the second split light beam 2620d will exit the polarizing beamsplitter 2602 through second angled facet 2608b to form the third split light beam 2620e. A second portion of the second split light beam 2620d will be reflected off the on second angled facet 2608b to form the fourth split light beam 2620f.

The fourth split light beam 2620f will be directed to a second portion of the bottom surface 2604 that is covered with a reflective coating 2614. The reflective coating 2614 may be formed form a high reflectance material such as a metal (e.g., aluminum, silver, or the like) that promotes reflection of light off of the bottom surface 2604. In this way, the fourth split light beam 2620f may be incident on the bottom surface 2604 at an angle that may otherwise cause a portion of the fourth split light beam 2620f to exit the polarizing beamsplitter 2602, but is instead reflected by the reflective coating 2614. The reflective coating 2614 may redirect the fourth split light beam 2620f toward the top surface 2606, where the fourth split light beam 2620f is incident on the third angled facet 2608c. The third angled facet 2608c may be angled (e.g., via the third wedge angle) such that the fourth split light beam 2620f exits the polarizing beamsplitter 2602 via the third angled facet 2608c.

In instances where the polarizing beamsplitter 2602 is configured to split off a reference light beam as part of beam generator, the first split light beam 2620c may be used to form an output light beam generated by the beam generator The fourth split light beam 2620f may be used as the reference light beam, such as discussed in more detail herein. In some instances, the optical measurement system may not have use for the third split light beam 2620e, in which case the optical measurement system may treat the third split light beam 2620e as a waste beam. In these instances, the third split light beam 2620e may be directed to an absorber or another portion of the optical measurement system such that this light does not interfere with the spectroscopic measurement performed by the optical measurement system.

While the first, second, and third angled facets 2608a-2608c are shown in FIG. 26 as being formed at flat surfaces, it should be appreciated that some or all of these angled facets may be curved in a first dimension, such as describe with respect to FIGS. 23 and 24. Additionally, one or more portions of the top surface 2606 of the substrate 2603 may be include an AR coating configured to adjust the magnitude of reflections occurring at the various angled facets. For example, in the variation shown in FIG. 26, an AR coating 2612 may cover each of the first, second, and third angled facets 2608a-2608c. This AR coating 2612 may be a single layer quarter-wave coating as described herein, which may change the relative intensity between the first split light beam 2620c and the second split light beam 2620d (e.g., occurring via the first Fresnel reflection) as well as the relative intensity between the third split light beam 2620e and the fourth split light beam 2620f (e.g., occurring via the second Fresnel reflection). In some variations, the third angled facet 2608c may instead be covered with a second AR coating (not shown), such as a multi-layer AR coating, that has a higher transmissivity than the AR coating 2612. This may act to reduce stray light that may be generated as the fourth split light beam 2620f exits the third angled facet 2608c. In still other variations, one or both of the first and second angled facets 2608a, 2608b may be uncoated, and the third angled facet 2608c may be coated with an AR coating.

In some variations, it may be desirable for the polarizer of a polarizing beamsplitter to be immersed within the polarizing beamsplitter, such that a cover layer separates the polarizer from the surrounding environment. In these instances, the cover layer may help to protect the polarizer from damage that may occur during handling of the polarizing beamsplitter and/or that may occur from exposure to dust, moisture, or other environmental conditions. Accordingly, any of the polarizing beamsplitters described herein, such as those described with respect to FIGS. 21A-26, may be configured to include an immersed polarizer.

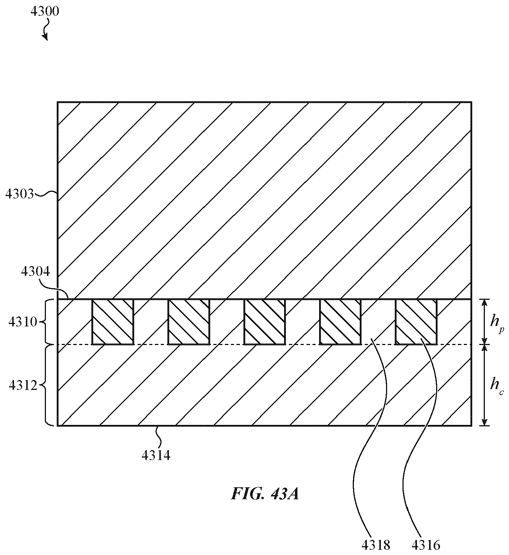

FIG. 43A an example of a portion of a polarizing beamsplitter 4300 that includes a substrate 4303 and an immersed polarizer 4310 positioned on a bottom surface 4304 of the substrate 4303. The polarizing beamsplitter 4300 further includes a cover layer 4312, such that the polarizer 4310 is positioned between cover layer 4312 and the substrate 4303, thereby encapsulating the polarizer 4310 within the polarizing beamsplitter 4300. It should be appreciated that the polarizers of the polarization beamsplitters described herein with respect to FIGS. 21A-26 may be configured in this manner.

The polarizer 4310 includes an array of electrically conductive nanowires 4316 that define the polarizer 4310 (e.g., to form a wire grid polarizer). The nanowires 4316 may be made from any suitable electrically conductive material, such as copper, aluminum, gold, or the like. A dielectric filler 4318 is positioned to fill the space between adjacent nanowires 4316. In some variations, the dielectric filler 4318 may be formed from the same material as the cover layer 4312. In some of these variations, the dielectric filler 4318 may be formed as part of a common manufacturing step with the cover layer 4312. In these variations, the array of nanowires 4316 may be defined on the bottom surface 4304 of the substrate 4303, and a dielectric material may be deposited on the bottom surface 4304 of the substrate 4303. A portion of the dielectric material may fill space between adjacent nanowires 4316 to define the dielectric filler 4318, whereas an additional portion of the dielectric material is positioned below the nanowires 4316 and the dielectric filler 4318 to define the cover layer 4312.

In some variations, each of the dielectric filler 4318, the cover layer 4312, and the substrate 4303 may be formed from the same material. For example, in some variations, the substrate 4303, the dielectric filler 4318, and the cover layer 4312 may each be formed from silicon dioxide. In some of these variations, the substrate 4303 may be formed from silicon dioxide as fused silica, whereas the dielectric filler 4138 and the cover layer 4312 are formed as deposited silicon dioxide

The cover layer 4312 may form an interface 4314 of the polarizing beamsplitter 4300 between the polarizing beamsplitter 4300 and its surrounding environment (e.g., air). Accordingly, when the polarizing beamsplitter 4300 is positioned to receive an input light beam that will pass through the polarizer 4310, such as described in more detail herein, the input light beam will first pass through the cover layer 4312 via the interface 4314. The polarization extinction ratio of the polarizer 4310, as well as the relative amounts of light that are respectively reflected and absorbed as an input light beam passes through the cover layer 4312 and the polarizer 4310, depends at least in part on the dimension of the polarizer 4310 and the cover layer 4312. For example, the cover layer 4312 may have a thickness *h_{c}* and the polarizer 4310 may have a thickness *hₚ.* Additionally, each of the nanowires 4316 may have a corresponding width (which may be a common width across the array of nanowires 4316) and may be separated from an adjacent nanowire by a corresponding pitch (which may be a common pitch across the array of nanowires 4316).

In some instances, it may be desirable to increase the thickness *hₚ* of the polarizer 4310 and/or the relative width of the nanowires 4316 for a given pitch (also referred to herein as the "duty cycle" of the polarizer 4310) to increase the polarization extinction ratio of the polarizer 4310. Increasing the thickness and/or the duty cycle of the polarizer 4310 may also increase the amount of light absorbed by the polarizer 4310 (e.g., light of a polarization that the polarizer 4310 is configured to pass), thereby increasing the amount of optical loss associated with the operation of the polarizing beamsplitter 4300. In some variations, it may be desirable to decrease the amount of light that is reflected by the polarizing beamsplitter 4300 (e.g., light of a polarization that the polarizer 4310 is configured to pass) as it passes through the cover layer 4312 and the polarizer 4310.

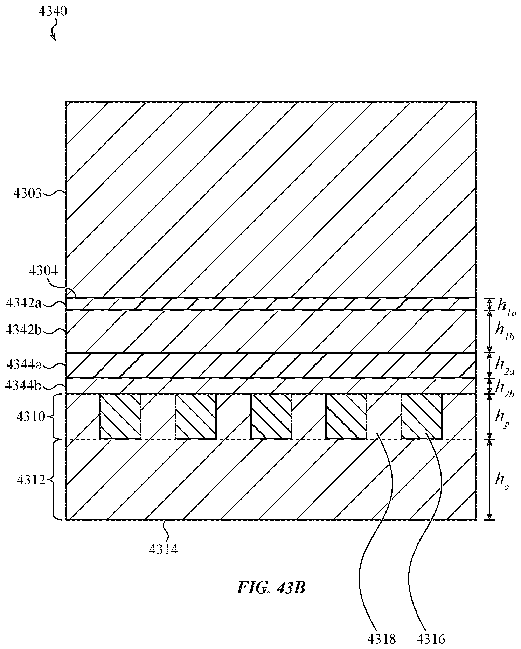

For example, FIG. 43B shows another variation of a portion of a polarizing beamsplitter 4340 that is configured the same as the polarizing beamsplitter 4300 of FIG. 43A, except that the polarizing beamsplitter 4340 includes a set of layer groups positioned between the polarizer 4310 and the substrate 4303. Each layer group includes plurality of layers (e.g., at least a first layer and a second layer), wherein the plurality of layers is formed from a corresponding plurality of materials having different refractive indices. Collectively, each layer group has an effective refractive index that is greater than a corresponding refractive index of the substrate 4303 and less than a corresponding refractive index of the polarizer 4310. In this way, the set of layer groups may provide a refractive index transition between the polarizer 4310 and the substrate 4303, which may reduce reflections that may otherwise occur at an interface between the polarizer 4310 and the bottom surface 4304 of the substrate 4303 (e.g., such as shown in the polarizing beamsplitter 4300 of FIG. 43A).

In some variations, the polarizing beamsplitter 4340 may include a single layer group positioned between polarizer 4310 and the substrate 4303. In the variation shown in FIG. 43B, the set of layer groups includes a plurality of layer groups having a first layer group 4342a-4342b and a second layer group 4344a-4344b. The first layer group 4342a-4342b may be formed on the bottom surface 4304 of the substrate 4303, and the second layer group 4344a-4344b may be formed on the first layer group 4342a-4342b. Accordingly, the first layer group 4342a-4342b is positioned between the substrate 4303 and the second layer group 4344a-4344b, and the second layer group 4344a-4344b is positioned between the first layer group 4342a-4342b and the polarizer 4310. In these variations, the first layer group 4342a-4342b may have a first effective refractive index that is greater than the corresponding refractive index of the substrate 4303, and the second layer group 4344a-4344b has a second effective refractive index that is both greater than the first effective refractive index and less than the corresponding refractive index of the polarizer 4310. It should be appreciated that polarizing beamsplitter 4340 may include additional layer groups positioned between the polarizer 4310 and the substrate 4303, if so desired.

In some variations, the first layer group 4342a-4342b and the second layer group 4344a-4344b may be formed from a common set of materials. For example, the first layer group 4342a-4324b may include a corresponding first layer 4342a formed from a first material and a corresponding second layer 4342b formed from a second material, where the second material has a lower refractive index than a refractive index of the first material. Similarly, the second layer group 4344a-4344b includes a corresponding first layer 4344a formed from the first material and a corresponding second layer 4344b formed from the second material. In some variations, the second material may be the same material that is used to form the substrate 4303. For example, in some variations where the substrate 4303 is formed from silicon dioxide (e.g., as fused silica), the second layer 4342b of the first layer group 4342a-4323b and the second layer 4344b of the second layer group 4344a-4344b may each also be formed from silicon dioxide (e.g., as deposited silicon dioxide). In some of these variations, the first layer 4342a of the first layer group 4342a-4323b and the first layer 4344a of the second layer group 4344a-4344b may each be formed from silicon.

To provide different effective refractive indices, the first layer group 4342a-4342b and the second layer group 4344a-4344b may have different relative thicknesses between their corresponding layers. For example, in some variations the first layer 4342a of the first layer group 4342a-4324b may have a thickness *h₁ₐ* that is less than a thickness *h₂ₐ* of the first layer 4344a of the second layer group 4344a-4344b, and the second layer 4342b of the first layer group 4342a-4324b may have a thickness *h_{1b}* that is larger than a thickness *h_{2b}* of the second layer 4344b of the second layer group 4344a-4344b. Additionally, these thicknesses may be selected in conjunction with the thickness *hₚ* of the polarizer 4310 and the thickness *h_{c}* to further tailor the amount of light that is reflected as light enters the polarizing beamsplitter 4340.

In some variations, in addition to or instead of the set of layer groups positioned between the polarizer 4310 and the substrate 4303, the polarizing beamsplitter 4340 may include a set of layer groups between the polarizer 4310 and the cover layer 4312. In these variations, each layer group may include a plurality of layers, where the layers are formed from respective materials having different refractive indices. Accordingly, the layer group may have an effective refractive index between the corresponding refractive indices of the polarizer 4310 and the cover layer 4312. For example, when the cover layer 4312 forms an interface 4314 with air, it may be desirable to form the cover layer 4312 from a material having a relatively low refractive index (e.g., to reduce reflection at the interface 4314). The presence of layer groups between the cover layer 4312 and the polarizer 4310 may reduce reflections that may otherwise occur at an interface between the cover layer 4312 and the polarizer 4310.

### Beam shifting optics and Optical Measurement Systems Incorporating the Same

When optical components such as lenses are used to shape a light beam within an optical measurement system, there may be constraints on how close optical components may be positioned to each other while still achieving the desired beam shaping. For example, in the variation of the beam generator 1302 shown in FIG. 13A, it may be desirable for the input light beam 1324 to have a particular dimensions (e.g., a first beam width in the first dimension and a second beam width in the second dimension) as it reaches the diffuser 1318. For example, to achieve the second beam width in the second dimension, it may be necessary for each of the plurality of individual light beams 1322a-1322c to diverge to a particular beam width in the second dimension as it reaches the slow axis collimating lens 1328. Accordingly, the spacing between the photonic integrated circuit 1314, the slow axis collimating lens 1328, and the diffuser 1318 may be determined at least in part on the beam size and divergence of the individual light beams 1322a-1322c as the exit the corresponding outcouplers 1320a-1320c, as well as the focal length of the slow axis collimating lens in the second dimension. This spacing defines a track length of the beam generator 1302, which represents a distance that light travels between the photonic integrated circuit 1314 and the diffuser 1318. Similarly, this track length may at least partially define a track length of the launch architecture 1300, which represents a distance that the light travel between the photonic integrated circuit 1314 and the launch site 1308 of the sampling interface.

When the photonic integrated circuit 1314, the slow axis collimating lens 1328, and the diffuser 1318 are positioned such that the input light beam 1324 travels along a single axis (e.g., along the X-axis shown in FIG. 13B), the entire track length of the beam generator 1302 is positioned along a single axis. This may thereby place a minimum size constraint on the beam generator 1302 (and thereby the launch architecture 1300) along this axis. Conversely, folding the input light beam 1324 (e.g., redirecting the input light beam 1324 such that it travels along another direction) may reduce the distance that the input light beam 1324 may travel in any particular direction. Accordingly, some optical measurement systems as described herein may incorporate a beam shifting optic that is configured to fold a light beam along multiple axes. The beam shifting optic may help to direct a light beam (such as the input light beam 1324 of beam generator 1302) in a manner that makes efficient use of available space within an optical measurement system.

The beam shifting optics described herein utilize a plurality of angled facets to laterally shift a light beam. In this way, a light beam may travel along the same direction or similar directions as it enters the and exits the beam shifting optic, but will be laterally shifted relative to each other. This may provide additional flexibility in designing an optical measurement system, as a beam shifting optic may allow the track length of a portion of an optical measurement system (e.g., a beam generator) to be split between multiple directions, thereby reducing the overall size in any single direction. Additionally, a beam shifting optic as described herein may be used to laterally shift light emitted by a photonic integrated circuit, which may provide flexibility in where the photonic integrated circuit is positioned within an optical measurement system.

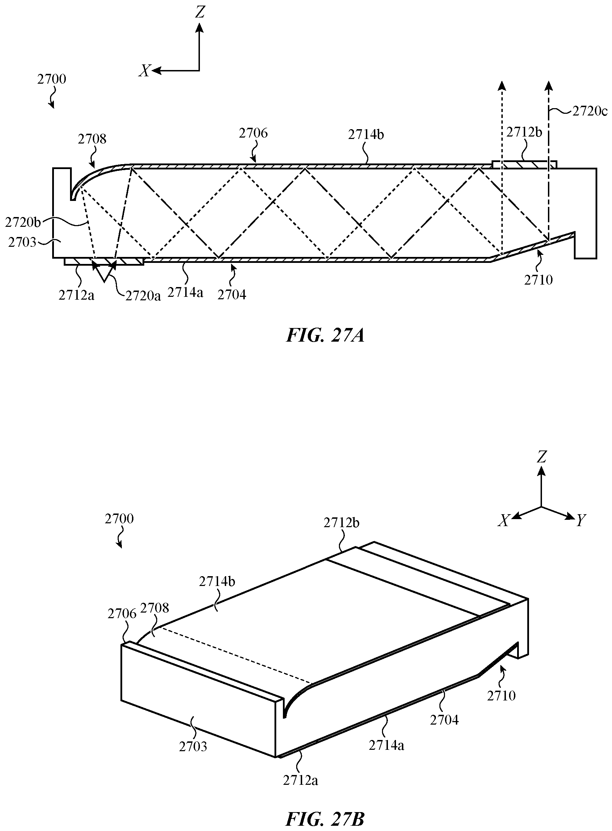

FIGS. 27A-33B depict variations of beam shifting optics, as well as beam generators incorporating these beam shifting optics. It should be appreciated that these beam shifting optics and associated beam generators may be incorporated into any of the optical measurement systems described herein (e.g., such as those described herein with respect to FIGS. 1A-26), or may be utilized as part of any other optical measurement system in which it is desirable to redirect light within an optical measurement system.

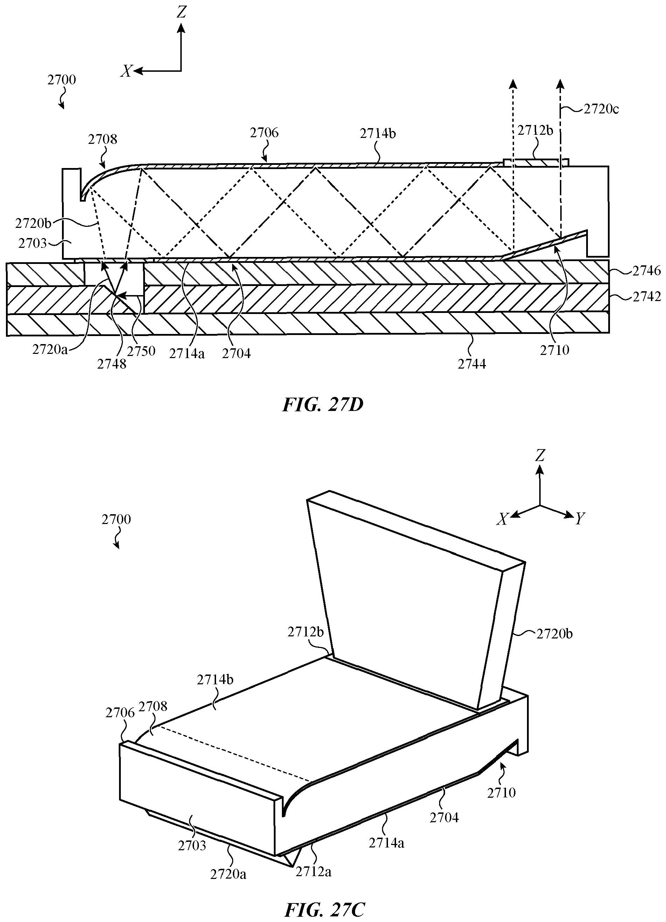

FIGS. 27A and 27B show side and perspective views, respectively, of a beam shifting optic 2700 as described herein. The beam shifting optic is configured to receive and laterally shift a light beam. For the purpose of illustration, the light beam is shown in FIG. 27A as having a first portion that is incident on the beam shifting optic 2700 (referred to herein as the "incoming portion 2720a" of the light beam), a second portion that is carried by the beam shifting optic 2700 (referred to herein as the "intermediate portion 2720b" of the light beam), and a third portion that exits the beam shifting optic 2700 (referred to herein as the "outgoing portion 2720c" of the light beam). FIG. 27C shows a perspective view of the beam shifting optic 2700 with the incoming portion 2720a entering the beam shifting optic 2700 and the outgoing portion 2720c exiting the beam shifting optic 2700.

The beam shifting optic 2700 may be formed from a substrate 2703. The substrate 2703 may be formed from a material that is transparent at any wavelength (or wavelengths) that will be received by the beam shifting optic 2700 (e.g., any of the measurement wavelengths used by an optical measurement system, simultaneously or sequentially, to generate an emission light beam). For example, the substrate 2703 may be formed from a silicon wafer, a glass wafer, or the like.

The substrate 2703 may include a bottom surface 2704 and a top surface 2706 opposite the bottom surface 2704. The bottom surface 2704 includes at least one planar region that is parallel to at least one planar region of the top surface 2706. The beam shifting optic 2700 may be positioned within a portion of an optical measurement system (e.g., a beam generator of the optical measurement systems described herein), such that an incoming portion 2720a of light beam is incident on an input region of the bottom surface 2704 (e.g., a portion of a planar region of the bottom surface 2704) with a predetermined angle of incidence. The top surface 2706 may be processed or otherwise shaped to define a first angled facet 2708. The first angled facet 2708 may be orientated at a first non-zero wedge angle relative to input region the bottom surface 2704 (e.g., in a first dimension along the XZ plane of the cartesian coordinate system shown there), such that the first angled facet 2708 is not parallel to the input region of the bottom surface 2704.

When the incoming portion 2720a of the light beam is incident on the input region of the bottom surface 2704 with the predetermined angle of incidence, the intermediate portion 2720b of the light beam enters the substrate 2703. The intermediate portion 2720b will travel from the bottom surface 2704 to the top surface 2706, where the intermediate portion 2720b will be incident on the first angled facet 2708. The angled facet 2708 is angled (e.g., at the first wedge angle) such that, when the intermediate portion 2720b is incident on the angled facet 2708, the intermediate portion 2720b is redirected toward the bottom surface 2704 in the first dimension. The intermediate portion 2720b will reflect back and forth between the bottom surface 2704 and the top surface 2706 until the intermediate portion 2720b is incident on an output region of the top surface 2706.

Specifically, the bottom surface 2704 may be processed or otherwise shaped to define a second angled facet 2710. The second angled facet 2710 may be orientated at a second non-zero wedge angle (in the first dimension) relative to output region of the top surface 2706, such that the second angled facet 2710 is not parallel to the output region of the top surface 2706. The relative angles of the first and second angled facets 2708, 2710 may at least partially control how the intermediate portion 2720b of the light beam travels through the beam shifting optic 2700. For example, the first wedge angle of the first angled facet 2708 may be selected such that the intermediate portion 2720b, after a certain number of reflections off the top and bottom surfaces 2706, 2704, will be incident on the second angled facet 2710. The second wedge angle of the second angled facet 2710 may be selected to decrease the angle of incidence of the intermediate portion 2720b as it reaches the output region of the top surface 2706. This may facilitate light exiting the beam shifting optic 2700 through the output region of the top surface 2706 at a near-normal incidence. In the variation shown in FIGS. 27A-27C, the input region of the bottom surface 2704 is parallel to the output region of the top surface 2706. In this way, the light beam may enter and exit the light beam along approximately the same direction, depending on the exact angles of incidence at which the light beam enters and exits the beam shifting optic 2700.

Specifically, it may be desirable for the light beam to both enter and exit the beam shifting optic 2700 at near-normal angles of incidence (e.g., within ten degrees of normal incidence), which may help control aberrations and reduce reflections occurring at these interfaces. It may also be desirable for these angles of incidence to be non-normal angles of incidence, which may reduce the likelihood that etalons are generated due to interaction of light with the beam shifting optic 2700. For example, the beam shifting optic 2700 may be positioned within an optical measurement system such that the incoming portion 2720a of the light beam may be incident on the input region of the bottom surface 2704 at a first non-normal angle of incidence that is less than ten degrees from normal incidence. In some of these variations, the first non-normal angle of incidence is less than six degrees from normal incidence.

Similarly, when the incoming portion 2720a is incident on the input region of the bottom surface 2704 at the first non-normal angle of incidence, the first and second angled facets 2708, 2710 may be angled such that the intermediate portion 2720b is incident on the output region of the top surface 2706 at a second non-normal angle of incidence. The second non-normal angle of incidence may be the same as or different from the first non-normal angle of incidence. In some variations, the second non-normal angle of incidence is less than ten degrees from normal incidence. In some of these variations, the second non-normal angle of incidence is less than six degrees from normal incidence.

In some variations one or both of the top and bottom surfaces 2706, 2704 of the beam shifting optic 2700 may be at least partially coated with an AR coating. For example, in the variation shown in FIGS. 27A-27C, the beam shifting optic 2700 includes a first AR coating 2712a positioned on the bottom surface 2704 and a second AR coating 2712b positioned on the top surface 2706. The first AR coating 2712a may be positioned over the input region of the bottom surface 2704, such that the light beam passes through the first AR coating 2712a as it enters the beam shifting optic 2700. Similarly, the second AR coating 2712b may be positioned over the output region of the top surface 2706, such that the light beam passes through the second AR coating 2712b as it exits the beam shifting optic 2700. Collectively, the first and second AR coatings 2712a-2712b may act to reduce reflections that would otherwise occur as light enters and exits the beam shifting optic 2700.

The beam shifting optic 2700 may be configured such that the intermediate portion 2720b may, while traveling through the beam shifting optic 2700, reflect off the top and bottom surfaces 2706, 2704 in any suitable manner. For example, in some variations the first wedge angle of the first angled facet 2708 and the second wedge angle of the second angled facet 2710 are selected such that each reflection of the light beam that occurs within the beam shifting optic 2700 occurs via total internal reflection. In this instances, the entire bottom surface 2704 may be covered by the first AR coating 2712a and/or the entire top surface 2706 may be covered by the second AR coating 2712b. This may reduce processing complexity as compared to instances in which one or both of these coatings are patterned to cover a subset of the respective surfaces.

In other variations, one or both of the top and bottom surfaces 2706, 2704 may be partially covered by a corresponding reflective coating, such as those described with respect to the polarizing beamsplitter 2602 of FIG. 26. For example, in the variation shown in FIGS. 27A-27C, the beam shifting optic 2700 includes a first reflective coating 2714a positioned over a portion of the bottom surface 2704 and a second reflective coating 2714b positioned over a portion of the top surface 2706. The first reflective coating 2714a, which in some variations may be positioned over the second angled facet 2710, may promote reflection off of the bottom surface 2704. Similarly, the second reflective coating 2714b, which in some variations may be positioned over the first angled facet 2708, may promote reflections off of the top surface 2706. This may allow the intermediate portion 2720b of the light beam to reflect off of the top surface 2706 and/or the bottom surface 2704 at angles that would not result in total internal reflection (and would thereby cause some light to exit the beam shifting optic 2700).

In some variations, the first angled facet 2708 may be formed from a surface that is curved in a first dimension (e.g., along an XZ plane using the cartesian coordinate system depicted in FIGS. 27A-27C). In these instances, the curved first angled facet 2708 may be used to change the divergence of the intermediate portion 2720b of the light beam in the first dimension as it is reflected off of the first angled facet 2708. For example, in the variation shown in FIGS. 27A-27C, the first angled facet 2708 is a curved surface that is convex in the first dimension. In these instances, a curved first angled facet 2708 that is convex in the first dimension may act to reduce the divergence of the intermediate portion 2720b of the light beam as it reflects off of the curved first angled facet 2708. If the incoming portion 2720a of the input light beam is diverging in the first dimension as it reaches the bottom surface 2704, the intermediate portion 2720b may also diverge in the first dimension as the light beam approaches the first angled facet 2708. A convex first angled facet 2708 may at least partially collimate the intermediate portion 2720b in the first dimension. Accordingly, the first angled facet 2708 may at least partially determine the beam width and the beam vergence of the light beam in the first dimension as it exits the beam shifting optic 2700.

In some variations, the second angled facet 2710 may also be curved in the first dimension. For example, the second angled facet 2710 may be a curved surface that is convex in the first dimension, such that the second angled facet 2710 also at least partially collimates the intermediate portion 2720b in the first dimension. In these instances, the curved first and second angled facets 2708, 2710 may collectively shape the light beam, such that the light beam is at least partially collimated as it exits the beam shifting optic 2700. For example, the first angled facet 2708 may partially collimate the intermediate portion 2720b in the first dimension such that the light beam is partially collimated as it reaches the second angled facet 2710. The second angled facet 2710 may further collimate the intermediate portion 2720b in the first dimension (e.g., to fully collimate the light beam in the first dimension). This may provide greater flexibility in providing a desired beam width and beam vergence in the first dimension as the light beam exits the beam shifting optic 2700.

In some variations, the beam shifting optic 2700 may be incorporated into an optical measurement system as described herein, and may be used to laterally shift a light beam generated by another component of the optical measurement system. For example, FIG. 27D shows a partially cross-sectional side view of a variation of a beam generator 2730 that includes the beam shifting optic 2700 of FIGS. 27A-27D and a photonic integrated circuit 2740.

In the variation shown in FIG. 27D, the photonic integrated circuit 2740 is configured to emit light vertically out of a top surface of the photonic integrated circuit 2740. Specifically, the photonic integrated circuit 2740 may include a waveguide layer 2742 that may be positioned on a first cladding layer 2744. The waveguide layer 2742 may be etched or patterned to define one or more waveguides. Light may be confined in the one or more waveguides to carry light within the photonic integrated circuit 2740. In some instances, the photonic integrated circuit 2740 may include one or more additional cladding layers (e.g., a second cladding layer 2746) that may further assist in confining light in the waveguides of the waveguide layer 2742.

The photonic integrated circuit 2740 includes a vertical output coupler 2748 that is configured to redirect light (e.g., using a reflective surface or an angled facet) out of the plane of the waveguide layer 2742 and emit the light from the photonic integrated circuit 2740 as light beam 2750. In other words, light may travel laterally within the photonic integrated circuit 2740 (e.g., along the X-axis as shown in FIG. 27D), and may exit the photonic integrated circuit 2740 (e.g., along the Z-axis as shown in FIG. 27D). The beam shifting optic 2700 may be positioned relative to the photonic integrated circuit 2740 such that the light beam 2750 redirected by the vertical output coupler 2748 forms the incoming portion 2720a of the light beam received by the beam shifting optic 2700. Accordingly, while the light beam 2750 may leave the photonic integrated circuit 2740 (e.g., along the Z-axis) at a first location along the X-axis, the light beam 2750 may be laterally shifted by the beam shifting optic 2700 to exit the beam shifting optic 2700 (e.g., along the Z-axis) at a second location along the X-axis. This may allow some flexibility in the placement of the of the vertical output coupler 2748 within the photonic integrated circuit 2740. Additionally, because the light beam 2750 is laterally shifted by the beam shifting optic 2700, other elements of the beam generator 2730 may be placed closer to the photonic integrated circuit 2740 along the Z-axis.

For example, the beam shifting optic 2700 may be incorporated into the beam generator 1302 described herein with respect to FIGS. 13A-13F. In these instances, the beam shifting optic 2700 may receive the input light beam 1324 and laterally shift the input light beam 1324, for example along the X-axis as shown in FIGS. 13B-13F, such that the input light beam 1324 enters the beam shifting optic 2700 at a first position along the X-axis and exits the beam shifting optic 2700 at a second position along the X-axis. In this way, the input light beam 1324 will travel a certain distance while being laterally shifted along the X-axis. This distances may depend on the width of the substrate 2703 as well as the number of reflections that occur within the beam shifting optic 2700, and may count as part of the track length of the beam generator 1302. Accordingly, this may allow the slow axis collimating lens 1328 to be positioned closer to the photonic integrated circuit 1314 along the Z-axis. This may reduce the overall size of the beam generator 1302 in the Z-axis, which may reduce the overall size a launch architecture (e.g., launch architectures 1300) that incorporates the beam generator 1302.

In instances where the first angled facet 2708 and/or the second angled facet are curved in a first dimension, the beam shifting optic 2700 may also act as the fast axis collimating lens 1326 of the beam generator 1302. In this way, the beam shifting optic 2700 may be positioned to receive the input light beam 1324 as it is emitted from the photonic integrated circuit 1314, and may at least partially collimate the input light beam 1324 in the first dimension (in the same manner as the fast axis collimating lens 1326 would otherwise have at least partially collimated the input light beam 1324 in the variations described in FIGS. 13B-13F). In some instances, the beam shifting optic 2700 is configured to fully collimate the input light beam 1324 in the first dimension such that the input light beam 1324 is fully collimated in the first dimension as it exits the beam shifting optic 2700. In other variations, the beam shifting optic 2700 is configured to partially collimate the input light beam 1324, such that the input light beam 1324 is partially collimated in the first dimension as it exits the beam shifting optic 2700.

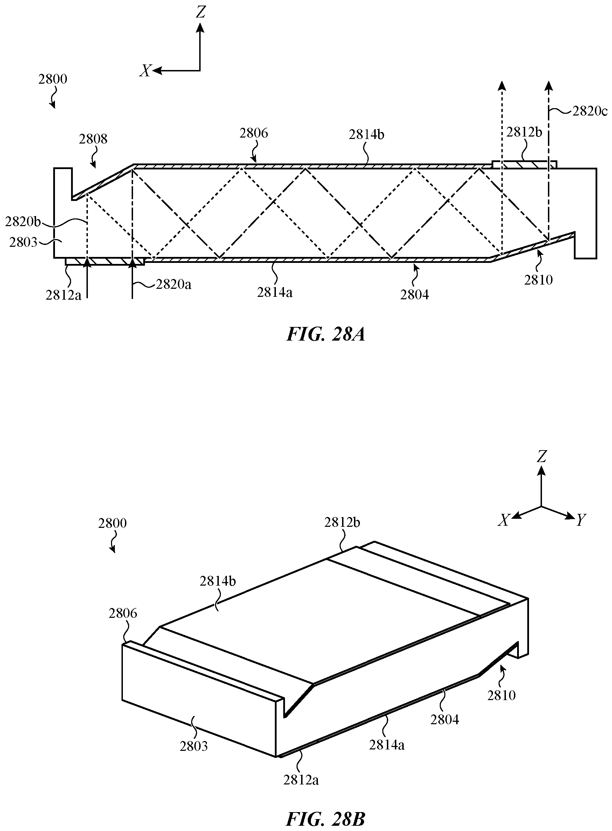

In other variations, both the first angled facet 2708 and the second angled facet may be flat surfaces. For example, FIGS. 28A and 28B show one such variation of a beam shifting optic 2800 as described herein. The beam shifting optic 2800 includes a substrate 2803 having a bottom surface 2804 and a top surface 2806. The top surface 2806 may be processed or otherwise shaped to define a first angled facet 2808, and the bottom surface 2804 may be processed or otherwise shaped to define a first angled facet 2810. The beam shifting optic 2800 may be configured in any manner as described above with respect to the beam shifting optic 2700 of FIGS. 27A-27D, except that the both the first angled facet 2808 and the second facet 2810 are each flat surfaces. In these instances, a light beam traveling through the beam shifting optic 2800 may maintain its beam vergence as the light beam reflects of the top and bottom surfaces 2806, 2804 of the beam shifting optic 2800.

For example, the beam shifting optic 2800 may be positioned within an optical measurement system such that the incoming portion 2820a of a light beam may be incident on an input region of the bottom surface 2804 (e.g., at a first predetermined non-normal angle of incidence such as those described herein with respect to FIGS. 27A-27C). An intermediate portion 2820b of the light beam will enter the substrate 2803a via the input region of the bottom surface 2804. The intermediate portion 2802b will travel from the bottom surface 2804 to the top surface 2806 such that the intermediate portion 2820b is incident on the first angled facet 2808. The intermediate portion 2820b reflect off of the first angled facet 2808 toward the bottom surface 2804, and will reflect back and forth between the bottom and top surfaces 2804, 2806 until the intermediate portion 2820b is incident on the second angled facet 2810. The second angled facet 2810 is angled such that the intermediate portion 2820b, after reflecting off of the second angled facet 2810, is incident on an output region of the top surface 2806 (e.g., at a second non-normal angle of incidence, such as those described herein with respect to FIGS. 27A-27C). An outgoing portion 2820c of the light beam exits the beam shifting optic 2800 through the output region of the top surface 2806.

In the variation shown in FIGS. 28A and 28B, the beam shifting optic 2800 includes a first AR coating 2812a positioned over the input region of the bottom surface 2804 and a first reflective coating 2814a positioned over another portion of the bottom surface 2804. The beam shifting optic 2800 includes a second AR coating 2812b positioned over the output region of the top surface 2806 and a second reflective coating 2814b positioned over another portion of the top surface 2806. In other instances, the entire top surface 2806 and/or the entire bottom surface 2804 may be coated with a corresponding AR coating, such as described in more detail with respect to FIGS. 27A-27C.

Because the light beam only interacts with flat surfaces as it enters, reflects within, and exits the beam shifting optic 2800, the beam shifting optic 2800 will not change the beam vergence (aside from the temporary change in beam vergence that occurs due to the refractive index of the substrate 2803 as the intermediate portion 2820b traverses the substrate 2803). Accordingly, when beam shifting optic 2800 is incorporated into the beam generator 1302 described herein with respect to FIGS. 13A-13F, it may be desirable to at least partially collimate the input light beam 1324 along its fast axis before the input light beam 1324 enters the beam shifting optic 2800.

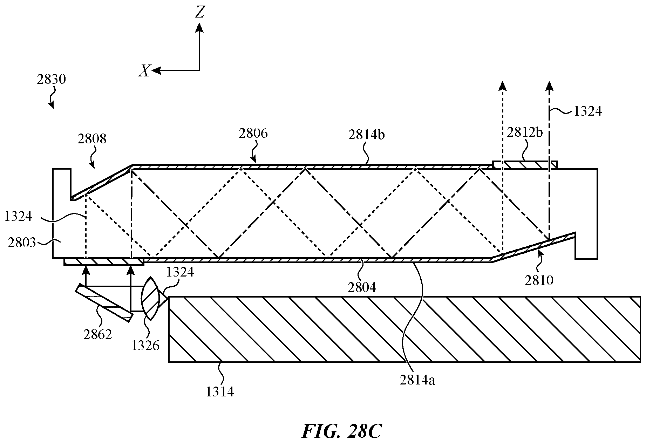

For example, FIG. 28C shows a side view of a variation of a portion of a beam generator 2830. Specifically, the beam generator 2830 may include the beam shifting optic 2800 of FIGS. 28A and 28B, as well as the components of the beam generator 1300 described with respect to FIGS. 13A-13F (only the photonic integrated circuit 1314 and the fast axis collimating lens 1326 are depicted in FIG. 28C). The photonic integrated circuit 1314 emits the input light beam 1324 from a side surface of the photonic integrated circuit 1314, and the fast axis collimating lens 1326 is positioned to receive the input light beam 1324. The fast axis collimating lens 1326 is configured to at least partially collimate the input light beam 1324 along a first dimension (e.g., along its fast axis). The beam shifting optic 2800 is positioned within the beam generator 2830 such that it receives the input light beam 1324 after the input light beam 1324 has passed through the fast axis collimating lens 1326.

Accordingly, the input light beam 1324 may be at least partially collimated in the first dimension as it enters the beam shifting optic 2800. If the input light beam 1324 is fully collimated in the first dimension as it enters the beam shifting optic 2800, the input light beam 1324 will remain collimated in the first dimension as it passes through the beam shifting optic 2800. In these instances, the beam width of the input light beam 1324 in the first dimension be the same as it enters and exits the beam shifting optic 2800. Conversely, when the input light beam 1324 is partially collimated in the first dimension as it enters the beam shifting optic 2800, the input light beam 1324 will continue to diverge in the first dimension as it passes through the beam shifting optic 2800. Accordingly, the beam width of the input light beam 1324 in the first dimension will increase between when it enters and exits the beam shifting optic 2800. Additionally, because the input light beam 1324 is diverging along a second dimension (e.g., its slow axis) as it enters the beam shifting optic 2800, the input light beam 1324 will continue to diverge in the second dimension as it passes through the beam shifting optic 2800. In this way, the beam width of the input light beam 1324 in the second dimension will increase between when it enters and exits the beam shifting optic 2800.

In some instances, it may be desirable to change the direction of the input light beam 1324 between when it is emitted from the photonic integrated circuit 1314 and when it enters the beam shifting optic 2800. This may allow the input light beam 1324 to be emitted from a side surface of the photonic integrated circuit 1314 without requiring the photonic integrated circuit 1314 to be positioned such that is side surface faces the sampling interface of an optical measurement system (such as shown in FIGS. 13A-13F). This may help to reduce the height (e.g., along the Z-axis) of the optical measurement system. In some variations, such as shown in FIG. 28C, the beam generator 2830 includes a mirror 2862. The mirror may be positioned such that it receives the input light beam 1324 along a first direction (e.g. along the X-axis as shown in FIG. 28C) after it has been at least partially collimated by the fast axis collimating lens 1326, and reflects the input light beam 1324 along a second direction (e.g., along the Z-axis as shown in FIG. 28C). The mirror 2862 and the beam shifting optic 2800 may be positioned such that the reflected input light beam 1324 forms the incoming portion of the light beam that is received by the beam shifting optic 2800. In other words, the beam generator 2830 may be configured such that the input light beam 1324 enters the input region of the bottom surface 2804 of the beam shifting optic 2800 (e.g., at a first predetermined non-normal angle of incidence as described herein). The beam shifting optic 2800 will laterally shift the input light beam, such that it will enter and exit the beam shifting optic 2800 at different positions along the X-axis. While the beam shifting optic 2800 is shown as spaced from the photonic integrated circuit 1314 along the Z-axis, it should be appreciated that in some instances the beam shifting optic 2800 may be positioned such that that bottom surface 2804 is positioned in contact with a top surface of the photonic integrated circuit 1314 (such as illustrated with respect to the beam shifting optic 2700 of FIG. 27D).

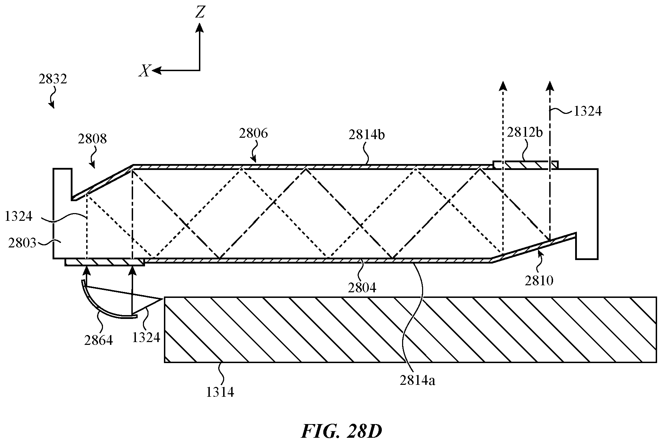

While the mirror 2862 shown in FIG. 28C is flat, in other instances the mirror 2862 may curved in a first dimension (e.g., corresponding to the fast axis of the input light beam 1324) such that the mirror 2862 may act to at least partially collimate the input light beam in the first dimension. FIG. 28D shows a side view of a portion of a beam generator 2832 that may be configured the same as the beam generator 2830 of FIG. 28C, except that the flat mirror 2862 and fast axis collimating lens 1326 have been replaced by a curved mirror 2864. The curved mirror 2864 is curved in a first dimension, such that it may act as the fast axis collimating lens 1326 to at least partially collimates the input light beam 1324 in the first dimension. The curved mirror 2864 may be positioned such that it receives the input light beam 1324 along a first direction (e.g. along the X-axis as shown in FIG. 28D) after it has been emitted by the photonic integrated circuit 1314, and reflects the input light beam 1324 along a second direction (e.g., along the Z-axis as shown in FIG. 28D) toward the beam shifting optic 2800. The input light beam 1324 is at least partially collimated in the first dimension as it is reflected off of the curved mirror 2864, such that input light beam is at least partially collimated in the first dimension as it enters the beam shifting optic 2800. In this way, the curved mirror 2864 may perform the redirecting and collimating functions, respectively, of the flat mirror 2862 and the fast axis collimating lens 1326 of FIG. 28C.

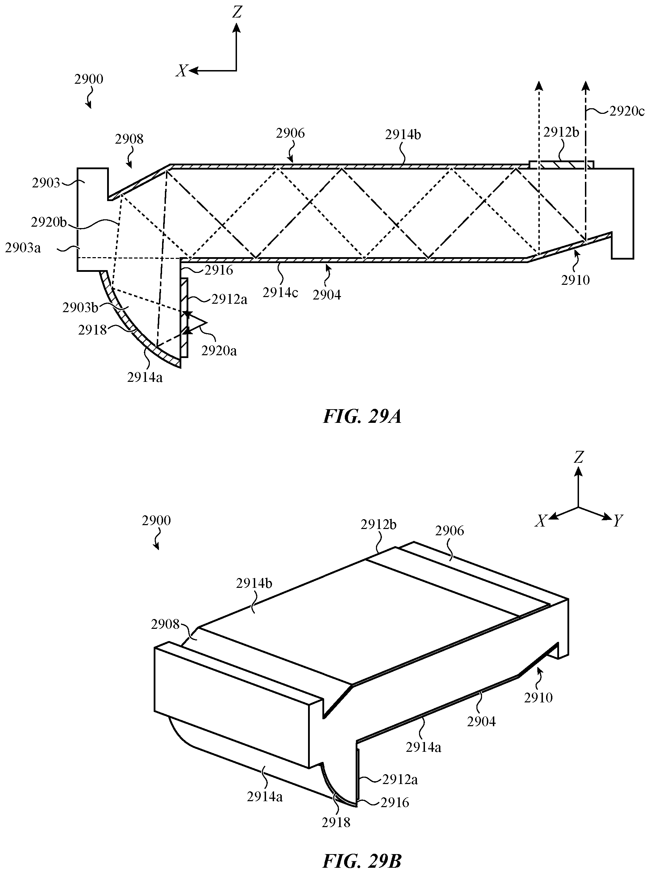

In still other variations, it may be desirable to incorporate the functionality of the curved mirror 2864 into a beam shifting optic, such that the beam shifting optic may both i) turn a light beam and ii) laterally shift the light beam. FIGS. 29A and 29B show side and perspective views, respectively, of a variation of a beam shifting optic 2900 having perpendicular entry and exit surfaces. Specifically, the beam shifting optic 2900 may be formed from a substrate 2903 that has a beam shifting section 2903a and a beam turning section 2903b. The beam shifting section 2903a may be formed with a bottom surface 2904 and a top surface 2906. The top surface 2906 may be processed or otherwise shaped to define a first angled facet 2908 and the bottom surface 2904 may be processed or otherwise shaped to define a second angled facet 2910. The beam turning section 2903b may define a first side surface 2916 and a second side surface 2918. In the variation shown in FIGS. 29A and 29B, the first side surface 2916 is flat and the second side surface 2918 is curved.

The first side surface 2916 forms an input region of the beam shifting optic 2900. Accordingly, the beam shifting optic 2900 may be positioned (e.g., within an optical measurement system) to receive an incoming portion 2920a of a light beam at the first side surface 2916 (e.g., at a first predetermined angle of incidence as described herein). In some variations, the beam shifting optic 2900 may comprise a first AR coating 2912a positioned on the first side surface 2916, such that the light beam passes through the first AR coating 2912a as it enters the beam shifting optic 2900. The second side surface 2918 is positioned opposite the first side surface 2916, such that when the light beam enters the beam shifting optic 2900, an intermediate portion 2920b of the light beam will be incident on the second side surface 2918.

The curve of the second side surface 2918 is selected such that the intermediate portion 2920b of the light beam will, upon reflecting off of the second side surface 2918, be redirected toward the first angled facet 2908. Additionally, when the incoming portion 2920a of the light beam is diverging in a first dimension (e.g., along the fast axis of the light beam), the second side surface 2918 may also at least partially collimate the intermediate portion 2920b of the light beam in the first dimension. Depending on the design of optical measurement system that incorporates the beam shifting optic 2900, it may be desirable to fully collimate the light beam or partially collimate the light beam in the first dimension. In some variations, the beam shifting optic 2900 may include a first reflective coating 2914a positioned on the curved second side surface 2918, which may promote reflection of the intermediate portion 2920b of the light beam off of the curved second side surface 2918.

The beam shifting optic 2900 is further configured such that when the light beam enters the beam shifting optic 2900 through the input region on the first side surface 2916 at the predetermined angle of incidence, the intermediate portion 2920b will be incident on the first angled facet 2908 after reflecting off of the curved second side surface 2918. The first angled facet 2908 may be angled such that the intermediate portion 2920b of the light beam may reflected off of the first angled facet 2908 toward the bottom surface 2904. The intermediate portion 2920b of the light beam may reflect back and forth between the bottom and top surfaces 2904, 2906 until it is incident on an output region of the top surface 2906. The light beam may exit the beam shifting optic 2900 (e.g., as the outgoing portion 2920c of the light beam) through the output region of the top surface 2906. As part of this, the intermediate portion 2920b of the light beam may be incident on the second angled facet 2910, which may decrease the angle of incidence of the light beam as it reaches the output region of the top surface 2706.

The beam shifting optic 2900 may also include a second AR coating 2912b positioned over the output region of the top surface 2706, such that the outgoing portion 2920c of the light beam passes through the second AR coating 2912b as it exits the beam shifting optic 2900. Depending on the angle or angles at which the intermediate portion 2920b of the light beam reflects off of the top surface 2906 (e.g., at the first angled facet 2908), the second AR coating 2912b may cover the entire top surface 2906. Alternatively, the second AR coating 2912b may be patterned cover a first portion of the top surface 2906 (e.g., corresponding to the output region) and a second reflective coating 2914b may be positioned over a second portion of the top surface 2906. In some of these variations, the second reflective coating 2914b may be positioned on the first angled facet 2809. Additionally or alternatively, the beam shifting optic 2900 may include a third reflective coating 2914c may be positioned to cover some or all of the bottom surface 2904. It should also be appreciated that one or both of the first and/or second angled facets 2908, 2910 may be curved in the first dimension, such that these facets may also act to change the divergence (e.g. decrease the divergence) of the light beam in the first dimension.

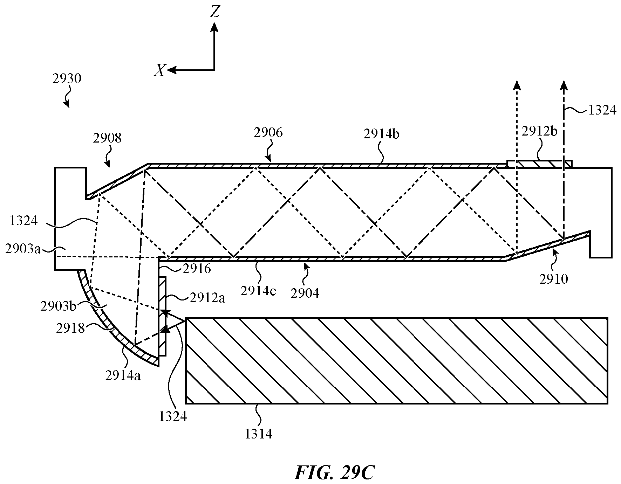

FIG. 29C shows a side view of a variation of a portion of a beam generator 2930 that incorporates the beam shifting optic 2900 of FIGS. 29A and 29B, as well as the components of the beam generator 1300 described with respect to FIGS. 13A-13F (only the photonic integrated circuit 1314 is depicted). In this example, the beam shifting optic 2900 may act as the fast axis collimating lens 1326. Accordingly, the beam shifting optic 2900 may be positioned relative to the photonic integrated circuit 1314 such the first side surface 2916 faces a side surface of the photonic integrated circuit 1314. In this way, the photonic integrated circuit 1314 is positioned such that, when the photonic integrated circuit 1314 emits the input light beam 1324 from the side surface of the photonic integrated circuit 1314, the input light beam 1324 will be directed toward the input region of the first side surface 2916. Accordingly, the input light beam 1324 will act as the incoming portion of the light beam received by the beam shifting optic 2900.

The beam shifting optic 2900 will at least partially collimate the input light beam 1324 in the first dimension (e.g., along its fast axis), redirect the beam, and laterally shift the beam. As shown in FIG. 29C, the input light beam 1324 may exit the photonic integrated circuit 1314 at a first point along the X-axis, such that the input light beam 1324 is travelling along the X-axis. After leaving the beam shifting optic 2900, the input light beam 1324 may be traveling along the Z-axis at a different point along the X-axis. Accordingly when the beam shifting optic 2900 is incorporated into the beam generator 1300 of FIGS. 13A-13F, this may allow the photonic integrated circuit 1314 to be rotated 90 degree relative its orientation shown in FIGS. 13A-13F. This may further reduce the length of the beam generator 1300 along the Z-axis. While the beam shifting optic 2900 is shown as spaced from the photonic integrated circuit 1314 along the Z-axis, it should be appreciated that in some instances the beam shifting optic 2900 may be positioned such that that bottom surface 2904 is positioned in contact with a top surface of the photonic integrated circuit 1314 (such as illustrated with respect to the beam shifting optic 2700 of FIG. 27D).

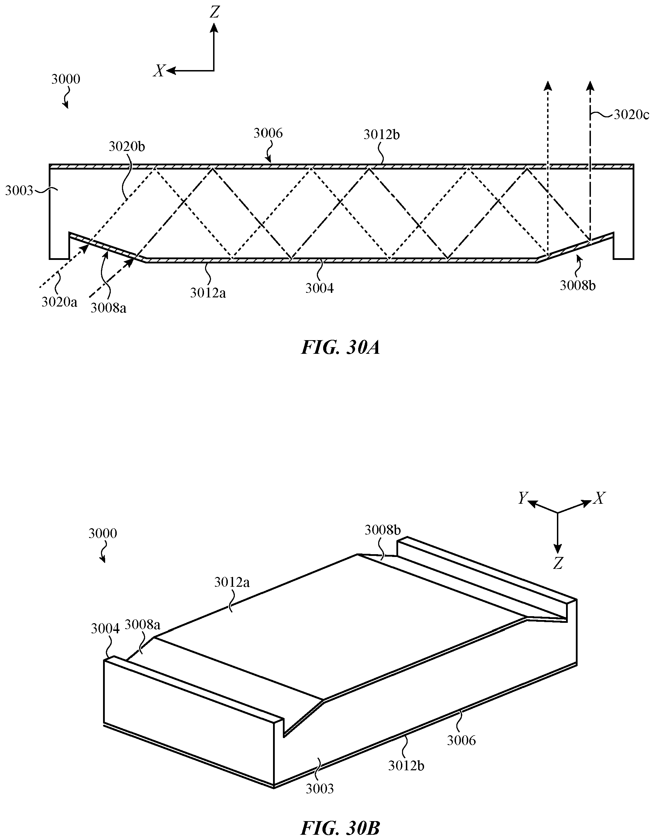

In some variations, it may be desirable for a beam shifting optic as described here to have a top surface without any angled facets, which may simplify the manufacturing of the beam shifting optic. For example, FIGS. 30A and 30B show one such variation of a beam shifting optic 3000. As shown there, the beam shifting optic 3000 includes a substrate 3003 having a bottom surface 3004 and a top surface 3006. The entire top surface 3006 may be a planar surface, such that the top surface 3006 does not include any angled facets. The bottom surface 3004 may be processed or otherwise shaped to define a first angled facet 3008a and a second angled facet 3008b.

The beam shifting optic 3000 may be positioned to receive an incoming portion 3020a of a light beam through an input region of the first angled facet 3008a (e.g., at a first predetermined angle of incidence such as described herein). The beam shifting optic 3000 may be configured such that, when the incoming portion 3020a of the light beam enters the beam shifting optic 3000 through the input region of the first angled facet 3008a, an outgoing portion 3020c of the light beam will exit the beam shifting optic 3000 through an output region of the top surface 3006. Specifically, the first angled facet 3008a may be orientated at a first non-zero wedge angle relative to the top surface 3006 (e.g., in a first dimension along the XZ plane of the cartesian coordinate system shown there), such that the first angled facet 3008a is not parallel to the output region of the top surface 3006. Similarly, the second angled facet 3008b may be orientated at a second non-zero wedge angle relative to the top surface 3006 in the first dimension, such that the second angled facet 3008b is not parallel to the output region of the top surface 3006.

As the light beam enters the beam shifting optic 3000 through the first angled facet 3008a, an intermediate portion 3020b of the light beam is directed toward the top surface 3006. The intermediate portion 3020b of the light beam may reflect back and forth off of the top and bottom surfaces 3006, 3004, until the intermediate portion 3020b of the light beam is incident on the second angled facet 3008b. The second angled facet 3008b may direct the light beam toward the output region of the top surface 3006, such that the light beam exits the beam shifting optic 3000. Specifically, the second angled facet 3008b may, along with the first angled facet 3008a, determine the angle of incidence of the intermediate portion 3020b as it reaches the output region of the top surface 3006. Accordingly, the beam shifting optic 3000 may be configured such that the incoming portion 3020a of the light beam enters the beam shifting optic at a first non-normal angle of incidence (such as those described herein with respect to FIGS. 27A-27C) and the intermediate portion 3020b of the light beam is incident on the output region of the top surface 3006 at a second non-normal angle of incidence (such as those described herein with respect to FIGS. 27A-27C).

The beam shifting optic 3000 may comprise a first AR coating 3012a positioned on the bottom surface 3004 and a second AR coating 3012b positioned on the top surface 3006. The first AR coating 3012a may be positioned on the input region of the first angled facet 3008a, such that the light beam passes through the first AR coating 3012a as it enters the beam shifting optic 3000. Similarly, the second AR coating 3012b may be positioned on the output region of the top surface 3006, such that the light beam passes through the second AR coating 3012b as it exits the beam shifting optic 3000.

In some variations, the first angled facet 3008a may be angled such that the intermediate portion 3020b of the light beam will enter the beam shifting optic 3000 at an angle that allows the light beam to reflect off of the top surface 3006 via total internal reflection. In these instances, the additional reflections off of the top surface 3006 and bottom surface 3004 may be at the same angle and thereby continue to reflect via total internal reflection until the intermediate portion 3020b of the light beam reaches the second angled facet 3008b. In some of these variations, the second AR coating 3012b may be positioned to cover the entire top surface 3006. In some variations, the second angled facet 3008b may be angled such that the intermediate portion 3020b of the light beam reflects off of the second angled facet 3008b via total internal reflection. In these instances, the first AR coating 3012a may be positioned to cover the entire bottom surface 3004. It should also be appreciated that one or both of the top and bottom surfaces 3006, 3004 may include a corresponding reflective coating positioned thereon (e.g., positioned at a portion of a corresponding surface at which the intermediate portion 3020b of the light beam is incidence thereon at an angle that would not result in total internal reflection).

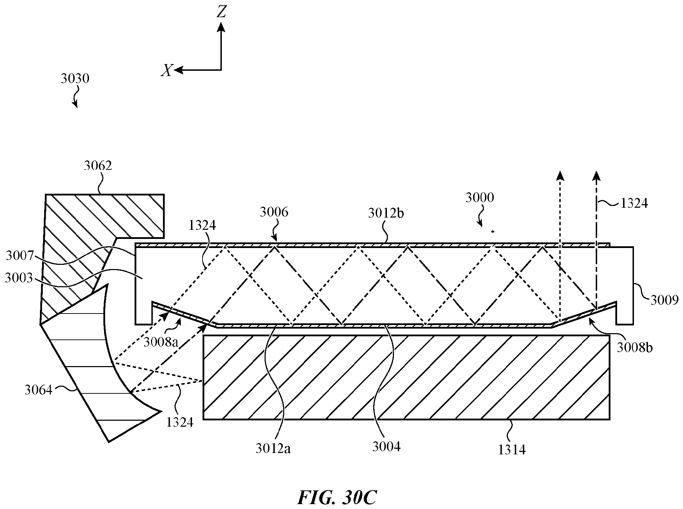

Additionally, because the input region of the first angled facet 3008a is not parallel to the output region of the top surface 3006, the light beam may enter and leave the beam shifting optic 3000 along different directions. For example, FIG. 30C shows a side view of a variation of a portion of a beam generator 3030 that incorporates the beam shifting optic 3000 of FIGS. 30A and 30B, as well as the components of the beam generator 1300 described with respect to FIGS. 13A-13F (only the photonic integrated circuit 1314 is depicted). In this example, a curved mirror 3064 may act as the fast axis collimating lens 1326.

As shown there, the photonic integrated circuit 1314 may emit the input light beam 1324 from a side surface of the photonic integrated circuit 1314, and the curved mirror 3064 may face the side surface of the photonic integrated circuit 1314 such that it receives and redirects the input light beam toward the beam shifting optic 3000. In this way, the input light beam 1324 may form the incoming portion of the light beam that is laterally shifted and redirected by the beam shifting optic 3000. Additionally, the curved mirror 3064 may at least partially collimate the input light beam 1324 in the first dimension, such as discussed with respect to the curved mirror 2864 of FIG. 28D. The beam shifting optic 3000 may be positioned relative to the photonic integrated circuit 1314 such that the first angled facet 3008a of the beam shifting optic 3000 and the side surface of the photonic integrated circuit 1314 that emits the input light beam 1324 are positioned at an oblique angle relative to each other. In these instances, this angle may relax the curvature requirements needed for the curved mirror 3064 need to redirect and at least partially collimate the input light beam 1324 in the first dimension.

While the beam shifting optic 3000 is shown as spaced from the photonic integrated circuit 1314 along the Z-axis, it should be appreciated that in some instances the beam shifting optic 3000 may be positioned such that that bottom surface 3004 is positioned in contact with a top surface of the photonic integrated circuit 1314 (such as illustrated with respect to the beam shifting optic 2700 of FIG. 27D). Additionally or alternatively, to position the curved mirror 3064 relative to the beam shifting optic 3000 and the photonic integrated circuit 1314, the beam generator 3030 may include a mounting structure 3062 that may be connected to both the beam shifting optic 3000 (e.g., the mounting structure 3062 may be attached to the top surface 3006 of the beam shifting optic 3000 and act as a hanging connector) and the curved mirror 3064, which may set the relative placement between the curved mirror 3064 and the beam shifting optic 3000. Additionally, the relative placement between the beam shifting optic 3000 and the photonic integrated circuit 1314 may at least partially determine the relative positioning between the curved mirror 3064 and the photonic integrated circuit 1314.

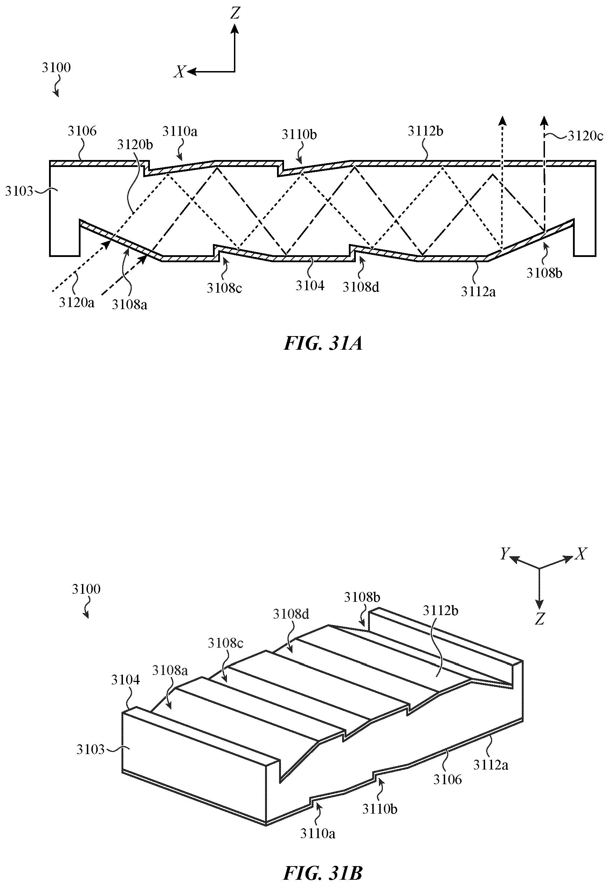

It should be appreciated that any of the beam shifting optics described herein may include additional angled facets as may be desired to assist in laterally shifting a beam of light. For example, FIGS. 31A and 31B show one such variation of a beam shifting optic 3100. The beam shifting optic 3100 includes a substrate 3103 having a bottom surface 3104 and a top surface 3106. The bottom surface 3104 may be processed or otherwise shaped to define a first plurality of angled facets 3108a-3108d and the top surface 3106 may be processed or otherwise shaped to define a second plurality of angled facets 3110a-3110b.

In the variation shown FIGS. 31A and 31B, the first plurality of angled facets 3108a-3108d includes a first angled facet 3108a and a second angled facet 3108b. The beam shifting optic 3100 may be positioned to receive an incoming portion 3120a of a light beam through an input region of the first angled facet 3108a (e.g., at a first predetermined angle of incidence such as described herein). The beam shifting optic 3100 may be configured such that, when the incoming portion 3120a of the light beam enters the beam shifting optic 3100 through the input region of the first angled facet 3108a, an outgoing portion 3120c of the light beam will exit the beam shifting optic 3100 through an output region of the top surface 3106. In the variation shown in FIGS. 31A and 31B, the first angled facet 3108a may be orientated at a first non-zero wedge angle relative to the top surface 3106 (e.g., in a first dimension along the XZ plane of the cartesian coordinate system shown there), such that the first angled facet 3108a is not parallel to the output region of the top surface 3106. Similarly, the second angled facet 3108b may be orientated at a second non-zero wedge angle relative to the top surface 3106 in the first dimension, such that the second angled facet 3108b is not parallel to the output region of the top surface 3106.

In the variation shown in FIGS. 31A and 31B, the first plurality of angled facets 3108a-3108d also includes a third angled facet 3108c and a fourth angled facet 3108d, and the second plurality of angled facets 3110a-3 1 10b includes a fifth angled facet 31 10a and a sixth angled facet 3110b. The first angled facet 3108a may be angled such that an intermediate portion 3120b of the light beam entering through the first angled facet 3108a will be incident on the fifth angled facet 31 10a. The fifth angled facet 31 10a may be positioned at a non-zero angle relative to the first angled face 3108a, such that light beam is reflected off of the fifth angled facet 3110a and directed toward the third angled facet 3108c. The third angled facet 3108c may angled relative to the fifth angled facet 3110a such that the light beam is reflected toward the sixth angled facet 3110b. The sixth angled facet 3110b may be angled relative to the third angled facet 3108c such that the light beam is reflected toward the fourth angled facet 3108d. In this way, the intermediate portion 3120b of the light beam may reflect back-and-forth between different angled facets. In this way, the overall angle change that occurs between the incoming portion 3120a and the outgoing portion 3120c of the light beam may be distributed across several angled facets, which may provide additional design and manufacturing flexibility in laterally shifting the light beam.

Additionally, the corresponding wedge angle of each angled facet of the first and second pluralities of angled facets may be selected such that the intermediate portion 3120b of the light beam may reflect off each angled facet via total internal reflection. In these instances, the entire bottom surface 3104 may be coated with a first AR coating 3112a and the entire top surface 3106 may be coated with a second AR coating 3112b. In other variations, a corresponding portion of one or both of the top and bottom surfaces 3106, 3104 may be coated with a corresponding reflective coating such, as described in more detail herein with respect to the beam shifting optic 2800 of FIGS. 28A and 28B.

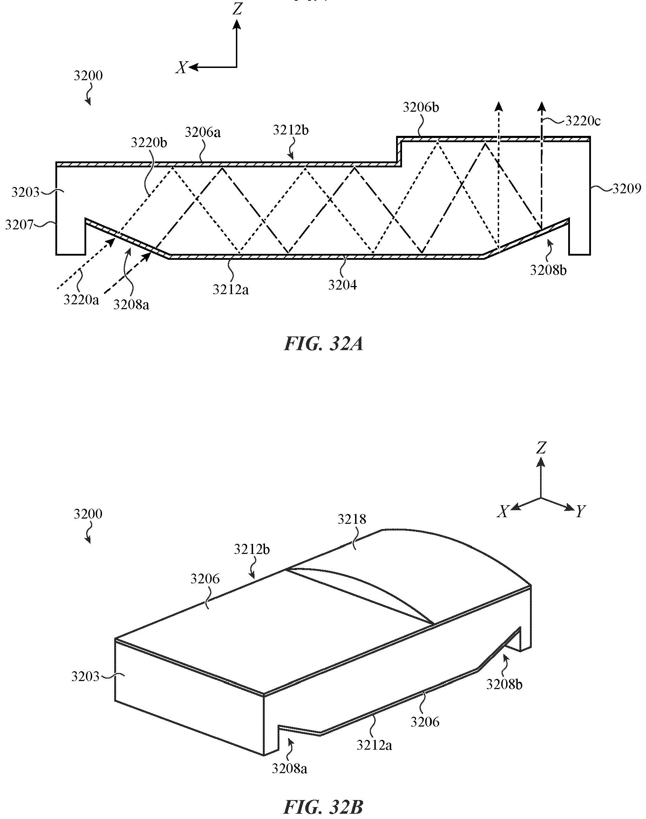

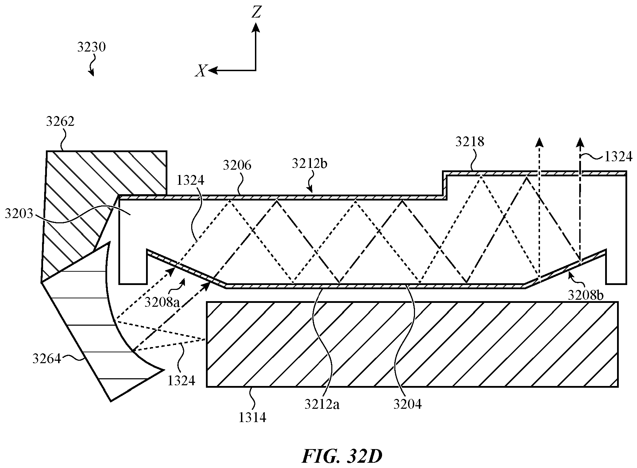

While some of the beam shifting optics described herein (e.g., beam shifting optic 2700 of FIGS. 27A-27D and beam shifting optic 2900 of FIGS. 29A-29C) are configured to change the divergence of an incoming light beam along a first dimension (e.g., a fast axis) of an incoming light beam, it should be appreciated that in some instances a beam shifting optic as described herein may additionally or alternatively be configured to change a beam divergence of the incoming light beam along a second dimension (e.g., a slow axis). For example, FIG. 32A and 32B show a variation of a beam shifting optic 3200 that is configured to laterally shift an incoming light beam along a first dimension and to at least partially collimate the light beam along a second dimension perpendicular to the first dimension. Specifically, the beam shifting optic 3200 may an incoming portion 3220a receive a light beam having a fast axis in the first dimension and a slow axis the second dimension, such as the input light beam 1324 discussed herein with respect to FIGS. 13A-13F. For example, the light beam may have a wider beam width along the slow axis than a corresponding beam width along the fast axis.

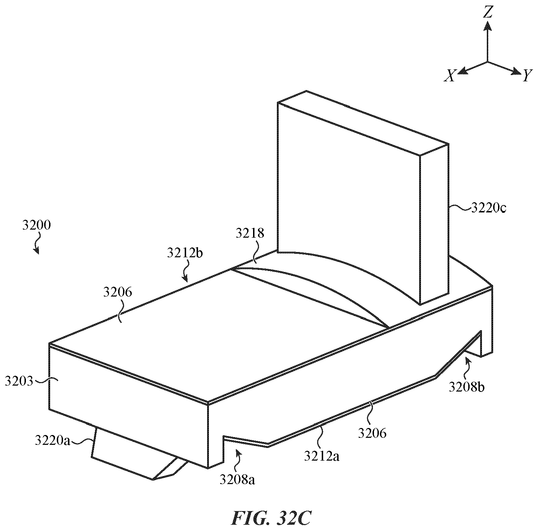

The incoming portion 3220a of the light beam may diverge in the slow axis as the light beam enters the beam shifting optic 3200, and an intermediate portion 3220b of the light beam may be at least partially collimated in the second dimension by the beam shifting optic 3200. In this way, the beam shifting optic 3200 at least partially collimates the light beam in the second dimension, such that an outgoing portion 3220c of the light beam is laterally shifted in the first dimension and at least partially collimated in the second dimension (as compared to the incoming portion 3220a). For example, FIG. 32C shows a perspective view of the beam shifting optic 3200 with the incoming portion 3220a entering the beam shifting optic 3200 and the outgoing portion 3220c exiting the beam shifting optic 3200.

The beam shifting optic 3200 includes a substrate 3203 having a bottom surface 3204 and a top surface 3206. The top surface 3206 may be processed or otherwise shaped to define a lensed facet 3218. The lensed facet 3218 may be curved along the second dimension, such that the lensed facet 3128 may change the divergence of the light beam in the second dimension as the light beam reflects off of or passes through the lensed facet 3128. For example, the lensed facet 3128 may form a cylinder lens.

The incoming portion 3220a of the light beam may enter the beam shifting optic 3200 via an input region (such as an input region of the bottom surface) at a first predetermined angle of incidence such as described herein, and the beam shifting optic 3200 may be configured such that the outgoing portion 3220c of light beam exits the beam shifting optic 3200 through an output region on the lensed facet 3218. The top and/or bottom surfaces may include one or more angled facets, such as those described herein with respect to FIGS. 27A-3 1B that are angled in the first dimension to laterally shift the light beam along the first dimension. For example, in the variation shown in FIGS. 32A-32C, the bottom surface 3204 is processed or otherwise shaped to define a first angled facet 3208a and a second angled facet 3208b.

The beam shifting optic 3200 may be positioned to receive an incoming portion 3220a of a light beam through an input region of the first angled facet 3208a (e.g., at a first predetermined angle of incidence such as described herein). The beam shifting optic 3200 may be configured such that, when the incoming portion 3220a of the light beam enters the beam shifting optic 3200 through the input region of the first angled facet 3208a, an outgoing portion 3220c of the light beam will exit the beam shifting optic 3200 through the output region of the lensed facet 3218. Specifically, the first angled facet 3208a may be orientated at a first non-zero wedge angle in the first dimension relative to the top surface 3206 such that the first angled facet 3208a is not parallel to the output region of the lensed facet 3218 in the first dimension. Similarly, the second angled facet 3208b may be orientated at a second non-zero wedge angle relative to the top surface 3206 in the first dimension, such that the second angled facet 3208b is not parallel to the output region of the lensed facet 3218 in the first dimension.

As the light beam enters the beam shifting optic 3200 through the first angled facet 3208a, an intermediate portion 3220b of the light beam is directed toward the top surface 3206. The intermediate portion 3220b of the light beam may reflect back and forth off of the top and bottom surfaces 3206, 3204, until the intermediate portion 3220b of the light beam is incident on the second angled facet 3208b. This may act to laterally shift the light beam along the first dimension. The second angled facet 3208b may direct the light beam toward the output region of the lensed facet 3218, such that the light beam exits the beam shifting optic 3200 through the lensed facet 3218. The lensed facet 3218 may define a convex curve along the second dimension, such that the lensed facet 3218 at least partially collimates the light beam as it exits the beam shifting optic 3200.

It should be appreciated that one or more portions of the beam shifting optic 3200 may be coated with an AR coating. For example, in the variation shown in FIGS. 32A-32D, the entire bottom surface 3204 may be coated with a first AR coating 3212a and the entire top surface 3206 (including the lensed facet 3218) may be coated with a second AR coating 3212b. In other variations, a corresponding portion of one or both of the top and bottom surfaces 3206, 3204 may be coated with a corresponding reflective coating such, as described in more detail herein with respect to the beam shifting optic 2800 of FIGS. 28A and 28B.

FIG. 32C shows a side view of a variation of a portion of a beam generator 3230 that incorporates the beam shifting optic 3200 of FIGS. 32A and 32B, as well as the components of the beam generator 1300 described with respect to FIGS. 13A-13F (only the photonic integrated circuit 1314 is depicted). In this example, a curved mirror 3264 may act as the fast axis collimating lens 1326, and the beam shifting optic 3200 may act as the slow axis collimating lens 1328.

As shown there, the photonic integrated circuit 1314 may emit the input light beam 1324 from a side surface of the photonic integrated circuit 1314, and the curved mirror 3264 may face the side surface of the photonic integrated circuit 1314 such that it receives and redirects the input light beam toward the beam shifting optic 3200. In this way, the input light beam 1324 may form the incoming portion of the light beam that is laterally shifted along the first dimension by the beam shifting optic 3200. Additionally, the curved mirror 3264 may at least partially collimate the input light beam 1324 in the first dimension, such as discussed with respect to the curved mirror 2864 of FIG. 28D. In this way, the input light beam 1324 may be fully or partially collimated in the first dimension as it enters the beam shifting optic 3200, and may be diverging as it enters the beam shifting optic 3200. The beam shifting optic 3200 may at least partially collimate the input light beam 1324 in the second dimension. In this way, the input light beam 1324 may be at least partially collimated in both the first and second dimensions as it exits the beam shifting optic 3200.

While the beam shifting optic 3200 is shown as spaced from the photonic integrated circuit 1314 along the Z-axis, it should be appreciated that in some instances the beam shifting optic 3200 may be positioned such that that bottom surface 3204 is positioned in contact with a top surface of the photonic integrated circuit 1314 (such as illustrated with respect to the beam shifting optic 2700 of FIG. 27D). Additionally or alternatively, the beam generator 3230 may include a mounting structure 3262 that may set a relative position between the beam shifting optic 3200 and the curved mirror 3064.

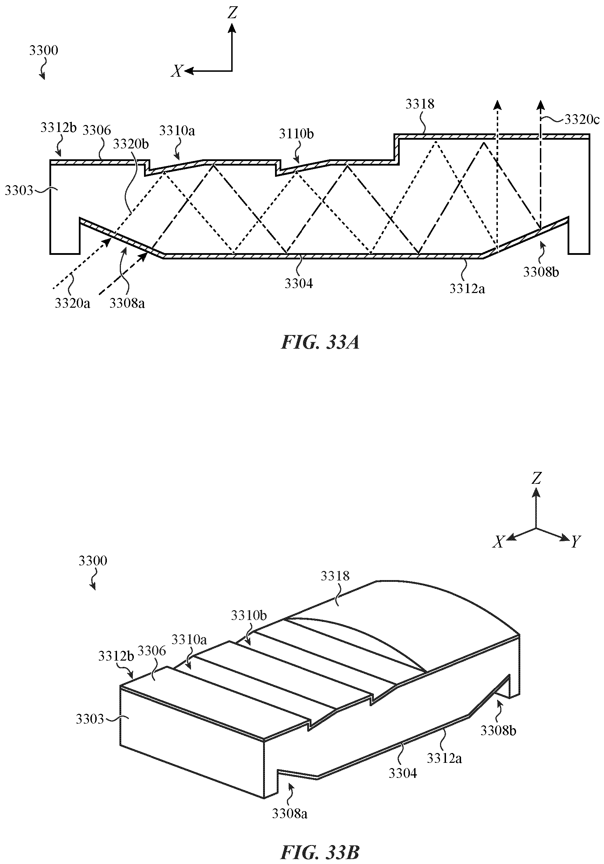

FIGS. 33A and 33B show another variation of a beam shifting optic 3300 that is configured to laterally shift a light beam in a first dimension and at least partially collimate the light beam in a second dimension. Specifically the beam shifting optic 3300 includes a substrate 3303 having a bottom surface 3304 and a top surface 3306. The bottom surface 3304 defines a first angled facet 3308a and a second angled facet 3308b, and may be at least partially coated with a first AR coating 3312a. The top surface 3306 defines a lensed facet 3318 and may at least partially be boated by a second AR coating 3312b. These components may be configured in any manner as describe herein with respect to the beam shifting optic 3200 of FIGS. 32A-32D to laterally shift a light beam (e.g. having an incoming portion 3320a that enters the beam shifting optic 3300, an intermediate portion 3320b that travels through the beam shifting optic 3300, and an outgoing portion 3320c that exits the beam shifting optic 3300). Additionally the top surface 3306 of the beam shifting optic 3300 defines a set of angled facets 3310a-3310b, which in the variation shown in FIGS. 33A and 33B include a third angled facet 3310a and a fourth angled facet 3310b. These angled facets may cooperate with the first and second angled facets 3308a, 3308b to help reflect the intermediate portion 3320b of the light beam, such as described with respect to the beam shifting optic 3100 of FIGS. 31A and 31B.

It should be appreciated that any of the beam shifting optics described herein may include additional angled facets as may be desired to assist in laterally shifting a beam of light.

In some variations, the beam shifting optic 3200 is configured such that the intermediate portion 3220b of the light beam reflects off of the lensed facet 3218 before it reaches the second angled facet 3208b. In these variations, the divergence of the light beam may decrease in the second dimension as it reflects off of the lensed facet 3218, then may be further decreased in the second dimension as it exits through the lensed facet 3218. In other words the reflection off of the lensed facet 3218 may at least partially collimate the light beam such that the intermediate portion 3220b of the light beam is partially collimated in the second dimension as it reflects off of the second angled facet 3208b. The light beam is further collimated as it exits the beam shifting optic 3200 through the lensed facet 3218. In this way, the principal plane of collimation may occur within the beam shifting optic 3200, which may further reduce the track length required in a particular dimension (e.g., along the Z-axis as shown in FIGS. 32A-32C). Overall, the outgoing portion 3220c of the light beam may be fully collimated or partially collimated in the second dimension depending on the desired beam vergence of the light beam.

In instances where an optical measurement system includes multiple measurement subsystems, it should be appreciated that the beam shifting optics described herein may be shared between different measurement subsystems. For example, a beam shifting optic may be shared between a first beam generator of a first measurement subsystem and a second beam generator of a second measurement subsystem. In these instances, the beam shifting optic may be configured to laterally shift a first input light beam generated by the first beam generator and may also be configured to laterally shift a second input light beam generated by the second beam generator. Using the beam shifting optic 2700 of FIGS. 27A-27D as an example, in some instances the width of the beam shifting optic 2700 along the Y-axis is sized such that the input region of the bottom surface 2704 is wide enough to accommodate both the first input light beam and the second input light beam. In this way, the first and second input light beam may enter the input region of the bottom surface 2704 in a side-by-side manner (each at a corresponding predetermined angle of incidence such as described herein), and may follow a similar trajectory through the beam shifting optic 2700. Specifically, the first input light beam may enter the beam shifting optic 2700 through a first subregion of the input region of bottom surface 2704 (e.g., such that the input light beam passes through the first AR coating 2712a), reflects off of the first angled facet 2708, back and forth between the top and bottom surfaces 2706, 2704, and off of the second angled facet 2710 before exiting through a first sub-region of the output region of the top surface 2706. The second input light beam may enter the beam shifting optic through a second subregion (displaced on the Y-axis as compared to the first subregion) of the output region of the bottom surface 2704, and take a similar trajectory through the beam shifting optic 2700 before exiting out a similarly-displaced second sub-region of the output region of the top surface 2706. Accordingly, if the first and second light beams have a common beam size and beam vergence (in one or both dimensions) as they enter the beam shifting optic 2700, they may also leave the beam shifting optic 2700 with a common beam size and beam vergence in these dimensions.

### Optical Measurement Systems with Multiple Measurement Subsystems

When an optical measurement system as described herein includes a plurality of measurement subsystems, the optical measurement system may be configured to generate a different emission light beam for each measurement subsystem. Accordingly, each measurement subsystem may include a different corresponding launch architecture that is configured to generate and emit a corresponding emission light beam from the optical measurement system. Similarly, each measurement subsystem may include a different corresponding collection architecture that is configured to collect and measure a portion of the corresponding emission light beam that is returned from the sample. It should be appreciated, however, that different measurement subsystems may share one or more components.

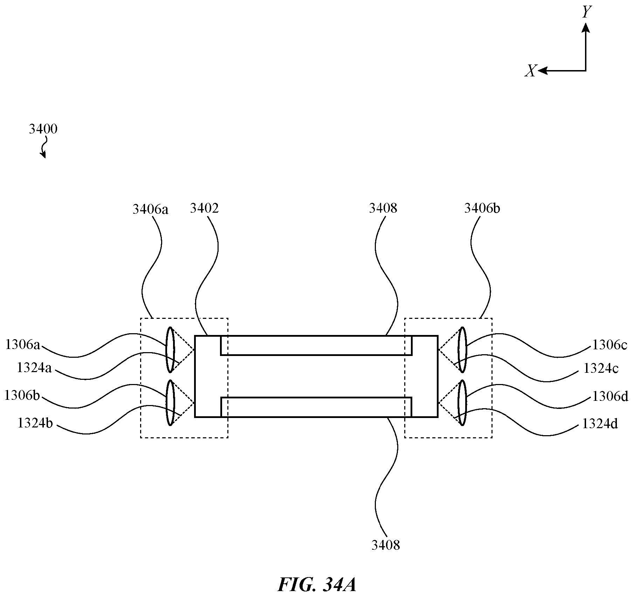

For example, in some variations a photonic integrated circuit may be used to generate multiple input light beams. Each light beam may be used a beam generator of a corresponding measurement subsystem. In this way, the photonic integrated circuit may be shared between the beam generators of multiple measurement subsystems. For example, when a photonic integrated circuit utilizes edge couplers to emit light from a side surface of the photonic integrated circuit, the photonic integrated circuit may be configured generate different input light beams from two or more side surfaces. For example, FIGS. 34A-34C depict variations of optical measurement systems in which a photonic integrated circuit is used to generate input light beams from two or more side surfaces thereof.

FIG. 34A shows a portion of an optical measurement system 3400 that includes a photonic integrated circuit 3402. The photonic integrated circuit 3400 may be configured to emit a plurality of input light beams 1324a-1324d. For example, each input light beam may be formed from a plurality of individual light beams (each emitted from a corresponding edge coupler) that at least partially overlap to collectively form the input light beam. The optical measurement system 3400 may include a plurality of measurement subsystems, each of which includes a corresponding beam generator that i) receives a corresponding one of the plurality of input light beams 1324a-1324d and ii) generates a corresponding output light beam therefrom. Each beam generator may be configured to generate an output light beam from an input light beam in any manner as described herein, such as described with respect to the beam generator 1302 described herein with respect to FIGS. 13A-13F. Collectively, the measurement subsystems may generate a plurality of output light beams 1306a-1306d, each of which may be used to generate a corresponding emission light beam.

In the variation shown in FIG. 34A, the photonic integrated circuit 3402 is configured to emit a first set of input light beams 1324a-1324b from a first side surface of the photonic integrated circuit 3402, and a second set of light beams 1324c-1324d from a second side surface of the photonic integrated circuit 3402. For example, the first side surface and the second side surface may be opposite sides of the photonic integrated circuit 3422. In the variation shown in FIG. 34A, the first set of input light beams 1324a-1324b includes multiple input light beams, specifically a first input light beam 1324a and a second input light beam 1324b. The second set of input light beams 1324c-1324d may also include multiple light beams, specifically a third input light beam 1324c and a fourth input light beam 1324d. A first beam generator of a first measurement subsystem may generate a first output light beam 1306a from the first input light beam 1324a, a second beam generator of a second measurement subsystem may generate a second output light beam 1306b from the second input light beam 1324b, a third beam generator of a third measurement subsystem may generate a third output light beam 1306c from the third input light beam 1324c, and a fourth beam generator of a fourth measurement subsystem may generate a fourth output light beam 1306d from the fourth input light beam 1324d. In this way, the photonic integrated circuit 3402 may act as shared component of four different beam generators, and is thus shared by four different measurement subsystems.

In some instances, the photonic integrated circuit 3402, as well as any of the other photonic integrated circuits described herein, may include one or more connection regions 3408 that allows the photonic integrated circuit 3402 to be electrically connected to another portion of the optical measurement system 3400. For example, wire bonds may be connected to a given connection region to provide an electrical connection between the photonic integrated circuit 3402 and another component (e.g., such as interposer 120). This may allow the photonic integrated circuit 3402 to be electrically connected to a controller, which may operate to control operation of the photonic integrated circuit 3402. These connection regions may be placed along edges of the photonic integrated circuit 3402 where the photonic integrated circuit 3402 is not being used to generate the input light beams 1324a-1324d, such that any wire bonds or other electrical connections do not interfere with or otherwise block the light emitted by the photonic integrated circuit 3402. It should be appreciated that the placement of the connection regions 3408 in the photonic integrated circuits of FIGS. 34A-39B are provided as illustrative examples, and are not intended to be limiting. It should be appreciated that these photonic integrated circuits may include any or all of the connection regions 3408 depicted in these figures, and/or may include other connection regions along portions of the photonic integrated circuits as may be desired.

In some variations, the optical measurement system 3400 may include one or more electromagnetic actuator arrangements, each of which includes at least one carrier that carries at least one diffuser and is moveably connected to a stationary base via a set of suspension elements, such as described in more detail herein with respect to FIGS. 19A-20E. The beam generators of multiple measurement subsystems may share a single electromagnetic actuator arrangement, such that multiple input light beams pass through a given carrier (e.g., pass through one or more diffusers carried by the carrier) to generate the output light beams. For example, in the variation shown in FIG. 34A, the first set of input light beams 1324a-1324b may each pass through a first carrier 3406a of a first electromagnetic actuator arrangement to generate a first set of output light beams 1306a-1306b, and the second set of input light beams 1324c-1324c may pass through a second carrier 3406b of a second electromagnetic actuator arrangement to generate a second set of output light beams 1306c-1306d. Depending on the configuration of the first carrier 3406a, the first and second input light beams 1324a, 1324b may pass through a common diffuser or different diffusers carried by the first carrier 3406a. Similarly, the third and fourth input light beams 1324c-1324d may pass through a common diffuser or different diffusers carried by the second carrier 3406.

It should be appreciated that the input light beams 1324a-1324d may be redirected (e.g. using a mirror or other beam turning component as described herein) to pass through the carriers in a different direction. For example, the first and second carriers 3406a, 3406b may be positioned over a top surface of the photonic integrated circuit 1314, and the input light beams 1324a-1324b may be redirected out of the page as shown in FIG. 34A. Additionally, in instances where an optical measurement system includes multiple carriers, these carriers may be formed as part of different electromagnetic actuator arrangements or as part of a common electromagnetic actuator arrangement. For example, the first carrier 3406a and the second carrier 3406b may be moveably connected to a common component (e.g., the same stationary base) as part of a single electromagnetic actuator arrangement, such that the first carrier 3406a and the second carrier 3406b may individually moveable relative to each other and relative to the common component.

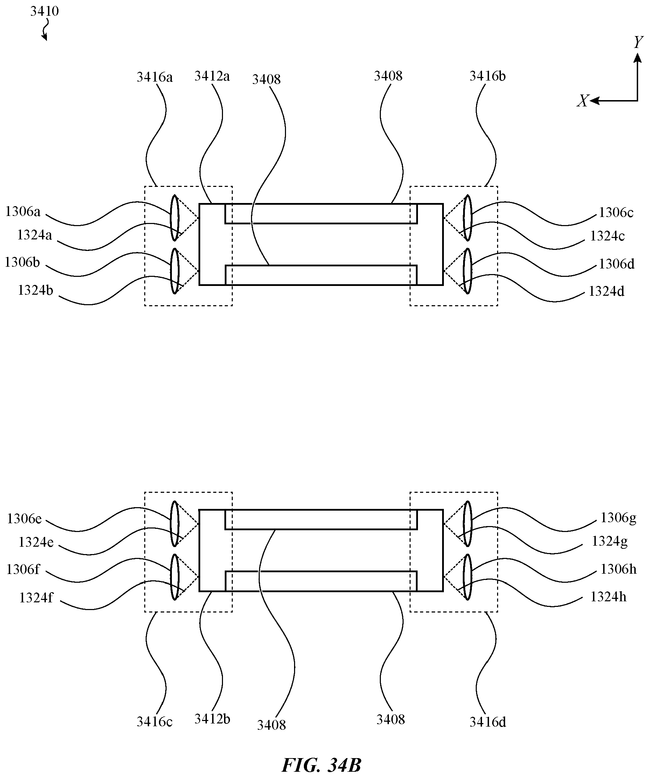

In some instances, the number of input light beams emitted by the photonic integrated circuit 3400 may be increased to accommodate a larger number of measurement subsystems. Additionally or alternatively, an optical measurement system may utilize multiple photonic integrated circuits to collectively emit a larger number of input light beams. For example, FIG. 34B shows a variation of an optical measurement system 3410 in which two photonic integrated circuits are collectively used to generate a plurality of input light beams 1324a-1324h. Specifically, the optical measurement system 3410 includes a first photonic integrated circuit 3412a and a second photonic integrated circuit 3142b, each of which is configured the same as the photonic integrated circuit 3402 of FIG. 34B. Accordingly, whereas the photonic integrated circuit 3402 of FIG. 34A is configured to emit four different input light beams 1324a-1324d, the first and second photonic integrated circuits 3412a, 3412b may collectively emit eight different input light beams 1324a-1324h. This may facilitate the generation of eight different output light beams 1306a-1306h that may be utilized by eight measurement subsystems to generate corresponding emission light beams.

Also shown there a plurality of carriers 3416a-3416d through which the plurality of input light beams 1324a-1324h may pass to form the corresponding output light beams 1306a-1306h. For example, a first set of input light beams (e.g., a first input light beam 1324a and second input light beam 1324b) emitted from a first side surface of the first photonic integrated circuit 3412a may pass through a first carrier 3416a to generate a first set of output light beams (e.g., a first output light beam 1306a and a second output light beam 1306b). Similarly, a second set of input light beams (e.g., a third input light beam 1324c and fourth input light beam 1324d) emitted from a second side surface of the first photonic integrated circuit 3412a may pass through a second carrier 3416b to generate a second set of output light beams (e.g., a third output light beam 1306c and a fourth output light beam 1306d). A third set of input light beams 1324e-1324f and a fourth set of input light beams 1324g-1324h emitted from different side surfaces of the second photonic integrated circuit 3412b may pass through a third carrier 3416c and a fourth carrier 3416d, respectively, to generate a third set of output light beams 1306e-1306f and a fourth set of output light beams 1306g-1306h.

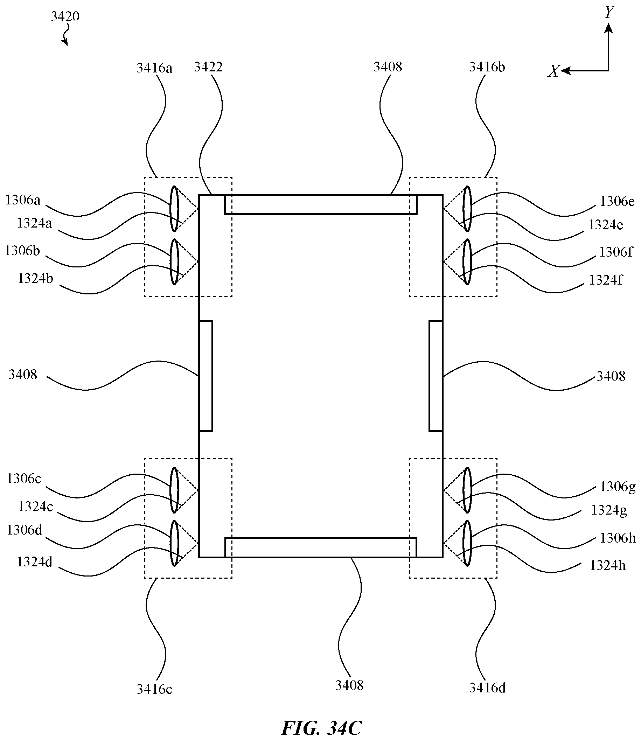

In some variations, different input light beams emitted from a given side of a photonic integrated circuit may be routed to different carriers. For example, FIG. 34C shows a variation of an optical measurement system 3420 that includes a photonic integrated circuit 3422 that is configured to emit a plurality of input light beams 1324a-1324h that are routed through the same plurality of carriers 3416a-3416d of the optical measurement system 3410 of FIG. 34B.

The photonic integrated circuit 3422 is configured to emit two sets of input light beams from a first side of the photonic integrated circuit 3422, each of which is routed to pass through a different carrier. For example, a first set of input light beams (e.g., including a first input light beam 1324a and a second input light beam 1324b) may be emitted from a first side surface of the photonic integrated circuit 3422 as part of a first set of measurement subsystems (e.g., a first measurement subsystem and a second measurement subsystem). The first set of input light beams may be routed to pass through the first carrier 3416a to generate a first set of output light beams (e.g., a first output light beam 1306a and a second output light beam 1306b). A second set of input light beams (e.g., including a third input light beam 1324c and a fourth input light beam 1324d) may be emitted from the same first side surface of the photonic integrated circuit 3422 as part of a second set of measurement subsystems (e.g., a first measurement subsystem and a second measurement subsystem). The second set of input light beams may be routed to pass through the third carrier 3416c to generate a second set of output light beams (e.g., a third output light beam 1306c and a fourth output light beam 1306d). In some of these variations, a connection region 3408 may be positioned along or near an edge of the first side surface, such that one or more wire bonds may be positioned between the first and second sets of input light beams.

Additionally, the photonic integrated circuit 3422 may be configured to emit a third set of input light beams 1324e-1324f and a fourth set of input light beams 1324g-1324h from a second side surface of the photonic integrated circuit 3422. The third set of light beams 1324e-1324f may be routed to pass through the second carrier 1316b to generate a third set of output light beams 1306e-1306f, and the fourth set of light beams 1324g-1324h may be routed to pass through the fourth carrier 1316d to generate a fourth set of output light beams 1306g-1306h.

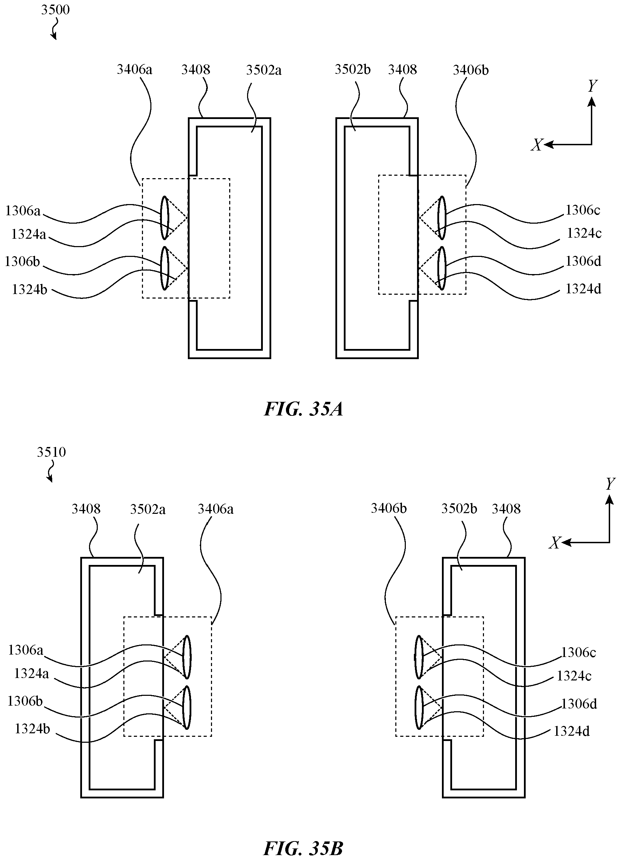

Depending on the number of measurement subsystems of an optical measurement system, as well as the number of shared carriers used by the beam generators of these measurement subsystems, there may be multiple ways in which one or more photonic integrated circuits may generate a desired number of input light beams. For example, FIGS. 35A-35C show additional ways in which a set of photonic integrated circuits may be used to generate two sets of light beams (e.g., the first set of input light beams 1324a-1324b and the second set of input light beams 1324c-1324d of FIG. 34A) that are routed to different carriers (e.g., the first carrier 4306a and the second carrier 4306b of FIG. 34A). For example, FIG. 35A shows a variation of an optical measurement system 3500 in which a first photonic integrated circuit 3502a emits the first set of input light beams 1324a-1324b from a corresponding side surface of the first photonic integrated circuit 3502a and a second photonic integrated circuit 3502b emits the second set of input light beams 1324c-1324d from a corresponding side surface of the second photonic integrated circuit 3502b. The first set of input light beams 1324a-1324b is routed to pass through the first carrier 3406a to generate the first set of output light beams 1306a-1306b, and the second set of input light beams 1324c-1324d are routed to pass through the second carrier 3406b to generate the second set of output light beams 1306c-1306d.

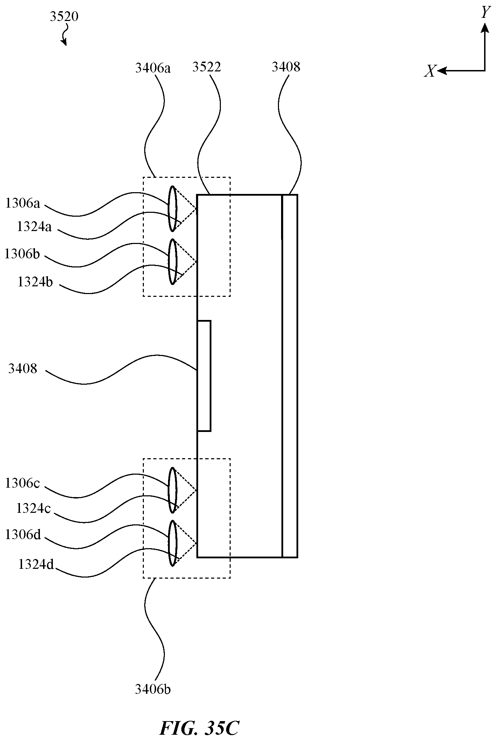

In the variation shown in FIG. 35A, the first and second photonic integrated circuits 3502a, 3502b are oriented such that the first set of input light beams 1324a-1324b is emitted from the first photonic integrated circuit 3502a in a direction away from the second photonic integrated circuit 3502b, and the second set of input light beams 1324c-1324d is emitted from the second photonic integrated circuit 3502b in a direction away from the first photonic integrated circuit 3502a. FIG. 35B shows another variation of an optical measurement system 3510, which is configured the same as the optical measurement system 3500 of FIG. 35A except that the first set of input light beams 1324a-1324b is emitted from the first photonic integrated circuit 3502a in a direction toward the second photonic integrated circuit 3502, and the second set of input light beams 1324c-1324d is emitted from the second photonic integrated circuit 3502b in a direction toward the first photonic integrated circuit 3502a. In still other variations, the first and second photonic integrated circuits 3502a, 3502b may be oriented such that the first set of input light beams 1324a-1324b and the second set of input light beams 1324c-1324d are emitted in a common direction. FIG. 35C shows another variation of an optical measurement system 3520 having a photonic integrated circuit 3522 that is configured to emit the first set of input light beams 1324a-1324b and the second set of input light beams 1324c-1324d from the same side surface of the photonic integrated circuit 3522.

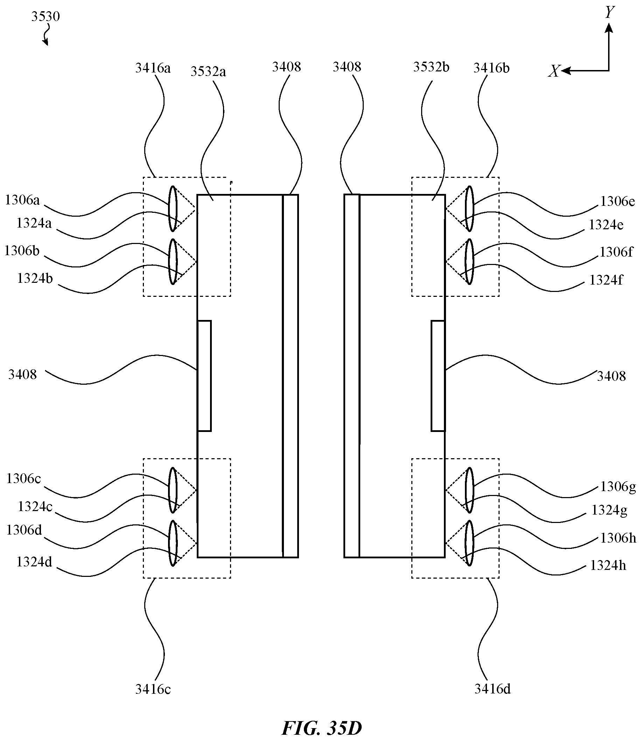

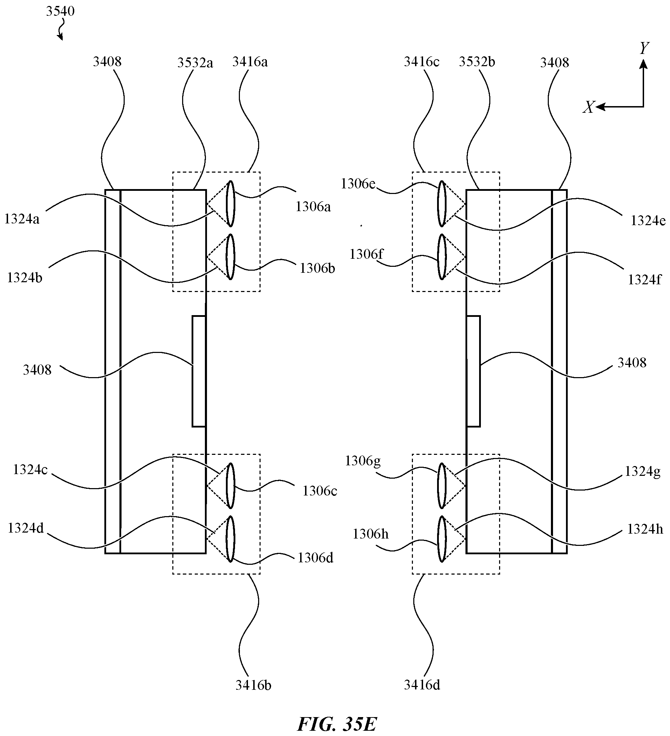

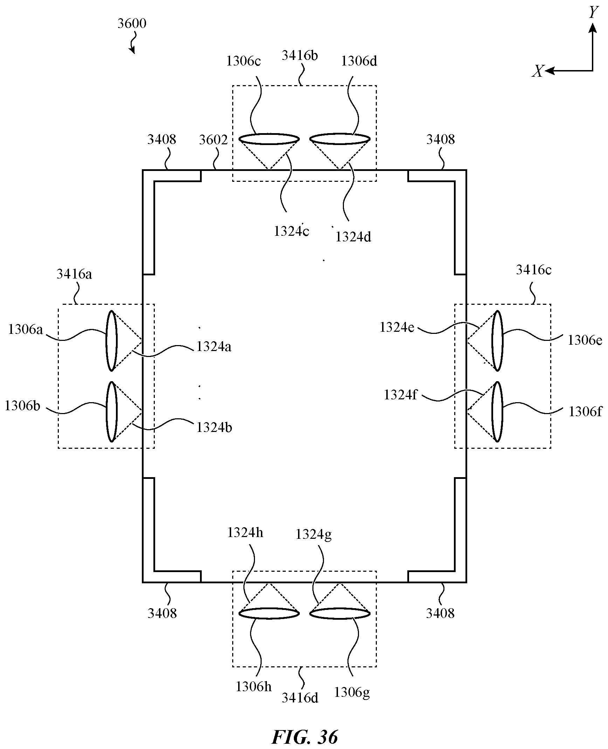

FIGS. 35D, 35E, and 36 show additional ways in which a set of photonic integrated circuits may be used to generate four sets of light beams, specifically the first set of input light beams 1324a-1324b, the second set of input light beams 1324c-1324d, the third set of input light beams 1324e-1324f, and the fourth set of input light beams 1324g-1324h of FIG. 34B, that are routed to the four carriers 3416a-3416d of FIG. 34B. Specifically, FIG. 35D shows a variation of an optical measurement system 3530 in which a first photonic integrated circuit 3532a is configured to emit the first set of input light beams 1324a-1324b and the second set of input light beams 1324c-1324d from the same side surface of the first photonic integrated circuit 3532a. A second photonic integrated circuit 3532b is configured to emit the third set of input light beams 1324e-1324f and the fourth set of input light beams 1324g-1324h from the same side of the second photonic integrated circuit 3532b. In this variation, first and second photonic integrated circuits 3532a, 3532b are oriented such that the first and second sets of light beams 1324a-1324b, 1324c-1324d are emitted from the first photonic integrated circuit 3532a in a direction away from the second photonic integrated circuit 3532b, and the third and fourth sets of light beams 1324e-1324f, 1324g-1324h are emitted from the second photonic integrated circuit 3532b in a direction away from the first photonic integrated circuit 3532a.

FIG. 35E shows another variation of an optical measurement system 3540, which is configured the same as the optical measurement system 3530 of FIG. 35D except that the first and second sets of light beams 1324a-1324b, 1324c-1324d are emitted from the first photonic integrated circuit 3532a in a direction toward the second photonic integrated circuit 3532b, and the third and fourth sets of light beams 1324e-1324f, 1324g-1324h are emitted from the second photonic integrated circuit 3532b in a direction toward the first photonic integrated circuit 3532a. In still other variations, the first and second photonic integrated circuits 3532a, 3532b may be oriented such that all of the set of input light beams are emitted in a common direction.

FIG. 36 shows another variation of an optical measurement system 3600 in which each of the four sets of input light beams 1324a-1324b, 1324c-1324d, 1324e-1324f, 1324g-1324h is emitted from a different corresponding side of a photonic integrated circuit 3602. In these variations, the four carriers 3416a-3416d may be positioned in a different arrangement as compared to the optical measurement systems shown in FIG. 34B and FIGS. 35D and 35E.

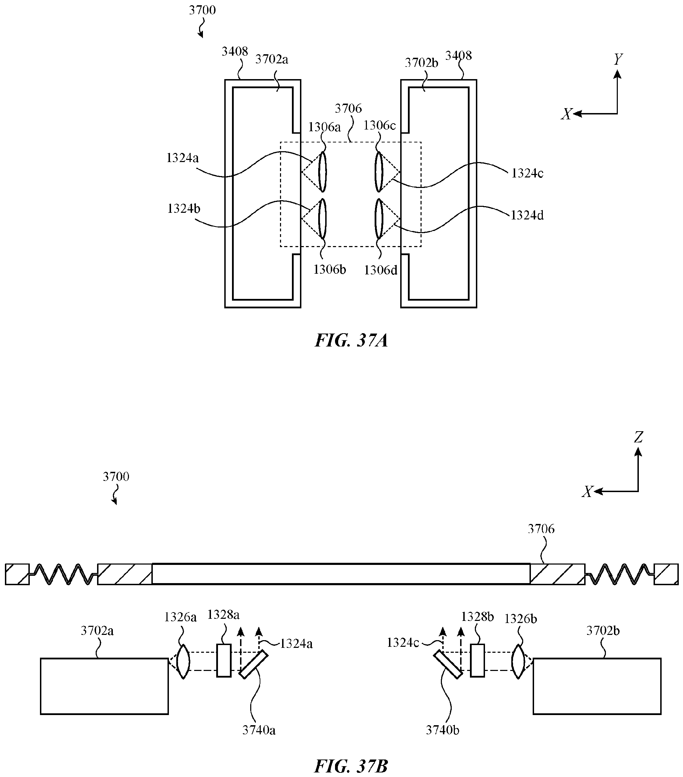

In some variations, it may be desirable to route multiple sets of input light beams to a common carrier, where different sets of these input light beams are emitted from different side surfaces of one or more photonic integrated circuits. This may help to decrease spacing between the different sets of input light beams, as well as to increase the number of output light beams that may be generated using a given carrier. This may also allow the optical measurement system to emit multiple emission light beams in relatively close proximity to each other, which may help to reduce the size of the sampling interface of the optical measurement. For example, FIG. 37A shows a variation of an optical measurement system 3700 that includes a first photonic integrated circuit 3702a and a second photonic integrated circuit 3702b. The first photonic integrated circuit 3702a is configured to emit a first set of input light beams 1324a-1324b from a corresponding side surface of the first photonic integrated circuit 3702a, and the second photonic integrated circuit 3702b is configured to emit a second set of input light beams 1324c-1324d from a corresponding side surface of the second photonic integrated circuit 3702b. The side surface of the first photonic integrated circuit 3702a that emits the first set of input light beams 1324a-1324b may face the second photonic integrated circuit 3702b, and the side surface of the second photonic integrated circuit 3702b that emits the second set of input light beams 1324c-1324d may face the first photonic integrated circuit 3702a. Accordingly, the first and second sets of input light beams 1324a-1324b, 1324c-1324d may each be routed to pass through a first carrier 3706 to generate a first set of output light beams 1306a-1306b and a second set of output light beams 1306c-1306d, respectively.

To route the input light beams to the first carrier 3706, each measurement subsystem may include an optical unit that is configured to receive a corresponding input light beam and route the input light beam to the first carrier 3706. For example, FIG. 37B shows a partial side view of the optical measurement system 3700 and includes components of first optical unit of a first measurement subsystem and a second optical unit of a second measurement subsystem. Specifically, the first measurement subsystem may include, as part of a first beam generator, a first optical unit that includes a first fast axis collimating lens 1326a and a first slow axis collimating lens 1328a, such as described in more detail with respect to the beam generator 1302 of FIGS. 13A-13F. Specifically, the first beam generator may be positioned to receive an input light beam 1324a of the first set of input light beams 1324a-1324b. The first fast axis collimating lens 1326a may be positioned to receive the input light beam 1324a and at least partially collimate the input light beam 1324a along its fast axis. The first slow axis collimating lens 1328a may receive the input light beam 1324a after it has passed through the fast axis collimating lens 1326a, and may at least partially collimate the input light beam 1324a along its slow axis. The first optical unit may further include a first mirror 3740a, which may reflect and thereby redirect the input light beam 1324a toward the first carrier 3706.

Similarly, the second measurement subsystem may include, as part of a first beam generator, a second optical unit that includes a second fast axis collimating lens 1326b, a second slow axis collimating lens 1328b, and a second mirror 3740b. The second optical unit may be positioned to receive an input light beam 1324c of the second set of input light beams 1324c, 1324d, such that the optical unit at least partially collimates the input light beam 1324c along its fast axis via the second fast axis collimating lens 1326b, at least partially collimates the input light beam 1324c along its slow axis via the second slow axis collimating lens 1328b, and redirects the light beam 1324c toward the first carrier 3706 using the second mirror 3740b. Additional measurement subsystems may be similarly configured to shape and redirect other input light beams generated by the optical measurement system 3700.

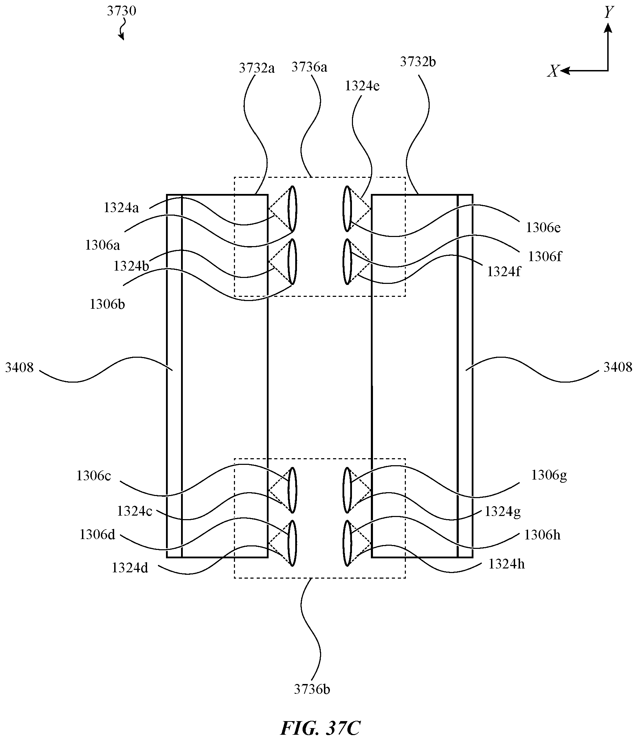

FIG. 37C shows another variation of an optical measurement system 3730 in which input light beams from multiple photonic integrated circuits are routed to pass through a common carrier. The optical measurement system 3730 includes a first photonic integrated circuit 3732a and a second photonic integrated circuit 3732b. The first photonic integrated circuit 3732a is configured to emit a first set of input light beams 1324a-1324b and a second set of input light beams 1324c-1324d from a corresponding side surface of the first photonic integrated circuit 3732a, and the second photonic integrated circuit 3732b is configured to emit a third set of input light beams 1324e-1324f and a fourth set of input light beams 1324g-1324h from a corresponding side surface of the second photonic integrated circuit 3732b. The side surface of the first photonic integrated circuit 3732a that emits the first and second sets of input light beams 1324a-1324b, 1324c-1324d may face the second photonic integrated circuit 3732b, and the side surface of the second photonic integrated circuit 3732b that emits the third and fourth sets of input light beams 1324e-1324f, 1324g-1324h faces the first photonic integrated circuit 3732a. Accordingly, the first and third sets of input light beams 1324a-1324b, 1324e-1324f may each be routed to pass through a first carrier 3736a to generate corresponding output light beams 1306a-1306b, 1306e-1306f. Similarly, the second and fourth sets of input light beams 1324c-1324d, 1324g-1324h may each be routed to pass through a second carrier 3736b to generate corresponding output light beams 1306c-1306d, 1306g-1306h.

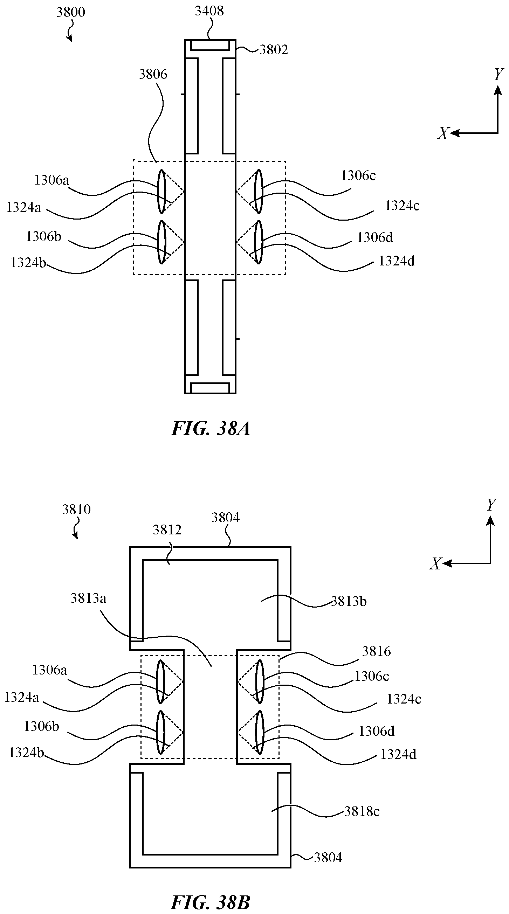

In some instances, input light beams emitted from different surfaces of a single photonic integrated circuit may be routed to a common carrier. For example, FIG. 38A shows a variation of an optical measurement system 3800 that includes a photonic integrated circuit 3802 that is configured to emit a first set of input light beams 1324a-1324b from a first side surface of the photonic integrated circuit 3802 and to emit a second set of input light beams 1324c-1324d from a second side surface of the photonic integrated circuit 3802. In these instances, the first and second sets of input light beams 1324a-1324b, 1324c-1324d may be emitted from opposite sides of the photonic integrated circuit 3802. Accordingly, if the photonic integrated circuit 3802 is sufficiently narrow, the first and second sets of input light beams 1324a-1324b, 1324c-1324d may be routed to pass through a common carrier 3806.

In instances where the photonic integrated circuit 3802 has a rectangular shape, this may limit the width of the photonic integrated circuit 3802 in order to allow the first and second sets of input light beams 1324a-1324b, 1324c-1324d to be routed to pass through the carrier 3806. This may constrain how light is generated and routed within the photonic integrated circuit. In some variations, the photonic integrated circuit 3802 may be wider than pictured in FIG. 38A, and the optical measurement system 3800 may incorporate one or more beam shifting optics that may be used to laterally shift one or more of the input light beams. For example, the optical measurement system 3800 may include a first beam shifting optic (not shown), such as those described with respect to FIGS. 27A-33B, that is positioned to receive the first set of input light beams 1324a-1324b. The first beam shifting optic may laterally shift these light beams to be closer to the second set of input light beams 1324c-1324d as they pass through the carrier 3806. Additionally or alternatively, the optical measurement system 3800 may include a second beam shifting optic (not shown), such as those described with respect to FIGS. 27A-33B, that is positioned to receive the second set of input light beams 1324c-1324d. The second beam shifting optic may laterally shift these light beams to be closer to the first set of input light beams 1324a-1324b as they pass through the carrier 3806.

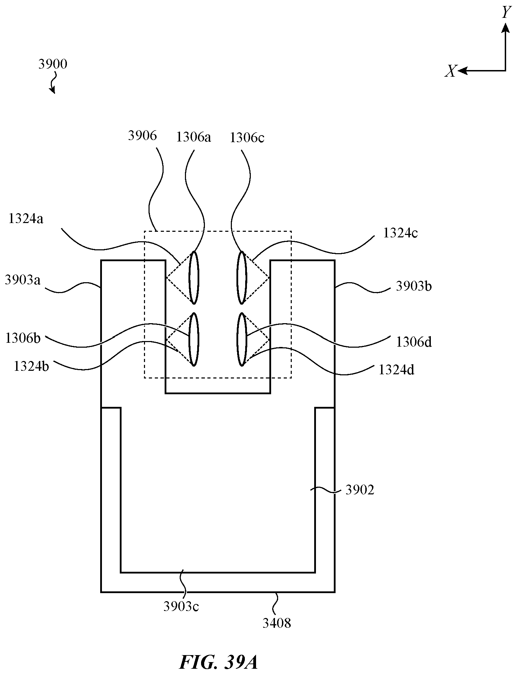

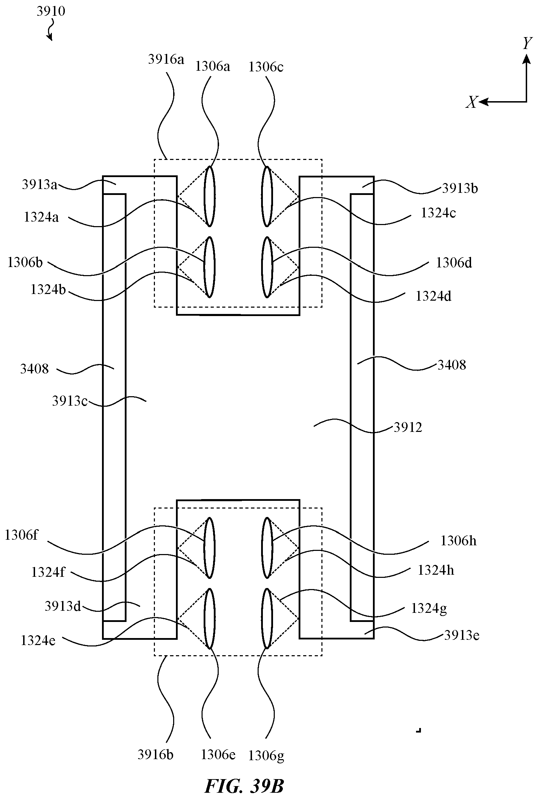

In some variations, a photonic integrated circuit may have a non-rectangular shape, which may provide additional flexibility in emitting the input light beams. For example, a photonic integrated circuit may be singulated from a wafer using a manufacturing technique such as plasma dicing, which may allow the photonic integrated circuit to be formed with a non-rectangular shape. For example, FIGS. 38B-38D and FIGS. 39A and 39B show examples of optical measurement systems that include non-rectangular photonic integrated circuits. For example, FIG. 38B shows a variation of an optical measurement system 3810 having a non-rectangular photonic integrated circuit 3812 having a first portion 3813a having a first width, a second portion 3813b having a second width, and a third portion 3813c having a third width. The first portion 3813a may be positioned between the second and third portions 3813b, 3813c, and may have a narrower first width as compared to the corresponding second and third widths of the respective second and third portions 3813b, 3813c. The photonic integrated circuit 3812 may be configured such that a first set of input light beams 1324a-1324b is emitted from a first side surface of the first portion 3813a and a second set of input light beams 1324c-1324d is emitted from an opposite second side surface of the first portion 3813a. In this way, the first width may be selected to facilitate routing of first and second sets of input light beams 1324a-1324b, 1324c-1324d through a common carrier 3816. The second and third portions 3813b, 3813c, by virtue of their relatively larger widths, may provide additional area for generating and/or routing light within the photonic integrated circuit 3812.

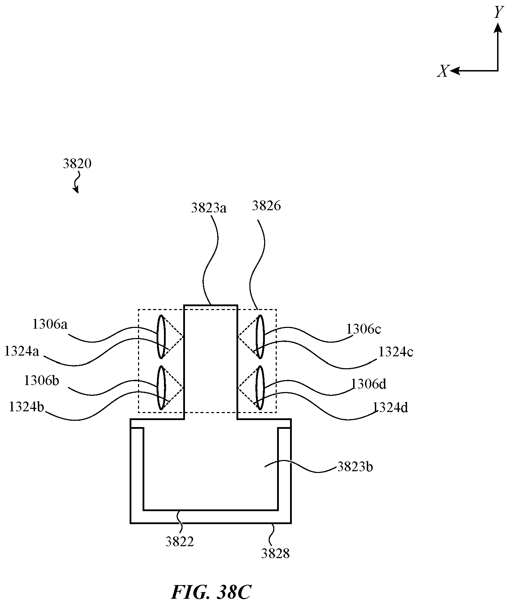

FIG. 38C shows another variation of an optical measurement system 3820 having a non-rectangular photonic integrated circuit 3822. In this variation, the photonic integrated circuit 3822 has a first portion 3823a having a first width and a second portion 3823b having a second width that is larger than the first width. As with the photonic integrated circuit 3812 of FIG. 38B, a first set of input light beams 1324a-1324b is emitted from a first side surface of the first portion 3823a and a second set of input light beams 1324c-1324d is emitted from an opposite second side surface of the first portion 3823a. The first and second sets of input light beams 1324a-1324b, 1324c-1324d may similarly be routed through a common carrier 3826.

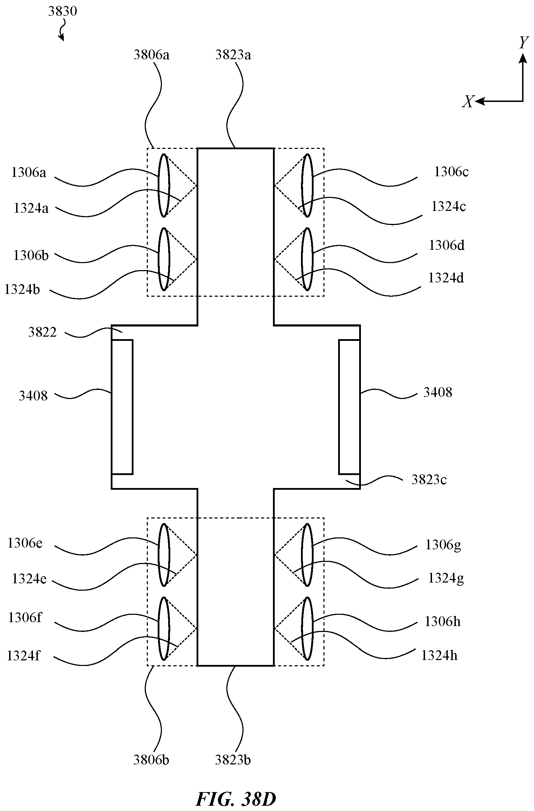

FIG. 38D shows another variation of an optical measurement system 3830 having a non-rectangular photonic integrated circuit 3832. In this variation, the photonic integrated circuit 3832 has a first portion 3823a having a first width, a second portion 3823b having a second width, and a third portion 3823c having a third width. The third portion 3823c may be positioned between the first and second portions 3823a, 3823b, and the third width may be larger than the first and second widths of the respective first and second portions 3823a, 3823b. The first portion 3823a is configured such that a first set of input light beams 1324a-1324b is emitted from a first side surface of the first portion 3823a and a second set of input light beams 1324c-1324d is emitted from an opposite second side surface of the first portion 3823a. The first and second sets of light beams 1324a-1324b, 1324c-1324d may be routed to pass through a first carrier 3836a. Similarly, the second portion 3823b is configured such that a third set of input light beams 1324e-1324f is emitted from a first side surface of the second portion 3823b and a fourth set of input light beams 1324g-1324h is emitted from an opposite second side surface of the second portion 3823b. The third and fourth sets of light beams 1324e-1324f, 1324g-1324h may be routed to pass through a second carrier 3836b.

In other variations, different input light beams may be emitted from one side surface of photonic integrated circuit in a direction toward another side surface of the photonic integrated circuit. For example, FIG. 39A shows a variation of an optical measurement system 3900 that includes a non-rectangular photonic integrated circuit 3902 having a first portion 3903a having a first width, a second portion 3903b having a second width, and a third portion 3903c having a third width larger than the first width and the second width. The first and second portions 3903a, 3903b may be connected to the third portion 3903c and may be positioned to define a gap between the first and second portions 3903a, 3903b. Specifically, a first side surface of the first portion 3903a may face a corresponding second side surface of the second portion 3903b. The photonic integrated circuit 3902 may be configured to emit a first set of input light beams 1324a-1324b from the first side surface of the first portion 3903a (e.g., in a direction toward the second portion 3903b), and to emit a second set of input light beams 1324c-1324d from the second side surface of the second portion 3903b (e.g., in a direction toward the first portion 3903a). The first and second sets of light beams 1324a-1324b, 1324c-1324d may be routed to pass through a first carrier 3906a. In these instances, one or more optical components (e.g., fast axis collimating lens, slow axis collimating lens, mirrors, etc.) of the measurement subsystems associated with the first and second sets of light beams 1324a-1324b, 1324c-1324d may also be positioned in the gap between the first and second portions 3903a, 3903b of the photonic integrated circuit 2902.

FIG. 39B shows another variation of a photonic integrated circuit 3910 having a non-rectangular photonic integrated circuit 3912. In this variation, the photonic integrated circuit 3912 may have a H shape, and may be configured to output multiple sets of light beams that are routed through a first carrier 3916a and multiple sets of light beams that are routed through a second carrier 3916b. Specifically, the photonic integrated circuit 3912 may include a first portion 3913a and a second portion 3913b that are connected to a third portion 3913c, such that the first and second portions 3913a, 3913b are spaced apart by a first gap. Specifically, a first side surface of the first portion 3913a may face a corresponding second side surface of the second portion 3913b. The photonic integrated circuit 3912 may be configured to emit a first set of input light beams 1324a-1324b from the first side surface of the first portion 3913a (e.g., in a direction toward the second portion 3913b), and to emit a second set of input light beams 1324c-1324d from the second side surface of the second portion 3913b (e.g., in a direction toward the first portion 3913a). The first and second sets of light beams 1324a-1324b, 1324c-1324d may be routed to pass through a first carrier 3916a.

Similarly, the photonic integrated circuit 3912 may include a fourth portion 3913d and a fifth portion 3913e that are connected to the third portion 3913c, such that the fourth and fifth portions 3913d, 3913e are spaced apart by a second gap. Specifically, a third side surface of the fourth portion 3913d may face a corresponding fourth side surface of the fifth portion 3913e. The photonic integrated circuit 3912 may be configured to emit a third set of input light beams 1324e-1324f from the third side surface of the fourth portion 3913d (e.g., in a direction toward the fifth portion 3913e), and to emit a fourth set of input light beams 1324g-1324h from the fourth side surface of the fifth portion 3913e (e.g., in a direction toward the fourth portion 3913d). The fourth and fifth sets of light beams 1324e-1324f, 1324g-1324h may be routed to pass through a second carrier 3916b.

When a side surface of a photonic integrated circuit is used to emit multiple input light beams, the input light beams may be emitted side-by-side (e.g., from a common waveguide layer in the photonic integrated circuit). Depending on the requirements of the input light beam (e.g., a desired beam size in one or more dimensions as it reaches a diffuser of a beam generator), there may be limitations on how closely two input light beams may be positioned within an optical measurement system. Accordingly, in some instances it may be desirable to emit and route input light beams to stagger the resulting output light beams, such that different output light beams have different lateral spacing relative to the photonic integrated circuit.

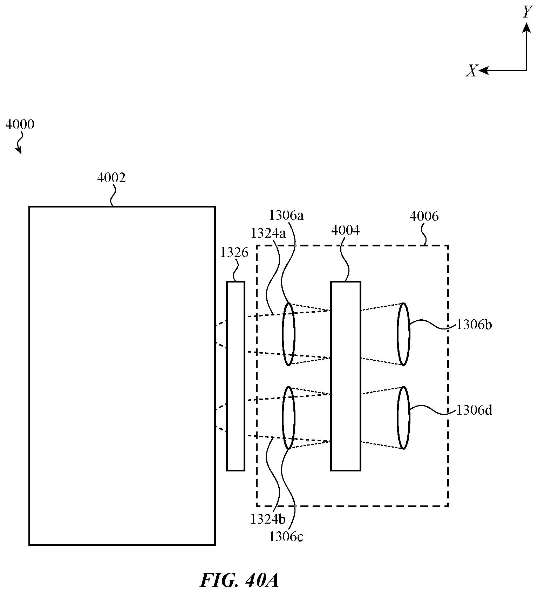

For example, in some variations it may be desirable to use a single emission location on a photonic integrated circuit to generate output light beams for multiple measurement subsystems. For example, FIG. 40A shows a top view of a variation of an optical measurement system 4000 that has a plurality of measurement subsystems. Specifically, the optical measurement system 4000 includes a photonic integrated circuit 4002 that is configured to emit a set of input light beams from a first side surface of the photonic integrated circuit 4002. For example, in the variation shown in FIG. 40A, the photonic integrated circuit 4002 is configured to emit a first input light beam 1324a from a first emission location of the first side surface and to emit a second input light beam 1324b from a second emission location of the second side surface. The optical measurement system 4000 is configured such that a first output light beam 1306a of a first measurement subsystem and a second output light beam 1306b of a second measurement subsystem may each be generated from the first input light beam 1324a. Similarly, the optical measurement system 4000 is configured such that a third output light beam 1306c of a third measurement subsystem and a fourth output light beam 1306d of a fourth measurement subsystem may each be generated from the second input light beam 1324b. In some instances, light from the first and second input light beams 1324a, 1324b pass through a common carrier 4006 to generate the plurality of output light beams 1306a-1306. In this way, each emission site on the first side surface of the photonic integrated circuit 4002 may be used to generate output light beams for multiple measurement subsystems.

For example, as shown in FIG. 40A, the optical measurement system 4000 may include a beam directing structure 4004 that is configured to route light from the first input light beam 1324a between the first and second measurement subsystems. The same beam directing structure 4004 (or an additional beam directing structure) may similarly route light from the second input light beam 1324b between the third and fourth measurement subsystems. In some variations, the optical measurement system may be configured such that the first and second input light beams 1324a, 1324b each pass through a corresponding fast axis collimating lens before reaching the beam directing structure 4004. Accordingly, the first and second input light beams 1324a, 1324b may be at least partially collimated in a first dimension before reaching the beam directing structure 4004. In the variation show in FIG. 40A, both input light beams 1324a, 1324b may pass through a single fast axis collimating lens 1326 (e.g., the fast axis collimating lens 1326 is shared between the four measurement subsystems). In other variations, each of the input light beams 1324a, 1324b may pass through different separate fast axis collimating lenses.

In some instances, the beam directing structure 4004 is configured to dynamically selectively route each input light beam to a single measurement subsystem at a time. For example, depending on the operation of the beam directing structure 4004, the first input light beam 1324a may be routed to either the first measurement subsystem or the second measurement subsystem. During a first period of time, the first input light beam 1324a may be routed to the first measurement subsystem, and the first measurement subsystem may generate the first output light beam 1306a using the first input light beam 1324a. During a second period of time, the first input light beam 1324a may be routed to the second measurement subsystem, and the second measurement subsystem may generate the second output light beam 1306b using the first input light beam 1324b. In this way, only one of these measurement subsystems may generate an output light beam at a time, and the first and second measurement subsystems may be unable to simultaneously perform individual measurements. The active measurement subsystem, however, may be able to use the entire first input light beam 1324a to generate the second output light beam.

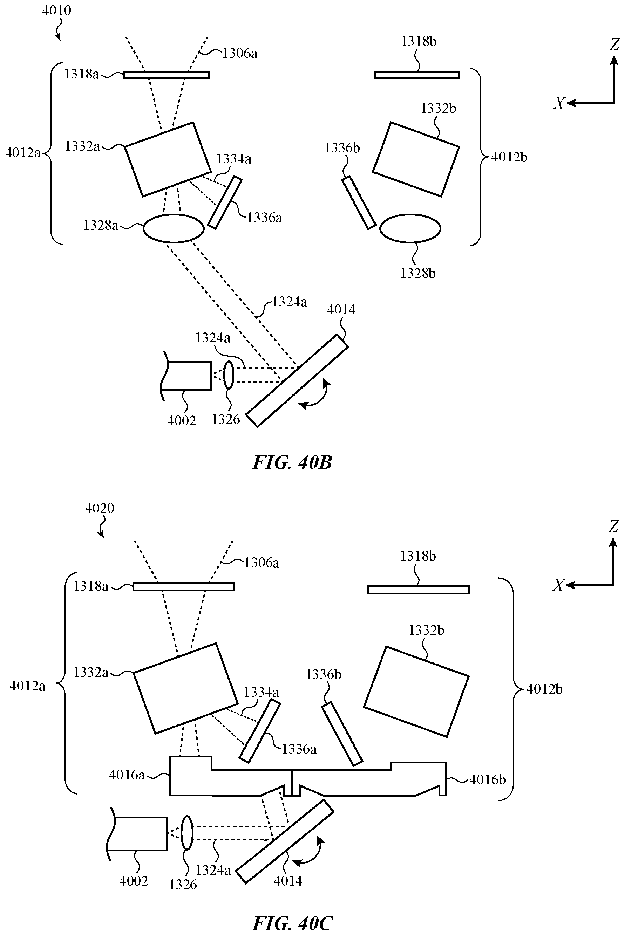

FIGS. 40B and 40C show variations of optical measurement systems that include a dynamic beam directing structure that is controllable to selectively route an input light beam between different measurement subsystems. FIG. 40B shows a first variation of an optical measurement system 4010 that may be configured the same as the optical measurement system of FIG. 40A, except that the optical measurement system 4010 includes a rotating mirror 4014 as a dynamic beam directing structure. Specifically, the rotating mirror 4014 may be controlled to rotate or move between two states (e.g., via an actuator, such as a mems actuator or the like). The rotating mirror 4014 may be positioned such that, when the photonic integrated circuit 4002 emits the first input light beam 1324a, the first input light beam 1324a will reflect off of the mirror 4014. The rotating mirror 4014, when in a first state, will route the first input light beam 1324a to a first measurement subsystem to generate the first output light beam 1306a, such as shown in FIG. 40B. When the rotating mirror 4014 is changed to the second state, the rotating mirror 4014 will route the input light beam 1324a to the second measurement subsystem.

As shown in FIG. 40B, the first measurement subsystem includes a first beam generator 4012a that is configured to generate the first output light beam 1306a from the input light beam 1324a. The first beam generator 4012a may be configured in any manner as described herein with respect to the beam generator 1302 of FIGS. 13A-13F. For example, the first beam generator 4012a may include a first slow axis collimating lens 1328a, a first beamsplitter 1332a, and a first diffuser 1318a. When the first beamsplitter 1332a receives the first input light beam 1324a, it may generate a first reference light beam 1334a (e.g., by splitting the first input light beam 1324a) that may be measured by a first reference detector 1336a. These components may cooperate to form the first output light beam 1036a, such as described in more detail herein. In some variations the first beamsplitter 1332a may be a polarizing beamsplitter such as those described herein with respect to FIGS. 21A-26. In these instances, the first beam generator 4012a may generate a polarized first output light beam 1306a. When the first beam generator 4012a receives the first input light beam 1324a from the rotating mirror 4014, the first measurement subsystem may emit a corresponding first emission light beam to perform one or more individual measurements of a spectroscopic measurement such as described herein.

Similarly, the second measurement subsystem includes a second beam generator 4012b that is configured to generate the second output light beam 1306b from the input light beam 1324a. The second beam generator 4012b may be configured in any manner as described herein with respect to the beam generator 1302 of FIGS. 13A-13F. For example, the second beam generator 4012b may include a second slow axis collimating lens 1328b, a second beamsplitter 1332b, and a second diffuser 1318b. When the second beamsplitter 1332b receives the first input light beam 1324a, it may generate a second reference light beam (not shown), by splitting the first input light beam 1324a, that may be measured by a second reference detector 1336b. These components may cooperate to form the second output light beam 1036b, such as described in more detail herein. As with the first beam generator, in some variations the second beamsplitter 1332b may be a polarizing beamsplitter such as those described herein with respect to FIGS. 21A-26. In these instances, the second beam generator 4012b may generate a second output light beam 1306b. When the second beam generator 4012b receives the first input light beam 1324a from the rotating mirror 4014, the second measurement subsystem may emit a corresponding second emission light beam to perform one or more individual measurements of a spectroscopic measurement such as described herein. In some instances, the optical measurement system 4010 includes a fast axis collimating lens 1326 that is positioned between the photonic integrated circuit 4002 and the rotating mirror 4014, and is configured to at least partially collimate the first input light beam 1324a in a first dimension (e.g., in the XY plane of the cartesian coordinate system shown in FIG. 40B). Accordingly, the photonic integrated circuit 4002 and the fast axis collimating lens 1326 are shared between the first and second beam generators 4012a, 4012b. Additionally, it should be appreciated that the first and second diffusers 1318a, 1318bb may be carried by the same carrier 4006, which may be configured, for example, as discussed with respect to the electromagnetic actuator arrangements of FIGS. 20C-20E.

FIG. 40C shows another variation of an optical measurement system 4020, which may be configured the same as the optical measurement system 4010 of FIG. 40B except that the first slow axis collimating lens 1328a of the first beam generator 4012a is replaced with a first beam shifting optic 4016a and the second slow axis collimating lens 1328b of the second beam generator 4012b is replaced with a second beam shifting optic 4016b. In these instances, the first beam shifting optic 4016a may receive the input light beam 1324a from the rotating mirror 4014 to laterally shift the first input light beam 1324a (e.g., along the X-axis as shown in FIG. 40C). This may act to reduce a height of the first beam generator 4012a along the Z-axis. Similarly the second beam shifting optic 4016b may laterally shift the first input light beam 1324a to reduce the height of the second beam generator 4012b along the Z-axis. In instances where the first and second beam shifting optics 4016a, 4016b include lensed facets, these beam shifting optics may each act as a slow axis collimating lens. In other instances, the first and second beam generators 4012a, 4012b may include the respective first and second slow axis collimating lenses 1328a, 1328b of FIG. 40B in addition to the first and second beam shifting optics 4016a, 4016b.

In other instances, the beam directing structure 4004 of the optical measurement system 4000 of FIG. 40A is configured to split an input light beam into two separate beams, such that that two measurement subsystems simultaneously receive a corresponding portion of the input light beam. For example, the first measurement subsystem may receive a first portion of the first input light beam 1324a while the second measurement subsystem simultaneously receives a second portion of the first input light beam 1324b. Accordingly, the first and second measurement subsystems may simultaneously generate the first and second output light beams 1306a, 1306b (and thus may simultaneously perform individual measurements). Each measurement subsystem may receive light having a lower intensity as compared to instances in which the same input light beam is routed to a single measurement subsystem. Additionally, if there are instances where the optical measurement system 4000 performs a measurement with only one of the measurement subsystems, the light routed to the other measurement subsystem may be wasted.

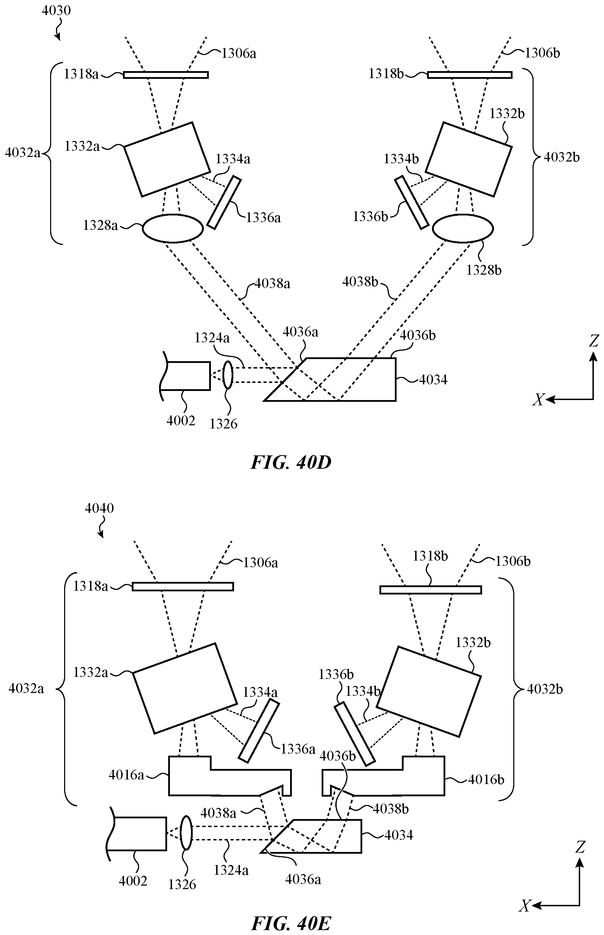

FIGS. 40D and 40E show variations of optical measurement systems that include a beam directing structure that splits an input light beam between different measurement subsystems. FIG. 40D shows a first variation of an optical measurement system 4030 that may be configured the same as the optical measurement system of FIG. 40A, except that the optical measurement system 4010 includes a beamsplitter 4034 as a beam directing structure. Specifically, the beamsplitter 4034 may split the first input light beam 1324a into a first split light beam 4038a and a second split light beam 4038b. For example, the beamsplitter 4034 may have a first facet 4036a that is positioned such that, when the first input light beam 1324a is incident on the first facet 4036a, a Fresnel reflection occurs that splits the first input light beam 1324a into the first split light beam 4038a and the second split light beam 4038b. Specifically, the portion of the first input light beam 1324a that reflects off of the first facet 4036a may form the first split light beam 4038a that is directed to the first measurement subsystem. The portion of the first input light beam 1324b that enters the beamsplitter 4034 may form the second split light beam 4038b. The beamsplitter 4034 may be configured such that the second split light beam 4038b exits the beamsplitter 4034 through a second facet 4036b at an angle such that it is received by the second measurement subsystem. Accordingly, the first and second measurement systems simultaneously receive the respective first and second split light beams 4038a, 4038b.

As shown in FIG. 40D, the first measurement subsystem includes a first beam generator 4032a that is configured to generate the first output light beam 1306a from the first split light beam 4038a. The first beam generator 4032a may be configured in any manner as described herein with respect to the beam generator 1302 of FIGS. 13A-13F. For example, the first beam generator 4012a may include a first slow axis collimating lens 1328a, a first beamsplitter 1332a, and a first diffuser 1318a. When the first beamsplitter 1332a receives the first split light beam 4038a, it may generate a first reference light beam 1334a (e.g., by splitting the first split light beam 4038a) that may be measured by a first reference detector 1336a. These components may cooperate to form the first output light beam 1036a, such as described in more detail herein. In some variations, the first beamsplitter 1332a may be a polarizing beamsplitter such as those described herein with respect to FIGS. 21A-26. In these instances, the first beam generator 4032a may generate a polarized first output light beam 1306a.

Similarly, the second measurement subsystem includes a second beam generator 4032b that is configured to generate the second output light beam 1306b from the second split light beam 4038b. The second beam generator 4012b may be configured in any manner as described herein with respect to the beam generator 1302 of FIGS. 13A-13F. For example, the second beam generator 4032b may include a second slow axis collimating lens 1328b, a second beamsplitter 1332b, and a second diffuser 1318b. When the second beamsplitter 1332b receives the second split light beam 4038b, it may generate a second reference light beam 1334b (e.g., by splitting the second split light beam 4038b) that may be measured by a second reference detector 1336b. These components may cooperate to form the second output light beam 1036b, such as described in more detail herein. As with the first beam generator, in some variations the second beamsplitter 1332b may be a polarizing beamsplitter such as those described herein with respect to FIGS. 21A-26. In these instances, the second beam generator 4032b may generate a second output light beam 1306b.

In some instances, the optical measurement system 4010 includes a fast axis collimating lens 1326 that is positioned between the photonic integrated circuit 4002 and beamsplitter 4034, and is configured to at least partially collimate the first input light beam 1324a in a first dimension (e.g., in the XY plane of the cartesian coordinate system shown in FIG. 40D). Accordingly, the photonic integrated circuit 4002 and the fast axis collimating lens 1326 are shared between the first and second beam generators 4032a, 4032b. Additionally, it should be appreciated that the first and second diffusers 1318a, 1318b may be carried by the same carrier 4006, which may be configured, for example, as discussed with respect to the electromagnetic actuator arrangements of FIGS. 20C-20E.

FIG. 40E shows another variation of an optical measurement system 4040, which may be configured the same as the optical measurement system 4030 of FIG. 40D except that the first slow axis collimating lens 1328a of the first beam generator 4032a is replaced with a first beam shifting optic 4016a and the second slow axis collimating lens 1328b of the second beam generator 4032b is replaced with a second beam shifting optic 4016b. In these instances, the first beam shifting optic 4016a may receive the first split light beam 4038a from the beamsplitter 4034 to laterally shift the first split light beam 4038a (e.g., along the X-axis as shown in FIG. 40E). This may act to reduce a height of the first beam generator 4032a along the Z-axis. Similarly the second beam shifting optic 4016b may laterally shift the second split light beam 4038b to reduce the height of the second beam generator 4032b along the Z-axis. In instances where the first and second beam shifting optics 4016a, 4016b include lensed facets, these beam shifting optics may each act as a slow axis collimating lens. In other instances, the first and second beam generators 4032a, 4032b may include the respective first and second slow axis collimating lenses 1328a, 1328b of FIG. 40D in addition to the first and second beam shifting optics 4016a, 4016b.

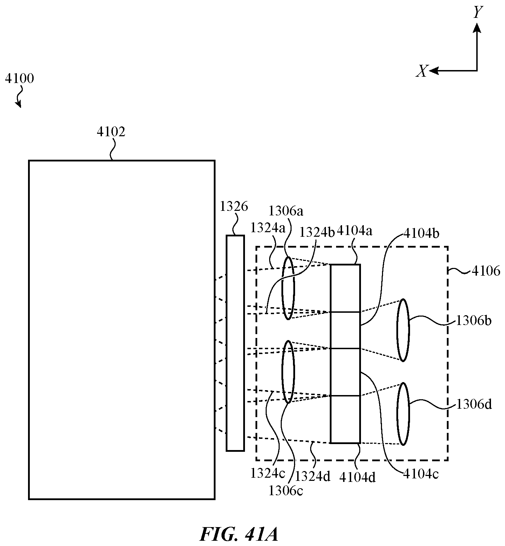

Additionally or alternatively, the optical measurement systems described herein may be configured to stagger adjacent input light beams emitted from a photonic integrated circuit. For example, FIG. 41A shows a top view of a variation of an optical measurement system 4100 that has a plurality of measurement subsystems. Specifically, the optical measurement system 4100 includes a photonic integrated circuit 4102 that is configured to emit a plurality of input light beams 1324a-1324d from a first side surface of the photonic integrated circuit 4102. For example, in the variation shown in FIG. 41A, the photonic integrated circuit 4102 is configured to emit four input light beams 1324a-1324d and the optical measurement system is configured to route the input light beams 1324a-1324d through a carrier 4106 to generate a corresponding plurality of output light beams 1306a-1306d.

The optical measurement system 4100 is configured to redirect adjacent input light beams in different directions, such that the resulting output light beams are generated in a staggered relationship. Indeed, instead of all four output light beams 1306a-1306d be generated in a single row, the first and third output light beams 1306a, 1306c are generated in a first row, and the second and fourth output light beams 1306b, 1306d are generated in a second row. For example, the first and third input light beams 1324a, 1324c may pass through a first diffuser (not shown) carried by the carrier 4106 to generate the first and third output light beams 1306a, 1306c, and the second and fourth input light beams 1324b, 1324d may pass through a second diffuser (not shown) carried by the carrier 4106 to generate the second and fourth output light beams 1306b, 1306d.

For example, as shown in FIG. 41A, the optical measurement system 4000 may include a plurality of alternating mirrors 4104a-4104d. Each of the mirrors 4104a-4104d is positioned to receive a corresponding input light beam, and collecting the set of mirrors are arranged to direct immediately adjacent input light beams emitted from a surface of the photonic integrated circuit 4102 in different directions. Each mirror is positioned at a corresponding angle relative to the corresponding incoming input light beam, which may at least partially determine the direction along which the input light beam is directed.

For example, a first mirror 4104a may be positioned to receive the first input light beam 1324a and reflect the first input light beam 1324a in a first direction. A second mirror 4104b next to the first mirror 4104a may be positioned to receive the second input light beam 1324b (which is next to the first input light beam 1324a) and reflect the second input light beam 1324b in a second direction that is different from the first direction. A third mirror 4104c next to the second mirror 4104b may be positioned to receive the third input light beam 1324c (which is next to the second input light beam 1324b) and reflect the third input light beam 1324c in a third direction that is different from the second direction (which, in the example shown in FIG. 41A is the same as the first direction). Similarly, a fourth mirror 4104d next to the third mirror 4104c may be positioned to receive the fourth input light beam 1324d (which is next to the third input light beam 1324c) and reflect the fourth input light beam 1324d in a fourth direction that is different from the third direction (which, in the example shown in FIG. 41A is the same as the second direction).

In some variations, the optical measurement system 4100 may be configured such that the plurality of input light beams 1324a-1324d each pass through a corresponding fast axis collimating lens before reaching a corresponding mirror of the plurality of alternating mirrors 4104a-4104d. Accordingly, the plurality of input light beams 1324a-1324d may each be at least partially collimated in a first dimension before reaching the corresponding mirror of the plurality of alternating mirrors 4104a-4104d. In the variation show in FIG. 41A, the plurality of input light beams 1324a-1324d may pass through a single fast axis collimating lens 1326 (e.g., the fast axis collimating lens 1326 is shared between the four measurement subsystems). In other variations, some or all of the input light beams 1324a-1324d may pass through different separate fast axis collimating lenses.

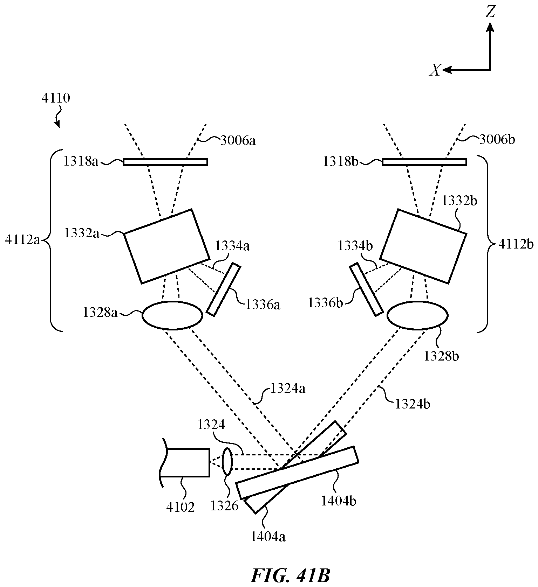

FIG. 41B shows a partial side view of first variation of an optical measurement system 4110 that may be configured the same as the optical measurement system 4100 of FIG. 41A. In this variation, the optical measurement system includes a first beam generator 4112a that is configured to generate the first output light beam 1306a from the first input light beam 1324a after it is reflected from the first mirror 4104a. The first beam generator 4112a may be configured in any manner as described herein with respect to the beam generator 1302 of FIGS. 13A-13F. For example, the first beam generator 4012a may include a first slow axis collimating lens 1328a, a first beamsplitter 1332a, and a first diffuser 1318a. When the first beamsplitter 1332a receives the first input light beam 1324a, it may generate a first reference light beam 1334a (e.g., by splitting the first input light beam 1324a) that may be measured by a first reference detector 1336a. These components may cooperate to form the first output light beam 1036a, such as described in more detail herein. In some variations, the first beamsplitter 1332a may be a polarizing beamsplitter such as those described herein with respect to FIGS. 21A-26. In these instances, the first beam generator 4112a may generate a polarized first output light beam 1306a.

Similarly, the second beam generator 4112b is configured to generate the second output light beam 1306b from the second input light beam 1324b after it is reflected from the second mirror 1404. The second beam generator 4112b may be configured in any manner as described herein with respect to the beam generator 1302 of FIGS. 13A-13F. For example, the second beam generator 4112b may include a second slow axis collimating lens 1328b, a second beamsplitter 1332b, and a second diffuser 1318b. When the second beamsplitter 1332b receives the second input light beam 1324b, it may generate a second reference light beam 1334b (e.g., by splitting the second input light beam 1324b) that may be measured by a second reference detector 1336b. These components may cooperate to form the second output light beam 1036b, such as described in more detail herein. As with the first beam generator, in some variations the second beamsplitter 1332b may be a polarizing beamsplitter such as those described herein with respect to FIGS. 21A-26. In these instances, the second beam generator 4032b may generate a second output light beam 1306b.

In some instances, the optical measurement system 4010 includes a fast axis collimating lens 1326 that is positioned between the photonic integrated circuit 4002 and the first and second mirrors 4104a, 4104b, and is configured to at least partially collimate the first and second input light beams (shown collectively as 1324 in FIG. 41B) in a first dimension (e.g., in the XY plane of the cartesian coordinate system shown in FIG. 41B). Accordingly, the photonic integrated circuit 4102 and the fast axis collimating lens 1326 are shared between the first and second beam generators 4112a, 4112b. Additionally, it should be appreciated that the first and second diffusers 1318a, 1318b may be carried by the same carrier 4106, which may be configured, for example, as discussed with respect to the electromagnetic actuator arrangements of FIGS. 20C-20E.

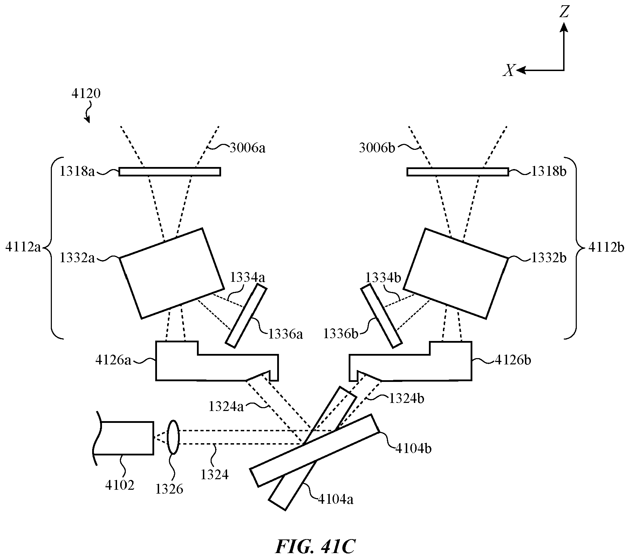

FIG. 41C shows another variation of an optical measurement system 4120, which may be configured the same as the optical measurement system 4110 of FIG. 40B except that the first slow axis collimating lens 1328a of the first beam generator 4112a is replaced with a first beam shifting optic 4116a and the second slow axis collimating lens 1328b of the second beam generator 4112b is replaced with a second beam shifting optic 4016b. In these instances, the first beam shifting optic 4116a may receive the first input light beam 1324a from the first mirror 4104a to laterally shift the first input light beam 1324a (e.g., along the X-axis as shown in FIG. 41C). This may act to reduce a height of the first beam generator 4112a along the Z-axis. Similarly the second beam shifting optic 4116b may laterally shift the second input light beam 1324b to reduce the height of the second beam generator 4112b along the Z-axis. In instances where the first and second beam shifting optics 4016a, 4016b include lensed facets, these beam shifting optics may each act as a slow axis collimating lens. In other instances, the first and second beam generators 4112a, 4112b may include the respective first and second slow axis collimating lenses 1328a, 1328b of FIG. 41B in addition to the first and second beam shifting optics 4016a, 4016b.

In other variations, an optical measurement system may include staggered mirrors that are positioned at different distances from a photonic integrated circuit. This may cause neighboring input light beams to be redirected toward a carrier at different distances, which may act to stagger the output light beams generated from these input light beams. This may, however, cause different input light beams to have different sizes in one or more dimensions as these input light beams reach their respective diffusers. Accordingly, this may result in the generation of output light beams having different sizes for measurement subsystems. In instances where it is desirable to generate output light beams having the same size, it may be desirable to account for this differential change in beam size.

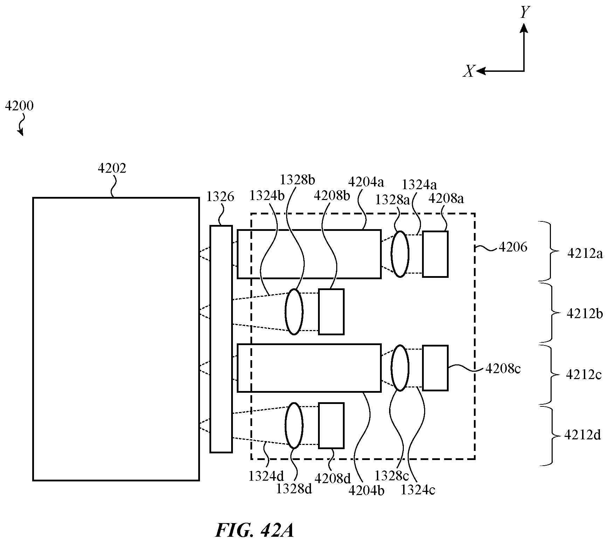

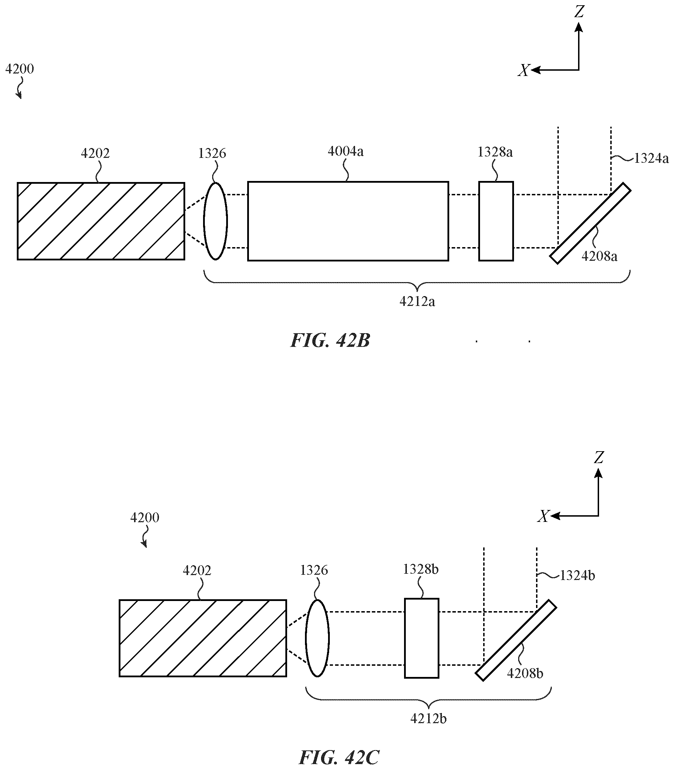

For example, FIG. 42A shows a front view and FIGS. 42B and 42C show partial cross-sectional side views of an optical measurement system 4200 that has a plurality of measurement subsystems. Specifically, the optical measurement system 4200 includes a photonic integrated circuit 4202 that is configured to emit a plurality of input light beams 1324a-1324d from a first side surface of the photonic integrated circuit 4202. For example, in the variation shown in FIG. 42A, the photonic integrated circuit 4202 is configured to emit four input light beams 1324a-1324d and the optical measurement system is configured to route the input light beams 1324a-1324d through a carrier 4206 to generate a corresponding plurality of output light beams 1306a-1306d, such as described in more detail herein.

Specifically, each of the input light beams 1324a-1324d is used to generate a corresponding output light beam for a different measurement subsystem. Each measurement subsystem may include a beam generator configured to route its corresponding light beam to the carrier 4206 in order to generate its corresponding output light beam. In the variation shown in FIGS. 42A-42C, each measurement subsystem includes a fast axis collimating lens (shown in FIGS. 42A-42C as a single fast axis collimating lens 1326 that is shared between the measurement subsystems), a slow axis collimator, and a beam turning structure configured to change the direction of the light beam (e.g., from the X-axis to the Z-axis in the cartesian coordinates system shown in FIGS. 42A-42C). The beam turning structure for each measurement subsystem is shown in FIGS. 42A-42C as being a mirror, it should be appreciated that the beam turning structure may be any optical component are arrangement of optical components that is capable of redirecting a corresponding input light beam (e.g., a prism or the like).

As shown in FIG. 42A, the optical measurement system 4200 may include a first measurement subsystem with a first beam generator 4212a that includes the fast axis collimating lens 1326, a first slow axis collimating lens 1328a, and a first mirror 4208a, as well as a second measurement subsystem with a second beam generator 4212b that includes the fast axis collimating lens 1326, a second slow axis collimating lens 1328b, and a second mirror 4208b. FIG. 42B shows a partial cross-sectional view illustrating portions of the first beam generator 4212a, while FIG. 42C shows a partial cross-sectional view illustrating portions of the second beam generator 4212b. Additionally, the optical measurement system 4200 may include a third measurement subsystem with a third beam generator 4212c that includes the fast axis collimating lens 1326, a third slow axis collimating lens 1328c, and a third mirror 4208c, as well as a fourth measurement subsystem with a fourth beam generator 4212d that includes the fast axis collimating lens 1326, a fourth slow axis collimating lens 1328d, and a fourth mirror 4208c.

Using the first measurement subsystem as an example, the fast axis collimating lens may at least partially collimate a first input light beam 1324a in a first dimension (e.g., along the XZ plane), the first slow axis collimating lens 1328b may at least partially collimate the first input light beam 1324a in a second dimension (e.g., along the XY plane), and the first mirror 4208a redirects the first input light beam 4208 within the first dimension to direct the first input light beam 4208 toward the carrier 4206. The first beam generator 4212a may generate an output light beam (not shown) as it passes through the carrier 4206 (e.g., by passing through a diffuser carrier by the carrier). The second, third, and fourth beam generators 4212b-4212d may operate similarly to generate corresponding output light beams from the second, third, and fourth input light beams 1324b-1324d.

In some instances, it may be desirable to allow each input light beam to diverge to a particular beam size before redirecting the beam using a beam turning component. Accordingly, in the variation shown FIGS. 42A-42C, the mirrors 4208a-4208d are positioned in a staggered arrangement such that mirrors associated with at least some neighboring input light beams are positioned at different distances from the side surface of the photonic integrated circuit that emits the input light beams 1324a-1324d. For example, in the variation shown in FIGS. 42A-42C, the first and third mirrors 4208a, 4208c are each positioned at a first distance from the photonic integrated circuit 4202, and the second and fourth mirrors 4208b, 4208d are each positioned at a second distance from the photonic integrated circuit 4202 that is less than the first distance. This may allow for the generation of staggered output light beams, which may be arranged similarly to the output light beams 1306a-1306d depicted in FIG. 41A.

Additionally, the first and third slow axis collimating lenses 1328a, 1328c are each positioned at third distance from the photonic integrated circuit 4202, and the second and fourth slow axis collimating lenses 1328b, 1328d are each positioned at a fourth distance from the photonic integrated circuit 4202 that is less than the third distance. Assuming each input light beam has approximately the same beam vergence in the second dimension when it is emitted from the photonic integrated circuit 4202, the first and third input light beams 1324a, 1324c may, by virtue of traveling a long distance to reach the respective first and third slow axis collimating lenses 1328a, 1328c, may have a larger beam size in the second dimension when they are collimated in the second dimension as compared to the second and fourth light beams 1324b, 1324d.

To help compensate for this, the optical measurement system may include a set of optical displacers 4204a-4204b that are configured to temporarily change the beam vergence of certain input light beams. Specifically, the optical measurement system 4202 shown in FIGS. 42A-42C includes a first optical displacer 4204a and a second optical displacer 4204b. The first optical displacer 4204a is positioned between the photonic integrated circuit 4202 and the first slow axis collimating lens 1328a, and is positioned such that the first input light beam 1324a travels through the first optical displacer 4204a before reaching the first slow axis collimating lens 1328a. Similarly, the second optical displacer 4204b is positioned between the photonic integrated circuit 4202 and the second slow axis collimating lens 1328b, and is positioned such that the second input light beam 1324b travels through the second optical displacer 4204b before reaching the second slow axis collimating lens 1328b. The optical displacers may each be formed from a piece of material that has a refractive index higher than the surrounding air or other medium through which the plurality of input light beams 1324a-1324b are traveling. Accordingly, the first input light beam 1324a will temporarily have a slower beam vergence in the second dimension as it travels through the first optical displacer 4204a, and thus the beam width of the first input light beam 1324a in the second dimension will increase at a slower rate in the first optical beam displacer 4204a. In this way, the dimensions and the materials of the optical beam displacers 4204a, 4204b may be selected such that each of the input light beams 1324a-1324d will have the same beam size as it is incident on its respective mirror. Overall, the slow axis collimating lenses 1328a-1328d and mirrors 4208a-4208d may be staggered relative to the photonic integrated circuit 4202, but may still be used to generate output light beams having a common beam size.

It should be appreciated that in some instances, one or more of the optical beam displacers 4204a, 4204b and/or the fast axis collimating lens 1326 (or multiple fast axis collimating lenses in instances where the some or all of the beam generators 4214a-4214d include a different fast axis collimating lens) may be configured to steer the one or more of the plurality of input light beams 1324a-1324b in the second dimension. This may facilitate routing the input light beams to allow the resulting output light beams to be positioned closer to each other.

Additionally, while the various photonic integrated circuits described with respect to FIGS. 34A-42C are configured to emit multiple input light beams, it should be appreciated that these photonic integrated circuits need not be configured to generate all of this input light beams simultaneously. For example, certain sets of input light beams are individually controllable (e.g., the photonic integrated circuit may be controllable to emit these input light beams without also needing to emit other light beams). Additionally or alternatively, certain sets of input light beams are controlled as a group (e.g., the photonic integrated circuit is not able to emit one input light beam within such a set without also emitting the other light beams in the set). Overall, a given photonic integrated circuit may be operated to emit various input light beams at different times to facilitate the individual measurements of a spectroscopic measurement as described herein.

The foregoing description, for purposes of explanation, used specific nomenclature to provide a thorough understanding of the described embodiments. However, it will be apparent to one skilled in the art that the specific details are not required in order to practice the described embodiments. Thus, the foregoing descriptions of the specific embodiments described herein are presented for purposes of illustration and description. They are not targeted to be exhaustive or to limit the embodiments to the precise forms disclosed. It will be apparent to one of ordinary skill in the art that many modifications and variations are possible in view of the above teachings.

Although the disclosed examples have been fully described with reference to the accompanying drawings, it is to be noted that various changes and modifications will become apparent to those skilled in the art. Such changes and modifications are to be understood as being included within the scope of the disclosed examples as defined by the appended claims.

Exemplary embodiments of the present disclosure are set out in the following seven sets of items. The sets of items comprise independent embodiments and dependent embodiments. It is noted that each of the dependent embodiments can also refer to the other independent embodiments.

### Optical Measurement Systems Configured to Measure Angle-Independent Sample Regions

1. An optical measurement system, comprising:
   a sampling interface defining a launch site and a set of collection sites each laterally spaced from the launch site;
   launch architecture configured to emit an emission light beam that exits the sampling interface through the launch site; and
   collection architecture comprising a set of detector elements positioned to measure light that has entered the sampling interface through the set of collection sites, wherein:
      the set of detector elements forms a set of measurement channels when the launch architecture emits the emission light beam;
      the emission light beam converges in a first dimension as it exits the sampling interface; and
      the emission light beam, if emitted into a target sample, projects to a transition region in the target sample that is angle independent for at least one of the set of measurement channels.
2. The optical measurement system of item 1, wherein:
   the launch architecture is configured such that the emission light beam has a beam vergence half-angle of at least 15 degrees in the first dimension as the emission light beam exits the sampling interface.
3. The optical measurement system of item 2, wherein:
   the launch architecture is configured such that the emission light beam has a beam vergence half-angle of at least 30 degrees in the first dimension as the emission light beam exits the sampling interface.
4. The optical measurement system of any of items 1-3, wherein:
   the set of collection sites comprises a first collection site and a second collection site;
   the launch site is positioned between the first collection site and the second collection site; and
   the set of detector elements comprises:
      a first set of detector elements positioned to measure light that has entered the sampling interface through the first collection site; and
      a second set of detector elements positioned to measure light that has entered the sampling interface through the second collection site.
5. The optical measurement system of item 4, wherein:
   the first set of detector elements forms a first set of measurement channels when the launch architecture emits the emission light beam;
   the second set of detector elements forms a second set of measurement channels when the launch architecture emits the emission light beam; and
   the transition region in the target sample is angle independent for the first set of measurement channels and the second set of measurement channels.
6. The optical measurement system of item 5, wherein:
   the launch architecture is configured such that the emission light beam has, as the emission light beam exits the sampling interface, a first beam width in the first dimension and a second beam width in a second dimension perpendicular to the first dimension; and
   the second beam width is at least eight times the first beam width.
7. An optical measurement system, comprising:
   a sampling interface defining a launch site and a first collection site laterally spaced from the launch site;
   launch architecture configured to emit an emission light beam that exits the sampling interface through the launch site; and
   collection architecture comprising a first detector element and configured to:
      collect a first return light beam that enters the sampling interface through the first collection site; and
      direct the return light beam to the first detector element, wherein:
   the first detector element forms a first measurement channel when the launch architecture emits the emission light beam;
   the first return light beam diverges in a first dimension as it enters the sampling interface; and
   the first return light beam, if collected from a target sample, projects from a first transition region in the target sample that is angle independent for the first measurement channel.
8. The optical measurements system of item 7, wherein:
   the collection architecture is configured such that the first return light beam has a beam vergence half-angle of at least 15 degrees in the first dimension as the return light beam enters the sampling interface.
9. The optical measurements system of item 8, wherein:
   the collection architecture is configured such that the first return light beam has a beam vergence half-angle of at least 30 degrees in the first dimension as the return light beam enters the sampling interface.
10. The optical measurement system of any of items 7-9, wherein:
   the collection architecture comprises a second detector element and is configured to:
      collect a second return light beam that enters the sampling interface through the first collection site; and
      direct the second return light beam to the second detector element, wherein:
   the second detector element forms a second measurement channel when the launch architecture emits the emission light beam; and
   the second return light beam diverges in the first dimension as it enters the sampling interface.
11. The optical measurement system of item 10, wherein:
   the second return light beam, if collected from the target sample, projects from a second transition region in the target sample that is angle independent for the second measurement channel.
12. The optical measurement system of any of items 7-9, wherein:
   the sampling interface defines a second collection site positioned such that the launch site is positioned between the first collection site and the second collection site;
   the collection architecture comprises a second detector element and is configured to:
      collect a second return light beam that enters the sampling interface through the second collection site; and
      direct the second return light beam to the second detector element;
   the second detector element forms a second measurement channel when the launch architecture emits the emission light beam;
   the second return light beam diverges in the first dimension as it enters the sampling interface; and
   the second return light beam, if collected from the target sample, projects from a second transition region in the target sample that is angle independent for the second measurement channel.
13. The optical measurement system of any of items 7-12, wherein:
   the collection architecture is configured such that the first return light beam has, as the first return light beam enters the sampling interface, a first beam width in the first dimension and a second beam width in a second dimension perpendicular to the first dimension; and
   the second beam width is at least eight times the first beam width.
14. An optical measurement system, comprising:
   a sampling interface defining a launch site and a first collection site laterally spaced from the launch site;
   launch architecture configured to emit an emission light beam that exits the sampling interface through the launch site; and
   collection architecture comprising a first detector element and configured to:
      collect a first return light beam that enters the first sampling interface through the collection site; and
      direct the return light beam to the first detector element, wherein:
   the first detector element forms a measurement channel when the launch architecture emits the emission light beam;
   the emission light beam converges in a first dimension as it exits the sampling interface;
   the return light beam diverges in the first dimension as it enters the sampling interface; and
   the optical measurement system is configured such that, if used to measure a target sample characteristics:
      the emission light beam projects to a first transition region in the target sample that is angle independent for the measurement channel; and
      the return light beam projects from a second transition region in the target sample that is angle independent for the measurement channel.
15. The optical measurement system of item 14, wherein:
   the launch architecture is configured such that the emission light beam has a beam vergence half-angle of at least 15 degrees in the first dimension as the emission light beam exits the sampling interface.
16. The optical measurement system of item 14 or item 15, wherein:
   the collection architecture is configured such that the first return light beam has a beam vergence half-angle of at least 15 degrees in the first dimension as the return light beam enters the sampling interface.
17. The optical measurement system of any of items 14-16, wherein:
   the collection architecture comprises a second detector element and is configured to:
      collect a second return light beam that enters the sampling interface through the first collection site; and
      direct the second return light beam to the second detector element, wherein:
   the second detector element forms a second measurement channel when the launch architecture emits the emission light beam; and
   the second return light beam diverges in the first dimension as it enters the sampling interface.
18. The optical measurement system of item 17, wherein:
   the first transition region is angle independent for the second measurement channel.
19. The optical measurement system of item 17 or item 18, wherein:
   the second return light beam, if collected from the target sample, projects from a third transition region in the target sample that is angle independent for the second measurement channel.
20. The optical measurement system of any of items 14-16, wherein:
   the sampling interface defines a second collection site positioned such that the launch site is positioned between the first collection site and the second collection site;
   the collection architecture comprises a second detector element and is configured to:
      collect a second return light beam that enters the sampling interface through the second collection site; and
      direct the second return light beam to the second detector element;
   the second detector element forms a second measurement channel when the launch architecture emits the emission light beam;
   the second return light beam diverges in the first dimension as it enters the sampling interface; and
   the second return light beam, if collected from the target sample, projects from a third transition region in the target sample that is angle independent for the second measurement channel.

### Launch Architectures for Optical Measurement Systems

1. An optical measurement system comprising:
   a sampling interface defining a launch site and a set of collection sites each laterally spaced from the launch site;
   launch architecture configured to emit an emission light beam that exits the sampling interface through the launch site into a measured sample; and
   collection architecture comprising a set of detector elements positioned to measure light that has entered the sampling interface through the set of collection sites, wherein:
      the emission light beam converges in a first dimension as it exits the sampling interface and is projected to a transition region in the measured sample; and
      the launch architecture comprises:
         a beam generator comprising a light source unit and configured to generate a diverging output light beam; and
         a launch optical subassembly configured to collimate the diverging input light beam in the first dimension to generate a plurality of collimated ray bundles that collectively form the emission light beam and are projected to intersect at the transition region.
2. The optical measurement system of item 1, wherein:
   the beam generator is configured to generate an input light beam and comprises a diffuser; and
   the diffuser positioned to receive the input light beam and generate the diverging output light beam from the input light beam.
3. The optical measurement system of item 2, wherein:
   the diffuser comprises an emission surface that forms the diverging output light beam; and
   the emission surface of the diffuser is positioned at a hyper-telecentric position relative to the launch optical subassembly.
4. The optical measurement system of item 2 or item 3, wherein:
   the beam generator comprises a photonic integrated circuit configured to generate the input light beam.
5. The optical measurement system of item 4, wherein:
   the beam generator comprises an optical unit positioned between the photonic integrated circuit and the diffuser; and
   the optical unit comprises one or more lenses.
6. The optical measurement system of item 5, wherein:
   the optical unit is configured to collimate the input light beam in the first dimension.
7. The optical measurement system of item 5 or item 6, wherein:
   the optical unit is configured to collimate the input light beam in a second dimension that is perpendicular to the first dimension.
8. The optical measurement system of any of items 1-7, wherein:
   the launch architecture is configured such that the emission light beam has a beam vergence half-angle of at least 15 degrees in the first dimension as the emission light beam exits the sampling interface.
9. An optical measurement system comprising:
   a sampling interface defining a launch site and a set of collection sites each laterally spaced from the launch site;
   launch architecture comprising:
      a diffuser positioned to receive an input light beam and generate an output light beam that diverges in a first dimension; and
      a set of lens configured to collimate the diverging output light beam in the first dimension to generate an emission light beam that converges in the first dimension; and
   collection architecture comprising a set of detector elements positioned to measure light that has entered the sampling interface through the set of collection sites, wherein
   the emission light beam comprises a plurality of collimated ray bundles that are projected, as the emission light beam exits the launch site, to intersect at a transition region in a measured sample.
10. The optical measurement system of item 9, wherein:
   the diffuser comprises an emission surface that forms the diverging output light beam; and
   the emission surface of the diffuser is positioned at a hyper-telecentric position relative to the set of lenses.
11. The optical measurement system of item 9 or item 10, wherein:
   the launch architecture comprises a fast axis collimating lens configured to at least partially collimate the input light beam in the first dimension.
12. The optical measurement system of item 11, wherein:
   the launch architecture comprises a slow axis collimating lens positioned between the diffuser and the fast axis collimating lens; and
   the slow axis collimating lens is configured to at least partially collimate the input light beam in a second dimension perpendicular to the first dimension.
13. The optical measurement system of any of items 9-12, wherein:
   the launch architecture is configured such that the emission light beam has, as the emission light beam exits the sampling interface, a first beam width in the first dimension and a second beam width in a second dimension perpendicular to the first dimension; and
   the second beam width is at least eight times the first beam width.
14. The optical measurement system of any of items 9-13, wherein:
   the launch architecture is configured such that the emission light beam has a beam vergence half-angle of at least 15 degrees in the first dimension as the emission light beam exits the sampling interface.
15. An optical measurement system comprising:
   a sampling interface defining a launch site and a set of collection sites each laterally spaced from the launch site;
   launch architecture comprising:
      a photonic integrated circuit configured to generate an input light beam;
      a diffuser positioned to receive an input light beam and generate an output light beam that diverges in a first dimension; and
      a set of lens configured to generate, from the output light beam, a plurality of collimated ray bundles that are projected to intersect at a transition region in a measured sample; and
   collection architecture comprising a set of detector elements positioned to measure light that has entered the sampling interface through the set of collection sites.
16. The optical measurement system of item 15, wherein:
   the photonic integrated circuit comprises a plurality output couplers that is configured to emit a plurality of individual light beams; and
   the plurality of individual light beams at least partially overlap and collectively form the input light beam.
17. The optical measurement system of item 16, wherein:
   the launch architecture comprises a slow axis collimating lens positioned between the photonic integrated circuit and the diffuser;
   the slow axis collimating lens is configured to collimate each of the plurality of individual light beams along a second dimension perpendicular to the first dimension, such that the plurality of individual light beams intersect at the diffuser.
18. The optical measurement of item 17, wherein:
   the slow axis collimating lens is configured to at least partially collimate the input light beam in the first dimension.
19. The optical measurement system of any of items 15-18, wherein:
   the launch architecture comprises a fast axis collimating lens positioned between the photonic integrated circuit and the diffuser; and
   the fast axis collimating lens is configured to at least partially collimate the input light beam in the first dimension.
20. The optical measurement system of any of items 15-19, wherein:
   the launch architecture is configured such that the emission light beam has, as the emission light beam exits the sampling interface, a first beam width in the first dimension and a second beam width in a second dimension perpendicular to the first dimension; and
   the second beam width is at least eight times the first beam width.
21. The optical measurement system of any of items 15-20, wherein:
   the launch architecture is configured such that the emission light beam has a beam vergence half-angle of at least 15 degrees in the first dimension as the emission light beam exits the sampling interface.

### Collection Architectures for Optical Measurement Systems

1. An optical measurement system, comprising:
   a sampling interface defining a launch site and a first collection site laterally spaced from the launch site;
   launch architecture configured to emit an emission light beam that exits the sampling interface through the launch site; and
   collection architecture comprising:
      a first optical collection subassembly comprising a first condenser lens; and
      a first plurality of detector elements, wherein:
   the collection architecture is configured to collect a first return light beam from the first collection site and direct the first return light beam to the first condenser lens;
   each detector of the first plurality of detector elements measures a corresponding portion of the first return light beam to form a corresponding measurement channel of a first plurality of measurement channels;
   the first return light beam diverges in a first dimension as it enters the first collection site; and
   the first return light beam, if collected from a target sample, projects from a first transition region in the target sample that is angle independent for each of the first plurality of measurement channels.
2. The optical measurement system of item 1, wherein:
   the launch architecture is configured such that the emission light beam converges in the first dimension as it exits the sampling interface.
3. The optical measurement system of item 1 or item 2, wherein:
   the collection architecture is configured such that the first return light beam has a beam vergence half-angle of at least 15 degrees in the first dimension as the return light beam enters the sampling interface.
4. The optical measurement system of any of items 1-3, wherein:
   the first optical collection subassembly comprises a second condenser lens;
   the collection architecture is configured to collect a second return light beam from the first collection site and direct the second return light beam to the second condenser lens;
   the collection architecture comprises a second plurality of detector elements;
   each detector of the second plurality of detector elements measures a corresponding portion of the second return light beam to form a corresponding measurement channel of a second plurality of measurement channels;
   the second return light beam diverges in the dimension as it enters the first collection site; and
   the second return light beam, if collected from a target sample, projects from a second transition region in the target sample that is angle independent for each of the second plurality of measurement channels.
5. The optical measurement system of any of items 1-3, wherein:
   the sampling interface defines a second collection site positioned such that the launch site is positioned between the first collection site and the second collection site;
      the collection architecture comprises:
      a second optical collection subassembly comprising a second condenser lens;
      a second plurality of detector elements;
   the collection architecture is configured to collect a second return light beam from the second collection site and direct the second return light beam to the first condenser lens;
   each detector of the second plurality of detector elements measures a corresponding portion of the second return light beam to form a corresponding measurement channel of a second plurality of measurement channels;
   the second return light beam diverges in the dimension as it enters the second collection site; and
   the second return light beam, if collected from a target sample, projects from a second transition region in the target sample that is angle independent for each of the second plurality of measurement channels.
6. The optical measurement system of any of items 1-5, wherein:
   the first optical subassembly comprises a first lens, a second lens, and an aperture positioned between the first lens and a second lens.
7. The optical measurement system of any of items 1-6, wherein:
   the condenser lens is an immersion condenser lens.
8. An optical measurement system, comprising:
   a sampling interface defining a launch site and a collection site laterally spaced from the launch site;
   launch architecture configured to emit an emission light beam that exits the sampling interface through the launch site; and
   collection architecture comprising:
      an optical collection subassembly comprising a plurality of condenser lenses; and
      multiple groups of detector elements, wherein each of the multiple groups of detector elements is positioned to receive light from a corresponding condenser lens of the plurality of condenser lens, wherein:
         the collection architecture is configured to collect a plurality of return light beams that enter the sampling interface through the collection site; and
         the collection architecture is configured to direct a portion of each of the plurality of return light beams to each of the plurality of condenser lenses, such that each of the multiple groups of detector elements measures light from each of the plurality of light beams.
9. The optical measurement system of item 8, wherein:
   the plurality of condenser lens is a plurality of immersion condenser lenses formed on a common substrate.
10. The optical measurement system of item 8 or 9, wherein:
   the collection architecture is configured such that, if the plurality of light beams is collected from a target sample, a first detector element of each of the multiple groups of detector elements measures light having a common path length distribution and a different sampling depth distribution.
11. The optical measurement system of any of item 8 or item 9, wherein:
   the plurality of return light beams comprise a first return light beam and a second return light beam;
   a first detector element of each of the multiple groups of detector elements measures a corresponding portion of the first return light beam; and
   a second detector element of each of the multiple groups of detector elements measures a corresponding portion of the second return light beam.
12. The optical measurement system of item 11, wherein:
   the collection architecture is configured such that the first light beam diverges in a first dimension as it enters the collection site and, if collected from a target sample, projects from a first transition region in the target sample.
13. The optical measurement system of item 12, wherein:
   the collection architecture is configured such that the second light beam diverges in the first dimension as it enters the collection site and, if collected from a target sample, projects from a second transition region in the target sample.
14. The optical measurement system of item 12 or item 13, wherein:
   the collection architecture is configured such that the first return light beam has a beam vergence half-angle of at least 15 degrees in the first dimension as the return light beam enters the sampling interface.
15. An optical measurement system, comprising:
   a sampling interface defining a launch site and a first collection site laterally spaced from the launch site;
   launch architecture configured to emit an emission light beam that exits the sampling interface through the launch site; and
   collection architecture comprising:
      a first optical collection subassembly comprising a first condenser lens and configured to receive light returned to the sampling interface through the first collection site; and
      a first plurality of detector elements positioned to receive light from the first condenser lens, wherein:
   the collection architecture is configured such that, if the collection architecture receives light from a target sample, each of the first plurality of detector elements measures light having a first common path length distribution and a different sampling depth distribution.
16. The optical measurement system of item 15, wherein:
   the launch architecture is configured such that the emission light beam converges in the first dimension as it exits the sampling interface.
17. The optical measurement system of item 15 or item 16, wherein:
   the first optical collection subassembly comprises a second condenser lens;
   the collection architecture comprises a second plurality of detector elements positioned to receive light from the second condenser lens;
   the collection architecture is configured such that, if the collection architecture receives light from the target sample, each of the second plurality of detector elements measures light having a second common path length distribution and a different sampling depth distribution.
18. The optical measurement system of item 15 or items 16 wherein:
   the sampling interface defines a second collection site positioned such that the launch site is positioned between the first collection site and the second collection site; and
   the collection architecture comprises:
      a second optical collection subassembly comprising a second condenser lens and configured to receive light returned to the sampling interface through the second collection site; and
      a second plurality of detector elements positioned to receive light from the second condenser lens.
19. The optical measurement system of item 18, wherein:
   the collection architecture is configured such that, if the collection architecture receives light from the target sample, each of the second plurality of detector elements measures light having a first common path length distribution and a different sampling depth distribution.
20. The optical measurement system of item 18, wherein:
   the collection architecture is configured such that, if the collection architecture receives light from the target sample, each of the second plurality of detector elements measures light having a second common path length distribution and a different sampling depth distribution.

### Electromagnetic actuator arrangements for moving a diffuser

1. An electromagnetic actuator arrangement comprising:
   a stationary base;
   a set of suspension elements;
   a carrier moveably connected to the stationary base via the set of suspension elements;
   a first diffuser carried by the carrier; and
   a set of actuators mounted to the carrier and controllable to move the carrier relative to the stationary base, wherein:
      the carrier defines a first carrier aperture that extends through the carrier.
2. The electromagnetic actuator arrangement of item 1, wherein:
   the first diffuser at least partially defines the first carrier aperture.
3. The electromagnetic actuator arrangement of item 1 or item 2, comprising:
   a second diffuser carried by the carrier.
4. The electromagnetic actuator arrangement of item 3, wherein:
   the first carrier aperture is positioned between the first aperture and the second aperture.
5. The electromagnetic actuator arrangement of any of items 1-4, wherein:
   the carrier defines a second carrier aperture that extends through the carrier.
6. The electromagnetic actuator arrangement of item 5, wherein:
   the first diffuser is positioned between the first carrier aperture and the second carrier aperture.
7. An optical measurement system comprising:
   a sampling interface defining a launch site and a first collection site laterally spaced from the launch site;
   launch architecture configured to emit an emission light beam that exits the sampling interface through the launch site, the launch architecture comprising:
      a beam generator comprising a diffuser, wherein the diffuser is positioned to receive an input light beam and generate an output light beam that diverges in a first dimension;
   collection architecture comprising a first set of detector elements positioned to measure light that has entered the sampling interface through the first collection site; and
   an electromagnetic actuator arrangement comprising:
      a stationary base;
      a carrier moveably connected to the stationary base; and
      a set of actuators mounted to the carrier and controllable to move the carrier relative to the stationary base, wherein:
         the diffuser is carried by the carrier;
         the carrier defines a first carrier aperture that extends through the carrier; and
         the first carrier aperture is positioned such that light measured by the first set of detector elements passes through the first carrier aperture.
8. The optical measurement system of item 7, wherein:
   the carrier defines a second carrier aperture that extends through the carrier; and
   the diffuser is positioned between the first carrier aperture and the second carrier aperture.
9. The optical measurement system of item 8, wherein:
   collection architecture comprising a second set of detector elements positioned to measure light that has entered the sampling interface through the second collection site; and
   the second carrier aperture is positioned such that light measured by the second set of detector elements passes through the second carrier aperture.
10. The optical measurement system of any of items 7-9, wherein:
   the collection architecture comprises a launch optical subassembly configured to generate the emission light beam from the output light beam; and
   the launch optical subassembly is positioned between the electromagnetic actuator arrangement and the sampling interface.
11. The optical measurement system of any of items 7-10, wherein:
   the launch architecture is configured such that the emission light beam converges in the first dimension as it exits the sampling interface.
12. The optical measurement system of any of items 7-11, wherein:
   the collection architecture comprises a first collection optical subassembly that is configured to collect a first set of return light beams that enters the sampling interface through the first collection site; and
   the first set of detector elements are positioned to measure the first set of return light beams.
13. The optical measurement system of item 12, wherein:
   the first collection optical subassembly comprises a set of lenses; and
   at least one lens of the set of lenses is positioned at least partially in the first carrier aperture.
14. An optical measurement system comprising:
   a first measurement subsystem configured to:
      emit a first emission light beam into a sample through a sampling interface; and
      measure a portion of the first emission light beam that is returned from the sample through the sampling interface;
   a second measurement subsystem configured to:
      emit a second emission light beam into the sample through the sampling interface; and
      measure a portion of the second emission light beam that is returned from the sample through the sampling interface; and
   an electromagnetic actuator arrangement comprising:
      a stationary base;
      a carrier moveably connected to the stationary base; and
      a set of actuators mounted to the carrier and controllable to move the carrier relative to the stationary base, wherein:
         the first measurement subsystem comprises a first diffuser;
         the second measurement subsystem comprises a second diffuser; and
         the carrier carries the first diffuser and the second diffuser.
15. The optical measurement system of item 14, wherein:
   the carrier defines a first carrier aperture that extends through the carrier.
16. The optical measurement system of item 15, wherein:
   the first carrier aperture is positioned between the first diffuser and the second diffuser.
17. The optical measurement system of item 15 or items 16, wherein:
   the first measurement subsystem collects a first set of light beams that passes through the first carrier aperture.
18. The optical measurement system of item 17, wherein:
   the second measurement subsystem collects a second set of light beams that passes through the first carrier aperture.
19. The optical measurement system of any of items 15-18, wherein:
   the carrier defines a second carrier aperture that extends through the carrier; and
   the first diffuser is positioned between the first carrier aperture and the second carrier aperture.
20. The optical measurement system of any of items 14-19, wherein the carrier comprises an optical barrier separating the first diffuser and the second diffuser.

### Optical Measurement Systems with Polarizing Beamsplitters

1. A polarizing beamsplitter comprising:
   a substrate comprising a top surface and a bottom surface; and
   a polarizer positioned on a first portion of the bottom surface, wherein:
      the top surface defines a first angled facet;
      the polarizing beamsplitter is configured such that when an incoming light beam is incident on a predetermined location of the bottom surface with a predetermined angle of incidence:
         the polarizer generates a polarized light beam from the incoming light beam;
         the first angled facet splits the polarized light beam into a first split light beam and a second split light beam;
         the first split light beam exits the polarizing beamsplitter through the first angled fact; and
         at least a portion of the second split light beam exits the polarizing beamsplitter through the bottom surface.
2. The polarizing beamsplitter of item 1, comprising:
   anti-reflection coating positioned on a second portion of the bottom surface and laterally spaced from the polarizer, wherein the polarizing beamsplitter is configured such that when the incoming light beam is incident on the predetermined location of the bottom surface with the predetermined angle of incidence:
      the second split light beam exits the bottom surface through the anti-reflection coating.
3. The polarizing beamsplitter of item 1 or item 2, comprising:
   a single-layer anti-reflection coating positioned on the first angled facet.
4. The polarizing beamsplitter of item 1, wherein the polarizing beamsplitter is configured such that when the incoming light beam is incident on the predetermined location of the bottom surface with the predetermined angle of incidence:
   the bottom surface splits the second split light beam into a third split light beam and a fourth split light beam; and
   the third split light beam exits the polarizing beamsplitter through the bottom surface.
5. The polarizing beamsplitter of item 3, wherein:
   the top surface defines a second angled facet and a third angled facet; and
   the polarizing beamsplitter is configured such that when the incoming light beam is incident on the predetermined location of the bottom surface with the predetermined angle of incidence:
      the first angled facet directs the second split light beam toward the bottom surface such that the second split light beam reflects off of the bottom surface toward the second angled facet; and
      the second angled facet redirects the second split light beam toward the bottom surface; and
      the fourth split light beam exits the polarizing beamsplitter through the third angled facet.
6. The polarizing beamsplitter of item 4, comprising:
   a single-layer anti-reflection coating positioned on the first angled facet, the second angled facet, and the third angled facet.
7. The polarizing beamsplitter of any of items 1-6, wherein:
   the first angled facet is formed as a curved surface.
8. A polarizing beamsplitter comprising:
   a substrate comprising a top surface and a bottom surface; and
   a polarizer positioned on a first portion of the bottom surface, wherein:
      the top surface defines a first angled facet and a second angled facet; and
      the polarizing beamsplitter is configured such that when an incoming light beam is incident on a predetermined location of the bottom surface with a predetermined angle of incidence:
         the polarizer generates a polarized light beam from the incoming light beam;
         the first angled facet splits the polarized light beam into a first split light beam and a second split light beam;
         the first split light beam exits the polarizing beamsplitter through the first angled fact; and
         at least a portion of the second split light beam exits the polarizing beamsplitter through the top surface.
9. The polarizing beamsplitter of item 8, wherein the polarizing beamsplitter is configured such that when the incoming light beam is incident on the predetermined location of the bottom surface with the predetermined angle of incidence:
   the split light beam exits the polarizing beamsplitter through the second angled facet.
10. The polarizing beamsplitter of item 8, wherein:
   the top surface defines a third angled facet; and
   the polarizing beamsplitter is configured such that when the incoming light beam is incident on the predetermined location of the bottom surface with the predetermined angle of incidence:
      at least a portion of the second split light beam exits the polarizing beamsplitter through the third angled facet.
11. The polarizing beamsplitter of item 10, wherein the polarizing beamsplitter is configured such that when the incoming light beam is incident on the predetermined location of the bottom surface with the predetermined angle of incidence:
   the second angled facet splits the second split light beam into a third split light beam and a fourth split light beam;
   the third split light beam exits the polarizing beamsplitter through the second angled facet; and
   the fourth split light beam exits the polarizing beamsplitter through the third angled facet.
12. The polarizing beamsplitter of item 11, comprising:
   a reflective coating positioned on a second portion of the bottom surface, wherein the polarizing beamsplitter is configured such that when the incoming light beam is incident on the predetermined location of the bottom surface with the predetermined angle of incidence:
   the second angled facet directs the fourth split light beam to the second portion of the bottom surface; and
   the fourth split light beam is reflected by the reflective coating.
13. The polarizing beamsplitter of any of items 8-12, comprising:
   a first anti-reflection coating positioned on the second angled facet.
14. The polarizing beamsplitter of item 13, wherein:
   the first anti-reflection coating is positioned on the first angled facet.
15. The polarizing beamsplitter of item 13, comprising:
   a second anti-reflection coating position on the first angled facet.
16. An optical measurement system comprising:
   a sampling interface defining a launch site and a set of collection sites each laterally spaced from the launch site;
   launch architecture comprising a beam generator and configured to emit an emission light beam that exits the sampling interface through the launch site into a measured sample; and
   collection architecture comprising a set of detector elements positioned to measure light that has entered the sampling interface through the set of collection sites, wherein:
      the beam generator is configured to generate an input light beam and generate an output light beam from the input light beam;
      the launch architecture is configured to generate the emission light beam from the output light beam;
      the beam generator comprises a reference detector and a polarizing beamsplitter;
      the polarizing beamsplitter comprises:
         a substrate comprising a top surface and a bottom surface, the top surface defining a first angled facet; and
         a polarizer positioned on a first portion of the bottom surface;
      the polarizing beamsplitter is positioned to receive the input light beam through the polarizer to generate a polarized light beam;
      the polarizing beamsplitter is configured to split the polarized light beam into a first split light beam and a second split light beam;
      the beam generator generates the output light beam from the first split light beam; and
      the reference detector is positioned to measure at least a portion of the second split light beam.
17. The optical measurement system of item 16, wherein:
   the polarizing beamsplitter comprises an anti-reflection coating positioned on a second portion of the bottom surface; and
   the second split light beam exits the polarizing beamsplitter through the anti-reflection coating.
18. The optical measurement system of item 16, wherein:
   the polarizing beamsplitter is configured to split the second split light beam into a third split light beam and a fourth split light beam; and
   the reference detector is positioned to measure at least a portion of the fourth split light beam.
19. The optical measurement system of item 18, wherein:
   the fourth split light beam exits the polarizing beamsplitter through the top surface.
20. The optical measurement system of item 18 or item 19, wherein:
   the third split light beam exits the polarizing beamsplitter through the bottom surface.

### Optical Measurement Systems with Beam shifting optics

1. A beam shifting optic comprising:
   a substrate comprising a top surface and a bottom surface, wherein:
   the bottom surface defines at least one angled facet that is angled in a first dimension;
   the top surface defines a lensed facet that is curved in a second dimension perpendicular to the first dimension; and
   the beam shifting optic is configured such that when a light beam enters the beam shifting optic through a predetermined input region and at a predetermined angle of incidence:
      the light beam reflects off of the at least one angled facet to redirect the light beam in the first dimension; and
      the light beam exits the beam shifting optic through an exit region of the lensed facet to at least partially collimate the light beam.
2. The beam shifting optic of item 1, wherein:
   the at least one angled facet comprises a first angled facet and a second angled facet.
3. The beam shifting optic of item 2, wherein:
   the input region is located on the first angled facet, such that the light beam enters the beam shifting optic through the first angled facet.
4. The beam shifting optic of any of items 1-3, wherein:
   the top surface defines at least one angled facet that is angled in the first dimension.
5. The beam shifting optic of any of items 1-4, wherein the beam shifting optic is configured such that when the light beam enters the beam shifting optic through the predetermined input region and at the predetermined angle of incidence:
   the light beam reflects off of the lensed facet before exiting the beam shifting optic.
6. The beam shifting optic of any of items 1-5, wherein:
   the substrate is formed from silicon.
7. The beam shifting optic of any of items 1-6, comprising:
   a anti-reflective coating positioned on the lensed facet.
8. An beam generator configured to generate an output light beam, the beam generator comprising:
   a photonic integrated circuit configured to emit an input light beam; and
   a beam shifting optic positioned to receive the input light beam at a predetermined input region and at a predetermined angle of incidence and configured to laterally shift the input light beam in a first dimension; and
   a diffuser positioned to receive the input light beam and generate the output light beam from the input light beam, wherein:
      the beam shifting optic comprising a substrate that comprises a top surface and a bottom surface;
      the bottom surface defines a first angled facet that is angled in the first dimension; and
      the beam shifting optic is configured such that when the input light beam enters the beam shifting optic through the predetermined input region and at the predetermined angle of incidence:
         the light beam reflects off of the first angled facet to redirect the light beam in the first dimension; and
         the light beam exits the beam shifting optic through an exit region of the beam shifting optic.
9. The beam generator of item 8, wherein:
   the bottom surface defines a second angled facet that is angled in the first dimension; and
   the input region is located on the second angled facet, such that the light beam enters the beam shifting optic through the second angled facet.
10. The beam generator of item 8, wherein:
   the top surface defines a second angled facet that is angled in the first dimension; and
   the beam shifting optic is configured such that when the input light beam enters the beam shifting optic through the predetermined input region and at the predetermined angle of incidence:
      the light beam reflects off of the second angled facet.
11. The beam generator of item 10, wherein:
   the second angled facet is curved in the first dimension.
12. The beam generator of item 11, wherein:
   the second angled facet is convex in the first dimension, such that the light beam is at least partially collimated in the first dimension as it reflects off of the second angled facet.
13. The beam generator of any of items 8-12, comprising:
   a mirror positioned to direct the input light beam toward the input region.
14. The beam generator of item 13, comprising:
   a fast axis collimating lens positioned between the mirror and the photonic integrated circuit.
15. The beam generator of item 13, wherein:
   the mirror is curved in the first dimension to at least partially collimated the input light beam in the first dimension.
16. An optical measurement system comprising:
   a sampling interface defining a launch site and a set of collection sites each laterally spaced from the launch site;
   launch architecture comprising a beam generator and configured to emit an emission light beam that exits the sampling interface through the launch site into a measured sample; and
   collection architecture comprising a set of detector elements positioned to measure light that has entered the sampling interface through the set of collection sites, wherein:
      the beam generator is configured to generate an input light beam and generate an output light beam from the input light beam;
      the launch architecture is configured to generate the emission light beam from the output light beam; and
      the beam generator comprises:
         a photonic integrated circuit configured to emit the input light beam; and
         a beam shifting optic configured to laterally shift the input light beam in a first dimension.
17. The optical measurement system of item 16, wherein:
   the beam shifting optic is configured to at least partially collimate the input light beam in the first dimension.
18. The optical measurement system of item 16 or item 17, wherein:
   the beam shifting optic is configured to at least partially collimate the input light beam in a second dimension perpendicular to the first dimension.
19. The optical measurement system of any of items 15-17 comprising:
   a diffuser positioned to receive the input light beam and generate the output light beam from the input light beam.
20. The optical measurement system of item 19, comprising:
   a beamsplitter positioned between the beam shifting optic and the diffuser; and
   a reference detector wherein:
      the beamsplitter is configured to generate a reference light beam from the input light beam; and
      the reference detector is positioned to measure the reference light beam.

### Optical Measurement Systems with Multiple Measurement Subsystems

1. An optical measurement system comprising:
   a first measurement subsystem configured to:
      emit a first emission light beam into a sample through a sampling interface; and
      measure a portion of the first emission light beam that is returned from the sample through the sampling interface;
   a second measurement subsystem configured to:
      emit a second emission light beam into the sample through the sampling interface; and
      measure a portion of the second emission light beam that is returned from the sample through the sampling interface;
   a set of photonic integrated circuits configured to emit a first input light beam and a second input light beam; and
   an electromagnetic actuator arrangement comprising a stationary base, a carrier moveably coupled to the stationary base, and at least one diffuser carried by the carrier, wherein:
      the first input light beam is routed through the at least one diffuser to generate a first output light beam and the second input light beam is routed through the at least one diffuser to generate a second output light beam;
      the first measurement subsystem generates the first emission light beam from the first output light beam; and
      the second measurement subsystem generates the second emission light beam form the second output light beam.
2. The optical measurement system of item 1, wherein:
   the set of photonic integrated circuits comprises a first photonic integrated circuit configured to emit the first input light beam and the second input light beam.
3. The optical measurement system of item 2, wherein:
   the first input light beam and the second input light beam are emitted from a common side surface of the photonic integrated circuit.
4. The optical measurement system of item 1, wherein:
   the set of photonic integrated circuits comprises a first photonic integrated circuit configured to emit the first input light beam and a second photonic integrated circuit configured to emit the second input light beam.
5. The optical measurement system of any of items 1-4, wherein:
   the first photonic integrated circuit has a non-rectangular shape.
6. The optical measurement system of any of items 1-5, wherein:
   the set of photonic integrated circuits is configured to emit a third input light beam and a fourth input light beam; and
   the third input light beam is routed through the at least one diffuser to generate a third output light beam and the fourth input light beam is routed through the at least one diffuser to generate a fourth output light beam.
7. An optical measurement system comprising:
   a first measurement subsystem configured to:
      emit a first emission light beam into a sample through a sampling interface; and
      measure a portion of the first emission light beam that is returned from the sample through the sampling interface;
   a second measurement subsystem configured to:
      emit a second emission light beam into the sample through the sampling interface; and
      measure a portion of the second emission light beam that is returned from the sample through the sampling interface;
   a photonic integrated circuit configured to emit a first input light beam from a first emission location on the photonic integrated circuit; and
   a beam redirecting structure configured to route light from the first input light beam between the first measurement subsystem and the second measurement subsystem.
8. The optical measurement system of item 7, wherein the beam directing structure is moveable to selectively route the input light beam to the first measurement subsystem during a first period of time and to the second measurement subsystem during a second period of time.
9. The optical measurement system of item 8, wherein the beam directing structure comprises a mirror that is moveable between a first state and a second state.
10. The optical measurement system of item 7, wherein:
   the beam directing structure comprises a beamsplitter that is positioned to split the input light beam into a first split light beam and a second split light beam; and
   the beam directing structure is configured to route the first split light beam to the first measurement subsystem and the second split light beam to the second measurement subsystem.
11. The optical measurement system of any of items 7-10 comprising a fast axis collimating lens positioned between the photonic integrated circuit and the beam directing structure, wherein the fast axis collimating lens is configured to at least partially collimate the input light beam in a first dimension.
12. The optical measurement system of any of items 7-11, wherein:
   the first measurement subsystem comprises a first diffuser positioned to generate a first output light beam from light of the first input light beam; and
   the second measurement subsystem comprises a second diffuser positioned to generate a second output light beam from light of the first input light beam.
13. The optical measurement system of item 12, comprising:
   an electromagnetic actuator arrangement comprising a stationary base and a carrier moveably coupled to the stationary base, wherein:
   the first diffuser and the second diffuser are carried by the carrier
14. The optical measurement system of any of items 7-13, comprising:
   a third measurement subsystem configured to:
      emit a third emission light beam into a sample through a sampling interface; and
      measure a portion of the third emission light beam that is returned from the sample through the sampling interface; and
   a fourth measurement subsystem configured to:
      emit a fourth emission light beam into the sample through the sampling interface; and
      measure a portion of the fourth emission light beam that is returned from the sample through the sampling interface; wherein:
         the photonic integrated circuit configured to emit a second input light beam from a second emission location on the photonic integrated circuit; and
         the beam redirecting structure is configured to route light from the second input light beam between the third measurement subsystem and the fourth measurement subsystem.
15. An optical measurement system comprising:
   a first measurement subsystem configured to:
      emit a first emission light beam into a sample through a sampling interface; and
      measure a portion of the first emission light beam that is returned from the sample through the sampling interface;
   a second measurement subsystem configured to:
      emit a second emission light beam into the sample through the sampling interface; and
      measure a portion of the second emission light beam that is returned from the sample through the sampling interface; and
   a photonic integrated circuit configured to emit a first input light beam and a second input light beam from a common surface of the photonic integrated circuit, wherein:
      the first measurement subsystem is configured to generate a first output light beam from the first input light beam;
      the second measurement subsystem is configured to generate a second output light beam from the first input light beam; and
      the first output light beam and the second output light beam are generated in a staggered relationship relative to the photonic integrated circuit.
16. The optical measurement system of item 15, wherein:
   the first measurement subsystem comprises a first mirror positioned to reflect the first input light beam in a first direction; and
   the second measurement subsystem comprises a second mirror positioned to reflect the second input light beam in a second direction that is different from the first direction.
17. The optical measurement system of item 15, comprising:
   a first mirror positioned to reflect the first input light beam; and
   a second input light beam to reflect the second input light beam, wherein:
      the first mirror is positioned at a first distance from the common surface of the photonic integrated circuit; and
      the first second is positioned at a second distance from the common surface of the photonic integrated circuit that is less than the first distance.
18. The optical measurement system of item 15, comprising:
   an optical displacer positioned between the first mirror and the common surface of the photonic integrated circuit such that the first input light beam passes through the optical displacer.
19. The optical measurement system of item 18, wherein:
   the first input light beam has a beam size at the first mirror that is the same size a beam of the second input light beam at the second mirror.
20. The optical measurement system of item 18 or 19, comprising:
   a fast axis collimating lens positioned between the optical displacer and the photonic integrated circuit.

## Claims

1. An optical measurement system, comprising:
a sampling interface defining a launch site and a set of collection sites each laterally spaced from the launch site;
launch architecture configured to emit an emission light beam that exits the sampling interface through the launch site; and
collection architecture comprising a set of detector elements positioned to measure light that has entered the sampling interface through the set of collection sites, wherein:
the set of detector elements forms a set of measurement channels when the launch architecture emits the emission light beam;
the emission light beam converges in a first dimension as it exits the sampling interface; and
the emission light beam, if emitted into a target sample, projects to a transition region in the target sample that is angle independent for at least one of the set of measurement channels.

2. The optical measurement system of claim 1, wherein:
the launch architecture is configured such that the emission light beam has a beam vergence half-angle of at least 15 degrees in the first dimension as the emission light beam exits the sampling interface.

3. The optical measurement system of claim 2, wherein:
the launch architecture is configured such that the emission light beam has a beam vergence half-angle of at least 30 degrees in the first dimension as the emission light beam exits the sampling interface.

4. The optical measurement system of any of claims 1-3, wherein:
the set of collection sites comprises a first collection site and a second collection site;
the launch site is positioned between the first collection site and the second collection site; and
the set of detector elements comprises:
a first set of detector elements positioned to measure light that has entered the sampling interface through the first collection site; and
a second set of detector elements positioned to measure light that has entered the sampling interface through the second collection site.

5. The optical measurement system of claim 4, wherein:
the first set of detector elements forms a first set of measurement channels when the launch architecture emits the emission light beam;
the second set of detector elements forms a second set of measurement channels when the launch architecture emits the emission light beam; and
the transition region in the target sample is angle independent for the first set of measurement channels and the second set of measurement channels.

6. The optical measurement system of claim 5, wherein:
the launch architecture is configured such that the emission light beam has, as the emission light beam exits the sampling interface, a first beam width in the first dimension and a second beam width in a second dimension perpendicular to the first dimension; and
the second beam width is at least eight times the first beam width.

7. An optical measurement system, comprising:
a sampling interface defining a launch site and a first collection site laterally spaced from the launch site;
launch architecture configured to emit an emission light beam that exits the sampling interface through the launch site; and
collection architecture comprising a first detector element and configured to:
collect a first return light beam that enters the sampling interface through the first collection site; and
direct the return light beam to the first detector element, wherein:
the first detector element forms a first measurement channel when the launch architecture emits the emission light beam;
the first return light beam diverges in a first dimension as it enters the sampling interface; and
the first return light beam, if collected from a target sample, projects from a first transition region in the target sample that is angle independent for the first measurement channel.

8. The optical measurements system of claim 7, wherein:
the collection architecture is configured such that the first return light beam has a beam vergence half-angle of at least 15 degrees in the first dimension as the return light beam enters the sampling interface.

9. The optical measurements system of claim 8, wherein:
the collection architecture is configured such that the first return light beam has a beam vergence half-angle of at least 30 degrees in the first dimension as the return light beam enters the sampling interface.

10. The optical measurement system of any of claims 7-9, wherein:
the collection architecture comprises a second detector element and is configured to:
collect a second return light beam that enters the sampling interface through the first collection site; and
direct the second return light beam to the second detector element, wherein:
the second detector element forms a second measurement channel when the launch architecture emits the emission light beam; and
the second return light beam diverges in the first dimension as it enters the sampling interface.

11. The optical measurement system of claim 10, wherein:
the second return light beam, if collected from the target sample, projects from a second transition region in the target sample that is angle independent for the second measurement channel.

12. The optical measurement system of any of claims 7-9, wherein:
the sampling interface defines a second collection site positioned such that the launch site is positioned between the first collection site and the second collection site;
the collection architecture comprises a second detector element and is configured to:
collect a second return light beam that enters the sampling interface through the second collection site; and
direct the second return light beam to the second detector element;
the second detector element forms a second measurement channel when the launch architecture emits the emission light beam;
the second return light beam diverges in the first dimension as it enters the sampling interface; and
the second return light beam, if collected from the target sample, projects from a second transition region in the target sample that is angle independent for the second measurement channel.

13. The optical measurement system of any of claims 7-12, wherein:
the collection architecture is configured such that the first return light beam has, as the first return light beam enters the sampling interface, a first beam width in the first dimension and a second beam width in a second dimension perpendicular to the first dimension; and
the second beam width is at least eight times the first beam width.

14. An optical measurement system, comprising:
a sampling interface defining a launch site and a first collection site laterally spaced from the launch site;
launch architecture configured to emit an emission light beam that exits the sampling interface through the launch site; and
collection architecture comprising a first detector element and configured to:
collect a first return light beam that enters the first sampling interface through the collection site; and
direct the return light beam to the first detector element, wherein:
the first detector element forms a measurement channel when the launch architecture emits the emission light beam;
the emission light beam converges in a first dimension as it exits the sampling interface;
the return light beam diverges in the first dimension as it enters the sampling interface; and
the optical measurement system is configured such that, if used to measure a target sample characteristics:
the emission light beam projects to a first transition region in the target sample that is angle independent for the measurement channel; and
the return light beam projects from a second transition region in the target sample that is angle independent for the measurement channel.

15. The optical measurement system of claim 14, wherein:
the launch architecture is configured such that the emission light beam has a beam vergence half-angle of at least 15 degrees in the first dimension as the emission light beam exits the sampling interface.
